# EUROPÄISCHE PATENTSCHRIFT

(11) **EP 1 908 841 B1**
(45) Veröffentlichungstag und Bekanntmachung des Hinweises auf die Patenterteilung: **25.01.2012**
(21) Anmeldenummer: 07123772.1
(22) Anmeldetag: 21.12.2005
(51) Int. Cl.: C12N 15/77, C07K 14/34

(54) **Mehrfachpromotoren und deren Verwendung zur Genexpression**
Multiple promoters and their use for gene expression
Promoteurs multiples et leur utilisation pour l'expression de gènes

(30) Priorität: 22.12.2004 DE 102004061846
(43) Veröffentlichungstag der Anmeldung: 09.04.2008
(62) Teilanmeldung aus: 05850322.8
(73) Patentinhaber: BASF SE, 67056 Ludwigshafen (DE)
(72) Erfinder: Haefner, Stefan, 67346 Speyer (DE); Schröder, Hartwig, 69226 Nußloch (DE); Zelder, Oskar, 67346 Speyer (DE); Klopprogge, Corinna, 68239 Mannheim (DE); Herold, Andrea, 68775 Ketsch (DE)

(56) Entgegenhaltungen:
- WO-A-2005/059143
- WO-A-2005/059144
- IVANOV I G ET AL: "EXPRESSION OF HUMAN ALPHA 1 INTERFERON GENES IN VECTORS CONTAINING TANDEMLY LOCATED PROMOTERS RECOGNIZED BY TWO DIFFERENT RNA POLYMERASES (ESCHERICHIA COLI AND T7)" FEMS MICROBIOLOGY LETTERS, AMSTERDAM, NL, Bd. 108, Nr. 2, 1993, Seiten 231-236, XP000923385 ISSN: 0378-1097
- FOLLETTIE MAXIMILLIAN T ET AL: "Gene structure and expression of the Corynebacterium flavum N13 ask-asd operon" JOURNAL OF BACTERIOLOGY, Bd. 175, Nr. 13, 1993, Seiten 4096-4103, XP002374574 ISSN: 0021-9193
- MURAKAMI Y ET AL: "CONSTRUCTION OF NEW EXCRETION VECTORS TWO AND THREE TANDEMLY LOCATED PROMOTERS ARE ACTIVE FOR EXTRACELLULAR PROTEIN PRODUCTION FROM ESCHERICHIA-COLI" APPLIED MICROBIOLOGY AND BIOTECHNOLOGY, Bd. 30, Nr. 6, 1989, Seiten 619-623, XP009064388 ISSN: 0175-7598
- KALINOWSKI J ET AL: "THE COMPLETE CORYNEBACTERIUM GLUTAMICUM ATCC 13032 GENONE SEQUENCE AND ITS IMPACT ON THE PRODUCTION OF L-ASPARTATE-DERIVED AMINO ACIDS AND VITAMINS" JOURNAL OF BIOTECHNOLOGY, ELSEVIER SCIENCE PUBLISHERS B.V., AMSTERDAM, NL, Bd. 104, Nr. 1-3, 4. September 2003 (2003-09-04), Seiten 5-25, XP001184752 ISSN: 0168-1656

### SEQUENCE LISTING

<110> BASF AG
<120> Mehrfachpromotoren
<130> M/45256
<160> 84
<170> PatentIn version 3.1
<210> 1
   <211> 177
   <212> DNA
   <213> Corynebacterium glutamicum
<220>
   <221> misc_feature
   <223> pGRO
<220>
   <221> misc_feature
   <222> (1)..(177)
   <223> promoter sequence if no further downstream promoter occurs
<220>
   <221> misc_feature
   <222> (1)..(155)
   <223> promoter sequence if a further promoter is attached downstream
<220>
   <221> misc_feature
   <222> (70)..(75)
   <223> putative -10 region
<220>
   <221> misc_feature
   <222> (165)..(172)
   <223> putative ribosome binding sequence
<400> 1
   cggcttaaag tttggctgcc atgtgaattt ttagcaccct caacagttga gtgctggcac     60
   tctcgggggt agagtgccaa ataggttgtt tgacacacag ttgttcaccc gcgacgacgg    120
   ctgtgctgga aacccacaac cggcacacac aaaatttttc tcatggaggg attcatc       177
<210> 2
   <211> 195
   <212> DNA
   <213> corynebacterium glutamicum
<220>
   <221> misc_feature
   <223> pEFTS
<220>
   <221> misc_feature
   <222> (1)..(195)
   <223> promoter sequence if no further downstream promoter occurs
<220>
   <221> misc_feature
   <222> (1)..(178)
   <223> promoter sequence if a further promoter is attached downstream
<220>
   <221> misc_feature
   <222> (147)..(152)
   <223> putative -10 region
<220>
   <221> misc_feature
   <222> (162)..(167)
   <223> putative -10 region
<220>
   <221> misc_feature
   <222> (179)..(185)
   <223> putative ribosome binding sequence
<400> 2
   cccccacgac aatggaactt tgacttttaa aatttcatcg ccgtgggggc tttttgggca     60
   gccagcccgc cgtgtcgcaa cgtaatcgac tgaatacctg tacgatcact ttttagacgg    120
   gcgggtaggg ctactgtgcc ctaacctaag cttgtaaagc attaattatc catacataag    180
   gaggatcgcc ccgta                                                     195
<210> 3
   <211> 199
   <212> DNA
   <213> corynebacterium glutamicum
<220>
   <221> misc_feature
   <223> pEFTU
<220>
   <221> misc_feature
   <222> (1)..(199)
   <223> promoter sequence if no further downstream promoter occurs
<220>
   <221> misc_feature
   <222> (1)..(176)
   <223> promoter sequence if a further promoter is attached downstream
<220>
<221> misc_feature
   <222> (36)..(41)
   <223> putative -10 region
<220>
   <221> misc_feature
   <222> (63)..(68)
   <223> putative -10 region
<220>
   <221> misc_feature
   <222> (92)..(97)
   <223> putative -10 region
<220>
   <221> misc_feature
   <222> (187)..(193)
   <223> putative ribosome binding sequence
<400> 3
   ggccgttacc ctgcgaatgt ccacagggta gctggtagtt tgaaaatcaa cgccgttgcc     60
   cttaggattc agtaactggc acattttgta atgcgctaga tctgtgtgct cagtcttcca    120
   ggctgcttat cacagtgaaa gcaaaaccaa ttcgtggctg cgaaagtcgt agccaccacg    180
   aagtccagga ggacataca                                                 199
<210> 4
   <211> 191
   <212> DNA
   <213> Corynebacterium glutamicum
<220>
   <221> misc_feature
   <223> pSOD
<220>
   <221> misc_feature
   <222> (1)..(191)
   <223> promoter sequence if no further downstream promoter occurs
<220>
   <221> misc_feature
   <222> (1)..(173)
   <223> promoter sequence if a further promoter is attached downstream
<220>
   <221> misc_feature
   <222> (65)..(70)
   <223> putative -10 region
<220>
   <221> nnsc_feature
   <222> (86)..(92)
   <223> putative -10 region
<220>
   <221> misc_feature
   <222> (175)..(181)
   <223> putative ribosome binding sequence
<400> 4
   agctgccaat tattccgggc ttgtgacccg ctacccgata aataggtcgg ctgaaaaatt 60
   tcgttgcaat atcaacaaaa aggcctatca ttgggaggtg tcgcaccaag tacttttgcg 120
   aagcgccatc tgacggattt tcaaaagatg tatatgctcg gtgcggaaac ctacgaaagg 180
   attttttacc c 191
<210> 5
   <211> 52
   <212> DNA
   <213> Artificial Sequence
<220>
   <223> Primer
<400> 5
   cccgggatcc gctagcggcg cgccggccgg cccggtgtga aataccgcac ag 52
<210> 6
   <211> 53
   <212> DNA
   <213> Artificial Sequence
<220>
   <223> Primer
<400> 6
   tctagactcg agcggccgcg gccggccttt aaattgaaga cgaaagggcc tcg 53
<210> 7
   <211> 47
   <212> DNA
   <213> Artificial Sequence
<220>
   <223> Primer
<400> 7
   gagatctaga cccggggatc cgctagcggg ctgctaaagg aagcgga 47
<210> 8
   <211> 38
   <212> DNA
   <213> Artificial Sequence
<220>
   <223> Primer
<400> 8
   gagaggcgcg ccgctagcgt gggcgaagaa ctccagca 38
<210> 9
   <211> 34
   <212> DNA
   <213> Artificial sequence
<220>
   <223> Primer
<400> 9
   gagagggcgg ccgcgcaaag tcccgcttcg tgaa 34
<210> 10
   <211> 34
   <212> DNA
   <213> Artificial Sequence
<220>
   <223> Primer
<400> 10
   gagagggcgg ccgctcaagt cggtcaagcc acgc 34
<210> 11
   <211> 140
   <212> DNA
   <213> Artificial Sequence
<220>
   <223> oligonucleotide
<400> 11
   tcgaatttaa atctcgagag gcctgacgtc gggcccggta ccacgcgtca tatgactagt 60
   tcggacctag ggatatcgtc gacatcgatg ctcttctgcg ttaattaaca attgggatcc 120
   tctagacccg ggatttaaat 140
<210> 12
   <211> 140
   <212> DNA
   <213> Artificial Sequence
<220>
   <223> oligonucleotide
<400> 12
   gatcatttaa atcccgggtc tagaggatcc caattgttaa ttaacgcaga agagcatcga 60
   tgtcgacgat atccctaggt ccgaactagt catatgacgc gtggtaccgg gcccgacgtc 120
   aggcctctcg agatttaaat 140
<210> 13
   <211> 4323
   <212> DNA
   <213> Artificial Sequence
<220>
   <223> Plasmid
   <220>
   <221> misc_feature
   <223> Plasmid pCLiK5MCS
<220>
   <221> misc_feature
   <222> (457)..(1248)
   <223> KanR
<220>
   <221> misc_feature
   <222> (3840)..(4302)
   <223> PsacB (complementary)
<220>
   <221> misc_feature
   <222> (2418)..(3839)
   <223> SacB (complementary)
<220>
   <221> misc_feature
   <222> (1515)..(2375)
   <223> Ori-EC (pMB) (complementary)
<400> 13
   tcgagaggcc tgacgtcggg cccggtacca cgcgtcatat gactagttcg gacctaggga     60
   tatcgtcgac atcgatgctc ttctgcgtta attaacaatt gggatcctct agacccggga    120
   tttaaatcgc tagcgggctg ctaaaggaag cggaacacgt agaaagccag tccgcagaaa    180
   cggtgctgac cccggatgaa tgtcagctac tgggctatct ggacaaggga aaacgcaagc    240
   gcaaagagaa agcaggtagc ttgcagtggg cttacatggc gatagctaga ctgggcggtt    300
   ttatggacag caagcgaacc ggaattgcca gctggggcgc cctctggtaa ggttgggaag    360
   ccctgcaaag taaactggat ggctttcttg ccgccaagga tctgatggcg caggggatca    420
   agatctgatc aagagacagg atgaggatcg tttcgcatga ttgaacaaga tggattgcac    480
   gcaggttctc cggccgcttg ggtggagagg ctattcggct atgactgggc acaacagaca    540
   atcggctgct ctgatgccgc cgtgttccgg ctgtcagcgc aggggcgccc ggttcttttt    600
   gtcaagaccg acctgtccgg tgccctgaat gaactgcagg acgaggcagc gcggctatcg    660
   tggctggcca cgacgggcgt tccttgcgca gctgtgctcg acgttgtcac tgaagcggga    720
   agggactggc tgctattggg cgaagtgccg gggcaggatc tcctgtcatc tcaccttgct    780
   cctgccgaga aagtatccat catggctgat gcaatgcggc ggctgcatac gcttgatccg    840
   gctacctgcc cattcgacca ccaagcgaaa catcgcatcg agcgagcacg tactcggatg    900
   gaagccggtc ttgtcgatca ggatgatctg gacgaagagc atcaggggct cgcgccagcc    960
   gaactgttcg ccaggctcaa ggcgcgcatg cccgacggcg aggatctcgt cgtgacccat   1020
   ggcgatgcct gcttgccgaa tatcatggtg gaaaatggcc gcttttctgg attcatcgac   1080
   tgtggccggc tgggtgtggc ggaccgctat caggacatag cgttggctac ccgtgatatt   1140
   gctgaagagc ttggcggcga atgggctgac cgcttcctcg tgctttacgg tatcgccgct   1200
   cccgattcgc agcgcatcgc cttctatcgc cttcttgacg agttcttctg agcgggactc   1260
   tggggttcga aatgaccgac caagcgacgc ccaacctgcc atcacgagat ttcgattcca   1320
   ccgccgcctt ctatgaaagg ttgggcttcg gaatcgtttt ccgggacgcc ggctggatga   1380
   tcctccagcg cggggatctc atgctggagt tcttcgccca cgctagcggc gcgccggccg   1440
   gcccggtgtg aaataccgca cagatgcgta aggagaaaat accgcatcag gcgctcttcc   1500
   gcttcctcgc tcactgactc gctgcgctcg gtcgttcggc tgcggcgagc ggtatcagct   1560
   cactcaaagg cggtaatacg gttatccaca gaatcagggg ataacgcagg aaagaacatg   1620
   tgagcaaaag gccagcaaaa ggccaggaac cgtaaaaagg ccgcgttgct ggcgtttttc   1680
   cataggctcc gcccccctga cgagcatcac aaaaatcgac gctcaagtca gaggtggcga   1740
   aacccgacag gactataaag ataccaggcg tttccccctg gaagctccct cgtgcgctct   1800
   cctgttccga ccctgccgct taccggatac ctgtccgcct ttctcccttc gggaagcgtg   1860
   gcgctttctc atagctcacg ctgtaggtat ctcagttcgg tgtaggtcgt tcgctccaag   1920
   ctgggctgtg tgcacgaacc ccccgttcag cccgaccgct gcgccttatc cggtaactat 1980
   cgtcttgagt ccaacccggt aagacacgac ttatcgccac tggcagcagc cactggtaac 2040
   aggattagca gagcgaggta tgtaggcggt gctacagagt tcttgaagtg gtggcctaac 2100
   tacggctaca ctagaaggac agtatttggt atctgcgctc tgctgaagcc agttaccttc 2160
   ggaaaaagag ttggtagctc ttgatccggc aaacaaacca ccgctggtag cggtggtttt 2220
   tttgtttgca agcagcagat tacgcgcaga aaaaaaggat ctcaagaaga tcctttgatc 2280
   ttttctacgg ggtctgacgc tcagtggaac gaaaactcac gttaagggat tttggtcatg 2340
   agattatcaa aaaggatctt cacctagatc cttttaaagg ccggccgcgg ccgccatcgg 2400
   cattttcttt tgcgttttta tttgttaact gttaattgtc cttgttcaag gatgctgtct 2460
   ttgacaacag atgttttctt gcctttgatg ttcagcagga agctcggcgc aaacgttgat 2520
   tgtttgtctg cgtagaatcc tctgtttgtc atatagcttg taatcacgac attgtttcct 2580
   ttcgcttgag gtacagcgaa gtgtgagtaa gtaaaggtta catcgttagg atcaagatcc 2640
   atttttaaca caaggccagt tttgttcagc ggcttgtatg ggccagttaa agaattagaa 2700
   acataaccaa gcatgtaaat atcgttagac gtaatgccgt caatcgtcat ttttgatccg 2760
   cgggagtcag tgaacaggta ccatttgccg ttcattttaa agacgttcgc gcgttcaatt 2820
   tcatctgtta ctgtgttaga tgcaatcagc ggtttcatca cttttttcag tgtgtaatca 2880
   tcgtttagct caatcatacc gagagcgccg tttgctaact cagccgtgcg ttttttatcg 2940
   ctttgcagaa gtttttgact ttcttgacgg aagaatgatg tgcttttgcc atagtatgct 3000
   ttgttaaata aagattcttc gccttggtag ccatcttcag ttccagtgtt tgcttcaaat 3060
   actaagtatt tgtggccttt atcttctacg tagtgaggat ctctcagcgt atggttgtcg 3120
   cctgagctgt agttgccttc atcgatgaac tgctgtacat tttgatacgt ttttccgtca 3180
   ccgtcaaaga ttgatttata atcctctaca ccgttgatgt tcaaagagct gtctgatgct 3240
   gatacgttaa cttgtgcagt tgtcagtgtt tgtttgccgt aatgtttacc ggagaaatca 3300
   gtgtagaata aacggatttt tccgtcagat gtaaatgtgg ctgaacctga ccattcttgt 3360
   gtttggtctt ttaggataga atcatttgca tcgaatttgt cgctgtcttt aaagacgcgg 3420
   ccagcgtttt tccagctgtc aatagaagtt tcgccgactt tttgatagaa catgtaaatc 3480
   gatgtgtcat ccgcattttt aggatctccg gctaatgcaa agacgatgtg gtagccgtga 3540
   tagtttgcga cagtgccgtc agcgttttgt aatggccagc tgtcccaaac gtccaggcct 3600
   tttgcagaag agatattttt aattgtggac gaatcaaatt cagaaacttg atatttttca 3660
   tttttttgct gttcagggat ttgcagcata tcatggcgtg taatatggga aatgccgtat 3720
   gtttccttat atggcttttg gttcgtttct ttcgcaaacg cttgagttgc gcctcctgcc 3780
   agcagtgcgg tagtaaaggt taatactgtt gcttgttttg caaacttttt gatgttcatc 3840
   gttcatgtct ccttttttat gtactgtgtt agcggtctgc ttcttccagc cctcctgttt 3900
   gaagatggca agttagttac gcacaataaa aaaagaccta aaatatgtaa ggggtgacgc 3960
   caaagtatac actttgccct ttacacattt taggtcttgc ctgctttatc agtaacaaac 4020
   ccgcgcgatt tacttttcga cctcattcta ttagactctc gtttggattg caactggtct 4080
   attttcctct tttgtttgat agaaaatcat aaaaggattt gcagactacg ggcctaaaga 4140
   actaaaaaat ctatctgttt cttttcattc tctgtatttt ttatagtttc tgttgcatgg 4200
   gcataaagtt gcctttttaa tcacaattca gaaaatatca taatatctca tttcactaaa 4260
   taatagtgaa cggcaggtat atgtgatggg ttaaaaagga tcggcggccg ctcgatttaa 4320
   atc 4323
<210> 14
   <211> 28
   <212> DNA
   <213> Artificial Sequence
<220>
   <223> Primer
<400> 14
   gcgcggtacc tagactcacc ccagtgct 28
<210> 15
   <211> 30
   <212> DNA
   <213> Artificial sequence
<220>
   <223> Primer
<400> 15
   ctctactagt ttagatgtag aactcgatgt 30
<210> 16
   <211> 6349
   <212> DNA
   <213> Artificial Sequence
<220>
   <223> Plasmid
   <220>
   <221> misc_feature
   <223> pCLiK5MCS PmetA metA
<220>
   <221> misc_feature
   <222> (1727)..(2518)
   <223> KanR
<220>
   <221> misc_feature
   <222> (4799)..(5920)
   <223> Rep Protein
<220>
   <221> misc_feature
   <222> (3791)..(4465)
   <223> Ori-Ec (pMB) (komplementär)
<220>
   <221> misc_feature
   <222> (2785)..(3645)
   <223> orf1
<220>
   <221> misc_feature
   <222> (42)..(177)
   <223> PmetA
<220>
   <221> misc_feature
   <222> (178)..(1311)
   <223> metA
<400> 16
   tcgatttaaa tctcgagagg cctgacgtcg ggcccggtac ctagactcac cccagtgctt      60
   aaagcgctgg gtttttcttt ttcagactcg tgagaatgca aactagacta gacagagctg     120
   tccatataca ctggacgaag ttttagtctt gtccacccag aacaggcggt tattttcatg     180
   cccaccctcg cgccttcagg tcaacttgaa atccaagcga tcggtgatgt ctccaccgaa     240
   gccggagcaa tcattacaaa cgctgaaatc gcctatcacc gctggggtga ataccgcgta     300
   gataaagaag gacgcagcaa tgtcgttctc atcgaacacg ccctcactgg agattccaac     360
   gcagccgatt ggtgggctga cttgctcggt cccggcaaag ccatcaacac tgatatttac     420
   tgcgtgatct gtaccaacgt catcggtggt tgcaacggtt ccaccggacc tggctccatg     480
   catccagatg gaaatttctg gggtaatcgc ttccccgcca cgtccattcg tgatcaggta      540
   aacgccgaaa aacaattcct cgacgcactc ggcatcacca cggtcgccgc agtacttggt     600
   ggttccatgg gtggtgcccg caccctagag tgggccgcaa tgtacccaga aactgttggc     660
   gcagctgctg ttcttgcagt ttctgcacgc gccagcgcct ggcaaatcgg cattcaatcc     720
   gcccaaatta aggcgattga aaacgaccac cactggcacg aaggcaacta ctacgaatcc     780
   ggctgcaacc cagccaccgg actcggcgcc gcccgacgca tcgcccacct cacctaccgt     840
   ggcgaactag aaatcgacga acgcttcggc accaaagccc aaaagaacga aaacccactc     900
   ggtccctacc gcaagcccga ccagcgcttc gccgtggaat cctacttgga ctaccaagca     960
   gacaagctag tacagcgttt cgacgccggc tcctacgtct tgctcaccga cgccctcaac    1020
   cgccacgaca ttggtcgcga ccgcggaggc ctcaacaagg cactcgaatc catcaaagtt    1080
   ccagtccttg tcgcaggcgt agataccgat attttgtacc cctaccacca gcaagaacac    1140
   ctctccagaa acctgggaaa tctactggca atggcaaaaa tcgtatcccc tgtcggccac    1200
   gatgctttcc tcaccgaaag ccgccaaatg gatcgcatcg tgaggaactt cttcagcctc    1260
   atctccccag acgaagacaa cccttcgacc tacatcgagt tctacatcta aactagttcg    1320
   gacctaggga tatcgtcgac atcgatgctc ttctgcgtta attaacaatt gggatcctct    1380
   agacccggga tttaaatcgc tagcgggctg ctaaaggaag cggaacacgt agaaagccag    1440
   tccgcagaaa cggtgctgac cccggatgaa tgtcagctac tgggctatct ggacaaggga    1500
   aaacgcaagc gcaaagagaa agcaggtagc ttgcagtggg cttacatggc gatagctaga    1560
   ctgggcggtt ttatggacag caagcgaacc ggaattgcca gctggggcgc cctctggtaa    1620
   ggttgggaag ccctgcaaag taaactggat ggctttcttg ccgccaagga tctgatggcg    1680
   caggggatca agatctgatc aagagacagg atgaggatcg tttcgcatga ttgaacaaga    1740
   tggattgcac gcaggttctc cggccgcttg ggtggagagg ctattcggct atgactgggc    1800
   acaacagaca atcggctgct ctgatgccgc cgtgttccgg ctgtcagcgc aggggcgccc    1860
   ggttcttttt gtcaagaccg acctgtccgg tgccctgaat gaactgcagg acgaggcagc    1920
   gcggctatcg tggctggcca cgacgggcgt tccttgcgca gctgtgctcg acgttgtcac    1980
   tgaagcggga agggactggc tgctattggg cgaagtgccg gggcaggatc tcctgtcatc    2040
   tcaccttgct cctgccgaga aagtatccat catggctgat gcaatgcggc ggctgcatac    2100
   gcttgatccg gctacctgcc cattcgacca ccaagcgaaa catcgcatcg agcgagcacg    2160
   tactcggatg gaagccggtc ttgtcgatca ggatgatctg gacgaagagc atcaggggct    2220
   cgcgccagcc gaactgttcg ccaggctcaa ggcgcgcatg cccgacggcg aggatctcgt    2280
   cgtgacccat ggcgatgcct gcttgccgaa tatcatggtg gaaaatggcc gcttttctgg    2340
   attcatcgac tgtggccggc tgggtgtggc ggaccgctat caggacatag cgttggctac    2400
   ccgtgatatt gctgaagagc ttggcggcga atgggctgac cgcttcctcg tgctttacgg    2460
   tatcgccgct cccgattcgc agcgcatcgc cttctatcgc cttcttgacg agttcttctg    2520
   agcgggactc tggggttcga aatgaccgac caagcgacgc ccaacctgcc atcacgagat    2580
   ttcgattcca ccgccgcctt ctatgaaagg ttgggcttcg gaatcgtttt ccgggacgcc    2640
   ggctggatga tcctccagcg cggggatctc atgctggagt tcttcgccca cgctagcggc    2700
   gcgccggccg gcccggtgtg aaataccgca cagatgcgta aggagaaaat accgcatcag    2760
   gcgctcttcc gcttcctcgc tcactgactc gctgcgctcg gtcgttcggc tgcggcgagc    2820
   ggtatcagct cactcaaagg cggtaatacg gttatccaca gaatcagggg ataacgcagg    2880
   aaagaacatg tgagcaaaag gccagcaaaa ggccaggaac cgtaaaaagg ccgcgttgct    2940
   ggcgtttttc cataggctcc gcccccctga cgagcatcac aaaaatcgac gctcaagtca    3000
   gaggtggcga aacccgacag gactataaag ataccaggcg tttccccctg gaagctccct    3060
   cgtgcgctct cctgttccga ccctgccgct taccggatac ctgtccgcct ttctcccttc    3120
   gggaagcgtg gcgctttctc atagctcacg ctgtaggtat ctcagttcgg tgtaggtcgt 3180
   tcgctccaag ctgggctgtg tgcacgaacc ccccgttcag cccgaccgct gcgccttatc 3240
   cggtaactat cgtcttgagt ccaacccggt aagacacgac ttatcgccac tggcagcagc 3300
   cactggtaac aggattagca gagcgaggta tgtaggcggt gctacagagt tcttgaagtg 3360
   gtggcctaac tacggctaca ctagaaggac agtatttggt atctgcgctc tgctgaagcc 3420
   agttaccttc ggaaaaagag ttggtagctc ttgatccggc aaacaaacca ccgctggtag 3480
   cggtggtttt tttgtttgca agcagcagat tacgcgcaga aaaaaaggat ctcaagaaga 3540
   tcctttgatc ttttctacgg ggtctgacgc tcagtggaac gaaaactcac gttaagggat 3600
   tttggtcatg agattatcaa aaaggatctt cacctagatc cttttaaagg ccggccgcgg 3660
   ccgcgcaaag tcccgcttcg tgaaaatttt cgtgccgcgt gattttccgc caaaaacttt 3720
   aacgaacgtt cgttataatg gtgtcatgac cttcacgacg aagtactaaa attggcccga 3780
   atcatcagct atggatctct ctgatgtcgc gctggagtcc gacgcgctcg atgctgccgt 3840
   cgatttaaaa acggtgatcg gatttttccg agctctcgat acgacggacg cgccagcatc 3900
   acgagactgg gccagtgccg cgagcgacct agaaactctc gtggcggatc ttgaggagct 3960
   ggctgacgag ctgcgtgctc ggccagcgcc aggaggacgc acagtagtgg aggatgcaat 4020
   cagttgcgcc tactgcggtg gcctgattcc tccccggcct gacccgcgag gacggcgcgc 4080
   aaaatattgc tcagatgcgt gtcgtgccgc agccagccgc gagcgcgcca acaaacgcca 4140
   cgccgaggag ctggaggcgg ctaggtcgca aatggcgctg gaagtgcgtc ccccgagcga 4200
   aattttggcc atggtcgtca cagagctgga agcggcagcg agaattatcg cgatcgtggc 4260
   ggtgcccgca ggcatgacaa acatcgtaaa tgccgcgttt cgtgtgccgt ggccgcccag 4320
   gacgtgtcag cgccgccacc acctgcaccg aatcggcagc agcgtcgcgc gtcgaaaaag 4380
   cgcacaggcg gcaagaagcg ataagctgca cgaatacctg aaaaatgttg aacgccccgt 4440
   gagcggtaac tcacagggcg tcggctaacc cccagtccaa acctgggaga aagcgctcaa 4500
   aaatgactct agcggattca cgagacattg acacaccggc ctggaaattt tccgctgatc 4560
   tgttcgacac ccatcccgag ctcgcgctgc gatcacgtgg ctggacgagc gaagaccgcc 4620
   gcgaattcct cgctcacctg ggcagagaaa atttccaggg cagcaagacc cgcgacttcg 4680
   ccagcgcttg gatcaaagac ccggacacgg agaaacacag ccgaagttat accgagttgg 4740
   ttcaaaatcg cttgcccggt gccagtatgt tgctctgacg cacgcgcagc acgcagccgt 4800
   gcttgtcctg gacattgatg tgccgagcca ccaggccggc gggaaaatcg agcacgtaaa 4860
   ccccgaggtc tacgcgattt tggagcgctg ggcacgcctg gaaaaagcgc cagcttggat 4920
   cggcgtgaat ccactgagcg ggaaatgcca gctcatctgg ctcattgatc cggtgtatgc 4980
   cgcagcaggc atgagcagcc cgaatatgcg cctgctggct gcaacgaccg aggaaatgac 5040
   ccgcgttttc ggcgctgacc aggctttttc acataggctg agccgtggcc actgcactct 5100
   ccgacgatcc cagccgtacc gctggcatgc ccagcacaat cgcgtggatc gcctagctga 5160
   tcttatggag gttgctcgca tgatctcagg cacagaaaaa cctaaaaaac gctatgagca 5220
   ggagttttct agcggacggg cacgtatcga agcggcaaga aaagccactg cggaagcaaa 5280
   agcacttgcc acgcttgaag caagcctgcc gagcgccgct gaagcgtctg gagagctgat 5340
   cgacggcgtc cgtgtcctct ggactgctcc agggcgtgcc gcccgtgatg agacggcttt 5400
   tcgccacgct ttgactgtgg gataccagtt aaaagcggct ggtgagcgcc taaaagacac 5460
   caagggtcat cgagcctacg agcgtgccta caccgtcgct caggcggtcg gaggaggccg 5520
   tgagcctgat ctgccgccgg actgtgaccg ccagacggat tggccgcgac gtgtgcgcgg 5580
   ctacgtcgct aaaggccagc cagtcgtccc tgctcgtcag acagagacgc agagccagcc 5640
   gaggcgaaaa gctctggcca ctatgggaag acgtggcggt aaaaaggccg cagaacgctg 5700
   gaaagaccca aacagtgagt acgcccgagc acagcgagaa aaactagcta agtccagtca 5760
   acgacaagct aggaaagcta aaggaaatcg cttgaccatt gcaggttggt ttatgactgt 5820
   tgagggagag actggctcgt ggccgacaat caatgaagct atgtctgaat ttagcgtgtc 5880
   acgtcagacc gtgaatagag cacttaaggt ctgcgggcat tgaacttcca cgaggacgcc 5940
   gaaagcttcc cagtaaatgt gccatctcgt aggcagaaaa cggttccccc gtagggtctc 6000
   tctcttggcc tcctttctag gtcgggctga ttgctcttga agctctctag gggggctcac 6060
   accataggca gataacgttc cccaccggct cgcctcgtaa gcgcacaagg actgctccca 6120
   aagatcttca aagccactgc cgcgactgcc ttcgcgaagc cttgccccgc ggaaatttcc 6180
   tccaccgagt tcgtgcacac ccctatgcca agcttctttc accctaaatt cgagagattg 6240
   gattcttacc gtggaaattc ttcgcaaaaa tcgtcccctg atcgcccttg cgacgttggc 6300
   gtcggtgccg ctggttgcgc ttggcttgac cgacttgatc agcggccgc 6349
<210> 17
   <211> 29
   <212> DNA
   <213> Artificial Sequence
<220>
   <223> Primer
<400> 17
   gagactcgag agctgccaat tattccggg 29
<210> 18
   <211> 41
   <212> DNA
   <213> Artificial Sequence
<220>
   <223> Primer
<400> 18
   cctgaaggcg cgagggtggg catgggtaaa aaatcctttc g 41
<210> 19
   <211> 19
   <212> DNA
   <213> Artificial Sequence
<220>
<223> Primer
<400> 19
   cccaccctcg cgccttcag 19
<210> 20
   <211> 18
   <212> DNA
   <213> Artificial Sequence
<220>
   <223> Primer
<400> 20
   ctgggtacat tgcggccc 18
<210> 21
   <211> 6386
   <212> DNA
   <213> Artificial Sequence
<220>
   <223> Plasmid
   <220>
   <221> misc_feature
   <223> pCLiK5MCS PSODmetA
<220>
   <221> misc_feature
   <222> (1752)..(2543)
   <223> KanR
<220>
   <221> misc_feature
   <222> (4824)..(5945)
   <223> Rep Protein
<220>
   <221> misc_feature
   <222> (2810)..(3670)
   <223> Ori-Ec (pMB) (complementary)
<220>
   <221> misc_feature
   <222> (3816)..(4490)
   <223> orf1
<220>
   <221> misc_feature
   <222> (6)..(196)
   <223> Psod
<220>
   <221> misc_feature
   <222> (197)..(1330)
   <223> metA
<400> 21
   tcgagagctg ccaattattc cgggcttgtg acccgctacc cgataaatag gtcggctgaa      60
   aaatttcgtt gcaatatcaa caaaaaggcc tatcattggg aggtgtcgca ccaagtactt     120
   ttgcgaagcg ccatctgacg gattttcaaa agatgtatat gctcggtgcg gaaacctacg     180
   aaaggatttt ttacccatgc ccaccctcgc gccttcaggt caacttgaaa tccaagcgat     240
   cggtgatgtc tccaccgaag ccggagcaat cattacaaac gctgaaatcg cctatcaccg     300
   ctggggtgaa taccgcgtag ataaagaagg acgcagcaat gtcgttctca tcgaacacgc     360
   cctcactgga gattccaacg cagccgattg gtgggctgac ttgctcggtc ccggcaaagc     420
   catcaacact gatatttact gcgtgatctg taccaacgtc atcggtggtt gcaacggttc     480
   caccggacct ggctccatgc atccagatgg aaatttctgg ggtaatcgct tccccgccac     540
   gtccattcgt gatcaggtaa acgccgaaaa acaattcctc gacgcactcg gcatcaccac     600
   ggtcgccgca gtacttggtg gttccatggg tggtgcccgc accctagagt gggccgcaat     660
   gtacccagaa actgttggcg cagctgctgt tcttgcagtt tctgcacgcg ccagcgcctg     720
   gcaaatcggc attcaatccg cccaaattaa ggcgattgaa aacgaccacc actggcacga     780
   aggcaactac tacgaatccg gctgcaaccc agccaccgga ctcggcgccg cccgacgcat     840
   cgcccacctc acctaccgtg gcgaactaga aatcgacgaa cgcttcggca ccaaagccca     900
   aaagaacgaa aacccactcg gtccctaccg caagcccgac cagcgcttcg ccgtggaatc     960
   ctacttggac taccaagcag acaagctagt acagcgtttc gacgccggct cctacgtctt    1020
   gctcaccgac gccctcaacc gccacgacat tggtcgcgac cgcggaggcc tcaacaaggc    1080
   actcgaatcc atcaaagttc cagtccttgt cgcaggcgta gataccgata ttttgtaccc    1140
   ctaccaccag caagaacacc tctccagaaa cctgggaaat ctactggcaa tggcaaaaat    1200
   cgtatcccct gtcggccacg atgctttcct caccgaaagc cgccaaatgg atcgcatcgt    1260
   gaggaacttc ttcagcctca tctccccaga cgaagacaac ccttcgacct acatcgagtt 1320
   ctacatctaa catatgacta gttcggacct agggatatcg tcgacatcga tgctcttctg 1380
   cgttaattaa caattgggat cctctagacc cgggatttaa atcgctagcg ggctgctaaa 1440
   ggaagcggaa cacgtagaaa gccagtccgc agaaacggtg ctgaccccgg atgaatgtca 1500
   gctactgggc tatctggaca agggaaaacg caagcgcaaa gagaaagcag gtagcttgca 1560
   gtgggcttac atggcgatag ctagactggg cggttttatg gacagcaagc gaaccggaat 1620
   tgccagctgg ggcgccctct ggtaaggttg ggaagccctg caaagtaaac tggatggctt 1680
   tcttgccgcc aaggatctga tggcgcaggg gatcaagatc tgatcaagag acaggatgag 1740
   gatcgtttcg catgattgaa caagatggat tgcacgcagg ttctccggcc gcttgggtgg 1800
   agaggctatt cggctatgac tgggcacaac agacaatcgg ctgctctgat gccgccgtgt 1860
   tccggctgtc agcgcagggg cgcccggttc tttttgtcaa gaccgacctg tccggtgccc 1920
   tgaatgaact gcaggacgag gcagcgcggc tatcgtggct ggccacgacg ggcgttcctt 1980
   gcgcagctgt gctcgacgtt gtcactgaag cgggaaggga ctggctgcta ttgggcgaag 2040
   tgccggggca ggatctcctg tcatctcacc ttgctcctgc cgagaaagta tccatcatgg 2100
   ctgatgcaat gcggcggctg catacgcttg atccggctac ctgcccattc gaccaccaag 2160
   cgaaacatcg catcgagcga gcacgtactc ggatggaagc cggtcttgtc gatcaggatg 2220
   atctggacga agagcatcag gggctcgcgc cagccgaact gttcgccagg ctcaaggcgc 2280
   gcatgcccga cggcgaggat ctcgtcgtga cccatggcga tgcctgcttg ccgaatatca 2340
   tggtggaaaa tggccgcttt tctggattca tcgactgtgg ccggctgggt gtggcggacc 2400
   gctatcagga catagcgttg gctacccgtg atattgctga agagcttggc ggcgaatggg 2460
   ctgaccgctt cctcgtgctt tacggtatcg ccgctcccga ttcgcagcgc atcgccttct 2520
   atcgccttct tgacgagttc ttctgagcgg gactctgggg ttcgaaatga ccgaccaagc 2580
   gacgcccaac ctgccatcac gagatttcga ttccaccgcc gccttctatg aaaggttggg 2640
   cttcggaatc gttttccggg acgccggctg gatgatcctc cagcgcgggg atctcatgct 2700
   ggagttcttc gcccacgcta gcggcgcgcc ggccggcccg gtgtgaaata ccgcacagat 2760
   gcgtaaggag aaaataccgc atcaggcgct cttccgcttc ctcgctcact gactcgctgc 2820
   gctcggtcgt tcggctgcgg cgagcggtat cagctcactc aaaggcggta atacggttat 2880
   ccacagaatc aggggataac gcaggaaaga acatgtgagc aaaaggccag caaaaggcca 2940
   ggaaccgtaa aaaggccgcg ttgctggcgt ttttccatag gctccgcccc cctgacgagc 3000
   atcacaaaaa tcgacgctca agtcagaggt ggcgaaaccc gacaggacta taaagatacc 3060
   aggcgtttcc ccctggaagc tccctcgtgc gctctcctgt tccgaccctg ccgcttaccg 3120
   gatacctgtc cgcctttctc ccttcgggaa gcgtggcgct ttctcatagc tcacgctgta 3180
   ggtatctcag ttcggtgtag gtcgttcgct ccaagctggg ctgtgtgcac gaaccccccg 3240
   ttcagcccga ccgctgcgcc ttatccggta actatcgtct tgagtccaac ccggtaagac 3300
   acgacttatc gccactggca gcagccactg gtaacaggat tagcagagcg aggtatgtag 3360
   gcggtgctac agagttcttg aagtggtggc ctaactacgg ctacactaga aggacagtat 3420
   ttggtatctg cgctctgctg aagccagtta ccttcggaaa aagagttggt agctcttgat 3480
   ccggcaaaca aaccaccgct ggtagcggtg gtttttttgt ttgcaagcag cagattacgc 3540
   gcagaaaaaa aggatctcaa gaagatcctt tgatcttttc tacggggtct gacgctcagt 3600
   ggaacgaaaa ctcacgttaa gggattttgg tcatgagatt atcaaaaagg atcttcacct 3660
   agatcctttt aaaggccggc cgcggccgcg caaagtcccg cttcgtgaaa attttcgtgc 3720
   cgcgtgattt tccgccaaaa actttaacga acgttcgtta taatggtgtc atgaccttca 3780
   cgacgaagta ctaaaattgg cccgaatcat cagctatgga tctctctgat gtcgcgctgg 3840
   agtccgacgc gctcgatgct gccgtcgatt taaaaacggt gatcggattt ttccgagctc 3900
   tcgatacgac ggacgcgcca gcatcacgag actgggccag tgccgcgagc gacctagaaa 3960
   ctctcgtggc ggatcttgag gagctggctg acgagctgcg tgctcggcca gcgccaggag 4020
   gacgcacagt agtggaggat gcaatcagtt gcgcctactg cggtggcctg attcctcccc 4080
   ggcctgaccc gcgaggacgg cgcgcaaaat attgctcaga tgcgtgtcgt gccgcagcca 4140
   gccgcgagcg cgccaacaaa cgccacgccg aggagctgga ggcggctagg tcgcaaatgg 4200
   cgctggaagt gcgtcccccg agcgaaattt tggccatggt cgtcacagag ctggaagcgg 4260
   cagcgagaat tatcgcgatc gtggcggtgc ccgcaggcat gacaaacatc gtaaatgccg 4320
   cgtttcgtgt gccgtggccg cccaggacgt gtcagcgccg ccaccacctg caccgaatcg 4380
   gcagcagcgt cgcgcgtcga aaaagcgcac aggcggcaag aagcgataag ctgcacgaat 4440
   acctgaaaaa tgttgaacgc cccgtgagcg gtaactcaca gggcgtcggc taacccccag 4500
   tccaaacctg ggagaaagcg ctcaaaaatg actctagcgg attcacgaga cattgacaca 4560
   ccggcctgga aattttccgc tgatctgttc gacacccatc ccgagctcgc gctgcgatca 4620
   cgtggctgga cgagcgaaga ccgccgcgaa ttcctcgctc acctgggcag agaaaatttc 4680
   cagggcagca agacccgcga cttcgccagc gcttggatca aagacccgga cacggagaaa 4740
   cacagccgaa gttataccga gttggttcaa aatcgcttgc ccggtgccag tatgttgctc 4800
   tgacgcacgc gcagcacgca gccgtgcttg tcctggacat tgatgtgccg agccaccagg 4860
   ccggcgggaa aatcgagcac gtaaaccccg aggtctacgc gattttggag cgctgggcac 4920
   gcctggaaaa agcgccagct tggatcggcg tgaatccact gagcgggaaa tgccagctca 4980
   tctggctcat tgatccggtg tatgccgcag caggcatgag cagcccgaat atgcgcctgc 5040
   tggctgcaac gaccgaggaa atgacccgcg ttttcggcgc tgaccaggct ttttcacata 5100
   ggctgagccg tggccactgc actctccgac gatcccagcc gtaccgctgg catgcccagc 5160
   acaatcgcgt ggatcgccta gctgatctta tggaggttgc tcgcatgatc tcaggcacag 5220
   aaaaacctaa aaaacgctat gagcaggagt tttctagcgg acgggcacgt atcgaagcgg 5280
   caagaaaagc cactgcggaa gcaaaagcac ttgccacgct tgaagcaagc ctgccgagcg 5340
   ccgctgaagc gtctggagag ctgatcgacg gcgtccgtgt cctctggact gctccagggc 5400
   gtgccgcccg tgatgagacg gcttttcgcc acgctttgac tgtgggatac cagttaaaag 5460
   cggctggtga gcgcctaaaa gacaccaagg gtcatcgagc ctacgagcgt gcctacaccg 5520
   tcgctcaggc ggtcggagga ggccgtgagc ctgatctgcc gccggactgt gaccgccaga 5580
   cggattggcc gcgacgtgtg cgcggctacg tcgctaaagg ccagccagtc gtccctgctc 5640
   gtcagacaga gacgcagagc cagccgaggc gaaaagctct ggccactatg ggaagacgtg 5700
   gcggtaaaaa ggccgcagaa cgctggaaag acccaaacag tgagtacgcc cgagcacagc 5760
   gagaaaaact agctaagtcc agtcaacgac aagctaggaa agctaaagga aatcgcttga 5820
   ccattgcagg ttggtttatg actgttgagg gagagactgg ctcgtggccg acaatcaatg 5880
   aagctatgtc tgaatttagc gtgtcacgtc agaccgtgaa tagagcactt aaggtctgcg 5940
   ggcattgaac ttccacgagg acgccgaaag cttcccagta aatgtgccat ctcgtaggca 6000
   gaaaacggtt cccccgtagg gtctctctct tggcctcctt tctaggtcgg gctgattgct 6060
   cttgaagctc tctagggggg ctcacaccat aggcagataa cgttccccac cggctcgcct 6120
   cgtaagcgca caaggactgc tcccaaagat cttcaaagcc actgccgcga ctgccttcgc 6180
   gaagccttgc cccgcggaaa tttcctccac cgagttcgtg cacaccccta tgccaagctt 6240
   ctttcaccct aaattcgaga gattggattc ttaccgtgga aattcttcgc aaaaatcgtc 6300
   ccctgatcgc ccttgcgacg ttggcgtcgg tgccgctggt tgcgcttggc ttgaccgact 6360
   tgatcagcgg ccgctcgatt taaatc 6386
<210> 22
   <211> 29
   <212> DNA
   <213> Artificial Sequence
<220>
   <223> Primer
<400> 22
   gagactcgag ggccgttacc ctgcgaatg 29
<210> 23
   <211> 40
   <212> DNA
   <213> Artificial Sequence
<220>
   <223> Primer
<400> 23
   cctgaaggcg cgagggtggg cattgtatgt cctcctggac 40
<210> 24
   <211> 19
   <212> DNA
   <213> Artificial Sequence
<220>
   <223> Primer
<400> 24
   cccaccctcg cgccttcag 19
<210> 25
   <211> 18
   <212> DNA
   <213> Artificial Sequence
<220>
   <223> Primer
<400> 25
   ctgggtacat tgcggccc 18
<210> 26
   <211> 6394
   <212> DNA
   <213> Artificial Sequence
<220>
   <223> Plasmid
   <220>
   <221> misc_feature
   <223> pCLiK5MCS P EFTUmetA
<220>
   <221> misc_feature
   <222> (1772)..(2563)
   <223> KanR
<220>
   <221> misc_feature
   <222> (4844)..(6965)
   <223> Rep Protein
<220>
   <221> misc_feature
   <222> (2830)..(3690)
   <223> Ori-Ec (pMB) (complementary)
<220>
   <221> misc_feature
   <222> (3836)..(4510)
   <223> orf1
<220>
   <221> misc_feature
   <222> (18)..(216)
   <223> Peftu
<220>
   <221> misc_feature
   <222> (217)..(1350)
   <223> metA
<400> 26
   tcgatttaaa tctcgagggc cgttaccctg cgaatgtcca cagggtagct ggtagtttga      60
   aaatcaacgc cgttgccctt aggattcagt aactggcaca ttttgtaatg cgctagatct     120
   gtgtgctcag tcttccaggc tgcttatcac agtgaaagca aaaccaattc gtggctgcga     180
   aagtcgtagc caccacgaag tccaggagga catacaatgc ccaccctcgc gccttcaggt     240
   caacttgaaa tccaagcgat cggtgatgtc tccaccgaag ccggagcaat cattacaaac     300
   gctgaaatcg cctatcaccg ctggggtgaa taccgcgtag ataaagaagg acgcagcaat     360
   gtcgttctca tcgaacacgc cctcactgga gattccaacg cagccgattg gtgggctgac     420
   ttgctcggtc ccggcaaagc catcaacact gatatttact gcgtgatctg taccaacgtc     480
   atcggtggtt gcaacggttc caccggacct ggctccatgc atccagatgg aaatttctgg     540
   ggtaatcgct tccccgccac gtccattcgt gatcaggtaa acgccgaaaa acaattcctc     600
   gacgcactcg gcatcaccac ggtcgccgca gtacttggtg gttccatggg tggtgcccgc     660
   accctagagt gggccgcaat gtacccagaa actgttggcg cagctgctgt tcttgcagtt     720
   tctgcacgcg ccagcgcctg gcaaatcggc attcaatccg cccaaattaa ggcgattgaa     780
   aacgaccacc actggcacga aggcaactac tacgaatccg gctgcaaccc agccaccgga     840
   ctcggcgccg cccgacgcat cgcccacctc acctaccgtg gcgaactaga aatcgacgaa     900
   cgcttcggca ccaaagccca aaagaacgaa aacccactcg gtccctaccg caagcccgac     960
   cagcgcttcg ccgtggaatc ctacttggac taccaagcag acaagctagt acagcgtttc    1020
   gacgccggct cctacgtctt gctcaccgac gccctcaacc gccacgacat tggtcgcgac    1080
   cgcggaggcc tcaacaaggc actcgaatcc atcaaagttc cagtccttgt cgcaggcgta    1140
   gataccgata ttttgtaccc ctaccaccag caagaacacc tctccagaaa cctgggaaat    1200
   ctactggcaa tggcaaaaat cgtatcccct gtcggccacg atgctttcct caccgaaagc    1260
   cgccaaatgg atcgcatcgt gaggaacttc ttcagcctca tctccccaga cgaagacaac    1320
   ccttcgacct acatcgagtt ctacatctaa catatgacta gttcggacct agggatatcg    1380
   tcgacatcga tgctcttctg cgttaattaa caattgggat cctctagacc cgggatttaa    1440
   atcgctagcg ggctgctaaa ggaagcggaa cacgtagaaa gccagtccgc agaaacggtg    1500
   ctgaccccgg atgaatgtca gctactgggc tatctggaca agggaaaacg caagcgcaaa    1560
   gagaaagcag gtagcttgca gtgggcttac atggcgatag ctagactggg cggttttatg    1620
   gacagcaagc gaaccggaat tgccagctgg ggcgccctct ggtaaggttg ggaagccctg    1680
   caaagtaaac tggatggctt tcttgccgcc aaggatctga tggcgcaggg gatcaagatc    1740
   tgatcaagag acaggatgag gatcgtttcg catgattgaa caagatggat tgcacgcagg 1800
   ttctccggcc gcttgggtgg agaggctatt cggctatgac tgggcacaac agacaatcgg 1860
   ctgctctgat gccgccgtgt tccggctgtc agcgcagggg cgcccggttc tttttgtcaa 1920
   gaccgacctg tccggtgccc tgaatgaact gcaggacgag gcagcgcggc tatcgtggct 1980
   ggccacgacg ggcgttcctt gcgcagctgt gctcgacgtt gtcactgaag cgggaaggga 2040
   ctggctgcta ttgggcgaag tgccggggca ggatctcctg tcatctcacc ttgctcctgc 2100
   cgagaaagta tccatcatgg ctgatgcaat gcggcggctg catacgcttg atccggctac 2160
   ctgcccattc gaccaccaag cgaaacatcg catcgagcga gcacgtactc ggatggaagc 2220
   cggtcttgtc gatcaggatg atctggacga agagcatcag gggctcgcgc cagccgaact 2280
   gttcgccagg ctcaaggcgc gcatgcccga cggcgaggat ctcgtcgtga cccatggcga 2340
   tgcctgcttg ccgaatatca tggtggaaaa tggccgcttt tctggattca tcgactgtgg 2400
   ccggctgggt gtggcggacc gctatcagga catagcgttg gctacccgtg atattgctga 2460
   agagcttggc ggcgaatggg ctgaccgctt cctcgtgctt tacggtatcg ccgctcccga 2520
   ttcgcagcgc atcgccttct atcgccttct tgacgagttc ttctgagcgg gactctgggg 2580
   ttcgaaatga ccgaccaagc gacgcccaac ctgccatcac gagatttcga ttccaccgcc 2640
   gccttctatg aaaggttggg cttcggaatc gttttccggg acgccggctg gatgatcctc 2700
   cagcgcgggg atctcatgct ggagttcttc gcccacgcta gcggcgcgcc ggccggcccg 2760
   gtgtgaaata ccgcacagat gcgtaaggag aaaataccgc atcaggcgct cttccgcttc 2820
   ctcgctcact gactcgctgc gctcggtcgt tcggctgcgg cgagcggtat cagctcactc 2880
   aaaggcggta atacggttat ccacagaatc aggggataac gcaggaaaga acatgtgagc 2940
   aaaaggccag caaaaggcca ggaaccgtaa aaaggccgcg ttgctggcgt ttttccatag 3000
   gctccgcccc cctgacgagc atcacaaaaa tcgacgctca agtcagaggt ggcgaaaccc 3060
   gacaggacta taaagatacc aggcgtttcc ccctggaagc tccctcgtgc gctctcctgt 3120
   tccgaccctg ccgcttaccg gatacctgtc cgcctttctc ccttcgggaa gcgtggcgct 3180
   ttctcatagc tcacgctgta ggtatctcag ttcggtgtag gtcgttcgct ccaagctggg 3240
   ctgtgtgcac gaaccccccg ttcagcccga ccgctgcgcc ttatccggta actatcgtct 3300
   tgagtccaac ccggtaagac acgacttatc gccactggca gcagccactg gtaacaggat 3360
   tagcagagcg aggtatgtag gcggtgctac agagttcttg aagtggtggc ctaactacgg 3420
   ctacactaga aggacagtat ttggtatctg cgctctgctg aagccagtta ccttcggaaa 3480
   aagagttggt agctcttgat ccggcaaaca aaccaccgct ggtagcggtg gtttttttgt 3540
   ttgcaagcag cagattacgc gcagaaaaaa aggatctcaa gaagatcctt tgatcttttc 3600
   tacggggtct gacgctcagt ggaacgaaaa ctcacgttaa gggattttgg tcatgagatt 3660
   atcaaaaagg atcttcacct agatcctttt aaaggccggc cgcggccgcg caaagtcccg 3720
   cttcgtgaaa attttcgtgc cgcgtgattt tccgccaaaa actttaacga acgttcgtta 3780
   taatggtgtc atgaccttca cgacgaagta ctaaaattgg cccgaatcat cagctatgga 3840
   tctctctgat gtcgcgctgg agtccgacgc gctcgatgct gccgtcgatt taaaaacggt 3900
   gatcggattt ttccgagctc tcgatacgac ggacgcgcca gcatcacgag actgggccag 3960
   tgccgcgagc gacctagaaa ctctcgtggc ggatcttgag gagctggctg acgagctgcg 4020
   tgctcggcca gcgccaggag gacgcacagt agtggaggat gcaatcagtt gcgcctactg 4080
   cggtggcctg attcctcccc ggcctgaccc gcgaggacgg cgcgcaaaat attgctcaga 4140
   tgcgtgtcgt gccgcagcca gccgcgagcg cgccaacaaa cgccacgccg aggagctgga 4200
   ggcggctagg tcgcaaatgg cgctggaagt gcgtcccccg agcgaaattt tggccatggt 4260
   cgtcacagag ctggaagcgg cagcgagaat tatcgcgatc gtggcggtgc ccgcaggcat 4320
   gacaaacatc gtaaatgccg cgtttcgtgt gccgtggccg cccaggacgt gtcagcgccg 4380
   ccaccacctg caccgaatcg gcagcagcgt cgcgcgtcga aaaagcgcac aggcggcaag 4440
   aagcgataag ctgcacgaat acctgaaaaa tgttgaacgc cccgtgagcg gtaactcaca 4500
   gggcgtcggc taacccccag tccaaacctg ggagaaagcg ctcaaaaatg actctagcgg 4560
   attcacgaga cattgacaca ccggcctgga aattttccgc tgatctgttc gacacccatc 4620
   ccgagctcgc gctgcgatca cgtggctgga cgagcgaaga ccgccgcgaa ttcctcgctc 4680
   acctgggcag agaaaatttc cagggcagca agacccgcga cttcgccagc gcttggatca 4740
   aagacccgga cacggagaaa cacagccgaa gttataccga gttggttcaa aatcgcttgc 4800
   ccggtgccag tatgttgctc tgacgcacgc gcagcacgca gccgtgcttg tcctggacat 4860
   tgatgtgccg agccaccagg ccggcgggaa aatcgagcac gtaaaccccg aggtctacgc 4920
   gattttggag cgctgggcac gcctggaaaa agcgccagct tggatcggcg tgaatccact 4980
   gagcgggaaa tgccagctca tctggctcat tgatccggtg tatgccgcag caggcatgag 5040
   cagcccgaat atgcgcctgc tggctgcaac gaccgaggaa atgacccgcg ttttcggcgc 5100
   tgaccaggct ttttcacata ggctgagccg tggccactgc actctccgac gatcccagcc 5160
   gtaccgctgg catgcccagc acaatcgcgt ggatcgccta gctgatctta tggaggttgc 5220
   tcgcatgatc tcaggcacag aaaaacctaa aaaacgctat gagcaggagt tttctagcgg 5280
   acgggcacgt atcgaagcgg caagaaaagc cactgcggaa gcaaaagcac ttgccacgct 5340
   tgaagcaagc ctgccgagcg ccgctgaagc gtctggagag ctgatcgacg gcgtccgtgt 5400
   cctctggact gctccagggc gtgccgcccg tgatgagacg gcttttcgcc acgctttgac 5460
   tgtgggatac cagttaaaag cggctggtga gcgcctaaaa gacaccaagg gtcatcgagc 5520
   ctacgagcgt gcctacaccg tcgctcaggc ggtcggagga ggccgtgagc ctgatctgcc 5580
   gccggactgt gaccgccaga cggattggcc gcgacgtgtg cgcggctacg tcgctaaagg 5640
   ccagccagtc gtccctgctc gtcagacaga gacgcagagc cagccgaggc gaaaagctct 5700
   ggccactatg ggaagacgtg gcggtaaaaa ggccgcagaa cgctggaaag acccaaacag 5760
   tgagtacgcc cgagcacagc gagaaaaact agctaagtcc agtcaacgac aagctaggaa 5820
   agctaaagga aatcgcttga ccattgcagg ttggtttatg actgttgagg gagagactgg 5880
   ctcgtggccg acaatcaatg aagctatgtc tgaatttagc gtgtcacgtc agaccgtgaa 5940
   tagagcactt aaggtctgcg ggcattgaac ttccacgagg acgccgaaag cttcccagta 6000
   aatgtgccat ctcgtaggca gaaaacggtt cccccgtagg gtctctctct tggcctcctt 6060
   tctaggtcgg gctgattgct cttgaagctc tctagggggg ctcacaccat aggcagataa 6120
   cgttccccac cggctcgcct cgtaagcgca caaggactgc tcccaaagat cttcaaagcc 6180
   actgccgcga ctgccttcgc gaagccttgc cccgcggaaa tttcctccac cgagttcgtg 6240
   cacaccccta tgccaagctt ctttcaccct aaattcgaga gattggattc ttaccgtgga 6300
   aattcttcgc aaaaatcgtc ccctgatcgc ccttgcgacg ttggcgtcgg tgccgctggt 6360
   tgcgcttggc ttgaccgact tgatcagcgg ccgc 6394
<210> 27
   <211> 30
   <212> DNA
   <213> Artificial Sequence
<220>
   <223> Primer
<400> 27
   gagactcgag cggcttaaag tttggctgcc 30
<210> 28
   <211> 41
   <212> DNA
   <213> Artificial sequence
<220>
   <223> Primer
<400> 28
   cctgaaggcg cgagggtggg catgatgaat ccctccatga g 41
<210> 29
   <211> 19
   <212> DNA
   <213> Artificial sequence
<220>
   <223> Primer
<400> 29
   cccaccctcg cgccttcag 19
<210> 30
   <211> 18
   <212> DNA
   <213> Artificial Sequence
<220>
<223> Primer
<400> 30
   ctgggtacat tgcggccc 18
<210> 31
   <211> 6372
   <212> DNA
   <213> Artificial Sequence
<220>
   <223> Plasmid
   <220>
   <221> misc_feature
   <223> pCLiK5MCS PGroESmetA
<220>
   <221> misc_feature
   <222> (1735)..(2526)
   <223> KanR
<220>
   <221> misc_feature
   <222> (4807)..(5928)
   <223> Rep Protein
<220>
   <221> misc_feature
   <222> (2793)..(3653)
   <223> Ori-EC (pMB) (complementary)
<220>
   <221> misc_feature
   <222> (3799)..(4473)
   <223> orf1
<220>
   <221> misc_feature
   <222> (3)..(179)
   <223> Pgro
<220>
   <221> misc_feature
   <222> (180)..(1313)
   <223> metA
<400> 31
   agcggcttaa agtttggctg ccatgtgaat ttttagcacc ctcaacagtt gagtgctggc 60
   actctcgggg gtagagtgcc aaataggttg tttgacacac agttgttcac ccgcgacgac 120
   ggctgtgctg gaaacccaca accggcacac acaaaatttt tctcatggag ggattcatca 180
   tgcccaccct cgcgccttca ggtcaacttg aaatccaagc gatcggtgat gtctccaccg 240
   aagccggagc aatcattaca aacgctgaaa tcgcctatca ccgctggggt gaataccgcg 300
   tagataaaga aggacgcagc aatgtcgttc tcatcgaaca cgccctcact ggagattcca 360
   acgcagccga ttggtgggct gacttgctcg gtcccggcaa agccatcaac actgatattt 420
   actgcgtgat ctgtaccaac gtcatcggtg gttgcaacgg ttccaccgga cctggctcca 480
   tgcatccaga tggaaatttc tggggtaatc gcttccccgc cacgtccatt cgtgatcagg 540
   taaacgccga aaaacaattc ctcgacgcac tcggcatcac cacggtcgcc gcagtacttg 600
   gtggttccat gggtggtgcc cgcaccctag agtgggccgc aatgtaccca gaaactgttg 660
   gcgcagctgc tgttcttgca gtttctgcac gcgccagcgc ctggcaaatc ggcattcaat 720
   ccgcccaaat taaggcgatt gaaaacgacc accactggca cgaaggcaac tactacgaat 780
   ccggctgcaa cccagccacc ggactcggcg ccgcccgacg catcgcccac ctcacctacc 840
   gtggcgaact agaaatcgac gaacgcttcg gcaccaaagc ccaaaagaac gaaaacccac 900
   tcggtcccta ccgcaagccc gaccagcgct tcgccgtgga atcctacttg gactaccaag 960
   cagacaagct agtacagcgt ttcgacgccg gctcctacgt cttgctcacc gacgccctca 1020
   accgccacga cattggtcgc gaccgcggag gcctcaacaa ggcactcgaa tccatcaaag 1080
   ttccagtcct tgtcgcaggc gtagataccg atattttgta cccctaccac cagcaagaac 1140
   acctctccag aaacctggga aatctactgg caatggcaaa aatcgtatcc cctgtcggcc 1200
   acgatgcttt cctcaccgaa agccgccaaa tggatcgcat cgtgaggaac ttcttcagcc 1260
   tcatctcccc agacgaagac aacccttcga cctacatcga gttctacatc taacatatga 1320
   ctagttcgga cctagggata tcgtcgacat cgatgctctt ctgcgttaat taacaattgg 1380
   gatcctctag acccgggatt taaatcgcta gcgggctgct aaaggaagcg gaacacgtag 1440
   aaagccagtc cgcagaaacg gtgctgaccc cggatgaatg tcagctactg ggctatctgg 1500
   acaagggaaa acgcaagcgc aaagagaaag caggtagctt gcagtgggct tacatggcga 1560
   tagctagact gggcggtttt atggacagca agcgaaccgg aattgccagc tggggcgccc 1620
   tctggtaagg ttgggaagcc ctgcaaagta aactggatgg ctttcttgcc gccaaggatc 1680
   tgatggcgca ggggatcaag atctgatcaa gagacaggat gaggatcgtt tcgcatgatt 1740
   gaacaagatg gattgcacgc aggttctccg gccgcttggg tggagaggct attcggctat 1800
   gactgggcac aacagacaat cggctgctct gatgccgccg tgttccggct gtcagcgcag 1860
   gggcgcccgg ttctttttgt caagaccgac ctgtccggtg ccctgaatga actgcaggac 1920
   gaggcagcgc ggctatcgtg gctggccacg acgggcgttc cttgcgcagc tgtgctcgac 1980
   gttgtcactg aagcgggaag ggactggctg ctattgggcg aagtgccggg gcaggatctc 2040
   ctgtcatctc accttgctcc tgccgagaaa gtatccatca tggctgatgc aatgcggcgg 2100
   ctgcatacgc ttgatccggc tacctgccca ttcgaccacc aagcgaaaca tcgcatcgag 2160
   cgagcacgta ctcggatgga agccggtctt gtcgatcagg atgatctgga cgaagagcat 2220
   caggggctcg cgccagccga actgttcgcc aggctcaagg cgcgcatgcc cgacggcgag 2280
   gatctcgtcg tgacccatgg cgatgcctgc ttgccgaata tcatggtgga aaatggccgc 2340
   ttttctggat tcatcgactg tggccggctg ggtgtggcgg accgctatca ggacatagcg 2400
   ttggctaccc gtgatattgc tgaagagctt ggcggcgaat gggctgaccg cttcctcgtg 2460
   ctttacggta tcgccgctcc cgattcgcag cgcatcgcct tctatcgcct tcttgacgag 2520
   ttcttctgag cgggactctg gggttcgaaa tgaccgacca agcgacgccc aacctgccat 2580
   cacgagattt cgattccacc gccgccttct atgaaaggtt gggcttcgga atcgttttcc 2640
   gggacgccgg ctggatgatc ctccagcgcg gggatctcat gctggagttc ttcgcccacg 2700
   ctagcggcgc gccggccggc ccggtgtgaa ataccgcaca gatgcgtaag gagaaaatac 2760
   cgcatcaggc gctcttccgc ttcctcgctc actgactcgc tgcgctcggt cgttcggctg 2820
   cggcgagcgg tatcagctca ctcaaaggcg gtaatacggt tatccacaga atcaggggat 2880
   aacgcaggaa agaacatgtg agcaaaaggc cagcaaaagg ccaggaaccg taaaaaggcc 2940
   gcgttgctgg cgtttttcca taggctccgc ccccctgacg agcatcacaa aaatcgacgc 3000
   tcaagtcaga ggtggcgaaa cccgacagga ctataaagat accaggcgtt tccccctgga 3060
   agctccctcg tgcgctctcc tgttccgacc ctgccgctta ccggatacct gtccgccttt 3120
   ctcccttcgg gaagcgtggc gctttctcat agctcacgct gtaggtatct cagttcggtg 3180
   taggtcgttc gctccaagct gggctgtgtg cacgaacccc ccgttcagcc cgaccgctgc 3240
   gccttatccg gtaactatcg tcttgagtcc aacccggtaa gacacgactt atcgccactg 3300
   gcagcagcca ctggtaacag gattagcaga gcgaggtatg taggcggtgc tacagagttc 3360
   ttgaagtggt ggcctaacta cggctacact agaaggacag tatttggtat ctgcgctctg 3420
   ctgaagccag ttaccttcgg aaaaagagtt ggtagctctt gatccggcaa acaaaccacc 3480
   gctggtagcg gtggtttttt tgtttgcaag cagcagatta cgcgcagaaa aaaaggatct 3540
   caagaagatc ctttgatctt ttctacgggg tctgacgctc agtggaacga aaactcacgt 3600
   taagggattt tggtcatgag attatcaaaa aggatcttca cctagatcct tttaaaggcc 3660
   ggccgcggcc gcgcaaagtc ccgcttcgtg aaaattttcg tgccgcgtga ttttccgcca 3720
   aaaactttaa cgaacgttcg ttataatggt gtcatgacct tcacgacgaa gtactaaaat 3780
   tggcccgaat catcagctat ggatctctct gatgtcgcgc tggagtccga cgcgctcgat 3840
   gctgccgtcg atttaaaaac ggtgatcgga tttttccgag ctctcgatac gacggacgcg 3900
   ccagcatcac gagactgggc cagtgccgcg agcgacctag aaactctcgt ggcggatctt 3960
   gaggagctgg ctgacgagct gcgtgctcgg ccagcgccag gaggacgcac agtagtggag 4020
   gatgcaatca gttgcgccta ctgcggtggc ctgattcctc cccggcctga cccgcgagga 4080
   cggcgcgcaa aatattgctc agatgcgtgt cgtgccgcag ccagccgcga gcgcgccaac 4140
   aaacgccacg ccgaggagct ggaggcggct aggtcgcaaa tggcgctgga agtgcgtccc 4200
   ccgagcgaaa ttttggccat ggtcgtcaca gagctggaag cggcagcgag aattatcgcg 4260
   atcgtggcgg tgcccgcagg catgacaaac atcgtaaatg ccgcgtttcg tgtgccgtgg 4320
   ccgcccagga cgtgtcagcg ccgccaccac ctgcaccgaa tcggcagcag cgtcgcgcgt 4380
   cgaaaaagcg cacaggcggc aagaagcgat aagctgcacg aatacctgaa aaatgttgaa 4440
   cgccccgtga gcggtaactc acagggcgtc ggctaacccc cagtccaaac ctgggagaaa   4500
   gcgctcaaaa atgactctag cggattcacg agacattgac acaccggcct ggaaattttc   4560
   cgctgatctg ttcgacaccc atcccgagct cgcgctgcga tcacgtggct ggacgagcga   4620
   agaccgccgc gaattcctcg ctcacctggg cagagaaaat ttccagggca gcaagacccg   4680
   cgacttcgcc agcgcttgga tcaaagaccc ggacacggag aaacacagcc gaagttatac   4740
   cgagttggtt caaaatcgct tgcccggtgc cagtatgttg ctctgacgca cgcgcagcac   4800
   gcagccgtgc ttgtcctgga cattgatgtg ccgagccacc aggccggcgg gaaaatcgag   4860
   cacgtaaacc ccgaggtcta cgcgattttg gagcgctggg cacgcctgga aaaagcgcca   4920
   gcttggatcg gcgtgaatcc actgagcggg aaatgccagc tcatctggct cattgatccg   4980
   gtgtatgccg cagcaggcat gagcagcccg aatatgcgcc tgctggctgc aacgaccgag   5040
   gaaatgaccc gcgttttcgg cgctgaccag gctttttcac ataggctgag ccgtggccac   5100
   tgcactctcc gacgatccca gccgtaccgc tggcatgccc agcacaatcg cgtggatcgc   5160
   ctagctgatc ttatggaggt tgctcgcatg atctcaggca cagaaaaacc taaaaaacgc   5220
   tatgagcagg agttttctag cggacgggca cgtatcgaag cggcaagaaa agccactgcg   5280
   gaagcaaaag cacttgccac gcttgaagca agcctgccga gcgccgctga agcgtctgga   5340
   gagctgatcg acggcgtccg tgtcctctgg actgctccag ggcgtgccgc ccgtgatgag   5400
   acggcttttc gccacgcttt gactgtggga taccagttaa aagcggctgg tgagcgccta   5460
   aaagacacca agggtcatcg agcctacgag cgtgcctaca ccgtcgctca ggcggtcgga   5520
   ggaggccgtg agcctgatct gccgccggac tgtgaccgcc agacggattg gccgcgacgt   5580
   gtgcgcggct acgtcgctaa aggccagcca gtcgtccctg ctcgtcagac agagacgcag   5640
   agccagccga ggcgaaaagc tctggccact atgggaagac gtggcggtaa aaaggccgca   5700
   gaacgctgga aagacccaaa cagtgagtac gcccgagcac agcgagaaaa actagctaag   5760
   tccagtcaac gacaagctag gaaagctaaa ggaaatcgct tgaccattgc aggttggttt   5820
   atgactgttg agggagagac tggctcgtgg ccgacaatca atgaagctat gtctgaattt   5880
   agcgtgtcac gtcagaccgt gaatagagca cttaaggtct gcgggcattg aacttccacg   5940
   aggacgccga aagcttccca gtaaatgtgc catctcgtag gcagaaaacg gttcccccgt   6000
   agggtctctc tcttggcctc ctttctaggt cgggctgatt gctcttgaag ctctctaggg   6060
   gggctcacac cataggcaga taacgttccc caccggctcg cctcgtaagc gcacaaggac   6120
   tgctcccaaa gatcttcaaa gccactgccg cgactgcctt cgcgaagcct tgccccgcgg   6180
   aaatttcctc caccgagttc gtgcacaccc ctatgccaag cttctttcac cctaaattcg   6240
   agagattgga ttcttaccgt ggaaattctt cgcaaaaatc gtcccctgat cgcccttgcg   6300
   acgttggcgt cggtgccgct ggttgcgctt ggcttgaccg acttgatcag cggccgctcg   6360
   atttaaatct cg                                                       6372
<210> 32
   <211> 32
   <212> DNA
   <213> Artificial Sequence
<220>
   <223> Primer
   <220>
   <221> misc_feature
   <223> internal designation: BK 1849
<400> 32
   gtgtgtcgac ttagatgtag aactcgatgt ag 32
<210> 33
   <211> 17
   <212> DNA
   <213> Artificial Sequence
<220>
   <223> Primer
   <220>
   <221> misc_feature
   <223> internal designation: BK 1862
<400> 33
   atgcccaccc tcgcgcc 17
<210> 34
   <211> 28
   <212> DNA
   <213> Artificial Sequence
<220>
   <223> Primer
   <220>
   <221> misc_feature
   <223> internal designation: Haf 26
<400> 34
   gagaggatcc cccccacgac aatggaac 28
<210> 35
   <211> 44
   <212> DNA
   <213> Artificial Sequence
<220>
   <223> Primer
   <220>
   <221> misc_feature
   <223> internal designation: Haf 27)
<400> 35
   cctgaaggcg cgagggtggg cattacgggg cgatcctcct tatg 44
<210> 36
   <211> 6389
   <212> DNA
   <213> Artificial Sequence
<220>
   <223> Plasmid
   <220>
   <221> misc_feature
   <223> pCLiK5MCS P EF-TS metA
<220>
   <221> misc_feature
   <222> (1767)..(2558)
   <223> KanR
<220>
   <221> misc_feature
   <222> (4839)..(5960)
   <223> Rep Protein
<220>
   <221> misc_feature
   <222> (2825)..(3685)
   <223> Ori-Ec (pMB) (complementary)
<220>
   <221> misc_feature
   <222> (3831)-..(4505)
   <223> Orf1
<220>
   <221> misc_feature
   <222> (1217)..(1411)
   <223> P EF-TS (complementary)
<220>
   <221> misc_feature
   <222> (83)..(1216)
   <223> metA (complementary)
<400> 36
   tcgatttaaa tctcgagagg cctgacgtcg ggcccggtac cacgcgtcat atgactagtt     60
   cggacctagg gatatcgtcg acttagatgt agaactcgat gtaggtcgaa gggttgtctt    120
   cgtctgggga gatgaggctg aagaagttcc tcacgatgcg atccatttgg cggctttcgg    180
   tgaggaaagc atcgtggccg acaggggata cgatttttgc cattgccagt agatttccca    240
   ggtttctgga gaggtgttct tgctggtggt aggggtacaa aatatcggta tctacgcctg    300
   cgacaaggac tggaactttg atggattcga gtgccttgtt gaggcctccg cggtcgcgac    360
   caatgtcgtg gcggttgagg gcgtcggtga gcaagacgta ggagccggcg tcgaaacgct    420
   gtactagctt gtctgcttgg tagtccaagt aggattccac ggcgaagcgc tggtcgggct    480
   tgcggtaggg accgagtggg ttttcgttct tttgggcttt ggtgccgaag cgttcgtcga    540
   tttctagttc gccacggtag gtgaggtggg cgatgcgtcg ggcggcgccg agtccggtgg    600
   ctgggttgca gccggattcg tagtagttgc cttcgtgcca gtggtggtcg ttttcaatcg    660
   ccttaatttg ggcggattga atgccgattt gccaggcgct ggcgcgtgca gaaactgcaa    720
   gaacagcagc tgcgccaaca gtttctgggt acattgcggc ccactctagg gtgcgggcac    780
   cacccatgga accaccaagt actgcggcga ccgtggtgat gccgagtgcg tcgaggaatt    840
   gtttttcggc gtttacctga tcacgaatgg acgtggcggg gaagcgatta ccccagaaat    900
   ttccatctgg atgcatggag ccaggtccgg tggaaccgtt gcaaccaccg atgacgttgg    960
   tacagatcac gcagtaaata tcagtgttga tggctttgcc gggaccgagc aagtcagccc   1020
   accaatcggc tgcgttggaa tctccagtga gggcgtgttc gatgagaacg acattgctgc   1080
   gtccttcttt atctacgcgg tattcacccc agcggtgata ggcgatttca gcgtttgtaa   1140
   tgattgctcc ggcttcggtg gagacatcac cgatcgcttg gatttcaagt tgacctgaag   1200
   gcgcgagggt gggcattacg gggcgatcct ccttatgtat ggataattaa tgctttacaa   1260
   gcttaggtta gggcacagta gccctacccg cccgtctaaa aagtgatcgt acaggtattc   1320
   agtcgattac gttgcgacac ggcgggctgg ctgcccaaaa agcccccacg gcgatgaaat   1380
   tttaaaagtc aaagttccat tgtcgtgggg gggatcctct agacccggga tttaaatcgc   1440
   tagcgggctg ctaaaggaag cggaacacgt agaaagccag tccgcagaaa cggtgctgac   1500
   cccggatgaa tgtcagctac tgggctatct ggacaaggga aaacgcaagc gcaaagagaa   1560
   agcaggtagc ttgcagtggg cttacatggc gatagctaga ctgggcggtt ttatggacag   1620
   caagcgaacc ggaattgcca gctggggcgc cctctggtaa ggttgggaag ccctgcaaag 1680
   taaactggat ggctttcttg ccgccaagga tctgatggcg caggggatca agatctgatc 1740
   aagagacagg atgaggatcg tttcgcatga ttgaacaaga tggattgcac gcaggttctc 1800
   cggccgcttg ggtggagagg ctattcggct atgactgggc acaacagaca atcggctgct 1860
   ctgatgccgc cgtgttccgg ctgtcagcgc aggggcgccc ggttcttttt gtcaagaccg 1920
   acctgtccgg tgccctgaat gaactgcagg acgaggcagc gcggctatcg tggctggcca 1980
   cgacgggcgt tccttgcgca gctgtgctcg acgttgtcac tgaagcggga agggactggc 2040
   tgctattggg cgaagtgccg gggcaggatc tcctgtcatc tcaccttgct cctgccgaga 2100
   aagtatccat catggctgat gcaatgcggc ggctgcatac gcttgatccg gctacctgcc 2160
   cattcgacca ccaagcgaaa catcgcatcg agcgagcacg tactcggatg gaagccggtc 2220
   ttgtcgatca ggatgatctg gacgaagagc atcaggggct cgcgccagcc gaactgttcg 2280
   ccaggctcaa ggcgcgcatg cccgacggcg aggatctcgt cgtgacccat ggcgatgcct 2340
   gcttgccgaa tatcatggtg gaaaatggcc gcttttctgg attcatcgac tgtggccggc 2400
   tgggtgtggc ggaccgctat caggacatag cgttggctac ccgtgatatt gctgaagagc 2460
   ttggcggcga atgggctgac cgcttcctcg tgctttacgg tatcgccgct cccgattcgc 2520
   agcgcatcgc cttctatcgc cttcttgacg agttcttctg agcgggactc tggggttcga 2580
   aatgaccgac caagcgacgc ccaacctgcc atcacgagat ttcgattcca ccgccgcctt 2640
   ctatgaaagg ttgggcttcg gaatcgtttt ccgggacgcc ggctggatga tcctccagcg 2700
   cggggatctc atgctggagt tcttcgccca cgctagcggc gcgccggccg gcccggtgtg 2760
   aaataccgca cagatgcgta aggagaaaat accgcatcag gcgctcttcc gcttcctcgc 2820
   tcactgactc gctgcgctcg gtcgttcggc tgcggcgagc ggtatcagct cactcaaagg 2880
   cggtaatacg gttatccaca gaatcagggg ataacgcagg aaagaacatg tgagcaaaag 2940
   gccagcaaaa ggccaggaac cgtaaaaagg ccgcgttgct ggcgtttttc cataggctcc 3000
   gcccccctga cgagcatcac aaaaatcgac gctcaagtca gaggtggcga aacccgacag 3060
   gactataaag ataccaggcg tttccccctg gaagctccct cgtgcgctct cctgttccga 3120
   ccctgccgct taccggatac ctgtccgcct ttctcccttc gggaagcgtg gcgctttctc 3180
   atagctcacg ctgtaggtat ctcagttcgg tgtaggtcgt tcgctccaag ctgggctgtg 3240
   tgcacgaacc ccccgttcag cccgaccgct gcgccttatc cggtaactat cgtcttgagt 3300
   ccaacccggt aagacacgac ttatcgccac tggcagcagc cactggtaac aggattagca 3360
   gagcgaggta tgtaggcggt gctacagagt tcttgaagtg gtggcctaac tacggctaca 3420
   ctagaaggac agtatttggt atctgcgctc tgctgaagcc agttaccttc ggaaaaagag 3480
   ttggtagctc ttgatccggc aaacaaacca ccgctggtag cggtggtttt tttgtttgca 3540
   agcagcagat tacgcgcaga aaaaaaggat ctcaagaaga tcctttgatc ttttctacgg 3600
   ggtctgacgc tcagtggaac gaaaactcac gttaagggat tttggtcatg agattatcaa 3660
   aaaggatctt cacctagatc cttttaaagg ccggccgcgg ccgcgcaaag tcccgcttcg 3720
   tgaaaatttt cgtgccgcgt gattttccgc caaaaacttt aacgaacgtt cgttataatg 3780
   gtgtcatgac cttcacgacg aagtactaaa attggcccga atcatcagct atggatctct 3840
   ctgatgtcgc gctggagtcc gacgcgctcg atgctgccgt cgatttaaaa acggtgatcg 3900
   gatttttccg agctctcgat acgacggacg cgccagcatc acgagactgg gccagtgccg 3960
   cgagcgacct agaaactctc gtggcggatc ttgaggagct ggctgacgag ctgcgtgctc 4020
   ggccagcgcc aggaggacgc acagtagtgg aggatgcaat cagttgcgcc tactgcggtg 4080
   gcctgattcc tccccggcct gacccgcgag gacggcgcgc aaaatattgc tcagatgcgt 4140
   gtcgtgccgc agccagccgc gagcgcgcca acaaacgcca cgccgaggag ctggaggcgg 4200
   ctaggtcgca aatggcgctg gaagtgcgtc cccgagcgaa aattttggcc atggtcgtca 4260
   cagagctgga agcggcagcg agaattatcg cgatcgtggc ggtgcccgca ggcatgacaa 4320
   acatcgtaaa tgccgcgttt cgtgtgccgt ggccgcccag gacgtgtcag cgccgccacc 4380
   acctgcaccg aatcggcagc agcgtcgcgc gtcgaaaaag cgcacaggcg gcaagaagcg 4440
   ataagctgca cgaatacctg aaaaatgttg aacgccccgt gagcggtaac tcacagggcg 4500
   tcggctaacc cccagtccaa acctgggaga aagcgctcaa aaatgactct agcggattca 4560
   cgagacattg acacaccggc ctggaaattt tccgctgatc tgttcgacac ccatcccgag 4620
   ctcgcgctgc gatcacgtgg ctggacgagc gaagaccgcc gcgaattcct cgctcacctg 4680
   ggcagagaaa atttccaggg cagcaagacc cgcgacttcg ccagcgcttg gatcaaagac 4740
   ccggacacgg agaaacacag ccgaagttat accgagttgg ttcaaaatcg cttgcccggt 4800
   gccagtatgt tgctctgacg cacgcgcagc acgcagccgt gcttgtcctg gacattgatg 4860
   tgccgagcca ccaggccggc gggaaaatcg agcacgtaaa ccccgaggtc tacgcgattt 4920
   tggagcgctg ggcacgcctg gaaaaagcgc cagcttggat cggcgtgaat ccactgagcg 4980
   ggaaatgcca gctcatctgg ctcattgatc cggtgtatgc cgcagcaggc atgagcagcc 5040
   cgaatatgcg cctgctggct gcaacgaccg aggaaatgac ccgcgttttc ggcgctgacc 5100
   aggctttttc acataggctg agccgtggcc actgcactct ccgacgatcc cagccgtacc 5160
   gctggcatgc ccagcacaat cgcgtggatc gcctagctga tcttatggag gttgctcgca 5220
   tgatctcagg cacagaaaaa cctaaaaaac gctatgagca ggagttttct agcggacggg 5280
   cacgtatcga agcggcaaga aaagccactg cggaagcaaa agcacttgcc acgcttgaag 5340
   caagcctgcc gagcgccgct gaagcgtctg gagagctgat cgacggcgtc cgtgtcctct 5400
   ggactgctcc agggcgtgcc gcccgtgatg agacggcttt tcgccacgct ttgactgtgg 5460
   gataccagtt aaaagcggct ggtgagcgcc taaaagacac caagggtcat cgagcctacg 5520
   agcgtgccta caccgtcgct caggcggtcg gaggaggccg tgagcctgat ctgccgccgg 5580
   actgtgaccg ccagacggat tggccgcgac gtgtgcgcgg ctacgtcgct aaaggccagc 5640
   cagtcgtccc tgctcgtcag acagagacgc agagccagcc gaggcgaaaa gctctggcca 5700
   ctatgggaag acgtggcggt aaaaaggccg cagaacgctg gaaagaccca aacagtgagt 5760
   acgcccgagc acagcgagaa aaactagcta agtccagtca acgacaagct aggaaagcta 5820
   aaggaaatcg cttgaccatt gcaggttggt ttatgactgt tgagggagag actggctcgt 5880
   ggccgacaat caatgaagct atgtctgaat ttagcgtgtc acgtcagacc gtgaatagag 5940
   cacttaaggt ctgcgggcat tgaacttcca cgaggacgcc gaaagcttcc cagtaaatgt 6000
   gccatctcgt aggcagaaaa cggttccccc gtagggtctc tctcttggcc tcctttctag 6060
   gtcgggctga ttgctcttga agctctctag gggggctcac accataggca gataacgttc 6120
   cccaccggct cgcctcgtaa gcgcacaagg actgctccca aagatcttca aagccactgc 6180
   cgcgactgcc ttcgcgaagc cttgccccgc ggaaatttcc tccaccgagt tcgtgcacac 6240
   ccctatgcca agcttctttc accctaaatt cgagagattg gattcttacc gtggaaattc 6300
   ttcgcaaaaa tcgtcccctg atcgcccttg cgacgttggc gtcggtgccg ctggttgcgc 6360
   ttggcttgac cgacttgatc agcggccgc 6389
<210> 37
   <211> 29
   <212> DNA
   <213> Artificial Sequence
<220>
   <223> Primer
   <220>
   <221> misc_feature
   <223> internal designation: BK 1753
<400> 37
   ggatctagag ttctgtgaaa aacaccgtg 29
<210> 38
   <211> 29
   <212> DNA
   <213> Artificial Sequence
<220>
   <223> Primer
   <220>
   <221> misc_feature
   <223> internal designation: BK 1754
<400> 38
   gcgactagtg ccccacaaat aaaaaacac 29
<210> 39
   <211> 5156
   <212> DNA
   <213> Artificial Sequence
<220>
   <223> Plasmid
   <220>
   <221> misc_feature
   <223> H247
<400> 39
   tcgatttaaa tctcgagagg cctgacgtcg ggcccggtac cacgcgtcat atgactagtt     60
   cggacctagg gatatcgtcg acatcgatgc tcttctgcgt taattaacaa ttgggatcct    120
   ctagagttct gtgaaaaaca ccgtggggca gtttctgctt cgcggtgttt tttatttgtg    180
   gggcactaga cccgggattt aaatcgctag cgggctgcta aaggaagcgg aacacgtaga    240
   aagccagtcc gcagaaacgg tgctgacccc ggatgaatgt cagctactgg gctatctgga    300
   caagggaaaa cgcaagcgca aagagaaagc aggtagcttg cagtgggctt acatggcgat    360
   agctagactg ggcggtttta tggacagcaa gcgaaccgga attgccagct ggggcgccct    420
   ctggtaaggt tgggaagccc tgcaaagtaa actggatggc tttcttgccg ccaaggatct    480
   gatggcgcag gggatcaaga tctgatcaag agacaggatg aggatcgttt cgcatgattg    540
   aacaagatgg attgcacgca ggttctccgg ccgcttgggt ggagaggcta ttcggctatg    600
   actgggcaca acagacaatc ggctgctctg atgccgccgt gttccggctg tcagcgcagg    660
   ggcgcccggt tctttttgtc aagaccgacc tgtccggtgc cctgaatgaa ctgcaggacg    720
   aggcagcgcg gctatcgtgg ctggccacga cgggcgttcc ttgcgcagct gtgctcgacg    780
   ttgtcactga agcgggaagg gactggctgc tattgggcga agtgccgggg caggatctcc    840
   tgtcatctca ccttgctcct gccgagaaag tatccatcat ggctgatgca atgcggcggc    900
   tgcatacgct tgatccggct acctgcccat tcgaccacca agcgaaacat cgcatcgagc    960
   gagcacgtac tcggatggaa gccggtcttg tcgatcagga tgatctggac gaagagcatc   1020
   aggggctcgc gccagccgaa ctgttcgcca ggctcaaggc gcgcatgccc gacggcgagg   1080
   atctcgtcgt gacccatggc gatgcctgct tgccgaatat catggtggaa aatggccgct   1140
   tttctggatt catcgactgt ggccggctgg gtgtggcgga ccgctatcag gacatagcgt   1200
   tggctacccg tgatattgct gaagagcttg gcggcgaatg ggctgaccgc ttcctcgtgc   1260
   tttacggtat cgccgctccc gattcgcagc gcatcgcctt ctatcgcctt cttgacgagt   1320
   tcttctgagc gggactctgg ggttcgaaat gaccgaccaa gcgacgccca acctgccatc   1380
   acgagatttc gattccaccg ccgccttcta tgaaaggttg ggcttcggaa tcgttttccg   1440
   ggacgccggc tggatgatcc tccagcgcgg ggatctcatg ctggagttct tcgcccacgc   1500
   tagcggcgcg ccggccggcc cggtgtgaaa taccgcacag atgcgtaagg agaaaatacc   1560
   gcatcaggcg ctcttccgct tcctcgctca ctgactcgct gcgctcggtc gttcggctgc   1620
   ggcgagcggt atcagctcac tcaaaggcgg taatacggtt atccacagaa tcaggggata   1680
   acgcaggaaa gaacatgtga gcaaaaggcc agcaaaaggc caggaaccgt aaaaaggccg   1740
   cgttgctggc gtttttccat aggctccgcc cccctgacga gcatcacaaa aatcgacgct   1800
   caagtcagag gtggcgaaac ccgacaggac tataaagata ccaggcgttt ccccctggaa   1860
   gctccctcgt gcgctctcct gttccgaccc tgccgcttac cggatacctg tccgcctttc   1920
   tcccttcggg aagcgtggcg ctttctcata gctcacgctg taggtatctc agttcggtgt   1980
   aggtcgttcg ctccaagctg ggctgtgtgc acgaaccccc cgttcagccc gaccgctgcg   2040
   ccttatccgg taactatcgt cttgagtcca acccggtaag acacgactta tcgccactgg   2100
   cagcagccac tggtaacagg attagcagag cgaggtatgt aggcggtgct acagagttct   2160
   tgaagtggtg gcctaactac ggctacacta gaaggacagt atttggtatc tgcgctctgc 2220
   tgaagccagt taccttcgga aaaagagttg gtagctcttg atccggcaaa caaaccaccg 2280
   ctggtagcgg tggttttttt gtttgcaagc agcagattac gcgcagaaaa aaaggatctc 2340
   aagaagatcc tttgatcttt tctacggggt ctgacgctca gtggaacgaa aactcacgtt 2400
   aagggatttt ggtcatgaga ttatcaaaaa ggatcttcac ctagatcctt ttaaaggccg 2460
   gccgcggccg cgcaaagtcc cgcttcgtga aaattttcgt gccgcgtgat tttccgccaa 2520
   aaactttaac gaacgttcgt tataatggtg tcatgacctt cacgacgaag tactaaaatt 2580
   ggcccgaatc atcagctatg gatctctctg atgtcgcgct ggagtccgac gcgctcgatg 2640
   ctgccgtcga tttaaaaacg gtgatcggat ttttccgagc tctcgatacg acggacgcgc 2700
   cagcatcacg agactgggcc agtgccgcga gcgacctaga aactctcgtg gcggatcttg 2760
   aggagctggc tgacgagctg cgtgctcggc cagcgccagg aggacgcaca gtagtggagg 2820
   atgcaatcag ttgcgcctac tgcggtggcc tgattcctcc ccggcctgac ccgcgaggac 2880
   ggcgcgcaaa atattgctca gatgcgtgtc gtgccgcagc cagccgcgag cgcgccaaca 2940
   aacgccacgc cgaggagctg gaggcggcta ggtcgcaaat ggcgctggaa gtgcgtcccc 3000
   cgagcgaaat tttggccatg gtcgtcacag agctggaagc ggcagcgaga attatcgcga 3060
   tcgtggcggt gcccgcaggc atgacaaaca tcgtaaatgc cgcgtttcgt gtgccgtggc 3120
   cgcccaggac gtgtcagcgc cgccaccacc tgcaccgaat cggcagcagc gtcgcgcgtc 3180
   gaaaaagcgc acaggcggca agaagcgata agctgcacga atacctgaaa aatgttgaac 3240
   gccccgtgag cggtaactca cagggcgtcg gctaaccccc agtccaaacc tgggagaaag 3300
   cgctcaaaaa tgactctagc ggattcacga gacattgaca caccggcctg gaaattttcc 3360
   gctgatctgt tcgacaccca tcccgagctc gcgctgcgat cacgtggctg gacgagcgaa 3420
   gaccgccgcg aattcctcgc tcacctgggc agagaaaatt tccagggcag caagacccgc 3480
   gacttcgcca gcgcttggat caaagacccg gacacggaga aacacagccg aagttatacc 3540
   gagttggttc aaaatcgctt gcccggtgcc agtatgttgc tctgacgcac gcgcagcacg 3600
   cagccgtgct tgtcctggac attgatgtgc cgagccacca ggccggcggg aaaatcgagc 3660
   acgtaaaccc cgaggtctac gcgattttgg agcgctgggc acgcctggaa aaagcgccag 3720
   cttggatcgg cgtgaatcca ctgagcggga aatgccagct catctggctc attgatccgg 3780
   tgtatgccgc agcaggcatg agcagcccga atatgcgcct gctggctgca acgaccgagg 3840
   aaatgacccg cgttttcggc gctgaccagg ctttttcaca taggctgagc cgtggccact 3900
   gcactctccg acgatcccag ccgtaccgct ggcatgccca gcacaatcgc gtggatcgcc 3960
   tagctgatct tatggaggtt gctcgcatga tctcaggcac agaaaaacct aaaaaacgct 4020
   atgagcagga gttttctagc ggacgggcac gtatcgaagc ggcaagaaaa gccactgcgg 4080
   aagcaaaagc acttgccacg cttgaagcaa gcctgccgag cgccgctgaa gcgtctggag 4140
   agctgatcga cggcgtccgt gtcctctgga ctgctccagg gcgtgccgcc cgtgatgaga 4200
   cggcttttcg ccacgctttg actgtgggat accagttaaa agcggctggt gagcgcctaa 4260
   aagacaccaa gggtcatcga gcctacgagc gtgcctacac cgtcgctcag gcggtcggag 4320
   gaggccgtga gcctgatctg ccgccggact gtgaccgcca gacggattgg ccgcgacgtg 4380
   tgcgcggcta cgtcgctaaa ggccagccag tcgtccctgc tcgtcagaca gagacgcaga 4440
   gccagccgag gcgaaaagct ctggccacta tgggaagacg tggcggtaaa aaggccgcag 4500
   aacgctggaa agacccaaac agtgagtacg cccgagcaca gcgagaaaaa ctagctaagt 4560
   ccagtcaacg acaagctagg aaagctaaag gaaatcgctt gaccattgca ggttggttta 4620
   tgactgttga gggagagact ggctcgtggc cgacaatcaa tgaagctatg tctgaattta 4680
   gcgtgtcacg tcagaccgtg aatagagcac ttaaggtctg cgggcattga acttccacga 4740
   ggacgccgaa agcttcccag taaatgtgcc atctcgtagg cagaaaacgg ttcccccgta 4800
   gggtctctct cttggcctcc tttctaggtc gggctgattg ctcttgaagc tctctagggg 4860
   ggctcacacc ataggcagat aacgttcccc accggctcgc ctcgtaagcg cacaaggact 4920
   gctcccaaag atcttcaaag ccactgccgc gactgccttc gcgaagcctt gccccgcgga 4980
   aatttcctcc accgagttcg tgcacacccc tatgccaagc ttctttcacc ctaaattcga 5040
   gagattggat tcttaccgtg gaaattcttc gcaaaaatcg tcccctgatc gcccttgcga 5100
   cgttggcgtc ggtgccgctg gttgcgcttg gcttgaccga cttgatcagc ggccgc 5156
<210> 40
   <211> 29
   <212> DNA
   <213> Artificial Sequence
<220>
   <223> Primer
   <220>
   <221> misc_feature
   <223> internal designation: BK 1848
<400> 40
   gagaactagt agctgccaat tattccggg 29
<210> 41
   <211> 32
   <212> DNA
   <213> Artificial Sequence
<220>
   <223> Primer
   <220>
   <221> misc_feature
   <223> internal designation: BK 1849
<400> 41
   gtgtgtcgac ttagatgtag aactcgatgt ag 32
<210> 42
   <211> 6464
   <212> DNA
   <213> Artificial Sequence
<220>
   <223> Plasmid
   <220>
   <221> misc_feature
   <223> internal designation: pG A4 (H344)
<400> 42
   tcgatttaaa tctcgagagg cctgacgtcg ggcccggtac cacgcgtcat atgactagta     60
   gctgccaatt attccgggct tgtgacccgc tacccgataa ataggtcggc tgaaaaattt    120
   cgttgcaata tcaacaaaaa ggcctatcat tgggaggtgt cgcaccaagt acttttgcga    180
   agcgccatct gacggatttt caaaagatgt atatgctcgg tgcggaaacc tacgaaagga    240
   ttttttaccc atgcccaccc tcgcgccttc aggtcaactt gaaatccaag cgatcggtga    300
   tgtctccacc gaagccggag caatcattac aaacgctgaa atcgcctatc accgctgggg    360
   tgaataccgc gtagataaag aaggacgcag caatgtcgtt ctcatcgaac acgccctcac    420
   tggagattcc aacgcagccg attggtgggc tgacttgctc ggtcccggca aagccatcaa    480
   cactgatatt tactgcgtga tctgtaccaa cgtcatcggt ggttgcaacg gttccaccgg    540
   acctggctcc atgcatccag atggaaattt ctggggtaat cgcttccccg ccacgtccat    600
   tcgtgatcag gtaaacgccg aaaaacaatt cctcgacgca ctcggcatca ccacggtcgc    660
   cgcagtactt ggtggttcca tgggtggtgc ccgcacccta gagtgggccg caatgtaccc    720
   agaaactgtt ggcgcagctg ctgttcttgc agtttctgca cgcgccagcg cctggcaaat    780
   cggcattcaa tccgcccaaa ttaaggcgat tgaaaacgac caccactggc acgaaggcaa    840
   ctactacgaa tccggctgca acccagccac cggactcggc gccgcccgac gcatcgccca    900
   cctcacctac cgtggcgaac tagaaatcga cgaacgcttc ggcaccaaag cccaaaagaa    960
   cgaaaaccca ctcggtccct accgcaagcc cgaccagcgc ttcgccgtgg aatcctactt   1020
   ggactaccaa gcagacaagc tagtacagcg tttcgacgcc ggctcctacg tcttgctcac   1080
   cgacgccctc aaccgccacg acattggtcg cgaccgcgga ggcctcaaca aggcactcga   1140
   atccatcaaa gttccagtcc ttgtcgcagg cgtagatacc gatattttgt acccctacca   1200
   ccagcaagaa cacctctcca gaaacctggg aaatctactg gcaatggcaa aaatcgtatc   1260
   ccctgtcggc cacgatgctt tcctcaccga aagccgccaa atggatcgca tcgtgaggaa   1320
   cttcttcagc ctcatctccc cagacgaaga caacccttcg acctacatcg agttctacat   1380
   ctaagtcgac atcgatgctc ttctgcgtta attaacaatt gggatcctct agagttctgt   1440
   gaaaaacacc gtggggcagt ttctgcttcg cggtgttttt tatttgtggg gcactagacc   1500
   cgggatttaa atcgctagcg ggctgctaaa ggaagcggaa cacgtagaaa gccagtccgc   1560
   agaaacggtg ctgaccccgg atgaatgtca gctactgggc tatctggaca agggaaaacg   1620
   caagcgcaaa gagaaagcag gtagcttgca gtgggcttac atggcgatag ctagactggg 1680
   cggttttatg gacagcaagc gaaccggaat tgccagctgg ggcgccctct ggtaaggttg 1740
   ggaagccctg caaagtaaac tggatggctt tcttgccgcc aaggatctga tggcgcaggg 1800
   gatcaagatc tgatcaagag acaggatgag gatcgtttcg catgattgaa caagatggat 1860
   tgcacgcagg ttctccggcc gcttgggtgg agaggctatt cggctatgac tgggcacaac 1920
   agacaatcgg ctgctctgat gccgccgtgt tccggctgtc agcgcagggg cgcccggttc 1980
   tttttgtcaa gaccgacctg tccggtgccc tgaatgaact gcaggacgag gcagcgcggc 2040
   tatcgtggct ggccacgacg ggcgttcctt gcgcagctgt gctcgacgtt gtcactgaag 2100
   cgggaaggga ctggctgcta ttgggcgaag tgccggggca ggatctcctg tcatctcacc 2160
   ttgctcctgc cgagaaagta tccatcatgg ctgatgcaat gcggcggctg catacgcttg 2220
   atccggctac ctgcccattc gaccaccaag cgaaacatcg catcgagcga gcacgtactc 2280
   ggatggaagc cggtcttgtc gatcaggatg atctggacga agagcatcag gggctcgcgc 2340
   cagccgaact gttcgccagg ctcaaggcgc gcatgcccga cggcgaggat ctcgtcgtga 2400
   cccatggcga tgcctgcttg ccgaatatca tggtggaaaa tggccgcttt tctggattca 2460
   tcgactgtgg ccggctgggt gtggcggacc gctatcagga catagcgttg gctacccgtg 2520
   atattgctga agagcttggc ggcgaatggg ctgaccgctt cctcgtgctt tacggtatcg 2580
   ccgctcccga ttcgcagcgc atcgccttct atcgccttct tgacgagttc ttctgagcgg 2640
   gactctgggg ttcgaaatga ccgaccaagc gacgcccaac ctgccatcac gagatttcga 2700
   ttccaccgcc gccttctatg aaaggttggg cttcggaatc gttttccggg acgccggctg 2760
   gatgatcctc cagcgcgggg atctcatgct ggagttcttc gcccacgcta gcggcgcgcc 2820
   ggccggcccg gtgtgaaata ccgcacagat gcgtaaggag aaaataccgc atcaggcgct 2880
   cttccgcttc ctcgctcact gactcgctgc gctcggtcgt tcggctgcgg cgagcggtat 2940
   cagctcactc aaaggcggta atacggttat ccacagaatc aggggataac gcaggaaaga 3000
   acatgtgagc aaaaggccag caaaaggcca ggaaccgtaa aaaggccgcg ttgctggcgt 3060
   ttttccatag gctccgcccc cctgacgagc atcacaaaaa tcgacgctca agtcagaggt 3120
   ggcgaaaccc gacaggacta taaagatacc aggcgtttcc ccctggaagc tccctcgtgc 3180
   gctctcctgt tccgaccctg ccgcttaccg gatacctgtc cgcctttctc ccttcgggaa 3240
   gcgtggcgct ttctcatagc tcacgctgta ggtatctcag ttcggtgtag gtcgttcgct 3300
   ccaagctggg ctgtgtgcac gaaccccccg ttcagcccga ccgctgcgcc ttatccggta 3360
   actatcgtct tgagtccaac ccggtaagac acgacttatc gccactggca gcagccactg 3420
   gtaacaggat tagcagagcg aggtatgtag gcggtgctac agagttcttg aagtggtggc 3480
   ctaactacgg ctacactaga aggacagtat ttggtatctg cgctctgctg aagccagtta 3540
   ccttcggaaa aagagttggt agctcttgat ccggcaaaca aaccaccgct ggtagcggtg 3600
   gtttttttgt ttgcaagcag cagattacgc gcagaaaaaa aggatctcaa gaagatcctt 3660
   tgatcttttc tacggggtct gacgctcagt ggaacgaaaa ctcacgttaa gggattttgg 3720
   tcatgagatt atcaaaaagg atcttcacct agatcctttt aaaggccggc cgcggccgcg 3780
   caaagtcccg cttcgtgaaa attttcgtgc cgcgtgattt tccgccaaaa actttaacga 3840
   acgttcgtta taatggtgtc atgaccttca cgacgaagta ctaaaattgg cccgaatcat 3900
   cagctatgga tctctctgat gtcgcgctgg agtccgacgc gctcgatgct gccgtcgatt 3960
   taaaaacggt gatcggattt ttccgagctc tcgatacgac ggacgcgcca gcatcacgag 4020
   actgggccag tgccgcgagc gacctagaaa ctctcgtggc ggatcttgag gagctggctg 4080
   acgagctgcg tgctcggcca gcgccaggag gacgcacagt agtggaggat gcaatcagtt 4140
   gcgcctactg cggtggcctg attcctcccc ggcctgaccc gcgaggacgg cgcgcaaaat 4200
   attgctcaga tgcgtgtcgt gccgcagcca gccgcgagcg cgccaacaaa cgccacgccg 4260
   aggagctgga ggcggctagg tcgcaaatgg cgctggaagt gcgtcccccg agcgaaattt 4320
   tggccatggt cgtcacagag ctggaagcgg cagcgagaat tatcgcgatc gtggcggtgc 4380
   ccgcaggcat gacaaacatc gtaaatgccg cgtttcgtgt gccgtggccg cccaggacgt 4440
   gtcagcgccg ccaccacctg caccgaatcg gcagcagcgt cgcgcgtcga aaaagcgcac 4500
   aggcggcaag aagcgataag ctgcacgaat acctgaaaaa tgttgaacgc cccgtgagcg 4560
   gtaactcaca gggcgtcggc taacccccag tccaaacctg ggagaaagcg ctcaaaaatg 4620
   actctagcgg attcacgaga cattgacaca ccggcctgga aattttccgc tgatctgttc 4680
   gacacccatc ccgagctcgc gctgcgatca cgtggctgga cgagcgaaga ccgccgcgaa 4740
   ttcctcgctc acctgggcag agaaaatttc cagggcagca agacccgcga cttcgccagc 4800
   gcttggatca aagacccgga cacggagaaa cacagccgaa gttataccga gttggttcaa 4860
   aatcgcttgc ccggtgccag tatgttgctc tgacgcacgc gcagcacgca gccgtgcttg 4920
   tcctggacat tgatgtgccg agccaccagg ccggcgggaa aatcgagcac gtaaaccccg 4980
   aggtctacgc gattttggag cgctgggcac gcctggaaaa agcgccagct tggatcggcg 5040
   tgaatccact gagcgggaaa tgccagctca tctggctcat tgatccggtg tatgccgcag 5100
   caggcatgag cagcccgaat atgcgcctgc tggctgcaac gaccgaggaa atgacccgcg 5160
   ttttcggcgc tgaccaggct ttttcacata ggctgagccg tggccactgc actctccgac 5220
   gatcccagcc gtaccgctgg catgcccagc acaatcgcgt ggatcgccta gctgatctta 5280
   tggaggttgc tcgcatgatc tcaggcacag aaaaacctaa aaaacgctat gagcaggagt 5340
   tttctagcgg acgggcacgt atcgaagcgg caagaaaagc cactgcggaa gcaaaagcac 5400
   ttgccacgct tgaagcaagc ctgccgagcg ccgctgaagc gtctggagag ctgatcgacg 5460
   gcgtccgtgt cctctggact gctccagggc gtgccgcccg tgatgagacg gcttttcgcc 5520
   acgctttgac tgtgggatac cagttaaaag cggctggtga gcgcctaaaa gacaccaagg 5580
   gtcatcgagc ctacgagcgt gcctacaccg tcgctcaggc ggtcggagga ggccgtgagc 5640
   ctgatctgcc gccggactgt gaccgccaga cggattggcc gcgacgtgtg cgcggctacg 5700
   tcgctaaagg ccagccagtc gtccctgctc gtcagacaga gacgcagagc cagccgaggc 5760
   gaaaagctct ggccactatg ggaagacgtg gcggtaaaaa ggccgcagaa cgctggaaag 5820
   acccaaacag tgagtacgcc cgagcacagc gagaaaaact agctaagtcc agtcaacgac 5880
   aagctaggaa agctaaagga aatcgcttga ccattgcagg ttggtttatg actgttgagg 5940
   gagagactgg ctcgtggccg acaatcaatg aagctatgtc tgaatttagc gtgtcacgtc 6000
   agaccgtgaa tagagcactt aaggtctgcg ggcattgaac ttccacgagg acgccgaaag 6060
   cttcccagta aatgtgccat ctcgtaggca gaaaacggtt cccccgtagg gtctctctct 6120
   tggcctcctt tctaggtcgg gctgattgct cttgaagctc tctagggggg ctcacaccat 6180
   aggcagataa cgttccccac cggctcgcct cgtaagcgca caaggactgc tcccaaagat 6240
   cttcaaagcc actgccgcga ctgccttcgc gaagccttgc cccgcggaaa tttcctccac 6300
   cgagttcgtg cacaccccta tgccaagctt ctttcaccct aaattcgaga gattggattc 6360
   ttaccgtgga aattcttcgc aaaaatcgtc ccctgatcgc ccttgcgacg ttggcgtcgg 6420
   tgccgctggt tgcgcttggc ttgaccgact tgatcagcgg ccgc 6464
<210> 43
   <211> 29
   <212> DNA
   <213> Artificial sequence
<220>
   <223> Primer
   <220>
   <221> misc_feature
   <223> internal designation: BK 1695
<400> 43
   gagactcgag ggccgttacc ctgcgaatg 29
<210> 44
   <211> 30
   <212> DNA
   <213> Artificial Sequence
<220>
   <223> Primer
   <220>
   <221> misc_feature
   <223> internal designation: Haf16
<400> 44
   gagaactagt gtggctacga ctttcgcagc 30
<210> 45
   <211> 6604
   <212> DNA
   <213> Artificial sequence
<220>
   <223> Plasmid
   <220>
   <221> misc_feature
   <223> internal designation: pCliK5MCS PeftuPsod metA (H473)
<400> 45
   tcgatttaaa tctcgagggc cgttaccctg cgaatgtcca cagggtagct ggtagtttga 60
   aaatcaacgc cgttgccctt aggattcagt aactggcaca ttttgtaatg cgctagatct 120
   gtgtgctcag tcttccaggc tgcttatcac agtgaaagca aaaccaattc gtggctgcga 180
   aagtcgtagc cacactagta gctgccaatt attccgggct tgtgacccgc tacccgataa 240
   ataggtcggc tgaaaaattt cgttgcaata tcaacaaaaa ggcctatcat tgggaggtgt 300
   cgcaccaagt acttttgcga agcgccatct gacggatttt caaaagatgt atatgctcgg 360
   tgcggaaacc tacgaaagga ttttttaccc atgcccaccc tcgcgccttc aggtcaactt 420
   gaaatccaag cgatcggtga tgtctccacc gaagccggag caatcattac aaacgctgaa 480
   atcgcctatc accgctgggg tgaataccgc gtagataaag aaggacgcag caatgtcgtt 540
   ctcatcgaac acgccctcac tggagattcc aacgcagccg attggtgggc tgacttgctc 600
   ggtcccggca aagccatcaa cactgatatt tactgcgtga tctgtaccaa cgtcatcggt 660
   ggttgcaacg gttccaccgg acctggctcc atgcatccag atggaaattt ctggggtaat 720
   cgcttccccg ccacgtccat tcgtgatcag gtaaacgccg aaaaacaatt cctcgacgca 780
   ctcggcatca ccacggtcgc cgcagtactt ggtggttcca tgggtggtgc ccgcacccta 840
   gagtgggccg caatgtaccc agaaactgtt ggcgcagctg ctgttcttgc agtttctgca 900
   cgcgccagcg cctggcaaat cggcattcaa tccgcccaaa ttaaggcgat tgaaaacgac 960
   caccactggc acgaaggcaa ctactacgaa tccggctgca acccagccac cggactcggc 1020
   gccgcccgac gcatcgccca cctcacctac cgtggcgaac tagaaatcga cgaacgcttc 1080
   ggcaccaaag cccaaaagaa cgaaaaccca ctcggtccct accgcaagcc cgaccagcgc 1140
   ttcgccgtgg aatcctactt ggactaccaa gcagacaagc tagtacagcg tttcgacgcc 1200
   ggctcctacg tcttgctcac cgacgccctc aaccgccacg acattggtcg cgaccgcgga 1260
   ggcctcaaca aggcactcga atccatcaaa gttccagtcc ttgtcgcagg cgtagatacc 1320
   gatattttgt acccctacca ccagcaagaa cacctctcca gaaacctggg aaatctactg 1380
   gcaatggcaa aaatcgtatc ccctgtcggc cacgatgctt tcctcaccga aagccgccaa 1440
   atggatcgca tcgtgaggaa cttcttcagc ctcatctccc cagacgaaga caacccttcg 1500
   acctacatcg agttctacat ctaagtcgac atcgatgctc ttctgcgtta attaacaatt 1560
   gggatcctct agagttctgt gaaaaacacc gtggggcagt ttctgcttcg cggtgttttt 1620
   tatttgtggg gcactagacc cgggatttaa atcgctagcg ggctgctaaa ggaagcggaa 1680
   cacgtagaaa gccagtccgc agaaacggtg ctgaccccgg atgaatgtca gctactgggc 1740
   tatctggaca agggaaaacg caagcgcaaa gagaaagcag gtagcttgca gtgggcttac 1800
   atggcgatag ctagactggg cggttttatg gacagcaagc gaaccggaat tgccagctgg 1860
   ggcgccctct ggtaaggttg ggaagccctg caaagtaaac tggatggctt tcttgccgcc 1920
   aaggatctga tggcgcaggg gatcaagatc tgatcaagag acaggatgag gatcgtttcg 1980
   catgattgaa caagatggat tgcacgcagg ttctccggcc gcttgggtgg agaggctatt 2040
   cggctatgac tgggcacaac agacaatcgg ctgctctgat gccgccgtgt tccggctgtc 2100
   agcgcagggg cgcccggttc tttttgtcaa gaccgacctg tccggtgccc tgaatgaact 2160
   gcaggacgag gcagcgcggc tatcgtggct ggccacgacg ggcgttcctt gcgcagctgt 2220
   gctcgacgtt gtcactgaag cgggaaggga ctggctgcta ttgggcgaag tgccggggca 2280
   ggatctcctg tcatctcacc ttgctcctgc cgagaaagta tccatcatgg ctgatgcaat 2340
   gcggcggctg catacgcttg atccggctac ctgcccattc gaccaccaag cgaaacatcg 2400
   catcgagcga gcacgtactc ggatggaagc cggtcttgtc gatcaggatg atctggacga 2460
   agagcatcag gggctcgcgc cagccgaact gttcgccagg ctcaaggcgc gcatgcccga 2520
   cggcgaggat ctcgtcgtga cccatggcga tgcctgcttg ccgaatatca tggtggaaaa 2580
   tggccgcttt tctggattca tcgactgtgg ccggctgggt gtggcggacc gctatcagga 2640
   catagcgttg gctacccgtg atattgctga agagcttggc ggcgaatggg ctgaccgctt 2700
   cctcgtgctt tacggtatcg ccgctcccga ttcgcagcgc atcgccttct atcgccttct 2760
   tgacgagttc ttctgagcgg gactctgggg ttcgaaatga ccgaccaagc gacgcccaac 2820
   ctgccatcac gagatttcga ttccaccgcc gccttctatg aaaggttggg cttcggaatc 2880
   gttttccggg acgccggctg gatgatcctc cagcgcgggg atctcatgct ggagttcttc 2940
   gcccacgcta gcggcgcgcc ggccggcccg gtgtgaaata ccgcacagat gcgtaaggag 3000
   aaaataccgc atcaggcgct cttccgcttc ctcgctcact gactcgctgc gctcggtcgt 3060
   tcggctgcgg cgagcggtat cagctcactc aaaggcggta atacggttat ccacagaatc 3120
   aggggataac gcaggaaaga acatgtgagc aaaaggccag caaaaggcca ggaaccgtaa 3180
   aaaggccgcg ttgctggcgt ttttccatag gctccgcccc cctgacgagc atcacaaaaa 3240
   tcgacgctca agtcagaggt ggcgaaaccc gacaggacta taaagatacc aggcgtttcc 3300
   ccctggaagc tccctcgtgc gctctcctgt tccgaccctg ccgcttaccg gatacctgtc 3360
   cgcctttctc ccttcgggaa gcgtggcgct ttctcatagc tcacgctgta ggtatctcag 3420
   ttcggtgtag gtcgttcgct ccaagctggg ctgtgtgcac gaaccccccg ttcagcccga 3480
   ccgctgcgcc ttatccggta actatcgtct tgagtccaac ccggtaagac acgacttatc 3540
   gccactggca gcagccactg gtaacaggat tagcagagcg aggtatgtag gcggtgctac 3600
   agagttcttg aagtggtggc ctaactacgg ctacactaga aggacagtat ttggtatctg 3660
   cgctctgctg aagccagtta ccttcggaaa aagagttggt agctcttgat ccggcaaaca 3720
   aaccaccgct ggtagcggtg gtttttttgt ttgcaagcag cagattacgc gcagaaaaaa 3780
   aggatctcaa gaagatcctt tgatcttttc tacggggtct gacgctcagt ggaacgaaaa 3840
   ctcacgttaa gggattttgg tcatgagatt atcaaaaagg atcttcacct agatcctttt 3900
   aaaggccggc cgcggccgcg caaagtcccg cttcgtgaaa attttcgtgc cgcgtgattt 3960
   tccgccaaaa actttaacga acgttcgtta taatggtgtc atgaccttca cgacgaagta 4020
   ctaaaattgg cccgaatcat cagctatgga tctctctgat gtcgcgctgg agtccgacgc 4080
   gctcgatgct gccgtcgatt taaaaacggt gatcggattt ttccgagctc tcgatacgac 4140
   ggacgcgcca gcatcacgag actgggccag tgccgcgagc gacctagaaa ctctcgtggc 4200
   ggatcttgag gagctggctg acgagctgcg tgctcggcca gcgccaggag gacgcacagt 4260
   agtggaggat gcaatcagtt gcgcctactg cggtggcctg attcctcccc ggcctgaccc 4320
   gcgaggacgg cgcgcaaaat attgctcaga tgcgtgtcgt gccgcagcca gccgcgagcg 4380
   cgccaacaaa cgccacgccg aggagctgga ggcggctagg tcgcaaatgg cgctggaagt 4440
   gcgtcccccg agcgaaattt tggccatggt cgtcacagag ctggaagcgg cagcgagaat 4500
   tatcgcgatc gtggcggtgc ccgcaggcat gacaaacatc gtaaatgccg cgtttcgtgt 4560
   gccgtggccg cccaggacgt gtcagcgccg ccaccacctg caccgaatcg gcagcagcgt 4620
   cgcgcgtcga aaaagcgcac aggcggcaag aagcgataag ctgcacgaat acctgaaaaa 4680
   tgttgaacgc cccgtgagcg gtaactcaca gggcgtcggc taacccccag tccaaacctg 4740
   ggagaaagcg ctcaaaaatg actctagcgg attcacgaga cattgacaca ccggcctgga 4800
   aattttccgc tgatctgttc gacacccatc ccgagctcgc gctgcgatca cgtggctgga 4860
   cgagcgaaga ccgccgcgaa ttcctcgctc acctgggcag agaaaatttc cagggcagca 4920
   agacccgcga cttcgccagc gcttggatca aagacccgga cacggagaaa cacagccgaa 4980
   gttataccga gttggttcaa aatcgcttgc ccggtgccag tatgttgctc tgacgcacgc 5040
   gcagcacgca gccgtgcttg tcctggacat tgatgtgccg agccaccagg ccggcgggaa 5100
   aatcgagcac gtaaaccccg aggtctacgc gattttggag cgctgggcac gcctggaaaa 5160
   agcgccagct tggatcggcg tgaatccact gagcgggaaa tgccagctca tctggctcat 5220
   tgatccggtg tatgccgcag caggcatgag cagcccgaat atgcgcctgc tggctgcaac 5280
   gaccgaggaa atgacccgcg ttttcggcgc tgaccaggct ttttcacata ggctgagccg 5340
   tggccactgc actctccgac gatcccagcc gtaccgctgg catgcccagc acaatcgcgt 5400
   ggatcgccta gctgatctta tggaggttgc tcgcatgatc tcaggcacag aaaaacctaa 5460
   aaaacgctat gagcaggagt tttctagcgg acgggcacgt atcgaagcgg caagaaaagc 5520
   cactgcggaa gcaaaagcac ttgccacgct tgaagcaagc ctgccgagcg ccgctgaagc 5580
   gtctggagag ctgatcgacg gcgtccgtgt cctctggact gctccagggc gtgccgcccg 5640
   tgatgagacg gcttttcgcc acgctttgac tgtgggatac cagttaaaag cggctggtga 5700
   gcgcctaaaa gacaccaagg gtcatcgagc ctacgagcgt gcctacaccg tcgctcaggc 5760
   ggtcggagga ggccgtgagc ctgatctgcc gccggactgt gaccgccaga cggattggcc 5820
   gcgacgtgtg cgcggctacg tcgctaaagg ccagccagtc gtccctgctc gtcagacaga 5880
   gacgcagagc cagccgaggc gaaaagctct ggccactatg ggaagacgtg gcggtaaaaa 5940
   ggccgcagaa cgctggaaag acccaaacag tgagtacgcc cgagcacagc gagaaaaact 6000
   agctaagtcc agtcaacgac aagctaggaa agctaaagga aatcgcttga ccattgcagg 6060
   ttggtttatg actgttgagg gagagactgg ctcgtggccg acaatcaatg aagctatgtc 6120
   tgaatttagc gtgtcacgtc agaccgtgaa tagagcactt aaggtctgcg ggcattgaac 6180
   ttccacgagg acgccgaaag cttcccagta aatgtgccat ctcgtaggca gaaaacggtt 6240
   cccccgtagg gtctctctct tggcctcctt tctaggtcgg gctgattgct cttgaagctc 6300
   tctagggggg ctcacaccat aggcagataa cgttccccac cggctcgcct cgtaagcgca 6360
   caaggactgc tcccaaagat cttcaaagcc actgccgcga ctgccttcgc gaagccttgc 6420
   cccgcggaaa tttcctccac cgagttcgtg cacaccccta tgccaagctt ctttcaccct 6480
   aaattcgaga gattggattc ttaccgtgga aattcttcgc aaaaatcgtc ccctgatcgc 6540
   ccttgcgacg ttggcgtcgg tgccgctggt tgcgcttggc ttgaccgact tgatcagcgg 6600
   ccgc 6604
<210> 46
   <211> 29
   <212> DNA
   <213> Artificial Sequence
<220>
   <223> Primer
   <220>
   <221> misc_feature
   <223> internal designation: Haf64
<400> 46
   gagaactagt ggccgttacc ctgcgaatg 29
<210> 47
   <211> 36
   <212> DNA
   <213> Artificial Sequence
<220>
   <223> Primer
   <220>
   <221> misc_feature
   <223> internal designation: Haf65
<400> 47
   gcgcgagggt gggcattgta tgtcctcctg gacttc 36
<210> 48
   <211> 17
   <212> DNA
   <213> Artificial Sequence
<220>
   <223> Primer
   <220>
   <221> misc_feature
   <223> internal designation: BK1862)
<400> 48
   atgcccaccc tcgcgcc 17
<210> 49
   <211> 32
   <212> DNA
   <213> Artificial Sequence
<220>
   <223> Primer
   <220>
   <221> misc_feature
   <223> internal designation: BK1849)
<400> 49
   gtgtgtcgac ttagatgtag aactcgatgt ag 32
<210> 50
   <211> 29
   <212> DNA
   <213> Artificial Sequence
<220>
   <223> Primer
   <220>
   <221> misc_feature
   <223> internal designation: BK 1697
<400> 50
   gagactcgag agctgccaat tattccggg 29
<210> 51
   <211> 28
   <212> DNA
   <213> Artificial Sequence
<220>
   <223> Primer
   <220>
   <221> misc_feature
   <223> internal designation: Haf17)
<400> 51
   gagaactagt taggtttccg caccgagc 28
<210> 52
   <211> 6609
   <212> DNA
   <213> Artificial Sequence
<220>
   <223> Plasmid
   <220>
   <221> misc_feature
   <223> internal designation: pCLiK5MCS Psod Peft metA (H505)
<400> 52
   tcgatttaaa tctcgagagc tgccaattat tccgggcttg tgacccgcta cccgataaat     60
   aggtcggctg aaaaatttcg ttgcaatatc aacaaaaagg cctatcattg ggaggtgtcg    120
   caccaagtac ttttgcgaag cgccatctga cggattttca aaagatgtat atgctcggtg    180
   cggaaaccta actagtggcc gttaccctgc gaatgtccac agggtagctg gtagtttgaa    240
   aatcaacgcc gttgccctta ggattcagta actggcacat tttgtaatgc gctagatctg    300
   tgtgctcagt cttccaggct gcttatcaca gtgaaagcaa aaccaattcg tggctgcgaa    360
   agtcgtagcc accacgaagt ccaggaggac atacaatgcc caccctcgcg ccttcaggtc    420
   aacttgaaat ccaagcgatc ggtgatgtct ccaccgaagc cggagcaatc attacaaacg    480
   ctgaaatcgc ctatcaccgc tggggtgaat accgcgtaga taaagaagga cgcagcaatg    540
   tcgttctcat cgaacacgcc ctcactggag attccaacgc agccgattgg tgggctgact    600
   tgctcggtcc cggcaaagcc atcaacactg atatttactg cgtgatctgt accaacgtca    660
   tcggtggttg caacggttcc accggacctg gctccatgca tccagatgga aatttctggg    720
   gtaatcgctt ccccgccacg tccattcgtg atcaggtaaa cgccgaaaaa caattcctcg    780
   acgcactcgg catcaccacg gtcgccgcag tacttggtgg ttccatgggt ggtgcccgca    840
   ccctagagtg ggccgcaatg tacccagaaa ctgttggcgc agctgctgtt cttgcagttt    900
   ctgcacgcgc cagcgcctgg caaatcggca ttcaatccgc ccaaattaag gcgattgaaa    960
   acgaccacca ctggcacgaa ggcaactact acgaatccgg ctgcaaccca gccaccggac   1020
   tcggcgccgc ccgacgcatc gcccacctca cctaccgtgg cgaactagaa atcgacgaac   1080
   gcttcggcac caaagcccaa aagaacgaaa acccactcgg tccctaccgc aagcccgacc   1140
   agcgcttcgc cgtggaatcc tacttggact accaagcaga caagctagta cagcgtttcg   1200
   acgccggctc ctacgtcttg ctcaccgacg ccctcaaccg ccacgacatt ggtcgcgacc   1260
   gcggaggcct caacaaggca ctcgaatcca tcaaagttcc agtccttgtc gcaggcgtag   1320
   ataccgatat tttgtacccc taccaccagc aagaacacct ctccagaaac ctgggaaatc   1380
   tactggcaat ggcaaaaatc gtatcccctg tcggccacga tgctttcctc accgaaagcc   1440
   gccaaatgga tcgcatcgtg aggaacttct tcagcctcat ctccccagac gaagacaacc 1500
   cttcgaccta catcgagttc tacatctaag tcgacatcga tgctcttctg cgttaattaa 1560
   caattgggat cctctagagt tctgtgaaaa acaccgtggg gcagtttctg cttcgcggtg 1620
   tttttatttt gtggggcact agacccggga tttaaatcgc tagcgggctg ctaaaggaag 1680
   cggaacacgt agaaagccag tccgcagaaa cggtgctgac cccggatgaa tgtcagctac 1740
   tgggctatct ggacaaggga aaacgcaagc gcaaagagaa agcaggtagc ttgcagtggg 1800
   cttacatggc gatagctaga ctgggcggtt ttatggacag caagcgaacc ggaattgcca 1860
   gctggggcgc cctctggtaa ggttgggaag ccctgcaaag taaactggat ggctttcttg 1920
   ccgccaagga tctgatggcg caggggatca agatctgatc aagagacagg atgaggatcg 1980
   tttcgcatga ttgaacaaga tggattgcac gcaggttctc cggccgcttg ggtggagagg 2040
   ctattcggct atgactgggc acaacagaca atcggctgct ctgatgccgc cgtgttccgg 2100
   ctgtcagcgc aggggcgccc ggttcttttt gtcaagaccg acctgtccgg tgccctgaat 2160
   gaactgcagg acgaggcagc gcggctatcg tggctggcca cgacgggcgt tccttgcgca 2220
   gctgtgctcg acgttgtcac tgaagcggga agggactggc tgctattggg cgaagtgccg 2280
   gggcaggatc tcctgtcatc tcaccttgct cctgccgaga aagtatccat catggctgat 2340
   gcaatgcggc ggctgcatac gcttgatccg gctacctgcc cattcgacca ccaagcgaaa 2400
   catcgcatcg agcgagcacg tactcggatg gaagccggtc ttgtcgatca ggatgatctg 2460
   gacgaagagc atcaggggct cgcgccagcc gaactgttcg ccaggctcaa ggcgcgcatg 2520
   cccgacggcg aggatctcgt cgtgacccat ggcgatgcct gcttgccgaa tatcatggtg 2580
   gaaaatggcc gcttttctgg attcatcgac tgtggccggc tgggtgtggc ggaccgctat 2640
   caggacatag cgttggctac ccgtgatatt gctgaagagc ttggcggcga atgggctgac 2700
   cgcttcctcg tgctttacgg tatcgccgct cccgattcgc agcgcatcgc cttctatcgc 2760
   cttcttgacg agttcttctg agcgggactc tggggttcga aatgaccgac caagcgacgc 2820
   ccaacctgcc atcacgagat ttcgattcca ccgccgcctt ctatgaaagg ttgggcttcg 2880
   gaatcgtttt ccgggacgcc ggctggatga tcctccagcg cggggatctc atgctggagt 2940
   tcttcgccca cgctagcggc gcgccggccg gcccggtgtg aaataccgca cagatgcgta 3000
   aggagaaaat accgcatcag gcgctcttcc gcttcctcgc tcactgactc gctgcgctcg 3060
   gtcgttcggc tgcggcgagc ggtatcagct cactcaaagg cggtaatacg gttatccaca 3120
   gaatcagggg ataacgcagg aaagaacatg tgagcaaaag gccagcaaaa ggccaggaac 3180
   cgtaaaaagg ccgcgttgct ggcgtttttc cataggctcc gcccccctga cgagcatcac 3240
   aaaaatcgac gctcaagtca gaggtggcga aacccgacag gactataaag ataccaggcg 3300
   tttccccctg gaagctccct cgtgcgctct cctgttccga ccctgccgct taccggatac 3360
   ctgtccgcct ttctcccttc gggaagcgtg gcgctttctc atagctcacg ctgtaggtat 3420
   ctcagttcgg tgtaggtcgt tcgctccaag ctgggctgtg tgcacgaacc ccccgttcag 3480
   cccgaccgct gcgccttatc cggtaactat cgtcttgagt ccaacccggt aagacacgac 3540
   ttatcgccac tggcagcagc cactggtaac aggattagca gagcgaggta tgtaggcggt 3600
   gctacagagt tcttgaagtg gtggcctaac tacggctaca ctagaaggac agtatttggt 3660
   atctgcgctc tgctgaagcc agttaccttc ggaaaaagag ttggtagctc ttgatccggc 3720
   aaacaaacca ccgctggtag cggtggtttt tttgtttgca agcagcagat tacgcgcaga 3780
   aaaaaaggat ctcaagaaga tcctttgatc ttttctacgg ggtctgacgc tcagtggaac 3840
   gaaaactcac gttaagggat tttggtcatg agattatcaa aaaggatctt cacctagatc 3900
   cttttaaagg ccggccgcgg ccgcgcaaag tcccgcttcg tgaaaatttt cgtgccgcgt 3960
   gattttccgc caaaaacttt aacgaacgtt cgttataatg gtgtcatgac cttcacgacg 4020
   aagtactaaa attggcccga atcatcagct atggatctct ctgatgtcgc gctggagtcc 4080
   gacgcgctcg atgctgccgt cgatttaaaa acggtgatcg gatttttccg agctctcgat 4140
   acgacggacg cgccagcatc acgagactgg gccagtgccg cgagcgacct agaaactctc 4200
   gtggcggatc ttgaggagct ggctgacgag ctgcgtgctc ggccagcgcc aggaggacgc 4260
   acagtagtgg aggatgcaat cagttgcgcc tactgcggtg gcctgattcc tccccggcct 4320
   gacccgcgag gacggcgcgc aaaatattgc tcagatgcgt gtcgtgccgc agccagccgc 4380
   gagcgcgcca acaaacgcca cgccgaggag ctggaggcgg ctaggtcgca aatggcgctg 4440
   gaagtgcgtc ccccgagcga aattttggcc atggtcgtca cagagctgga agcggcagcg 4500
   agaattatcg cgatcgtggc ggtgcccgca ggcatgacaa acatcgtaaa tgccgcgttt 4560
   cgtgtgccgt ggccgcccag gacgtgtcag cgccgccacc acctgcaccg aatcggcagc 4620
   agcgtcgcgc gtcgaaaaag cgcacaggcg gcaagaagcg ataagctgca cgaatacctg 4680
   aaaaatgttg aacgccccgt gagcggtaac tcacagggcg tcggctaacc cccagtccaa 4740
   acctgggaga aagcgctcaa aaatgactct agcggattca cgagacattg acacaccggc 4800
   ctggaaattt tccgctgatc tgttcgacac ccatcccgag ctcgcgctgc gatcacgtgg 4860
   ctggacgagc gaagaccgcc gcgaattcct cgctcacctg ggcagagaaa atttccaggg 4920
   cagcaagacc cgcgacttcg ccagcgcttg gatcaaagac ccggacacgg agaaacacag 4980
   ccgaagttat accgagttgg ttcaaaatcg cttgcccggt gccagtatgt tgctctgacg 5040
   cacgcgcagc acgcagccgt gcttgtcctg gacattgatg tgccgagcca ccaggccggc 5100
   gggaaaatcg agcacgtaaa ccccgaggtc tacgcgattt tggagcgctg ggcacgcctg 5160
   gaaaaagcgc cagcttggat cggcgtgaat ccactgagcg ggaaatgcca gctcatctgg 5220
   ctcattgatc cggtgtatgc cgcagcaggc atgagcagcc cgaatatgcg cctgctggct 5280
   gcaacgaccg aggaaatgac ccgcgttttc ggcgctgacc aggctttttc acataggctg 5340
   agccgtggcc actgcactct ccgacgatcc cagccgtacc gctggcatgc ccagcacaat 5400
   cgcgtggatc gcctagctga tcttatggag gttgctcgca tgatctcagg cacagaaaaa 5460
   cctaaaaaac gctatgagca ggagttttct agcggacggg cacgtatcga agcggcaaga 5520
   aaagccactg cggaagcaaa agcacttgcc acgcttgaag caagcctgcc gagcgccgct 5580
   gaagcgtctg gagagctgat cgacggcgtc cgtgtcctct ggactgctcc agggcgtgcc 5640
   gcccgtgatg agacggcttt tcgccacgct ttgactgtgg gataccagtt aaaagcggct 5700
   ggtgagcgcc taaaagacac caagggtcat cgagcctacg agcgtgccta caccgtcgct 5760
   caggcggtcg gaggaggccg tgagcctgat ctgccgccgg actgtgaccg ccagacggat 5820
   tggccgcgac gtgtgcgcgg ctacgtcgct aaaggccagc cagtcgtccc tgctcgtcag 5880
   acagagacgc agagccagcc gaggcgaaaa gctctggcca ctatgggaag acgtggcggt 5940
   aaaaaggccg cagaacgctg gaaagaccca aacagtgagt acgcccgagc acagcgagaa 6000
   aaactagcta agtccagtca acgacaagct aggaaagcta aaggaaatcg cttgaccatt 6060
   gcaggttggt ttatgactgt tgagggagag actggctcgt ggccgacaat caatgaagct 6120
   atgtctgaat ttagcgtgtc acgtcagacc gtgaatagag cacttaaggt ctgcgggcat 6180
   tgaacttcca cgaggacgcc gaaagcttcc cagtaaatgt gccatctcgt aggcagaaaa 6240
   cggttccccc gtagggtctc tctcttggcc tcctttctag gtcgggctga ttgctcttga 6300
   agctctctag gggggctcac accataggca gataacgttc cccaccggct cgcctcgtaa 6360
   gcgcacaagg actgctccca aagatcttca aagccactgc cgcgactgcc ttcgcgaagc 6420
   cttgccccgc ggaaatttcc tccaccgagt tcgtgcacac ccctatgcca agcttctttc 6480
   accctaaatt cgagagattg gattcttacc gtggaaattc ttcgcaaaaa tcgtcccctg 6540
   atcgcccttg cgacgttggc gtcggtgccg ctggttgcgc ttggcttgac cgacttgatc 6600
   agcggccgc 6609
<210> 53
   <211> 30
   <212> DNA
   <213> Artificial Sequence
<220>
   <223> Primer
   <220>
   <221> misc_feature
   <223> internal designation: BK1701
<400> 53
   gagactcgag cggcttaaag tttggctgcc 30
<210> 54
   <211> 31
   <212> DNA
   <213> Artificial Sequence
<220>
   <223> Primer
   <220>
   <221> misc_feature
   <223> internal designation: Haf018
<400> 54
   gagaactagt attttgtgtg tgccggttgt g 31
<210> 55
   <211> 6583
   <212> DNA
   <213> Artificial Sequence
<220>
   <223> Plasmid
   <220>
   <221> misc_feature ,
   <223> internal designation: pCLiK5MCS PgroPsod metA (H472)
<400> 55
   tcgatttaaa tctcgagcgg cttaaagttt ggctgccatg tgaattttta gcaccctcaa     60
   cagttgagtg ctggcactct cgggggtaga gtgccaaata ggttgtttga cacacagttg    120
   ttcacccgcg acgacggctg tgctggaaac ccacaaccgg cacacacaaa atactagtag    180
   ctgccaatta ttccgggctt gtgacccgct acccgataaa taggtcggct gaaaaatttc    240
   gttgcaatat caacaaaaag gcctatcatt gggaggtgtc gcaccaagta cttttgcgaa    300
   gcgccatctg acggattttc aaaagatgta tatgctcggt gcggaaacct acgaaaggat    360
   tttttaccca tgcccaccct cgcgccttca ggtcaacttg aaatccaagc gatcggtgat    420
   gtctccaccg aagccggagc aatcattaca aacgctgaaa tcgcctatca ccgctggggt    480
   gaataccgcg tagataaaga aggacgcagc aatgtcgttc tcatcgaaca cgccctcact    540
   ggagattcca acgcagccga ttggtgggct gacttgctcg gtcccggcaa agccatcaac    600
   actgatattt actgcgtgat ctgtaccaac gtcatcggtg gttgcaacgg ttccaccgga    660
   cctggctcca tgcatccaga tggaaatttc tggggtaatc gcttccccgc cacgtccatt    720
   cgtgatcagg taaacgccga aaaacaattc ctcgacgcac tcggcatcac cacggtcgcc    780
   gcagtacttg gtggttccat gggtggtgcc cgcaccctag agtgggccgc aatgtaccca    840
   gaaactgttg gcgcagctgc tgttcttgca gtttctgcac gcgccagcgc ctggcaaatc    900
   ggcattcaat ccgcccaaat taaggcgatt gaaaacgacc accactggca cgaaggcaac    960
   tactacgaat ccggctgcaa cccagccacc ggactcggcg ccgcccgacg catcgcccac   1020
   ctcacctacc gtggcgaact agaaatcgac gaacgcttcg gcaccaaagc ccaaaagaac   1080
   gaaaacccac tcggtcccta ccgcaagccc gaccagcgct tcgccgtgga atcctacttg   1140
   gactaccaag cagacaagct agtacagcgt ttcgacgccg gctcctacgt cttgctcacc   1200
   gacgccctca accgccacga cattggtcgc gaccgcggag gcctcaacaa ggcactcgaa   1260
   tccatcaaag ttccagtcct tgtcgcaggc gtagataccg atattttgta cccctaccac   1320
   cagcaagaac acctctccag aaacctggga aatctactgg caatggcaaa aatcgtatcc   1380
   cctgtcggcc acgatgcttt cctcaccgaa agccgccaaa tggatcgcat cgtgaggaac   1440
   ttcttcagcc tcatctcccc agacgaagac aacccttcga cctacatcga gttctacatc   1500
   taagtcgaca tcgatgctct tctgcgttaa ttaacaattg ggatcctcta gagttctgtg   1560
   aaaaacaccg tggggcagtt tctgcttcgc ggtgtttttt atttgtgggg cactagaccc   1620
   gggatttaaa tcgctagcgg gctgctaaag gaagcggaac acgtagaaag ccagtccgca   1680
   gaaacggtgc tgaccccgga tgaatgtcag ctactgggct atctggacaa gggaaaacgc   1740
   aagcgcaaag agaaagcagg tagcttgcag tgggcttaca tggcgatagc tagactgggc 1800
   ggttttatgg acagcaagcg aaccggaatt gccagctggg gcgccctctg gtaaggttgg 1860
   gaagccctgc aaagtaaact ggatggcttt cttgccgcca aggatctgat ggcgcagggg 1920
   atcaagatct gatcaagaga caggatgagg atcgtttcgc atgattgaac aagatggatt 1980
   gcacgcaggt tctccggccg cttgggtgga gaggctattc ggctatgact gggcacaaca 2040
   gacaatcggc tgctctgatg ccgccgtgtt ccggctgtca gcgcaggggc gcccggttct 2100
   ttttgtcaag accgacctgt ccggtgccct gaatgaactg caggacgagg cagcgcggct 2160
   atcgtggctg gccacgacgg gcgttccttg cgcagctgtg ctcgacgttg tcactgaagc 2220
   gggaagggac tggctgctat tgggcgaagt gccggggcag gatctcctgt catctcacct 2280
   tgctcctgcc gagaaagtat ccatcatggc tgatgcaatg cggcggctgc atacgcttga 2340
   tccggctacc tgcccattcg accaccaagc gaaacatcgc atcgagcgag cacgtactcg 2400
   gatggaagcc ggtcttgtcg atcaggatga tctggacgaa gagcatcagg ggctcgcgcc 2460
   agccgaactg ttcgccaggc tcaaggcgcg catgcccgac ggcgaggatc tcgtcgtgac 2520
   ccatggcgat gcctgcttgc cgaatatcat ggtggaaaat ggccgctttt ctggattcat 2580
   cgactgtggc cggctgggtg tggcggaccg ctatcaggac atagcgttgg ctacccgtga 2640
   tattgctgaa gagcttggcg gcgaatgggc tgaccgcttc ctcgtgcttt acggtatcgc 2700
   cgctcccgat tcgcagcgca tcgccttcta tcgccttctt gacgagttct tctgagcggg 2760
   actctggggt tcgaaatgac cgaccaagcg acgcccaacc tgccatcacg agatttcgat 2820
   tccaccgccg ccttctatga aaggttgggc ttcggaatcg ttttccggga cgccggctgg 2880
   atgatcctcc agcgcgggga tctcatgctg gagttcttcg cccacgctag cggcgcgccg 2940
   gccggcccgg tgtgaaatac cgcacagatg cgtaaggaga aaataccgca tcaggcgctc 3000
   ttccgcttcc tcgctcactg actcgctgcg ctcggtcgtt cggctgcggc gagcggtatc 3060
   agctcactca aaggcggtaa tacggttatc cacagaatca ggggataacg caggaaagaa 3120
   catgtgagca aaaggccagc aaaaggccag gaaccgtaaa aaggccgcgt tgctggcgtt 3180
   tttccatagg ctccgccccc ctgacgagca tcacaaaaat cgacgctcaa gtcagaggtg 3240
   gcgaaacccg acaggactat aaagatacca ggcgtttccc cctggaagct ccctcgtgcg 3300
   ctctcctgtt ccgaccctgc cgcttaccgg atacctgtcc gcctttctcc cttcgggaag 3360
   cgtggcgctt tctcatagct cacgctgtag gtatctcagt tcggtgtagg tcgttcgctc 3420
   caagctgggc tgtgtgcacg aaccccccgt tcagcccgac cgctgcgcct tatccggtaa 3480
   ctatcgtctt gagtccaacc cggtaagaca cgacttatcg ccactggcag cagccactgg 3540
   taacaggatt agcagagcga ggtatgtagg cggtgctaca gagttcttga agtggtggcc 3600
   taactacggc tacactagaa ggacagtatt tggtatctgc gctctgctga agccagttac 3660
   cttcggaaaa agagttggta gctcttgatc cggcaaacaa accaccgctg gtagcggtgg 3720
   tttttttgtt tgcaagcagc agattacgcg cagaaaaaaa ggatctcaag aagatccttt 3780
   gatcttttct acggggtctg acgctcagtg gaacgaaaac tcacgttaag ggattttggt 3840
   catgagatta tcaaaaagga tcttcaccta gatcctttta aaggccggcc gcggccgcgc 3900
   aaagtcccgc ttcgtgaaaa ttttcgtgcc gcgtgatttt ccgccaaaaa ctttaacgaa 3960
   cgttcgttat aatggtgtca tgaccttcac gacgaagtac taaaattggc ccgaatcatc 4020
   agctatggat ctctctgatg tcgcgctgga gtccgacgcg ctcgatgctg ccgtcgattt 4080
   aaaaacggtg atcggatttt tccgagctct cgatacgacg gacgcgccag catcacgaga 4140
   ctgggccagt gccgcgagcg acctagaaac tctcgtggcg gatcttgagg agctggctga 4200
   cgagctgcgt gctcggccag cgccaggagg acgcacagta gtggaggatg caatcagttg 4260
   cgcctactgc ggtggcctga ttcctccccg gcctgacccg cgaggacggc gcgcaaaata 4320
   ttgctcagat gcgtgtcgtg ccgcagccag ccgcgagcgc gccaacaaac gccacgccga 4380
   ggagctggag gcggctaggt cgcaaatggc gctggaagtg cgtcccccga gcgaaatttt 4440
   ggccatggtc gtcacagagc tggaagcggc agcgagaatt atcgcgatcg tggcggtgcc 4500
   cgcaggcatg acaaacatcg taaatgccgc gtttcgtgtg ccgtggccgc ccaggacgtg 4560
   tcagcgccgc caccacctgc accgaatcgg cagcagcgtc gcgcgtcgaa aaagcgcaca 4620
   ggcggcaaga agcgataagc tgcacgaata cctgaaaaat gttgaacgcc ccgtgagcgg 4680
   taactcacag ggcgtcggct aacccccagt ccaaacctgg gagaaagcgc tcaaaaatga 4740
   ctctagcgga ttcacgagac attgacacac cggcctggaa attttccgct gatctgttcg 4800
   acacccatcc cgagctcgcg ctgcgatcac gtggctggac gagcgaagac cgccgcgaat 4860
   tcctcgctca cctgggcaga gaaaatttcc agggcagcaa gacccgcgac ttcgccagcg 4920
   cttggatcaa agacccggac acggagaaac acagccgaag ttataccgag ttggttcaaa 4980
   atcgcttgcc cggtgccagt atgttgctct gacgcacgcg cagcacgcag ccgtgcttgt 5040
   cctggacatt gatgtgccga gccaccaggc cggcgggaaa atcgagcacg taaaccccga 5100
   ggtctacgcg attttggagc gctgggcacg cctggaaaaa gcgccagctt ggatcggcgt 5160
   gaatccactg agcgggaaat gccagctcat ctggctcatt gatccggtgt atgccgcagc 5220
   aggcatgagc agcccgaata tgcgcctgct ggctgcaacg accgaggaaa tgacccgcgt 5280
   tttcggcgct gaccaggctt tttcacatag gctgagccgt ggccactgca ctctccgacg 5340
   atcccagccg taccgctggc atgcccagca caatcgcgtg gatcgcctag ctgatcttat 5400
   ggaggttgct cgcatgatct caggcacaga aaaacctaaa aaacgctatg agcaggagtt 5460
   ttctagcgga cgggcacgta tcgaagcggc aagaaaagcc actgcggaag caaaagcact 5520
   tgccacgctt gaagcaagcc tgccgagcgc cgctgaagcg tctggagagc tgatcgacgg 5580
   cgtccgtgtc ctctggactg ctccagggcg tgccgcccgt gatgagacgg cttttcgcca 5640
   cgctttgact gtgggatacc agttaaaagc ggctggtgag cgcctaaaag acaccaaggg 5700
   tcatcgagcc tacgagcgtg cctacaccgt cgctcaggcg gtcggaggag gccgtgagcc 5760
   tgatctgccg ccggactgtg accgccagac ggattggccg cgacgtgtgc gcggctacgt 5820
   cgctaaaggc cagccagtcg tccctgctcg tcagacagag acgcagagcc agccgaggcg 5880
   aaaagctctg gccactatgg gaagacgtgg cggtaaaaag gccgcagaac gctggaaaga 5940
   cccaaacagt gagtacgccc gagcacagcg agaaaaacta gctaagtcca gtcaacgaca 6000
   agctaggaaa gctaaaggaa atcgcttgac cattgcaggt tggtttatga ctgttgaggg 6060
   agagactggc tcgtggccga caatcaatga agctatgtct gaatttagcg tgtcacgtca 6120
   gaccgtgaat agagcactta aggtctgcgg gcattgaact tccacgagga cgccgaaagc 6180
   ttcccagtaa atgtgccatc tcgtaggcag aaaacggttc ccccgtaggg tctctctctt 6240
   ggcctccttt ctaggtcggg ctgattgctc ttgaagctct ctaggggggc tcacaccata 6300
   ggcagataac gttccccacc ggctcgcctc gtaagcgcac aaggactgct cccaaagatc 6360
   ttcaaagcca ctgccgcgac tgccttcgcg aagccttgcc ccgcggaaat ttcctccacc 6420
   gagttcgtgc acacccctat gccaagcttc tttcacccta aattcgagag attggattct 6480
   taccgtggaa attcttcgca aaaatcgtcc cctgatcgcc cttgcgacgt tggcgtcggt 6540
   gccgctggtt gcgcttggct tgaccgactt gatcagcggc cgc 6583
<210> 56
   <211> 20
   <212> DNA
   <213> Artificial Sequence
<220>
   <223> Primer
   <220>
   <221> misc_feature
   <223> internal designation: BK1782
<400> 56
   tcgagagatt ggattcttac 20
<210> 57
   <211> 30
   <212> DNA
   <213> Artificial Sequence
<220>
   <223> Primer
   <220>
   <221> misc_feature
   <223> internal designation: Haf63
<400> 57
   tctcggtacc ccgcaccgag catatacatc 30
<210> 58
   <211> 6450
   <212> DNA
   <213> Artificial Sequence
<220>
   <223> Plasmid
   <220>
   <221> misc_feature
   <223> internal designation: H479 (pCliK5MCS PEF-TS metA)
<400> 58
   aaatctcgag cttggtctat agtggctagg taccccccca cgacaatgga actttgactt 60
   ttaaaatttc atcgccgtgg gggctttttg ggcagccagc ccgccgtgtc gcaacgtaat 120
   cgactgaata cctgtacgat cactttttag acgggcgggt agggctactg tgccctaacc 180
   taagcttgta aagcattaat tatccataca taaggaggat cgccccgtaa tgcccaccct 240
   cgcgccttca ggtcaacttg aaatccaagc gatcggtgat gtctccaccg aagccggagc 300
   aatcattaca aacgctgaaa tcgcctatca ccgctggggt gaataccgcg tagataaaga 360
   aggacgcagc aatgtcgttc tcatcgaaca cgccctcact ggagattcca acgcagccga 420
   ttggtgggct gacttgctcg gtcccggcaa agccatcaac actgatattt actgcgtgat 480
   ctgtaccaac gtcatcggtg gttgcaacgg ttccaccgga cctggctcca tgcatccaga 540
   tggaaatttc tggggtaatc gcttccccgc cacgtccatt cgtgatcagg taaacgccga 600
   aaaacaattc ctcgacgcac tcggcatcac cacggtcgcc gcagtacttg gtggttccat 660
   gggtggtgcc cgcaccctag agtgggccgc aatgtaccca gaaactgttg gcgcagctgc 720
   tgttcttgca gtttctgcac gcgccagcgc ctggcaaatc ggcattcaat ccgcccaaat 780
   taaggcgatt gaaaacgacc accactggca cgaaggcaac tactacgaat ccggctgcaa 840
   cccagccacc ggactcggcg ccgcccgacg catcgcccac ctcacctacc gtggcgaact 900
   agaaatcgac gaacgcttcg gcaccaaagc ccaaaagaac gaaaacccac tcggtcccta 960
   ccgcaagccc gaccagcgct tcgccgtgga atcctacttg gactaccaag cagacaagct 1020
   agtacagcgt ttcgacgccg gctcctacgt cttgctcacc gacgccctca accgccacga 1080
   cattggtcgc gaccgcggag gcctcaacaa ggcactcgaa tccatcaaag ttccagtcct 1140
   tgtcgcaggc gtagataccg atattttgta cccctaccac cagcaagaac acctctccag 1200
   aaacctggga aatctactgg caatggcaaa aatcgtatcc cctgtcggcc acgatgcttt 1260
   cctcaccgaa agccgccaaa tggatcgcat cgtgaggaac ttcttcagcc tcatctcccc 1320
   agacgaagac aacccttcga cctacatcga gttctacatc taagtcgaca tcgatgctct 1380
   tctgcgttaa ttaacaattg ggatcctcta gagttctgtg aaaaacaccg tggggcagtt 1440
   tctgcttcgc ggtgtttttt atttgtgggg cactagaccc gggatttaaa tcgctagcgg 1500
   gctgctaaag gaagcggaac acgtagaaag ccagtccgca gaaacggtgc tgaccccgga 1560
   tgaatgtcag ctactgggct atctggacaa gggaaaacgc aagcgcaaag agaaagcagg 1620
   tagcttgcag tgggcttaca tggcgatagc tagactgggc ggttttatgg acagcaagcg 1680
   aaccggaatt gccagctggg gcgccctctg gtaaggttgg gaagccctgc aaagtaaact 1740
   ggatggcttt cttgccgcca aggatctgat ggcgcagggg atcaagatct gatcaagaga 1800
   caggatgagg atcgtttcgc atgattgaac aagatggatt gcacgcaggt tctccggccg 1860
   cttgggtgga gaggctattc ggctatgact gggcacaaca gacaatcggc tgctctgatg 1920
   ccgccgtgtt ccggctgtca gcgcaggggc gcccggttct ttttgtcaag accgacctgt 1980
   ccggtgccct gaatgaactg caggacgagg cagcgcggct atcgtggctg gccacgacgg 2040
   gcgttccttg cgcagctgtg ctcgacgttg tcactgaagc gggaagggac tggctgctat 2100
   tgggcgaagt gccggggcag gatctcctgt catctcacct tgctcctgcc gagaaagtat 2160
   ccatcatggc tgatgcaatg cggcggctgc atacgcttga tccggctacc tgcccattcg 2220
   accaccaagc gaaacatcgc atcgagcgag cacgtactcg gatggaagcc ggtcttgtcg 2280
   atcaggatga tctggacgaa gagcatcagg ggctcgcgcc agccgaactg ttcgccaggc 2340
   tcaaggcgcg catgcccgac ggcgaggatc tcgtcgtgac ccatggcgat gcctgcttgc 2400
   cgaatatcat ggtggaaaat ggccgctttt ctggattcat cgactgtggc cggctgggtg 2460
   tggcggaccg ctatcaggac atagcgttgg ctacccgtga tattgctgaa gagcttggcg 2520
   gcgaatgggc tgaccgcttc ctcgtgcttt acggtatcgc cgctcccgat tcgcagcgca 2580
   tcgccttcta tcgccttctt gacgagttct tctgagcggg actctggggt tcgaaatgac 2640
   cgaccaagcg acgcccaacc tgccatcacg agatttcgat tccaccgccg ccttctatga 2700
   aaggttgggc ttcggaatcg ttttccggga cgccggctgg atgatcctcc agcgcgggga 2760
   tctcatgctg gagttcttcg cccacgctag cggcgcgccg gccggcccgg tgtgaaatac 2820
   cgcacagatg cgtaaggaga aaataccgca tcaggcgctc ttccgcttcc tcgctcactg 2880
   actcgctgcg ctcggtcgtt cggctgcggc gagcggtatc agctcactca aaggcggtaa 2940
   tacggttatc cacagaatca ggggataacg caggaaagaa catgtgagca aaaggccagc 3000
   aaaaggccag gaaccgtaaa aaggccgcgt tgctggcgtt tttccatagg ctccgccccc 3060
   ctgacgagca tcacaaaaat cgacgctcaa gtcagaggtg gcgaaacccg acaggactat 3120
   aaagatacca ggcgtttccc cctggaagct ccctcgtgcg ctctcctgtt ccgaccctgc 3180
   cgcttaccgg atacctgtcc gcctttctcc cttcgggaag cgtggcgctt tctcatagct 3240
   cacgctgtag gtatctcagt tcggtgtagg tcgttcgctc caagctgggc tgtgtgcacg 3300
   aaccccccgt tcagcccgac cgctgcgcct tatccggtaa ctatcgtctt gagtccaacc 3360
   cggtaagaca cgacttatcg ccactggcag cagccactgg taacaggatt agcagagcga 3420
   ggtatgtagg cggtgctaca gagttcttga agtggtggcc taactacggc tacactagaa 3480
   ggacagtatt tggtatctgc gctctgctga agccagttac cttcggaaaa agagttggta 3540
   gctcttgatc cggcaaacaa accaccgctg gtagcggtgg tttttttgtt tgcaagcagc 3600
   agattacgcg cagaaaaaaa ggatctcaag aagatccttt gatcttttct acggggtctg 3660
   acgctcagtg gaacgaaaac tcacgttaag ggattttggt catgagatta tcaaaaagga 3720
   tcttcaccta gatcctttta aaggccggcc gcggccgcgc aaagtcccgc ttcgtgaaaa 3780
   ttttcgtgcc gcgtgatttt ccgccaaaaa ctttaacgaa cgttcgttat aatggtgtca 3840
   tgaccttcac gacgaagtac taaaattggc ccgaatcatc agctatggat ctctctgatg 3900
   tcgcgctgga gtccgacgcg ctcgatgctg ccgtcgattt aaaaacggtg atcggatttt 3960
   tccgagctct cgatacgacg gacgcgccag catcacgaga ctgggccagt gccgcgagcg 4020
   acctagaaac tctcgtggcg gatcttgagg agctggctga cgagctgcgt gctcggccag 4080
   cgccaggagg acgcacagta gtggaggatg caatcagttg cgcctactgc ggtggcctga 4140
   ttcctccccg gcctgacccg cgaggacggc gcgcaaaata ttgctcagat gcgtgtcgtg 4200
   ccgcagccag ccgcgagcgc gccaacaaac gccacgccga ggagctggag gcggctaggt 4260
   cgcaaatggc gctggaagtg cgtcccccga gcgaaatttt ggccatggtc gtcacagagc 4320
   tggaagcggc agcgagaatt atcgcgatcg tggcggtgcc cgcaggcatg acaaacatcg 4380
   taaatgccgc gtttcgtgtg ccgtggccgc ccaggacgtg tcagcgccgc caccacctgc 4440
   accgaatcgg cagcagcgtc gcgcgtcgaa aaagcgcaca ggcggcaaga agcgataagc 4500
   tgcacgaata cctgaaaaat gttgaacgcc ccgtgagcgg taactcacag ggcgtcggct 4560
   aacccccagt ccaaacctgg gagaaagcgc tcaaaaatga ctctagcgga ttcacgagac 4620
   attgacacac cggcctggaa attttccgct gatctgttcg acacccatcc cgagctcgcg 4680
   ctgcgatcac gtggctggac gagcgaagac cgccgcgaat tcctcgctca cctgggcaga 4740
   gaaaatttcc agggcagcaa gacccgcgac ttcgccagcg cttggatcaa agacccggac 4800
   acggagaaac acagccgaag ttataccgag ttggttcaaa atcgcttgcc cggtgccagt 4860
   atgttgctct gacgcacgcg cagcacgcag ccgtgcttgt cctggacatt gatgtgccga 4920
   gccaccaggc cggcgggaaa atcgagcacg taaaccccga ggtctacgcg attttggagc 4980
   gctgggcacg cctggaaaaa gcgccagctt ggatcggcgt gaatccactg agcgggaaat 5040
   gccagctcat ctggctcatt gatccggtgt atgccgcagc aggcatgagc agcccgaata 5100
   tgcgcctgct ggctgcaacg accgaggaaa tgacccgcgt tttcggcgct gaccaggctt 5160
   tttcacatag gctgagccgt ggccactgca ctctccgacg atcccagccg taccgctggc 5220
   atgcccagca caatcgcgtg gatcgcctag ctgatcttat ggaggttgct cgcatgatct 5280
   caggcacaga aaaacctaaa aaacgctatg agcaggagtt ttctagcgga cgggcacgta 5340
   tcgaagcggc aagaaaagcc actgcggaag caaaagcact tgccacgctt gaagcaagcc 5400
   tgccgagcgc cgctgaagcg tctggagagc tgatcgacgg cgtccgtgtc ctctggactg 5460
   ctccagggcg tgccgcccgt gatgagacgg cttttcgcca cgctttgact gtgggatacc 5520
   agttaaaagc ggctggtgag cgcctaaaag acaccaaggg tcatcgagcc tacgagcgtg 5580
   cctacaccgt cgctcaggcg gtcggaggag gccgtgagcc tgatctgccg ccggactgtg 5640
   accgccagac ggattggccg cgacgtgtgc gcggctacgt cgctaaaggc cagccagtcg 5700
   tccctgctcg tcagacagag acgcagagcc agccgaggcg aaaagctctg gccactatgg 5760
   gaagacgtgg cggtaaaaag gccgcagaac gctggaaaga cccaaacagt gagtacgccc 5820
   gagcacagcg agaaaaacta gctaagtcca gtcaacgaca agctaggaaa gctaaaggaa 5880
   atcgcttgac cattgcaggt tggtttatga ctgttgaggg agagactggc tcgtggccga 5940
   caatcaatga agctatgtct gaatttagcg tgtcacgtca gaccgtgaat agagcactta 6000
   aggtctgcgg gcattgaact tccacgagga cgccgaaagc ttcccagtaa atgtgccatc 6060
   tcgtaggcag aaaacggttc ccccgtaggg tctctctctt ggcctccttt ctaggtcggg 6120
   ctgattgctc ttgaagctct ctaggggggc tcacaccata ggcagataac gttccccacc 6180
   ggctcgcctc gtaagcgcac aaggactgct cccaaagatc ttcaaagcca ctgccgcgac 6240
   tgccttcgcg aagccttgcc ccgcggaaat ttcctccacc gagttcgtgc acacccctat 6300
   gccaagcttc tttcacccta aattcgagag attggattct taccgtggaa attcttcgca 6360
   aaaatcgtcc cctgatcgcc cttgcgacgt tggcgtcggt gccgctggtt gcgcttggct 6420
   tgaccgactt gatcagcggc cgctcgattt 6450
<210> 59
   <211> 6759
   <212> DNA
   <213> Artificial Sequence
<220>
   <223> Plasmid
   <220>
   <221> misc_feature
   <223> internal designation: H501 (pCliK5MCS Pgro Psod Pefts metA)
<400> 59
   aaatctcgag cggcttaaag tttggctgcc atgtgaattt ttagcaccct caacagttga     60
   gtgctggcac tctcgggggt agagtgccaa ataggttgtt tgacacacag ttgttcaccc    120
   gcgacgacgg ctgtgctgga aacccacaac cggcacacac aaaatactag tagctgccaa    180
   ttattccggg cttgtgaccc gctacccgat aaataggtcg gctgaaaaat ttcgttgcaa    240
   tatcaacaaa aaggcctatc attgggaggt gtcgcaccaa gtacttttgc gaagcgccat    300
   ctgacggatt ttcaaaagat gtatatgctc ggtgcggggt acccccccac gacaatggaa    360
   ctttgacttt taaaatttca tcgccgtggg ggctttttgg gcagccagcc cgccgtgtcg    420
   caacgtaatc gactgaatac ctgtacgatc actttttaga cgggcgggta gggctactgt    480
   gccctaacct aagcttgtaa agcattaatt atccatacat aaggaggatc gccccgtaat    540
   gcccaccctc gcgccttcag gtcaacttga aatccaagcg atcggtgatg tctccaccga    600
   agccggagca atcattacaa acgctgaaat cgcctatcac cgctggggtg aataccgcgt    660
   agataaagaa ggacgcagca atgtcgttct catcgaacac gccctcactg gagattccaa    720
   cgcagccgat tggtgggctg acttgctcgg tcccggcaaa gccatcaaca ctgatattta    780
   ctgcgtgatc tgtaccaacg tcatcggtgg ttgcaacggt tccaccggac ctggctccat    840
   gcatccagat ggaaatttct ggggtaatcg cttccccgcc acgtccattc gtgatcaggt    900
   aaacgccgaa aaacaattcc tcgacgcact cggcatcacc acggtcgccg cagtacttgg    960
   tggttccatg ggtggtgccc gcaccctaga gtgggccgca atgtacccag aaactgttgg   1020
   cgcagctgct gttcttgcag tttctgcacg cgccagcgcc tggcaaatcg gcattcaatc   1080
   cgcccaaatt aaggcgattg aaaacgacca ccactggcac gaaggcaact actacgaatc   1140
   cggctgcaac ccagccaccg gactcggcgc cgcccgacgc atcgcccacc tcacctaccg   1200
   tggcgaacta gaaatcgacg aacgcttcgg caccaaagcc caaaagaacg aaaacccact   1260
   cggtccctac cgcaagcccg accagcgctt cgccgtggaa tcctacttgg actaccaagc   1320
   agacaagcta gtacagcgtt tcgacgccgg ctcctacgtc ttgctcaccg acgccctcaa   1380
   ccgccacgac attggtcgcg accgcggagg cctcaacaag gcactcgaat ccatcaaagt   1440
   tccagtcctt gtcgcaggcg tagataccga tattttgtac ccctaccacc agcaagaaca   1500
   cctctccaga aacctgggaa atctactggc aatggcaaaa atcgtatccc ctgtcggcca   1560
   cgatgctttc ctcaccgaaa gccgccaaat ggatcgcatc gtgaggaact tcttcagcct   1620
   catctcccca gacgaagaca acccttcgac ctacatcgag ttctacatct aagtcgacat   1680
   cgatgctctt ctgcgttaat taacaattgg gatcctctag agttctgtga aaaacaccgt   1740
   ggggcagttt ctgcttcgcg gtgtttttta tttgtggggc actagacccg ggatttaaat   1800
   cgctagcggg ctgctaaagg aagcggaaca cgtagaaagc cagtccgcag aaacggtgct   1860
   gaccccggat gaatgtcagc tactgggcta tctggacaag ggaaaacgca agcgcaaaga   1920
   gaaagcaggt agcttgcagt gggcttacat ggcgatagct agactgggcg gttttatgga   1980
   cagcaagcga accggaattg ccagctgggg cgccctctgg taaggttggg aagccctgca   2040
   aagtaaactg gatggctttc ttgccgccaa ggatctgatg gcgcagggga tcaagatctg   2100
   atcaagagac aggatgagga tcgtttcgca tgattgaaca agatggattg cacgcaggtt   2160
   ctccggccgc ttgggtggag aggctattcg gctatgactg ggcacaacag acaatcggct   2220
   gctctgatgc cgccgtgttc cggctgtcag cgcaggggcg cccggttctt tttgtcaaga   2280
   ccgacctgtc cggtgccctg aatgaactgc aggacgaggc agcgcggcta tcgtggctgg   2340
   ccacgacggg cgttccttgc gcagctgtgc tcgacgttgt cactgaagcg ggaagggact   2400
   ggctgctatt gggcgaagtg ccggggcagg atctcctgtc atctcacctt gctcctgccg   2460
   agaaagtatc catcatggct gatgcaatgc ggcggctgca tacgcttgat ccggctacct   2520
   gcccattcga ccaccaagcg aaacatcgca tcgagcgagc acgtactcgg atggaagccg   2580
   gtcttgtcga tcaggatgat ctggacgaag agcatcaggg gctcgcgcca gccgaactgt   2640
   tcgccaggct caaggcgcgc atgcccgacg gcgaggatct cgtcgtgacc catggcgatg   2700
   cctgcttgcc gaatatcatg gtggaaaatg gccgcttttc tggattcatc gactgtggcc   2760
   ggctgggtgt ggcggaccgc tatcaggaca tagcgttggc tacccgtgat attgctgaag   2820
   agcttggcgg cgaatgggct gaccgcttcc tcgtgcttta cggtatcgcc gctcccgatt   2880
   cgcagcgcat cgccttctat cgccttcttg acgagttctt ctgagcggga ctctggggtt   2940
   cgaaatgacc gaccaagcga cgcccaacct gccatcacga gatttcgatt ccaccgccgc   3000
   cttctatgaa aggttgggct tcggaatcgt tttccgggac gccggctgga tgatcctcca   3060
   gcgcggggat ctcatgctgg agttcttcgc ccacgctagc ggcgcgccgg ccggcccggt   3120
   gtgaaatacc gcacagatgc gtaaggagaa aataccgcat caggcgctct tccgcttcct   3180
   cgctcactga ctcgctgcgc tcggtcgttc ggctgcggcg agcggtatca gctcactcaa   3240
   aggcggtaat acggttatcc acagaatcag gggataacgc aggaaagaac atgtgagcaa   3300
   aaggccagca aaaggccagg aaccgtaaaa aggccgcgtt gctggcgttt ttccataggc   3360
   tccgcccccc tgacgagcat cacaaaaatc gacgctcaag tcagaggtgg cgaaacccga   3420
   caggactata aagataccag gcgtttcccc ctggaagctc cctcgtgcgc tctcctgttc   3480
   cgaccctgcc gcttaccgga tacctgtccg cctttctccc ttcgggaagc gtggcgcttt   3540
   ctcatagctc acgctgtagg tatctcagtt cggtgtaggt cgttcgctcc aagctgggct   3600
   gtgtgcacga accccccgtt cagcccgacc gctgcgcctt atccggtaac tatcgtcttg   3660
   agtccaaccc ggtaagacac gacttatcgc cactggcagc agccactggt aacaggatta   3720
   gcagagcgag gtatgtaggc ggtgctacag agttcttgaa gtggtggcct aactacggct   3780
   acactagaag gacagtattt ggtatctgcg ctctgctgaa gccagttacc ttcggaaaaa   3840
   gagttggtag ctcttgatcc ggcaaacaaa ccaccgctgg tagcggtggt ttttttgttt   3900
   gcaagcagca gattacgcgc agaaaaaaag gatctcaaga agatcctttg atcttttcta   3960
   cggggtctga cgctcagtgg aacgaaaact cacgttaagg gattttggtc atgagattat   4020
   caaaaaggat cttcacctag atccttttaa aggccggccg cggccgcgca aagtcccgct 4080
   tcgtgaaaat tttcgtgccg cgtgattttc cgccaaaaac tttaacgaac gttcgttata 4140
   atggtgtcat gaccttcacg acgaagtact aaaattggcc cgaatcatca gctatggatc 4200
   tctctgatgt cgcgctggag tccgacgcgc tcgatgctgc cgtcgattta aaaacggtga 4260
   tcggattttt ccgagctctc gatacgacgg acgcgccagc atcacgagac tgggccagtg 4320
   ccgcgagcga cctagaaact ctcgtggcgg atcttgagga gctggctgac gagctgcgtg 4380
   ctcggccagc gccaggagga cgcacagtag tggaggatgc aatcagttgc gcctactgcg 4440
   gtggcctgat tcctccccgg cctgacccgc gaggacggcg cgcaaaatat tgctcagatg 4500
   cgtgtcgtgc cgcagccagc cgcgagcgcg ccaacaaacg ccacgccgag gagctggagg 4560
   cggctaggtc gcaaatggcg ctggaagtgc gtcccccgag cgaaattttg gccatggtcg 4620
   tcacagagct ggaagcggca gcgagaatta tcgcgatcgt ggcggtgccc gcaggcatga 4680
   caaacatcgt aaatgccgcg tttcgtgtgc cgtggccgcc caggacgtgt cagcgccgcc 4740
   accacctgca ccgaatcggc agcagcgtcg cgcgtcgaaa aagcgcacag gcggcaagaa 4800
   gcgataagct gcacgaatac ctgaaaaatg ttgaacgccc cgtgagcggt aactcacagg 4860
   gcgtcggcta acccccagtc caaacctggg agaaagcgct caaaaatgac tctagcggat 4920
   tcacgagaca ttgacacacc ggcctggaaa ttttccgctg atctgttcga cacccatccc 4980
   gagctcgcgc tgcgatcacg tggctggacg agcgaagacc gccgcgaatt cctcgctcac 5040
   ctgggcagag aaaatttcca gggcagcaag acccgcgact tcgccagcgc ttggatcaaa 5100
   gacccggaca cggagaaaca cagccgaagt tataccgagt tggttcaaaa tcgcttgccc 5160
   ggtgccagta tgttgctctg acgcacgcgc agcacgcagc cgtgcttgtc ctggacattg 5220
   atgtgccgag ccaccaggcc ggcgggaaaa tcgagcacgt aaaccccgag gtctacgcga 5280
   ttttggagcg ctgggcacgc ctggaaaaag cgccagcttg gatcggcgtg aatccactga 5340
   gcgggaaatg ccagctcatc tggctcattg atccggtgta tgccgcagca ggcatgagca 5400
   gcccgaatat gcgcctgctg gctgcaacga ccgaggaaat gacccgcgtt ttcggcgctg 5460
   accaggcttt ttcacatagg ctgagccgtg gccactgcac tctccgacga tcccagccgt 5520
   accgctggca tgcccagcac aatcgcgtgg atcgcctagc tgatcttatg gaggttgctc 5580
   gcatgatctc aggcacagaa aaacctaaaa aacgctatga gcaggagttt tctagcggac 5640
   gggcacgtat cgaagcggca agaaaagcca ctgcggaagc aaaagcactt gccacgcttg 5700
   aagcaagcct gccgagcgcc gctgaagcgt ctggagagct gatcgacggc gtccgtgtcc 5760
   tctggactgc tccagggcgt gccgcccgtg atgagacggc ttttcgccac gctttgactg 5820
   tgggatacca gttaaaagcg gctggtgagc gcctaaaaga caccaagggt catcgagcct 5880
   acgagcgtgc ctacaccgtc gctcaggcgg tcggaggagg ccgtgagcct gatctgccgc 5940
   cggactgtga ccgccagacg gattggccgc gacgtgtgcg cggctacgtc gctaaaggcc 6000
   agccagtcgt ccctgctcgt cagacagaga cgcagagcca gccgaggcga aaagctctgg 6060
   ccactatggg aagacgtggc ggtaaaaagg ccgcagaacg ctggaaagac ccaaacagtg 6120
   agtacgcccg agcacagcga gaaaaactag ctaagtccag tcaacgacaa gctaggaaag 6180
   ctaaaggaaa tcgcttgacc attgcaggtt ggtttatgac tgttgaggga gagactggct 6240
   cgtggccgac aatcaatgaa gctatgtctg aatttagcgt gtcacgtcag accgtgaata 6300
   gagcacttaa ggtctgcggg cattgaactt ccacgaggac gccgaaagct tcccagtaaa 6360
   tgtgccatct cgtaggcaga aaacggttcc cccgtagggt ctctctcttg gcctcctttc   6420
   taggtcgggc tgattgctct tgaagctctc taggggggct cacaccatag gcagataacg   6480
   ttccccaccg gctcgcctcg taagcgcaca aggactgctc ccaaagatct tcaaagccac   6540
   tgccgcgact gccttcgcga agccttgccc cgcggaaatt tcctccaccg agttcgtgca   6600
   cacccctatg ccaagcttct ttcaccctaa attcgagaga ttggattctt accgtggaaa   6660
   ttcttcgcaa aaatcgtccc ctgatcgccc ttgcgacgtt ggcgtcggtg ccgctggttg   6720
   cgcttggctt gaccgacttg atcagcggcc gctcgattt                           6759
<210> 60
   <211> 6780
   <212> DNA
   <213> Artificial Sequence
<220>
   <223> Plasmid
   <220>
   <221> misc_feature
   <223> internal designation: H502 (pCliK5MCS Peftu Psod Pefts metA)
<400> 60
   aaatctcgag ggccgttacc ctgcgaatgt ccacagggta gctggtagtt tgaaaatcaa 60
   cgccgttgcc cttaggattc agtaactggc acattttgta atgcgctaga tctgtgtgct 120
   cagtcttcca ggctgcttat cacagtgaaa gcaaaaccaa ttcgtggctg cgaaagtcgt 180
   agccacacta gtagctgcca attattccgg gcttgtgacc cgctacccga taaataggtc 240
   ggctgaaaaa tttcgttgca atatcaacaa aaaggcctat cattgggagg tgtcgcacca 300
   agtacttttg cgaagcgcca tctgacggat tttcaaaaga tgtatatgct cggtgcgggg 360
   taccccccca cgacaatgga actttgactt ttaaaatttc atcgccgtgg gggctttttg 420
   ggcagccagc ccgccgtgtc gcaacgtaat cgactgaata cctgtacgat cactttttag 480
   acgggcgggt agggctactg tgccctaacc taagcttgta aagcattaat tatccataca 540
   taaggaggat cgccccgtaa tgcccaccct cgcgccttca ggtcaacttg aaatccaagc 600
   gatcggtgat gtctccaccg aagccggagc aatcattaca aacgctgaaa tcgcctatca 660
   ccgctggggt gaataccgcg tagataaaga aggacgcagc aatgtcgttc tcatcgaaca 720
   cgccctcact ggagattcca acgcagccga ttggtgggct gacttgctcg gtcccggcaa 780
   agccatcaac actgatattt actgcgtgat ctgtaccaac gtcatcggtg gttgcaacgg 840
   ttccaccgga cctggctcca tgcatccaga tggaaatttc tggggtaatc gcttccccgc 900
   cacgtccatt cgtgatcagg taaacgccga aaaacaattc ctcgacgcac tcggcatcac 960
   cacggtcgcc gcagtacttg gtggttccat gggtggtgcc cgcaccctag agtgggccgc 1020
   aatgtaccca gaaactgttg gcgcagctgc tgttcttgca gtttctgcac gcgccagcgc 1080
   ctggcaaatc ggcattcaat ccgcccaaat taaggcgatt gaaaacgacc accactggca 1140
   cgaaggcaac tactacgaat ccggctgcaa cccagccacc ggactcggcg ccgcccgacg 1200
   catcgcccac ctcacctacc gtggcgaact agaaatcgac gaacgcttcg gcaccaaagc 1260
   ccaaaagaac gaaaacccac tcggtcccta ccgcaagccc gaccagcgct tcgccgtgga 1320
   atcctacttg gactaccaag cagacaagct agtacagcgt ttcgacgccg gctcctacgt 1380
   cttgctcacc gacgccctca accgccacga cattggtcgc gaccgcggag gcctcaacaa 1440
   ggcactcgaa tccatcaaag ttccagtcct tgtcgcaggc gtagataccg atattttgta 1500
   cccctaccac cagcaagaac acctctccag aaacctggga aatctactgg caatggcaaa 1560
   aatcgtatcc cctgtcggcc acgatgcttt cctcaccgaa agccgccaaa tggatcgcat 1620
   cgtgaggaac ttcttcagcc tcatctcccc agacgaagac aacccttcga cctacatcga 1680
   gttctacatc taagtcgaca tcgatgctct tctgcgttaa ttaacaattg ggatcctcta 1740
   gagttctgtg aaaaacaccg tggggcagtt tctgcttcgc ggtgtttttt atttgtgggg 1800
   cactagaccc gggatttaaa tcgctagcgg gctgctaaag gaagcggaac acgtagaaag 1860
   ccagtccgca gaaacggtgc tgaccccgga tgaatgtcag ctactgggct atctggacaa 1920
   gggaaaacgc aagcgcaaag agaaagcagg tagcttgcag tgggcttaca tggcgatagc 1980
   tagactgggc ggttttatgg acagcaagcg aaccggaatt gccagctggg gcgccctctg 2040
   gtaaggttgg gaagccctgc aaagtaaact ggatggcttt cttgccgcca aggatctgat 2100
   ggcgcagggg atcaagatct gatcaagaga caggatgagg atcgtttcgc atgattgaac 2160
   aagatggatt gcacgcaggt tctccggccg cttgggtgga gaggctattc ggctatgact 2220
   gggcacaaca gacaatcggc tgctctgatg ccgccgtgtt ccggctgtca gcgcaggggc 2280
   gcccggttct ttttgtcaag accgacctgt ccggtgccct gaatgaactg caggacgagg 2340
   cagcgcggct atcgtggctg gccacgacgg gcgttccttg cgcagctgtg ctcgacgttg 2400
   tcactgaagc gggaagggac tggctgctat tgggcgaagt gccggggcag gatctcctgt 2460
   catctcacct tgctcctgcc gagaaagtat ccatcatggc tgatgcaatg cggcggctgc 2520
   atacgcttga tccggctacc tgcccattcg accaccaagc gaaacatcgc atcgagcgag 2580
   cacgtactcg gatggaagcc ggtcttgtcg atcaggatga tctggacgaa gagcatcagg 2640
   ggctcgcgcc agccgaactg ttcgccaggc tcaaggcgcg catgcccgac ggcgaggatc 2700
   tcgtcgtgac ccatggcgat gcctgcttgc cgaatatcat ggtggaaaat ggccgctttt 2760
   ctggattcat cgactgtggc cggctgggtg tggcggaccg ctatcaggac atagcgttgg 2820
   ctacccgtga tattgctgaa gagcttggcg gcgaatgggc tgaccgcttc ctcgtgcttt 2880
   acggtatcgc cgctcccgat tcgcagcgca tcgccttcta tcgccttctt gacgagttct 2940
   tctgagcggg actctggggt tcgaaatgac cgaccaagcg acgcccaacc tgccatcacg 3000
   agatttcgat tccaccgccg ccttctatga aaggttgggc ttcggaatcg ttttccggga 3060
   cgccggctgg atgatcctcc agcgcgggga tctcatgctg gagttcttcg cccacgctag 3120
   cggcgcgccg gccggcccgg tgtgaaatac cgcacagatg cgtaaggaga aaataccgca 3180
   tcaggcgctc ttccgcttcc tcgctcactg actcgctgcg ctcggtcgtt cggctgcggc 3240
   gagcggtatc agctcactca aaggcggtaa tacggttatc cacagaatca ggggataacg 3300
   caggaaagaa catgtgagca aaaggccagc aaaaggccag gaaccgtaaa aaggccgcgt 3360
   tgctggcgtt tttccatagg ctccgccccc ctgacgagca tcacaaaaat cgacgctcaa 3420
   gtcagaggtg gcgaaacccg acaggactat aaagatacca ggcgtttccc cctggaagct 3480
   ccctcgtgcg ctctcctgtt ccgaccctgc cgcttaccgg atacctgtcc gcctttctcc 3540
   cttcgggaag cgtggcgctt tctcatagct cacgctgtag gtatctcagt tcggtgtagg 3600
   tcgttcgctc caagctgggc tgtgtgcacg aaccccccgt tcagcccgac cgctgcgcct 3660
   tatccggtaa ctatcgtctt gagtccaacc cggtaagaca cgacttatcg ccactggcag 3720
   cagccactgg taacaggatt agcagagcga ggtatgtagg cggtgctaca gagttcttga 3780
   agtggtggcc taactacggc tacactagaa ggacagtatt tggtatctgc gctctgctga 3840
   agccagttac cttcggaaaa agagttggta gctcttgatc cggcaaacaa accaccgctg 3900
   gtagcggtgg tttttttgtt tgcaagcagc agattacgcg cagaaaaaaa ggatctcaag 3960
   aagatccttt gatcttttct acggggtctg acgctcagtg gaacgaaaac tcacgttaag 4020
   ggattttggt catgagatta tcaaaaagga tcttcaccta gatcctttta aaggccggcc 4080
   gcggccgcgc aaagtcccgc ttcgtgaaaa ttttcgtgcc gcgtgatttt ccgccaaaaa 4140
   ctttaacgaa cgttcgttat aatggtgtca tgaccttcac gacgaagtac taaaattggc 4200
   ccgaatcatc agctatggat ctctctgatg tcgcgctgga gtccgacgcg ctcgatgctg 4260
   ccgtcgattt aaaaacggtg atcggatttt tccgagctct cgatacgacg gacgcgccag 4320
   catcacgaga ctgggccagt gccgcgagcg acctagaaac tctcgtggcg gatcttgagg 4380
   agctggctga cgagctgcgt gctcggccag cgccaggagg acgcacagta gtggaggatg 4440
   caatcagttg cgcctactgc ggtggcctga ttcctccccg gcctgacccg cgaggacggc 4500
   gcgcaaaata ttgctcagat gcgtgtcgtg ccgcagccag ccgcgagcgc gccaacaaac 4560
   gccacgccga ggagctggag gcggctaggt cgcaaatggc gctggaagtg cgtcccccga 4620
   gcgaaatttt ggccatggtc gtcacagagc tggaagcggc agcgagaatt atcgcgatcg 4680
   tggcggtgcc cgcaggcatg acaaacatcg taaatgccgc gtttcgtgtg ccgtggccgc 4740
   ccaggacgtg tcagcgccgc caccacctgc accgaatcgg cagcagcgtc gcgcgtcgaa 4800
   aaagcgcaca ggcggcaaga agcgataagc tgcacgaata cctgaaaaat gttgaacgcc 4860
   ccgtgagcgg taactcacag ggcgtcggct aacccccagt ccaaacctgg gagaaagcgc 4920
   tcaaaaatga ctctagcgga ttcacgagac attgacacac cggcctggaa attttccgct 4980
   gatctgttcg acacccatcc cgagctcgcg ctgcgatcac gtggctggac gagcgaagac 5040
   cgccgcgaat tcctcgctca cctgggcaga gaaaatttcc agggcagcaa gacccgcgac 5100
   ttcgccagcg cttggatcaa agacccggac acggagaaac acagccgaag ttataccgag 5160
   ttggttcaaa atcgcttgcc cggtgccagt atgttgctct gacgcacgcg cagcacgcag 5220
   ccgtgcttgt cctggacatt gatgtgccga gccaccaggc cggcgggaaa atcgagcacg 5280
   taaaccccga ggtctacgcg attttggagc gctgggcacg cctggaaaaa gcgccagctt 5340
   ggatcggcgt gaatccactg agcgggaaat gccagctcat ctggctcatt gatccggtgt 5400
   atgccgcagc aggcatgagc agcccgaata tgcgcctgct ggctgcaacg accgaggaaa 5460
   tgacccgcgt tttcggcgct gaccaggctt tttcacatag gctgagccgt ggccactgca 5520
   ctctccgacg atcccagccg taccgctggc atgcccagca caatcgcgtg gatcgcctag 5580
   ctgatcttat ggaggttgct cgcatgatct caggcacaga aaaacctaaa aaacgctatg 5640
   agcaggagtt ttctagcgga cgggcacgta tcgaagcggc aagaaaagcc actgcggaag 5700
   caaaagcact tgccacgctt gaagcaagcc tgccgagcgc cgctgaagcg tctggagagc 5760
   tgatcgacgg cgtccgtgtc ctctggactg ctccagggcg tgccgcccgt gatgagacgg 5820
   cttttcgcca cgctttgact gtgggatacc agttaaaagc ggctggtgag cgcctaaaag 5880
   acaccaaggg tcatcgagcc tacgagcgtg cctacaccgt cgctcaggcg gtcggaggag 5940
   gccgtgagcc tgatctgccg ccggactgtg accgccagac ggattggccg cgacgtgtgc 6000
   gcggctacgt cgctaaaggc cagccagtcg tccctgctcg tcagacagag acgcagagcc 6060
   agccgaggcg aaaagctctg gccactatgg gaagacgtgg cggtaaaaag gccgcagaac 6120
   gctggaaaga cccaaacagt gagtacgccc gagcacagcg agaaaaacta gctaagtcca 6180
   gtcaacgaca agctaggaaa gctaaaggaa atcgcttgac cattgcaggt tggtttatga 6240
   ctgttgaggg agagactggc tcgtggccga caatcaatga agctatgtct gaatttagcg 6300
   tgtcacgtca gaccgtgaat agagcactta aggtctgcgg gcattgaact tccacgagga 6360
   cgccgaaagc ttcccagtaa atgtgccatc tcgtaggcag aaaacggttc ccccgtaggg 6420
   tctctctctt ggcctccttt ctaggtcggg ctgattgctc ttgaagctct ctaggggggc 6480
   tcacaccata ggcagataac gttccccacc ggctcgcctc gtaagcgcac aaggactgct 6540
   cccaaagatc ttcaaagcca ctgccgcgac tgccttcgcg aagccttgcc ccgcggaaat 6600
   ttcctccacc gagttcgtgc acacccctat gccaagcttc tttcacccta aattcgagag 6660
   attggattct taccgtggaa attcttcgca aaaatcgtcc cctgatcgcc cttgcgacgt 6720
   tggcgtcggt gccgctggtt gcgcttggct tgaccgactt gatcagcggc cgctcgattt 6780
<210> 61
   <211> 35
   <212> DNA
   <213> Artificial Sequence
<220>
   <223> Primer
<400> 61
   gagagagaga cgcgtcccag tggctgagac gcatc 35
<210> 62
   <211> 34
   <212> DNA
   <213> Artificial sequence
<220>
   <223> Primer
<400> 62

   ctctctctgt cgacgaattc aatcttacgg cctg 34
<210> 63
   <211> 4323
   <212> DNA
   <213> Artificial Sequence
<220>
   <223> Plasmid
   <220>
   <221> misc_feature
   <223> internal designation: pCLiK5 MCS integrativ SacB (also referred t o as pcIS)
<220>
   <221> misc_feature
   <222> (457)..(1248)
   <223> KanR
<220>
   <221> misc_feature
   <222> (3840)..(4302)
   <223> PsacB (complementary)
<220>
   <221> misc_feature
   <222> (2418)..(3839)
   <223> SacB (complementary)
<220>
   <221> misc_feature
   <222> (1515)..(2375)
   <223> Ori-Ec (pMB) (complementary)
<400> 63
   tcgagaggcc tgacgtcggg cccggtacca cgcgtcatat gactagttcg gacctaggga      60
   tatcgtcgac atcgatgctc ttctgcgtta attaacaatt gggatcctct agacccggga     120
   tttaaatcgc tagcgggctg ctaaaggaag cggaacacgt agaaagccag tccgcagaaa     180
   cggtgctgac cccggatgaa tgtcagctac tgggctatct ggacaaggga aaacgcaagc     240
   gcaaagagaa agcaggtagc ttgcagtggg cttacatggc gatagctaga ctgggcggtt     300
   ttatggacag caagcgaacc ggaattgcca gctggggcgc cctctggtaa ggttgggaag     360
   ccctgcaaag taaactggat ggctttcttg ccgccaagga tctgatggcg caggggatca     420
   agatctgatc aagagacagg atgaggatcg tttcgcatga ttgaacaaga tggattgcac     480
   gcaggttctc cggccgcttg ggtggagagg ctattcggct atgactgggc acaacagaca     540
   atcggctgct ctgatgccgc cgtgttccgg ctgtcagcgc aggggcgccc ggttcttttt     600
   gtcaagaccg acctgtccgg tgccctgaat gaactgcagg acgaggcagc gcggctatcg 660
   tggctggcca cgacgggcgt tccttgcgca gctgtgctcg acgttgtcac tgaagcggga 720
   agggactggc tgctattggg cgaagtgccg gggcaggatc tcctgtcatc tcaccttgct 780
   cctgccgaga aagtatccat catggctgat gcaatgcggc ggctgcatac gcttgatccg 840
   gctacctgcc cattcgacca ccaagcgaaa catcgcatcg agcgagcacg tactcggatg 900
   gaagccggtc ttgtcgatca ggatgatctg gacgaagagc atcaggggct cgcgccagcc 960
   gaactgttcg ccaggctcaa ggcgcgcatg cccgacggcg aggatctcgt cgtgacccat 1020
   ggcgatgcct gcttgccgaa tatcatggtg gaaaatggcc gcttttctgg attcatcgac 1080
   tgtggccggc tgggtgtggc ggaccgctat caggacatag cgttggctac ccgtgatatt 1140
   gctgaagagc ttggcggcga atgggctgac cgcttcctcg tgctttacgg tatcgccgct 1200
   cccgattcgc agcgcatcgc cttctatcgc cttcttgacg agttcttctg agcgggactc 1260
   tggggttcga aatgaccgac caagcgacgc ccaacctgcc atcacgagat ttcgattcca 1320
   ccgccgcctt ctatgaaagg ttgggcttcg gaatcgtttt ccgggacgcc ggctggatga 1380
   tcctccagcg cggggatctc atgctggagt tcttcgccca cgctagcggc gcgccggccg 1440
   gcccggtgtg aaataccgca cagatgcgta aggagaaaat accgcatcag gcgctcttcc 1500
   gcttcctcgc tcactgactc gctgcgctcg gtcgttcggc tgcggcgagc ggtatcagct 1560
   cactcaaagg cggtaatacg gttatccaca gaatcagggg ataacgcagg aaagaacatg 1620
   tgagcaaaag gccagcaaaa ggccaggaac cgtaaaaagg ccgcgttgct ggcgtttttc 1680
   cataggctcc gcccccctga cgagcatcac aaaaatcgac gctcaagtca gaggtggcga 1740
   aacccgacag gactataaag ataccaggcg tttccccctg gaagctccct cgtgcgctct 1800
   cctgttccga ccctgccgct taccggatac ctgtccgcct ttctcccttc gggaagcgtg 1860
   gcgctttctc atagctcacg ctgtaggtat ctcagttcgg tgtaggtcgt tcgctccaag 1920
   ctgggctgtg tgcacgaacc ccccgttcag cccgaccgct gcgccttatc cggtaactat 1980
   cgtcttgagt ccaacccggt aagacacgac ttatcgccac tggcagcagc cactggtaac 2040
   aggattagca gagcgaggta tgtaggcggt gctacagagt tcttgaagtg gtggcctaac 2100
   tacggctaca ctagaaggac agtatttggt atctgcgctc tgctgaagcc agttaccttc 2160
   ggaaaaagag ttggtagctc ttgatccggc aaacaaacca ccgctggtag cggtggtttt 2220
   tttgtttgca agcagcagat tacgcgcaga aaaaaaggat ctcaagaaga tcctttgatc 2280
   ttttctacgg ggtctgacgc tcagtggaac gaaaactcac gttaagggat tttggtcatg 2340
   agattatcaa aaaggatctt cacctagatc cttttaaagg ccggccgcgg ccgccatcgg 2400
   cattttcttt tgcgttttta tttgttaact gttaattgtc cttgttcaag gatgctgtct 2460
   ttgacaacag atgttttctt gcctttgatg ttcagcagga agctcggcgc aaacgttgat 2520
   tgtttgtctg cgtagaatcc tctgtttgtc atatagcttg taatcacgac attgtttcct 2580
   ttcgcttgag gtacagcgaa gtgtgagtaa gtaaaggtta catcgttagg atcaagatcc 2640
   atttttaaca caaggccagt tttgttcagc ggcttgtatg ggccagttaa agaattagaa 2700
   acataaccaa gcatgtaaat atcgttagac gtaatgccgt caatcgtcat ttttgatccg 2760
   cgggagtcag tgaacaggta ccatttgccg ttcattttaa agacgttcgc gcgttcaatt 2820
   tcatctgtta ctgtgttaga tgcaatcagc ggtttcatca cttttttcag tgtgtaatca 2880
   tcgtttagct caatcatacc gagagcgccg tttgctaact cagccgtgcg ttttttatcg   2940
   ctttgcagaa gtttttgact ttcttgacgg aagaatgatg tgcttttgcc atagtatgct   3000
   ttgttaaata aagattcttc gccttggtag ccatcttcag ttccagtgtt tgcttcaaat   3060
   actaagtatt tgtggccttt atcttctacg tagtgaggat ctctcagcgt atggttgtcg   3120
   cctgagctgt agttgccttc atcgatgaac tgctgtacat tttgatacgt ttttccgtca    3180
   ccgtcaaaga ttgatttata atcctctaca ccgttgatgt tcaaagagct gtctgatgct   3240
   gatacgttaa cttgtgcagt tgtcagtgtt tgtttgccgt aatgtttacc ggagaaatca   3300
   gtgtagaata aacggatttt tccgtcagat gtaaatgtgg ctgaacctga ccattcttgt   3360
   gtttggtctt ttaggataga atcatttgca tcgaatttgt cgctgtcttt aaagacgcgg   3420
   ccagcgtttt tccagctgtc aatagaagtt tcgccgactt tttgatagaa catgtaaatc   3480
   gatgtgtcat ccgcattttt aggatctccg gctaatgcaa agacgatgtg gtagccgtga   3540
   tagtttgcga cagtgccgtc agcgttttgt aatggccagc tgtcccaaac gtccaggcct   3600
   tttgcagaag agatattttt aattgtggac gaatcaaatt cagaaacttg atatttttca   3660
   tttttttgct gttcagggat ttgcagcata tcatggcgtg taatatggga aatgccgtat   3720
   gtttccttat atggcttttg gttcgtttct ttcgcaaacg cttgagttgc gcctcctgcc   3780
   agcagtgcgg tagtaaaggt taatactgtt gcttgttttg caaacttttt gatgttcatc   3840
   gttcatgtct ccttttttat gtactgtgtt agcggtctgc ttcttccagc cctcctgttt    3900
   gaagatggca agttagttac gcacaataaa aaaagaccta aaatatgtaa ggggtgacgc   3960
   caaagtatac actttgccct ttacacattt taggtcttgc ctgctttatc agtaacaaac   4020
   ccgcgcgatt tacttttcga cctcattcta ttagactctc gtttggattg caactggtct   4080
   attttcctct tttgtttgat agaaaatcat aaaaggattt gcagactacg ggcctaaaga   4140
   actaaaaaat ctatctgttt cttttcattc tctgtatttt ttatagtttc tgttgcatgg   4200
   gcataaagtt gcctttttaa tcacaattca gaaaatatca taatatctca tttcactaaa   4260
   taatagtgaa cggcaggtat atgtgatggg ttaaaaagga tcggcggccg ctcgatttaa   4320
   atc                                                                 4323
<210> 64
   <211> 5860
   <212> DNA
   <213> Artificial Sequence
<220>
   <223> Plasmid
   <220>
   <221> misc_feature
   <222> (155)..(1420)
   <223> lysC
<220>
   <221> misc_feature
   <222> (3935)..(5356)
   <223> sacB (Bacillus subtilis)
<220>
   <221> misc_feature
   <222> (5357)..(5819)
   <223> Promotor sacB complement
<220>
   <221> misc_feature
   <222> (1974)..(2765)
   <223> kanR
<220>
   <221> misc_feature
   <222> (3032)..(3892)
   <223> Ori-EC complement
<220>
   <221> misc_feature
   <222> (3913)..(3934)
   <223> sacB downstream region (complement)
<400> 64
   cccggtacca cgcgtcccag tggctgagac gcatccgcta aagccccagg aaccctgtgc     60
   agaaagaaaa cactcctctg gctaggtaga cacagtttat aaaggtagag ttgagcgggt    120
   aactgtcagc acgtagatcg aaaggtgcac aaaggtggcc ctggtcgtac agaaatatgg    180
   cggttcctcg cttgagagtg cggaacgcat tagaaacgtc gctgaacgga tcgttgccac    240
   caagaaggct ggaaatgatg tcgtggttgt ctgctccgca atgggagaca ccacggatga    300
   acttctagaa cttgcagcgg cagtgaatcc cgttccgcca gctcgtgaaa tggatatgct    360
   cctgactgct ggtgagcgta tttctaacgc tctcgtcgcc atggctattg agtcccttgg    420
   cgcagaagcc caatctttca cgggctctca ggctggtgtg ctcaccaccg agcgccacgg    480
   aaacgcacgc attgttgatg tcactccagg tcgtgtgcgt gaagcactcg atgagggcaa    540
   gatctgcatt gttgctggtt tccagggtgt taataaagaa acccgcgatg tcaccacgtt    600
   gggtcgtggt ggttctgaca ccactgcagt tgcgttggca gctgctttga acgctgatgt    660
   gtgtgagatt tactcggacg ttgacggtgt gtataccgct gacccgcgca tcgttcctaa    720
   tgcacagaag ctggaaaagc tcagcttcga agaaatgctg gaacttgctg ctgttggctc    780
   caagattttg gtgctgcgca gtgttgaata cgctcgtgca ttcaatgtgc cacttcgcgt    840
   acgctcgtct tatagtaatg atcccggcac tttgattgcc ggctctatgg aggatattcc    900
   tgtggaagaa gcagtcctta ccggtgtcgc aaccgacaag tccgaagcca aagtaaccgt    960
   tctgggtatt tccgataagc caggcgaggc tgcgaaggtt ttccgtgcgt tggctgatgc   1020
   agaaatcaac attgacatgg ttctgcagaa cgtctcttct gtagaagacg gcaccaccga   1080
   catcaccttc acctgccctc gttccgacgg ccgccgcgcg atggagatct tgaagaagct   1140
   tcaggttcag ggcaactgga ccaatgtgct ttacgacgac caggtcggca aagtctccct   1200
   cgtgggtgct ggcatgaagt ctcacccagg tgttaccgca gagttcatgg aagctctgcg   1260
   cgatgtcaac gtgaacatcg aattgatttc cacctctgag attcgtattt ccgtgctgat   1320
   ccgtgaagat gatctggatg ctgctgcacg tgcattgcat gagcagttcc agctgggcgg   1380
   cgaagacgaa gccgtcgttt atgcaggcac cggacgctaa agttttaaag gagtagtttt   1440
   acaatgacca ccatcgcagt tgttggtgca accggccagg tcggccaggt tatgcgcacc   1500
   cttttggaag agcgcaattt cccagctgac actgttcgtt tctttgcttc cccacgttcc   1560
   gcaggccgta agattgaatt cgtcgacatc gatgctcttc tgcgttaatt aacaattggg   1620
   atcctctaga cccgggattt aaatcgctag cgggctgcta aaggaagcgg aacacgtaga   1680
   aagccagtcc gcagaaacgg tgctgacccc ggatgaatgt cagctactgg gctatctgga   1740
   caagggaaaa cgcaagcgca aagagaaagc aggtagcttg cagtgggctt acatggcgat   1800
   agctagactg ggcggtttta tggacagcaa gcgaaccgga attgccagct ggggcgccct   1860
   ctggtaaggt tgggaagccc tgcaaagtaa actggatggc tttcttgccg ccaaggatct   1920
   gatggcgcag gggatcaaga tctgatcaag agacaggatg aggatcgttt cgcatgattg   1980
   aacaagatgg attgcacgca ggttctccgg ccgcttgggt ggagaggcta ttcggctatg   2040
   actgggcaca acagacaatc ggctgctctg atgccgccgt gttccggctg tcagcgcagg   2100
   ggcgcccggt tctttttgtc aagaccgacc tgtccggtgc cctgaatgaa ctgcaggacg   2160
   aggcagcgcg gctatcgtgg ctggccacga cgggcgttcc ttgcgcagct gtgctcgacg   2220
   ttgtcactga agcgggaagg gactggctgc tattgggcga agtgccgggg caggatctcc   2280
   tgtcatctca ccttgctcct gccgagaaag tatccatcat ggctgatgca atgcggcggc   2340
   tgcatacgct tgatccggct acctgcccat tcgaccacca agcgaaacat cgcatcgagc   2400
   gagcacgtac tcggatggaa gccggtcttg tcgatcagga tgatctggac gaagagcatc   2460
   aggggctcgc gccagccgaa ctgttcgcca ggctcaaggc gcgcatgccc gacggcgagg   2520
   atctcgtcgt gacccatggc gatgcctgct tgccgaatat catggtggaa aatggccgct   2580
   tttctggatt catcgactgt ggccggctgg gtgtggcgga ccgctatcag gacatagcgt   2640
   tggctacccg tgatattgct gaagagcttg gcggcgaatg ggctgaccgc ttcctcgtgc   2700
   tttacggtat cgccgctccc gattcgcagc gcatcgcctt ctatcgcctt cttgacgagt   2760
   tcttctgagc gggactctgg ggttcgaaat gaccgaccaa gcgacgccca acctgccatc   2820
   acgagatttc gattccaccg ccgccttcta tgaaaggttg ggcttcggaa tcgttttccg   2880
   ggacgccggc tggatgatcc tccagcgcgg ggatctcatg ctggagttct tcgcccacgc   2940
   tagcggcgcg ccggccggcc cggtgtgaaa taccgcacag atgcgtaagg agaaaatacc   3000
   gcatcaggcg ctcttccgct tcctcgctca ctgactcgct gcgctcggtc gttcggctgc   3060
   ggcgagcggt atcagctcac tcaaaggcgg taatacggtt atccacagaa tcaggggata   3120
   acgcaggaaa gaacatgtga gcaaaaggcc agcaaaaggc caggaaccgt aaaaaggccg   3180
   cgttgctggc gtttttccat aggctccgcc cccctgacga gcatcacaaa aatcgacgct 3240
   caagtcagag gtggcgaaac ccgacaggac tataaagata ccaggcgttt ccccctggaa 3300
   gctccctcgt gcgctctcct gttccgaccc tgccgcttac cggatacctg tccgcctttc 3360
   tcccttcggg aagcgtggcg ctttctcata gctcacgctg taggtatctc agttcggtgt 3420
   aggtcgttcg ctccaagctg ggctgtgtgc acgaaccccc cgttcagccc gaccgctgcg 3480
   ccttatccgg taactatcgt cttgagtcca acccggtaag acacgactta tcgccactgg 3540
   cagcagccac tggtaacagg attagcagag cgaggtatgt aggcggtgct acagagttct 3600
   tgaagtggtg gcctaactac ggctacacta gaaggacagt atttggtatc tgcgctctgc 3660
   tgaagccagt taccttcgga aaaagagttg gtagctcttg atccggcaaa caaaccaccg 3720
   ctggtagcgg tggttttttt gtttgcaagc agcagattac gcgcagaaaa aaaggatctc 3780
   aagaagatcc tttgatcttt tctacggggt ctgacgctca gtggaacgaa aactcacgtt 3840
   aagggatttt ggtcatgaga ttatcaaaaa ggatcttcac ctagatcctt ttaaaggccg 3900
   gccgcggccg ccatcggcat tttcttttgc gtttttattt gttaactgtt aattgtcctt 3960
   gttcaaggat gctgtctttg acaacagatg ttttcttgcc tttgatgttc agcaggaagc 4020
   tcggcgcaaa cgttgattgt ttgtctgcgt agaatcctct gtttgtcata tagcttgtaa 4080
   tcacgacatt gtttcctttc gcttgaggta cagcgaagtg tgagtaagta aaggttacat 4140
   cgttaggatc aagatccatt tttaacacaa ggccagtttt gttcagcggc ttgtatgggc 4200
   cagttaaaga attagaaaca taaccaagca tgtaaatatc gttagacgta atgccgtcaa 4260
   tcgtcatttt tgatccgcgg gagtcagtga acaggtacca tttgccgttc attttaaaga 4320
   cgttcgcgcg ttcaatttca tctgttactg tgttagatgc aatcagcggt ttcatcactt 4380
   ttttcagtgt gtaatcatcg tttagctcaa tcataccgag agcgccgttt gctaactcag 4440
   ccgtgcgttt tttatcgctt tgcagaagtt tttgactttc ttgacggaag aatgatgtgc 4500
   ttttgccata gtatgctttg ttaaataaag attcttcgcc ttggtagcca tcttcagttc 4560
   cagtgtttgc ttcaaatact aagtatttgt ggcctttatc ttctacgtag tgaggatctc 4620
   tcagcgtatg gttgtcgcct gagctgtagt tgccttcatc gatgaactgc tgtacatttt 4680
   gatacgtttt tccgtcaccg tcaaagattg atttataatc ctctacaccg ttgatgttca 4740
   aagagctgtc tgatgctgat acgttaactt gtgcagttgt cagtgtttgt ttgccgtaat 4800
   gtttaccgga gaaatcagtg tagaataaac ggatttttcc gtcagatgta aatgtggctg 4860
   aacctgacca ttcttgtgtt tggtctttta ggatagaatc atttgcatcg aatttgtcgc 4920
   tgtctttaaa gacgcggcca gcgtttttcc agctgtcaat agaagtttcg ccgacttttt 4980
   gatagaacat gtaaatcgat gtgtcatccg catttttagg atctccggct aatgcaaaga 5040
   cgatgtggta gccgtgatag tttgcgacag tgccgtcagc gttttgtaat ggccagctgt 5100
   cccaaacgtc caggcctttt gcagaagaga tatttttaat tgtggacgaa tcaaattcag 5160
   aaacttgata tttttcattt ttttgctgtt cagggatttg cagcatatca tggcgtgtaa 5220
   tatgggaaat gccgtatgtt tccttatatg gcttttggtt cgtttctttc gcaaacgctt 5280
   gagttgcgcc tcctgccagc agtgcggtag taaaggttaa tactgttgct tgttttgcaa 5340
   actttttgat gttcatcgtt catgtctcct tttttatgta ctgtgttagc ggtctgcttc 5400
   ttccagccct cctgtttgaa gatggcaagt tagttacgca caataaaaaa agacctaaaa 5460
   tatgtaaggg gtgacgccaa agtatacact ttgcccttta cacattttag gtcttgcctg 5520
   ctttatcagt aacaaacccg cgcgatttac ttttcgacct cattctatta gactctcgtt 5580
   tggattgcaa ctggtctatt ttcctctttt gtttgataga aaatcataaa aggatttgca 5640
   gactacgggc ctaaagaact aaaaaatcta tctgtttctt ttcattctct gtatttttta 5700
   tagtttctgt tgcatgggca taaagttgcc tttttaatca caattcagaa aatatcataa 5760
   tatctcattt cactaaataa tagtgaacgg caggtatatg tgatgggtta aaaaggatcg 5820
   gcggccgctc gatttaaatc tcgagaggcc tgacgtcggg 5860
<210> 65
   <211> 38
   <212> DNA
   <213> Artificial Sequence
<220>
   <223> Primer
<400> 65
   cggcaccacc gacatcatct tcacctgccc tcgttccg 38
<210> 66
   <211> 38
   <212> DNA
   <213> Artificial Sequence
<220>
   <223> Primer
<400> 66
   cggaacgagg gcaggtgaag atgatgtcgg tggtgccg 38
<210> 67
   <211> 1263
   <212> DNA
   <213> Corynebacterium glutamicum
<220>
   <221> misc_feature
   <223> lysc gene
<400> 67
   gtggccctgg tcgtacagaa atatggcggt tcctcgcttg agagtgcgga acgcattaga 60
   aacgtcgctg aacggatcgt tgccaccaag aaggctggaa atgatgtcgt ggttgtctgc 120
   tccgcaatgg gagacaccac ggatgaactt ctagaacttg cagcggcagt gaatcccgtt 180
   ccgccagctc gtgaaatgga tatgctcctg actgctggtg agcgtatttc taacgctctc 240
   gtcgccatgg ctattgagtc ccttggcgca gaagcccaat ctttcacggg ctctcaggct 300
   ggtgtgctca ccaccgagcg ccacggaaac gcacgcattg ttgatgtcac tccaggtcgt 360
   gtgcgtgaag cactcgatga gggcaagatc tgcattgttg ctggtttcca gggtgttaat 420
   aaagaaaccc gcgatgtcac cacgttgggt cgtggtggtt ctgacaccac tgcagttgcg 480
   ttggcagctg ctttgaacgc tgatgtgtgt gagatttact cggacgttga cggtgtgtat 540
   accgctgacc cgcgcatcgt tcctaatgca cagaagctgg aaaagctcag cttcgaagaa 600
   atgctggaac ttgctgctgt tggctccaag attttggtgc tgcgcagtgt tgaatacgct 660
   cgtgcattca atgtgccact tcgcgtacgc tcgtcttata gtaatgatcc cggcactttg 720
   attgccggct ctatggagga tattcctgtg gaagaagcag tccttaccgg tgtcgcaacc 780
   gacaagtccg aagccaaagt aaccgttctg ggtatttccg ataagccagg cgaggctgcg 840
   aaggttttcc gtgcgttggc tgatgcagaa atcaacattg acatggttct gcagaacgtc 900
   tcttctgtag aagacggcac caccgacatc accttcacct gccctcgttc cgacggccgc 960
   cgcgcgatgg agatcttgaa gaagcttcag gttcagggca actggaccaa tgtgctttac 1020
   gacgaccagg tcggcaaagt ctccctcgtg ggtgctggca tgaagtctca cccaggtgtt 1080
   accgcagagt tcatggaagc tctgcgcgat gtcaacgtga acatcgaatt gatttccacc 1140
   tctgagattc gtatttccgt gctgatccgt gaagatgatc tggatgctgc tgcacgtgca 1200
   ttgcatgagc agttccagct gggcggcgaa gacgaagccg tcgtttatgc aggcaccgga 1260
   cgc 1263
<210> 68
   <211> 5860
   <212> DNA
   <213> Artificial Sequence
<220>
   <223> Plasmid
   <220>
   <221> misc_feature
   <223> internal designation: pCIS sysc thr311ile
<220>
   <221> misc_feature
   <222> (155)..(1420)
   <223> lysC
<220>
   <221> misc_feature
   <222> (3935)..(5356)
   <223> sacB (Bacillus subtilis) complement
<220>
   <221> misc_feature
   <222> (5357).'.(5819)
   <223> Promotor sacB complement
<220>
   <221> misc_feature
   <222> (1974)..(2765)
   <223> kanR
<220>
   <221> misc_feature
   <222> (3032)..(3892)
   <223> ori-EC complement
<400> 68
   cccggtacca cgcgtcccag tggctgagac gcatccgcta aagccccagg aaccctgtgc      60
   agaaagaaaa cactcctctg gctaggtaga cacagtttat aaaggtagag ttgagcgggt     120
   aactgtcagc acgtagatcg aaaggtgcac aaaggtggcc ctggtcgtac agaaatatgg     180
   cggttcctcg cttgagagtg cggaacgcat tagaaacgtc gctgaacgga tcgttgccac     240
   caagaaggct ggaaatgatg tcgtggttgt ctgctccgca atgggagaca ccacggatga     300
   acttctagaa cttgcagcgg cagtgaatcc cgttccgcca gctcgtgaaa tggatatgct     360
   cctgactgct ggtgagcgta tttctaacgc tctcgtcgcc atggctattg agtcccttgg     420
   cgcagaagcc caatctttca cgggctctca ggctggtgtg ctcaccaccg agcgccacgg     480
   aaacgcacgc attgttgatg tcactccagg tcgtgtgcgt gaagcactcg atgagggcaa     540
   gatctgcatt gttgctggtt tccagggtgt taataaagaa acccgcgatg tcaccacgtt     600
   gggtcgtggt ggttctgaca ccactgcagt tgcgttggca gctgctttga acgctgatgt     660
   gtgtgagatt tactcggacg ttgacggtgt gtataccgct gacccgcgca tcgttcctaa     720
   tgcacagaag ctggaaaagc tcagcttcga agaaatgctg gaacttgctg ctgttggctc     780
   caagattttg gtgctgcgca gtgttgaata cgctcgtgca ttcaatgtgc cacttcgcgt     840
   acgctcgtct tatagtaatg atcccggcac tttgattgcc ggctctatgg aggatattcc     900
   tgtggaagaa gcagtcctta ccggtgtcgc aaccgacaag tccgaagcca aagtaaccgt     960
   tctgggtatt tccgataagc caggcgaggc tgcgaaggtt ttccgtgcgt tggctgatgc    1020
   agaaatcaac attgacatgg ttctgcagaa cgtctcttct gtagaagacg gcaccaccga    1080
   catcatcttc acctgccctc gttccgacgg ccgccgcgcg atggagatct tgaagaagct    1140
   tcaggttcag ggcaactgga ccaatgtgct ttacgacgac caggtcggca aagtctccct    1200
   cgtgggtgct ggcatgaagt ctcacccagg tgttaccgca gagttcatgg aagctctgcg    1260
   cgatgtcaac gtgaacatcg aattgatttc cacctctgag attcgtattt ccgtgctgat    1320
   ccgtgaagat gatctggatg ctgctgcacg tgcattgcat gagcagttcc agctgggcgg 1380
   cgaagacgaa gccgtcgttt atgcaggcac cggacgctaa agttttaaag gagtagtttt 1440
   acaatgacca ccatcgcagt tgttggtgca accggccagg tcggccaggt tatgcgcacc 1500
   cttttggaag agcgcaattt cccagctgac actgttcgtt tctttgcttc cccacgttcc 1560
   gcaggccgta agattgaatt cgtcgacatc gatgctcttc tgcgttaatt aacaattggg 1620
   atcctctaga cccgggattt aaatcgctag cgggctgcta aaggaagcgg aacacgtaga 1680
   aagccagtcc gcagaaacgg tgctgacccc ggatgaatgt cagctactgg gctatctgga 1740
   caagggaaaa cgcaagcgca aagagaaagc aggtagcttg cagtgggctt acatggcgat 1800
   agctagactg ggcggtttta tggacagcaa gcgaaccgga attgccagct ggggcgccct 1860
   ctggtaaggt tgggaagccc tgcaaagtaa actggatggc tttcttgccg ccaaggatct 1920
   gatggcgcag gggatcaaga tctgatcaag agacaggatg aggatcgttt cgcatgattg 1980
   aacaagatgg attgcacgca ggttctccgg ccgcttgggt ggagaggcta ttcggctatg 2040
   actgggcaca acagacaatc ggctgctctg atgccgccgt gttccggctg tcagcgcagg 2100
   ggcgcccggt tctttttgtc aagaccgacc tgtccggtgc cctgaatgaa ctgcaggacg 2160
   aggcagcgcg gctatcgtgg ctggccacga cgggcgttcc ttgcgcagct gtgctcgacg 2220
   ttgtcactga agcgggaagg gactggctgc tattgggcga agtgccgggg caggatctcc 2280
   tgtcatctca ccttgctcct gccgagaaag tatccatcat ggctgatgca atgcggcggc 2340
   tgcatacgct tgatccggct acctgcccat tcgaccacca agcgaaacat cgcatcgagc 2400
   gagcacgtac tcggatggaa gccggtcttg tcgatcagga tgatctggac gaagagcatc 2460
   aggggctcgc gccagccgaa ctgttcgcca ggctcaaggc gcgcatgccc gacggcgagg 2520
   atctcgtcgt gacccatggc gatgcctgct tgccgaatat catggtggaa aatggccgct 2580
   tttctggatt catcgactgt ggccggctgg gtgtggcgga ccgctatcag gacatagcgt 2640
   tggctacccg tgatattgct gaagagcttg gcggcgaatg ggctgaccgc ttcctcgtgc 2700
   tttacggtat cgccgctccc gattcgcagc gcatcgcctt ctatcgcctt cttgacgagt 2760
   tcttctgagc gggactctgg ggttcgaaat gaccgaccaa gcgacgccca acctgccatc 2820
   acgagatttc gattccaccg ccgccttcta tgaaaggttg ggcttcggaa tcgttttccg 2880
   ggacgccggc tggatgatcc tccagcgcgg ggatctcatg ctggagttct tcgcccacgc 2940
   tagcggcgcg ccggccggcc cggtgtgaaa taccgcacag atgcgtaagg agaaaatacc 3000
   gcatcaggcg ctcttccgct tcctcgctca ctgactcgct gcgctcggtc gttcggctgc 3060
   ggcgagcggt atcagctcac tcaaaggcgg taatacggtt atccacagaa tcaggggata 3120
   acgcaggaaa gaacatgtga gcaaaaggcc agcaaaaggc caggaaccgt aaaaaggccg 3180
   cgttgctggc gtttttccat aggctccgcc cccctgacga gcatcacaaa aatcgacgct 3240
   caagtcagag gtggcgaaac ccgacaggac tataaagata ccaggcgttt ccccctggaa 3300
   gctccctcgt gcgctctcct gttccgaccc tgccgcttac cggatacctg tccgcctttc 3360
   tcccttcggg aagcgtggcg ctttctcata gctcacgctg taggtatctc agttcggtgt 3420
   aggtcgttcg ctccaagctg ggctgtgtgc acgaaccccc cgttcagccc gaccgctgcg 3480
   ccttatccgg taactatcgt cttgagtcca acccggtaag acacgactta tcgccactgg 3540
   cagcagccac tggtaacagg attagcagag cgaggtatgt aggcggtgct acagagttct 3600
   tgaagtggtg gcctaactac ggctacacta gaaggacagt atttggtatc tgcgctctgc 3660
   tgaagccagt taccttcgga aaaagagttg gtagctcttg atccggcaaa caaaccaccg 3720
   ctggtagcgg tggttttttt gtttgcaagc agcagattac gcgcagaaaa aaaggatctc 3780
   aagaagatcc tttgatcttt tctacggggt ctgacgctca gtggaacgaa aactcacgtt 3840
   aagggatttt ggtcatgaga ttatcaaaaa ggatcttcac ctagatcctt ttaaaggccg 3900
   gccgcggccg ccatcggcat tttcttttgc gtttttattt gttaactgtt aattgtcctt 3960
   gttcaaggat gctgtctttg acaacagatg ttttcttgcc tttgatgttc agcaggaagc 4020
   tcggcgcaaa cgttgattgt ttgtctgcgt agaatcctct gtttgtcata tagcttgtaa 4080
   tcacgacatt gtttcctttc gcttgaggta cagcgaagtg tgagtaagta aaggttacat 4140
   cgttaggatc aagatccatt tttaacacaa ggccagtttt gttcagcggc ttgtatgggc 4200
   cagttaaaga attagaaaca taaccaagca tgtaaatatc gttagacgta atgccgtcaa 4260
   tcgtcatttt tgatccgcgg gagtcagtga acaggtacca tttgccgttc attttaaaga 4320
   cgttcgcgcg ttcaatttca tctgttactg tgttagatgc aatcagcggt ttcatcactt 4380
   ttttcagtgt gtaatcatcg tttagctcaa tcataccgag agcgccgttt gctaactcag 4440
   ccgtgcgttt tttatcgctt tgcagaagtt tttgactttc ttgacggaag aatgatgtgc 4500
   ttttgccata gtatgctttg ttaaataaag attcttcgcc ttggtagcca tcttcagttc 4560
   cagtgtttgc ttcaaatact aagtatttgt ggcctttatc ttctacgtag tgaggatctc 4620
   tcagcgtatg gttgtcgcct gagctgtagt tgccttcatc gatgaactgc tgtacatttt 4680
   gatacgtttt tccgtcaccg tcaaagattg atttataatc ctctacaccg ttgatgttca 4740
   aagagctgtc tgatgctgat acgttaactt gtgcagttgt cagtgtttgt ttgccgtaat 4800
   gtttaccgga gaaatcagtg tagaataaac ggatttttcc gtcagatgta aatgtggctg 4860
   aacctgacca ttcttgtgtt tggtctttta ggatagaatc atttgcatcg aatttgtcgc 4920
   tgtctttaaa gacgcggcca gcgtttttcc agctgtcaat agaagtttcg ccgacttttt 4980
   gatagaacat gtaaatcgat gtgtcatccg catttttagg atctccggct aatgcaaaga 5040
   cgatgtggta gccgtgatag tttgcgacag tgccgtcagc gttttgtaat ggccagctgt 5100
   cccaaacgtc caggcctttt gcagaagaga tatttttaat tgtggacgaa tcaaattcag 5160
   aaacttgata tttttcattt ttttgctgtt cagggatttg cagcatatca tggcgtgtaa 5220
   tatgggaaat gccgtatgtt tccttatatg gcttttggtt cgtttctttc gcaaacgctt 5280
   gagttgcgcc tcctgccagc agtgcggtag taaaggttaa tactgttgct tgttttgcaa 5340
   actttttgat gttcatcgtt catgtctcct tttttatgta ctgtgttagc ggtctgcttc 5400
   ttccagccct cctgtttgaa gatggcaagt tagttacgca caataaaaaa agacctaaaa 5460
   tatgtaaggg gtgacgccaa agtatacact ttgcccttta cacattttag gtcttgcctg 5520
   ctttatcagt aacaaacccg cgcgatttac ttttcgacct cattctatta gactctcgtt 5580
   tggattgcaa ctggtctatt ttcctctttt gtttgataga aaatcataaa aggatttgca 5640
   gactacgggc ctaaagaact aaaaaatcta tctgtttctt ttcattctct gtatttttta 5700
   tagtttctgt tgcatgggca taaagttgcc tttttaatca caattcagaa aatatcataa 5760
   tatctcattt cactaaataa tagtgaacgg caggtatatg tgatgggtta aaaaggatcg 5820
   gcggccgctc gatttaaatc tcgagaggcc tgacgtcggg 5860
<210> 69
   <211> 29
   <212> DNA
   <213> Artificial sequence
<220>
   <223> Primer
   <220>
   <221> misc_feature
   <223> internal designation: Old38
<400> 69
   acatccatgg tggccgttac cctgcgaat 29
<210> 70
   <211> 20
   <212> DNA
   <213> Artificial Sequence
<220>
   <223> Primer
   <220>
   <221> misc_feature
   <223> internal designation: Old 39
<400> 70
   tgtatgtcct cctggacttc 20
<210> 71
   <211> 40
   <212> DNA
   <213> Artificial Sequence
<220>
   <223> Primer
   <220>
   <221> misc_feature
   <223> internal designation: old40
<400> 71
   gaagtccagg aggacataca atgaccaaca tccgcgtagc 40
<210> 72
   <211> 21
   <212> DNA
   <213> Artificial sequence
<220>
   <223> Primer
   <220>
   <221> misc_feature
   <223> internal designation: old 37
<400> 72
   gaaaccacac tgtttccttg c 21
<210> 73
   <211> 31
   <212> DNA
   <213> Artificial Sequence
<220>
   <223> Primer
   <220>
   <221> misc_feature
   <223> internal designation: old 32
<400> 73
   atcaacgcgt cgacaccaca tcatcaatca c 31
<210> 74
   <211> 33
   <212> DNA
   <213> Artificial Sequence
<220>
   <223> Primer
   <220>
   <221> misc_feature
   <223> internal designation: old33
<400> 74
   atcaccatgg gttcttgtaa tcctccaaaa ttg 33
   <210> 75
   <211> 6187
   <212> DNA
   <213> Artificial sequence
<220>
   <223> Plasmid
   <220>
   <221> misc_feature
   <223> internal designation: pCIS Peftu ddh (pCLiK int sacB Peftu ddh)
<400> 75
   tcgagctaaa ttagacgtcg cgtgcgatca gatcgtccaa gttctctggg gagagcaggt 60
   atggagcaac ttcgaggacg gtgaaagctc cgctttggcc ctgctgcttc atgcggtgag 120
   ctgcgcgacc gaaagcgatc tgtgaggaag cggtgaaatc tgggtttcgg tccagcttga 180
   ggatgtattc cacggtgtgg ttgaagccac cggtgtcgcc ggtggtaatc acgtggccac 240
   cgtgtggcat gccggtgtgc tcggagtcga aggttgcttc gtcgatgaag ttgacttcga 300
   cttcgtagcc aacgaagtaa tcaggcatgg tgcggatgtc gttttcgatg cgctcgtgat 360
   cggccgcgtc ggcaaccacg aagcattggc gcttgtgggt ttgctttccg gtaaggtcgc 420
   cggcttcgcc gcggcgggcc ttttccaggg cgtcttcgga tgggagggtg tactggactg 480
   ccttttgaac gccagggatg cgtcgcaaag catcggagtg gccctgtgac aaacctgggc 540
   cccagaaggt gtgctgctgg tgctcggcta agactgccgc tgcgtagacg cggttgatgg 600
   agaacattcc tggatcccag ccggtagaga ccagtgcaac gttgccggct gcggtggcgg 660
   cttcgttcat gacctggcgg tggcgtggga tgtcgcggtg gttgtcgtag gtgtctacgg 720
   tgcaggcgaa ctgcgcgaac tttggtgcct gctcagggat gtcggtggcg gagcccatgc 780
   acaggaacag cacgtccacg tcgtcggcgt gcttgtccac gtcggcgaca tcaaagactg 840
   gcgtctttgt gtcgagggtg gcccggcgcg agaagattcc tacaaggtcc atgtcgggct 900
   gcttggcaat aagcttttcg acgctgcgtc ccaggtttcc gtagcccacg atagctacgc 960
   ggatgttggt cattgtatgt cctcctggac ttcgtggtgg ctacgacttt cgcagccacg 1020
   aattggtttt gctttcactg tgataagcag cctggaagac tgagcacaca gatctagcgc 1080
   attacaaaat gtgccagtta ctgaatccta agggcaacgg cgttgatttt caaactacca 1140
   gctaccctgt ggacattcgc agggtaacgg ccaccatggg ttcttgtaat cctccaaaat 1200
   tgtggtggca ctgtcctggt cgagcttacc gagatgcata cttagatgat gattcaggga 1260
   catctctttc atcaggaccg aaagcgaacg tttcgtattg ttgagccttt tggttccacc 1320
   acggatgcgc tgatctattt tcatggctcc cagcagtcag gatctgtggg gcgcagcttc 1380
   accaacagga cttttgatcc gttgccgttc atggtggttt atccggatgg ggtggatcag 1440
   cattggaatg atgcgcggtt gggtttggat gaaaataccc gccatttagg cattgatgat 1500
   gtggggttct ttgtaaaact cgccacgcac ttgggcaaca cgtatggcat caagaggatc 1560
   tttattgttg gctattccaa cggtgggcag atggtgttgc ggctcatgca tgaggttccc 1620
   aagatgctca gtggcgctgc aaccattgca tccaacatgc cagttgcaga gaatacgctg 1680
   ccgcaggtga aaaccttcaa gacacatccg gtgccttatt tggcgatggc tggaactgcc 1740
   gatacttttt caccgtatga gggtggcgat gccggtattg gtcgcgaaca ccgccgtggc 1800
   gtgggcatgt ccgcctttga ttcagctgcc tatattgccg cccgaaacgg actgaccgaa 1860
   caccgccacg acgtgattga tgatgtggtg tcgacgcgtc atatgactag ttcggaccta 1920
   gggatatcgt cgacatcgat gctcttctgc gttaattaac aattgggatc ctctagaccc 1980
   gggatttaaa tcgctagcgg gctgctaaag gaagcggaac acgtagaaag ccagtccgca 2040
   gaaacggtgc tgaccccgga tgaatgtcag ctactgggct atctggacaa gggaaaacgc 2100
   aagcgcaaag agaaagcagg tagcttgcag tgggcttaca tggcgatagc tagactgggc 2160
   ggttttatgg acagcaagcg aaccggaatt gccagctggg gcgccctctg gtaaggttgg 2220
   gaagccctgc aaagtaaact ggatggcttt cttgccgcca aggatctgat ggcgcagggg 2280
   atcaagatct gatcaagaga caggatgagg atcgtttcgc atgattgaac aagatggatt 2340
   gcacgcaggt tctccggccg cttgggtgga gaggctattc ggctatgact gggcacaaca 2400
   gacaatcggc tgctctgatg ccgccgtgtt ccggctgtca gcgcaggggc gcccggttct 2460
   ttttgtcaag accgacctgt ccggtgccct gaatgaactg caggacgagg cagcgcggct 2520
   atcgtggctg gccacgacgg gcgttccttg cgcagctgtg ctcgacgttg tcactgaagc 2580
   gggaagggac tggctgctat tgggcgaagt gccggggcag gatctcctgt catctcacct 2640
   tgctcctgcc gagaaagtat ccatcatggc tgatgcaatg cggcggctgc atacgcttga 2700
   tccggctacc tgcccattcg accaccaagc gaaacatcgc atcgagcgag cacgtactcg 2760
   gatggaagcc ggtcttgtcg atcaggatga tctggacgaa gagcatcagg ggctcgcgcc 2820
   agccgaactg ttcgccaggc tcaaggcgcg catgcccgac ggcgaggatc tcgtcgtgac 2880
   ccatggcgat gcctgcttgc cgaatatcat ggtggaaaat ggccgctttt ctggattcat 2940
   cgactgtggc cggctgggtg tggcggaccg ctatcaggac atagcgttgg ctacccgtga 3000
   tattgctgaa gagcttggcg gcgaatgggc tgaccgcttc ctcgtgcttt acggtatcgc 3060
   cgctcccgat tcgcagcgca tcgccttcta tcgccttctt gacgagttct tctgagcggg 3120
   actctggggt tcgaaatgac cgaccaagcg acgcccaacc tgccatcacg agatttcgat 3180
   tccaccgccg ccttctatga aaggttgggc ttcggaatcg ttttccggga cgccggctgg 3240
   atgatcctcc agcgcgggga tctcatgctg gagttcttcg cccacgctag cggcgcgccg 3300
   gccggcccgg tgtgaaatac cgcacagatg cgtaaggaga aaataccgca tcaggcgctc 3360
   ttccgcttcc tcgctcactg actcgctgcg ctcggtcgtt cggctgcggc gagcggtatc 3420
   agctcactca aaggcggtaa tacggttatc cacagaatca ggggataacg caggaaagaa 3480
   catgtgagca aaaggccagc aaaaggccag gaaccgtaaa aaggccgcgt tgctggcgtt 3540
   tttccatagg ctccgccccc ctgacgagca tcacaaaaat cgacgctcaa gtcagaggtg 3600
   gcgaaacccg acaggactat aaagatacca ggcgtttccc cctggaagct ccctcgtgcg 3660
   ctctcctgtt ccgaccctgc cgcttaccgg atacctgtcc gcctttctcc cttcgggaag 3720
   cgtggcgctt tctcatagct cacgctgtag gtatctcagt tcggtgtagg tcgttcgctc 3780
   caagctgggc tgtgtgcacg aaccccccgt tcagcccgac cgctgcgcct tatccggtaa 3840
   ctatcgtctt gagtccaacc cggtaagaca cgacttatcg ccactggcag cagccactgg 3900
   taacaggatt agcagagcga ggtatgtagg cggtgctaca gagttcttga agtggtggcc 3960
   taactacggc tacactagaa ggacagtatt tggtatctgc gctctgctga agccagttac 4020
   cttcggaaaa agagttggta gctcttgatc cggcaaacaa accaccgctg gtagcggtgg 4080
   tttttttgtt tgcaagcagc agattacgcg cagaaaaaaa ggatctcaag aagatccttt 4140
   gatcttttct acggggtctg acgctcagtg gaacgaaaac tcacgttaag ggattttggt 4200
   catgagatta tcaaaaagga tcttcaccta gatcctttta aaggccggcc gcggccgcca 4260
   tcggcatttt cttttgcgtt tttatttgtt aactgttaat tgtccttgtt caaggatgct 4320
   gtctttgaca acagatgttt tcttgccttt gatgttcagc aggaagctcg gcgcaaacgt 4380
   tgattgtttg tctgcgtaga atcctctgtt tgtcatatag cttgtaatca cgacattgtt 4440
   tcctttcgct tgaggtacag cgaagtgtga gtaagtaaag gttacatcgt taggatcaag 4500
   atccattttt aacacaaggc cagttttgtt cagcggcttg tatgggccag ttaaagaatt 4560
   agaaacataa ccaagcatgt aaatatcgtt agacgtaatg ccgtcaatcg tcatttttga 4620
   tccgcgggag tcagtgaaca ggtaccattt gccgttcatt ttaaagacgt tcgcgcgttc 4680
   aatttcatct gttactgtgt tagatgcaat cagcggtttc atcacttttt tcagtgtgta 4740
   atcatcgttt agctcaatca taccgagagc gccgtttgct aactcagccg tgcgtttttt 4800
   atcgctttgc agaagttttt gactttcttg acggaagaat gatgtgcttt tgccatagta 4860
   tgctttgtta aataaagatt cttcgccttg gtagccatct tcagttccag tgtttgcttc 4920
   aaatactaag tatttgtggc ctttatcttc tacgtagtga ggatctctca gcgtatggtt 4980
   gtcgcctgag ctgtagttgc cttcatcgat gaactgctgt acattttgat acgtttttcc 5040
   gtcaccgtca aagattgatt tataatcctc tacaccgttg atgttcaaag agctgtctga 5100
   tgctgatacg ttaacttgtg cagttgtcag tgtttgtttg ccgtaatgtt taccggagaa 5160
   atcagtgtag aataaacgga tttttccgtc agatgtaaat gtggctgaac ctgaccattc 5220
   ttgtgtttgg tcttttagga tagaatcatt tgcatcgaat ttgtcgctgt ctttaaagac 5280
   gcggccagcg tttttccagc tgtcaataga agtttcgccg actttttgat agaacatgta 5340
   aatcgatgtg tcatccgcat ttttaggatc tccggctaat gcaaagacga tgtggtagcc 5400
   gtgatagttt gcgacagtgc cgtcagcgtt ttgtaatggc cagctgtccc aaacgtccag 5460
   gccttttgca gaagagatat ttttaattgt ggacgaatca aattcagaaa cttgatattt 5520
   ttcatttttt tgctgttcag ggatttgcag catatcatgg cgtgtaatat gggaaatgcc 5580
   gtatgtttcc ttatatggct tttggttcgt ttctttcgca aacgcttgag ttgcgcctcc 5640
   tgccagcagt gcggtagtaa aggttaatac tgttgcttgt tttgcaaact ttttgatgtt 5700
   catcgttcat gtctcctttt ttatgtactg tgttagcggt ctgcttcttc cagccctcct 5760
   gtttgaagat ggcaagttag ttacgcacaa taaaaaaaga cctaaaatat gtaaggggtg 5820
   acgccaaagt atacactttg ccctttacac attttaggtc ttgcctgctt tatcagtaac 5880
   aaacccgcgc gatttacttt tcgacctcat tctattagac tctcgtttgg attgcaactg 5940
   gtctattttc ctcttttgtt tgatagaaaa tcataaaagg atttgcagac tacgggccta 6000
   aagaactaaa aaatctatct gtttcttttc attctctgta ttttttatag tttctgttgc 6060
   atgggcataa agttgccttt ttaatcacaa ttcagaaaat atcataatat ctcatttcac 6120
   taaataatag tgaacggcag gtatatgtga tgggttaaaa aggatcggcg gccgctcgat 6180
   ttaaatc 6187
<210> 76
   <211> 29
   <212> DNA
   <213> Artificial sequence
<220>
   <223> Primer
   <220>
   <221> misc_feature
   <223> internal designation: CK461
<400> 76
   cctgacgtcg caatataggc agctgaatc 29
<210> 77
   <211> 29
   <212> DNA
   <213> Artificial Sequence
<220>
   <223> Primer
   <220>
   <221> misc feature
   <223> internal designation: CK466
<400> 77
   gcccaattgg ttcttgtaat cctccaaaa 29
<210> 78
   <211> 26
   <212> DNA
   <213> Artificial sequence
<220>
   <223> Primer
   <220>
   <221> misc_feature
   <223> internal designation: CK426
<400> 78
   cgccaattgt cgagggccgt taccct 26
<210> 79
   <211> 40
   <212> DNA
   <213> Artificial Sequence
<220>
   <223> Primer
   <220>
   <221> misc_feature
   <223> internal designation: Eck463
<400> 79
   gctacgcgga tgttggtcat gggtaaaaaa tcctttcgta 40
<210> 80
   <211> 40
   <212> DNA
   <213> Artificial Sequence
<220>
   <223> Primer
   <220>
   <221> misc_feature
   <223> internal designation: CK464
<400> 80
   tacgaaagga ttttttaccc atgaccaaca tccgcgtagc 40
<210> 81
   <211> 29
   <212> DNA
   <213> Artificial Sequence
<220>
   <223> Primer
   <220>
   <221> misc_feature
   <223> internal desigation: CK467
<400> 81
   agacccgggt tagacgtcgc gtgcgatca 29
<210> 82
   <211> 6233
   <212> DNA
   <213> Artificial Sequence
<220>
   <223> Plasmid
<220>
   <221> misc_feature
   <223> internal designation: pCIS PeftuPsod ddh = pclik int sacB PeftuPs od ddh
<400> 82
   gggatttaaa tcgctagcgg gctgctaaag gaagcggaac acgtagaaag ccagtccgca 60
   gaaacggtgc tgaccccgga tgaatgtcag ctactgggct atctggacaa gggaaaacgc 120
   aagcgcaaag agaaagcagg tagcttgcag tgggcttaca tggcgatagc tagactgggc 180
   ggttttatgg acagcaagcg aaccggaatt gccagctggg gcgccctctg gtaaggttgg 240
   gaagccctgc aaagtaaact ggatggcttt cttgccgcca aggatctgat ggcgcagggg 300
   atcaagatct gatcaagaga caggatgagg atcgtttcgc atgattgaac aagatggatt 360
   gcacgcaggt tctccggccg cttgggtgga gaggctattc ggctatgact gggcacaaca 420
   gacaatcggc tgctctgatg ccgccgtgtt ccggctgtca gcgcaggggc gcccggttct 480
   ttttgtcaag accgacctgt ccggtgccct gaatgaactg caggacgagg cagcgcggct 540
   atcgtggctg gccacgacgg gcgttccttg cgcagctgtg ctcgacgttg tcactgaagc 600
   gggaagggac tggctgctat tgggcgaagt gccggggcag gatctcctgt catctcacct 660
   tgctcctgcc gagaaagtat ccatcatggc tgatgcaatg cggcggctgc atacgcttga 720
   tccggctacc tgcccattcg accaccaagc gaaacatcgc atcgagcgag cacgtactcg 780
   gatggaagcc ggtcttgtcg atcaggatga tctggacgaa gagcatcagg ggctcgcgcc 840
   agccgaactg ttcgccaggc tcaaggcgcg catgcccgac ggcgaggatc tcgtcgtgac 900
   ccatggcgat gcctgcttgc cgaatatcat ggtggaaaat ggccgctttt ctggattcat 960
   cgactgtggc cggctgggtg tggcggaccg ctatcaggac atagcgttgg ctacccgtga 1020
   tattgctgaa gagcttggcg gcgaatgggc tgaccgcttc ctcgtgcttt acggtatcgc 1080
   cgctcccgat tcgcagcgca tcgccttcta tcgccttctt gacgagttct tctgagcggg 1140
   actctggggt tcgaaatgac cgaccaagcg acgcccaacc tgccatcacg agatttcgat 1200
   tccaccgccg ccttctatga aaggttgggc ttcggaatcg ttttccggga cgccggctgg 1260
   atgatcctcc agcgcgggga tctcatgctg gagttcttcg cccacgctag cggcgcgccg 1320
   gccggcccgg tgtgaaatac cgcacagatg cgtaaggaga aaataccgca tcaggcgctc 1380
   ttccgcttcc tcgctcactg actcgctgcg ctcggtcgtt cggctgcggc gagcggtatc 1440
   agctcactca aaggcggtaa tacggttatc cacagaatca ggggataacg caggaaagaa 1500
   catgtgagca aaaggccagc aaaaggccag gaaccgtaaa aaggccgcgt tgctggcgtt 1560
   tttccatagg ctccgccccc ctgacgagca tcacaaaaat cgacgctcaa gtcagaggtg 1620
   gcgaaacccg acaggactat aaagatacca ggcgtttccc cctggaagct ccctcgtgcg 1680
   ctctcctgtt ccgaccctgc cgcttaccgg atacctgtcc gcctttctcc cttcgggaag 1740
   cgtggcgctt tctcatagct cacgctgtag gtatctcagt tcggtgtagg tcgttcgctc 1800
   caagctgggc tgtgtgcacg aaccccccgt tcagcccgac cgctgcgcct tatccggtaa 1860
   ctatcgtctt gagtccaacc cggtaagaca cgacttatcg ccactggcag cagccactgg 1920
   taacaggatt agcagagcga ggtatgtagg cggtgctaca gagttcttga agtggtggcc 1980
   taactacggc tacactagaa ggacagtatt tggtatctgc gctctgctga agccagttac 2040
   cttcggaaaa agagttggta gctcttgatc cggcaaacaa accaccgctg gtagcggtgg 2100
   tttttttgtt tgcaagcagc agattacgcg cagaaaaaaa ggatctcaag aagatccttt 2160
   gatcttttct acggggtctg acgctcagtg gaacgaaaac tcacgttaag ggattttggt 2220
   catgagatta tcaaaaagga tcttcaccta gatcctttta aaggccggcc gcggccgcca 2280
   tcggcatttt cttttgcgtt tttatttgtt aactgttaat tgtccttgtt caaggatgct 2340
   gtctttgaca acagatgttt tcttgccttt gatgttcagc aggaagctcg gcgcaaacgt 2400
   tgattgtttg tctgcgtaga atcctctgtt tgtcatatag cttgtaatca cgacattgtt 2460
   tcctttcgct tgaggtacag cgaagtgtga gtaagtaaag gttacatcgt taggatcaag 2520
   atccattttt aacacaaggc cagttttgtt cagcggcttg tatgggccag ttaaagaatt 2580
   agaaacataa ccaagcatgt aaatatcgtt agacgtaatg ccgtcaatcg tcatttttga 2640
   tccgcgggag tcagtgaaca ggtaccattt gccgttcatt ttaaagacgt tcgcgcgttc 2700
   aatttcatct gttactgtgt tagatgcaat cagcggtttc atcacttttt tcagtgtgta 2760
   atcatcgttt agctcaatca taccgagagc gccgtttgct aactcagccg tgcgtttttt 2820
   atcgctttgc agaagttttt gactttcttg acggaagaat gatgtgcttt tgccatagta 2880
   tgctttgtta aataaagatt cttcgccttg gtagccatct tcagttccag tgtttgcttc 2940
   aaatactaag tatttgtggc ctttatcttc tacgtagtga ggatctctca gcgtatggtt 3000
   gtcgcctgag ctgtagttgc cttcatcgat gaactgctgt acattttgat acgtttttcc 3060
   gtcaccgtca aagattgatt tataatcctc tacaccgttg atgttcaaag agctgtctga 3120
   tgctgatacg ttaacttgtg cagttgtcag tgtttgtttg ccgtaatgtt taccggagaa 3180
   atcagtgtag aataaacgga tttttccgtc agatgtaaat gtggctgaac ctgaccattc 3240
   ttgtgtttgg tcttttagga tagaatcatt tgcatcgaat ttgtcgctgt ctttaaagac 3300
   gcggccagcg tttttccagc tgtcaataga agtttcgccg actttttgat agaacatgta 3360
   aatcgatgtg tcatccgcat ttttaggatc tccggctaat gcaaagacga tgtggtagcc 3420
   gtgatagttt gcgacagtgc cgtcagcgtt ttgtaatggc cagctgtccc aaacgtccag 3480
   gccttttgca gaagagatat ttttaattgt ggacgaatca aattcagaaa cttgatattt 3540
   ttcatttttt tgctgttcag ggatttgcag catatcatgg cgtgtaatat gggaaatgcc 3600
   gtatgtttcc ttatatggct tttggttcgt ttctttcgca aacgcttgag ttgcgcctcc 3660
   tgccagcagt gcggtagtaa aggttaatac tgttgcttgt tttgcaaact ttttgatgtt 3720
   catcgttcat gtctcctttt ttatgtactg tgttagcggt ctgcttcttc cagccctcct 3780
   gtttgaagat ggcaagttag ttacgcacaa taaaaaaaga cctaaaatat gtaaggggtg 3840
   acgccaaagt atacactttg ccctttacac attttaggtc ttgcctgctt tatcagtaac 3900
   aaacccgcgc gatttacttt tcgacctcat tctattagac tctcgtttgg attgcaactg 3960
   gtctattttc ctcttttgtt tgatagaaaa tcataaaagg atttgcagac tacgggccta 4020
   aagaactaaa aaatctatct gtttcttttc attctctgta ttttttatag tttctgttgc 4080
   atgggcataa agttgccttt ttaatcacaa ttcagaaaat atcataatat ctcatttcac 4140
   taaataatag tgaacggcag gtatatgtga tgggttaaaa aggatcggcg gccgctcgat 4200
   ttaaatctcg agaggcctga cgtcgcaata taggcagctg aatcaaaggc ggacatgccc 4260
   acgccacggc ggtgttcgcg accaataccg gcatcgccac cctcatacgg tgaaaaagta 4320
   tcggcagttc cagccatcgc caaataaggc accggatgtg tcttgaaggt tttcacctgc 4380
   ggcagcgtat tctctgcaac tggcatgttg gatgcaatgg ttgcagcgcc actgagcatc 4440
   ttgggaacct catgcatgag ccgcaacacc atctgcccac cgttggaata gccaacaata 4500
   aagatcctct tgatgccata cgtgttgccc aagtgcgtgg cgagttttac aaagaacccc 4560
   acatcatcaa tgcctaaatg gcgggtattt tcatccaaac ccaaccgcgc atcattccaa 4620
   tgctgatcca ccccatccgg ataaaccacc atgaacggca acggatcaaa agtcctgttg 4680
   gtgaagctgc gccccacaga tcctgactgc tgggagccat gaaaatagat cagcgcatcc 4740
   gtggtggaac caaaaggctc aacaatacga aacgttcgct ttcggtcctg atgaaagaga 4800
   tgtccctgaa tcatcatcta agtatgcatc tcggtaagct cgaccaggac agtgccacca 4860
   caattttgga ggattacaag aaccaattgt cgagggccgt taccctgcga atgtccacag 4920
   ggtagctggt agtttgaaaa tcaacgccgt tgcccttagg attcagtaac tggcacattt 4980
   tgtaatgcgc tagatctgtg tgctcagtct tccaggctgc ttatcacagt gaaagcaaaa 5040
   ccaattcgtg gctgcgaaag tcgtagccac actagtagct gccaattatt ccgggcttgt 5100
   gacccgctac ccgataaata ggtcggctga aaaatttcgt tgcaatatca acaaaaaggc 5160
   ctatcattgg gaggtgtcgc accaagtact tttgcgaagc gccatctgac ggattttcaa 5220
   aagatgtata tgctcggtgc ggaaacctac gaaaggattt tttacccatg accaacatcc 5280
   gcgtagctat cgtgggctac ggaaacctgg gacgcagcgt cgaaaagctt attgccaagc 5340
   agcccgacat ggaccttgta ggaatcttct cgcgccgggc caccctcgac acaaagacgc 5400
   cagtctttga tgtcgccgac gtggacaagc acgccgacga cgtggacgtg ctgttcctgt 5460
   gcatgggctc cgccaccgac atccctgagc aggcaccaaa gttcgcgcag ttcgcctgca 5520
   ccgtagacac ctacgacaac caccgcgaca tcccacgcca ccgccaggtc atgaacgaag 5580
   ccgccaccgc agccggcaac gttgcactgg tctctaccgg ctgggatcca ggaatgttct 5640
   ccatcaaccg cgtctacgca gcggcagtct tagccgagca ccagcagcac accttctggg 5700
   gcccaggttt gtcacagggc cactccgatg ctttgcgacg catccctggc gttcaaaagg 5760
   cagtccagta caccctccca tccgaagacg ccctggaaaa ggcccgccgc ggcgaagccg 5820
   gcgaccttac cggaaagcaa acccacaagc gccaatgctt cgtggttgcc gacgcggccg 5880
   atcacgagcg catcgaaaac gacatccgca ccatgcctga ttacttcgtt ggctacgaag 5940
   tcgaagtcaa cttcatcgac gaagcaacct tcgactccga gcacaccggc atgccacacg 6000
   gtggccacgt gattaccacc ggcgacaccg gtggcttcaa ccacaccgtg gaatacatcc 6060
   tcaagctgga ccgaaaccca gatttcaccg cttcctcaca gatcgctttc ggtcgcgcag 6120
   ctcaccgcat gaagcagcag ggccaaagcg gagctttcac cgtcctcgaa gttgctccat 6180
   acctgctctc cccagagaac ttggacgatc tgatcgcacg cgacgtctaa ccc 6233
<210> 83
   <211> 7
   <212> DNA
   <213> Corynebacterium glutamicum
<220>
   <221> misc_feature
   <223> ribosome binding sequence
<400> 83
   aggagga
<210> 84
   <211> 1365
   <212> DNA
   <213> corynebacterium glutamicum
<220>
   <221> misc_feature
   <223> RAX077
<400> 84
   atgaatgatg agaatattca aagctccaac tatcagccat tcccgagttt tgacgattgg 60
   aaacagatcg aggtgtcgct cttagatgtc atcgaatcct cacgccattt ttctgatttg 120
   aaagatagca ctgatcgttc tgcgttagat gctgcgctag agagagcaaa aagagctgcc 180
   gcagttgata ccaatgccat agaaggaatc ttccaaactg atcgcggttt tacccataca 240
   gttgcaacgc aggtaggggc ttgggagcaa caaatggcga tgaaaggcaa acatgttaag 300
   cctgcgtttg acgatactct agaaggcttt gagtatgttc tcgatgcagt aactggtaga 360
   actccaatct ctcagcaatg gattagaaat ttgcacgccg tcattctgcg gagccaagaa 420
   agccacgagg tttttacagc cgttggagtc caaaatcagg cgcttcagaa aggcgagtat 480
   aaaactcagc caaatagtcc acagcgctca gatggatctg tacatgcata cgccccagtt 540
   gaagatactc ctgctgaaat ggctagattt atttcagaac ttgaatctaa ggaattctta 600
   gcagccgaga aggttattca agctgcctat gcccactatg ctttcgtatg tattcatcct 660
   tttgcagatg ggaatggacg agttgcacga gccttggcta gtgtttttct atacaaagat 720
   cctggtgtcc ctctcgtaat ctaccaagat caacgcagag attacatcca tgctctagaa 780
   gcagcggaca agaataaccc gctcctgctg attagattct ttgctgaacg agtgaccgat 840
   actattaact ctattatcgt tgatctcact accccgatcg cgggtaaatc tggttcggct   900
   aagctttcgg atgcgctacg ccccactcgc gtattaccag aattacatga tgctgcacat   960
   aggctccaag aaagtttatt tacagaaatc cgatctcgat tggatgaaga aggaaaaagg  1020
   aatgggttgg agtttctact tcaacggatt tttatcggtt ccccattcaa tctgccagag  1080
   ggctataacg ctttccctga tagctattgt ctgaccttag ctttcaatag caactctcca  1140
   aaacaaatct tccacccgct atccatagta atagcagctc gagatgggaa aagagcgagc  1200
   agcgacctcg tggcagctac ttctattgga tacaactttc acgcttacgg acgtgaagtc  1260
   gagcctgttg ttactgaaag ctttcgagaa cgtgtgaaaa tttacgccga cgggattgta  1320
   gatcacttct taaccgaact ggctaaaaag tttcaacaga attaa                  1365

## Beschreibung

Die vorliegende Erfindung betrifft ein genetisch verändertes coryneformes Bakterium, transformiert mit wenigstens einem Vektor enthaltend wenigstens eine Expressionskassette, wobei die Expressionskassette in 5' - 3' -Richtung ein Sequenzmodul der foldenden allgemeinen Formel II umfasst:

5' -P₁-(-Aₓ-Pₓ-)ₙ-A_{y}-P_{y}-G-3' (II)

worin
n für einen ganzzahligen Wert von 0 bis 10 steht,
Aₓ und A_{y} gleich oder verschieden sind und für eine chemische Bindung oder eine Linker-Nukleinsäuresequenz stehen;
P₁, Pₓ und P_{y} für gleiche oder verschiedene Promotorsequenzen abgeleitet von gleichen oder verschiedenen coryneformen Bakterien kodieren, welche wenigstens einen RNA-Polymerase-bindenden Abschnitt umfassen; und wenigstens P_{y} einen die Ribosomenbindung vermittelnden, 3' -terminalen Sequenzabschnitt umfasst,
G für wenigstens eine kodierenden Ukleinsäuresequenz steht, welche mit der 5' - stromaufwärts gelegenen reulatorischen Sequenz funktionell verknüpft ist; sowie Verfahren zur Herstellung von biosynthetischen Produkten durch Kultivierung des genetisch veränderten coryneformen Bakteriums.

### HINTERGRUND DER ERFINDUNG

Verschiedene biosynthetische Produkte werden über natürliche Stoffwechselprozesse in Zellen hergestellt und werden in vielen Industriezweigen verwendet, einschließlich der Nahrungsmittel-, Futtermittel-, Kosmetik-, Feed-, Food- und pharmazeutischen Industrie. Diese Substanzen, die zusammen als Feinchemikalien/Proteine bezeichnet werden, umfassen unter anderem organische Säuren, sowohl proteinogene als auch

nicht-proteinogene Aminosäuren, Nukleotide und Nukleoside, Lipide und Fettsäuren, Diole, Kohlenhydrate, aromatische Verbindungen, Vitamine und Cofaktoren, sowie Proteine und Enzyme. Ihre Produktion erfolgt am zweckmäßigsten im Großmaßstab mittels Anzucht von Bakterienstämmen oder anderen Mikroorganismen, die entwickelt wurden, um große Mengen der jeweils gewünschten Substanz zu produzieren und zu sezernieren. Für diesen Zweck besonders geeignete Organismen sind coryneforme Bakterien, gram-positive nicht-pathogene Bakterien.

Es ist bekannt, dass Aminosäuren durch Fermentation von Stämmen coryneformer Bakterien, insbesondere Corynebacterium glutamicum, hergestellt werden können. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration während der Fermentation, oder die Aufarbeitung zum Produkt, beispielsweise durch lonenaustauschehromatographie aber auch Sprühtrocknung, oder die intrinsischen Leistungseigenschaften des Mikroorganismus selbst betreffen.

Seit einigen Jahren werden ebenfalls Methoden der rekombinanten DNA-Technik zur Stammverbesserung von Feinchemikalien/Proteine produzierender Stämme von Corynebacterium eingesetzt, indem man einzelne Gene amplifiziert und die Auswirkung auf die Produktion von Feinchemikalien/Proteine untersucht.

Andere Wege, um ein Verfahren für die Herstellung von Feinchemikalien oder Proteinen zu entwickeln, oder die Produktivität eines bereits existierenden Verfahrens für die Herstellung von Feinchemikalien oder Proteinen zu erhöhen bzw. zu verbessern, umfassen die Erhöhung bzw. die Veränderung der Expression eines oder mehrerer Gene und/oder die Beeinflussung der Translation einer mRNA durch geeignete Polynukleotidsequenzen. Beeinflussung kann in diesem Zusammenhang die Erhöhung, Verringerung oder auch andere Parameter der Expression von Genen, wie zeitliche Expressionsmuster, umfassen.

Dem Fachmann sind unterschiedliche Bestandteile von bakteriellen Regulationssequenzen bekannt. Man unterscheidet die Bindungsstellen von Regulatoren, auch Operatoren genannt, die Bindungsstellen von RNA-Polymerase-Holoenzymen, auch -35 und -10 Regionen genannt, und die Bindungsstelle von ribosomaler 16S-RNA, auch ribosomale Bindungsstelle (RBS) oder auch Shine-Dalgarno-Sequenz genannt.

In der Literatur (E. coli und S. typhimurium, Neidhardt F.C. 1995 ASM Press) wird beschrieben, dass sowohl die Zusammensetzung der Polynukleotidsequenz der Shine-Dalgarno-Sequenz, die Sequenzabfolge der Basen, aber auch der Abstand einer in der Shine-Dalgarno-Sequenz enthaltenen Polynukleotidsequenz zum Start-Codon einen wesentlichen Einfluss auf die Initiationstionsrate der Translation hat.

Nukleinsäuresequenzen mit Promotoraktivität können die Bildung von mRNA auf unterschiedliche Weise beeinflussen. Promotoren, deren Aktivität unabhängig von der physiologischen Wachstumsphase des Organismus sind, nennt man konstitutiv. Wiederum andere Promotoren reagieren auf externe chemische, wie physikalische Stimuli wie Sauerstoff, Metabolite, Hitze, pH, etc.. Wiederum andere zeigen in unterschiedlichen Wachstumsphasen eine starke Abhängigkeit ihrer Aktivität. Beispielsweise sind in der Literatur Promotoren beschrieben, die während der exponentiellen Wachstumsphase von Mikroorganismen eine besonders ausgeprägte Aktivität zeigen, oder aber auch genau in der stationären Phase des mikrobiellen Wachstums. Beide Charakteristika von Promotoren können für eine Produktion von Feinchemikalien und Proteine je nach Stoffwechselweg einen günstigen Einfluss auf die Produktivität haben.

In Corynebacterium-Spezies konnten bereits solche Nukleotidsequenzen isoliert werden, die für eine Erhöhung bzw. eine Abschwächung der Genexpression genutzt werden können. Diese regulierten Promotoren können die Rate, mit der ein Gen transkribiert wird, abhängig von den internen und/oder externen Bedingungen der Zelle erhöhen oder erniedrigen. Zum Teil kann die Anwesenheit eines bestimmten Faktors, bekannt als Induktor, die Rate der Transkription vom Promotor stimulieren. Induktoren können direkt oder aber indirekt die Transkription vom Promotor beeinflussen. Eine andere Klasse von Faktoren, bekannt als Suppressoren ist in der Lage, die Transkription vom Promotor zu reduzieren oder aber zu inhibieren. Wie auch die Induktoren, können auch die Suppressoren direkt oder indirekt wirken. Es sind jedoch auch Promotoren bekannt, die über die Temperatur reguliert werden. So kann der Level der Transkription solcher Promotoren zum Beispiel durch eine Erhöhung der Wachstumstemperatur über die normale Wachstumstemperatur der Zelle erhöht oder aber abgeschwächt werden.

Eine geringe Anzahl von Promotoren aus C. glutamicum wurde bis zum heutigen Tag beschrieben. Der Promotor des Malatsynthase-Gens aus C. glutamicum wurde in DE-A- 44 40 118 beschrieben. Dieser Promotor wurde einem für ein Protein kodierendes Strukturgen vorgeschaltet. Nach Transformation eines solchen Konstrukts in ein coryneformes Bakterium wird die Expression des dem Promotor nachgeschalteten Strukturgens reguliert. Die Expression des Strukturgens wird induziert, sobald dem Medium ein entsprechender Induktor zugesetzt wird.

Reinscheid et al., Microbiology 145:503 (1999) haben eine transkriptionelle Fusion zwischen dem pta-ack Promotor aus C. glutamicum und einem Reportergen (Chloramphenicol Acetyltransferase) beschrieben. Zellen von C. glutamicum, die eine solche transkriptionelle Fusion enthalten, wiesen eine erhöhte Expression des Reportergens bei Wachstum auf Acetat-haltigem Medium auf. Im Vergleich dazu zeigten transformierte Zellen, die auf Glucose wuchsen, keine erhöhte Expression dieses Reportergens.

In Patek et al., Microbiology 142:1297 (1996) wurden einige DNA Sequenzen aus C. glutamicum beschrieben, die die Expression eines Reportergens in C. glutamicum Zellen verstärken können, beschrieben. Diese Sequenzen wurden miteinander verglichen, um Consensus-Sequenzen für C. glutamicum Promotoren zu definieren.

Weitere DNA-Sequenzen aus C. glutamicum, die zur Regulation der Genexpression genutzt werden können, sind in der WO 02/40679 beschrieben worden. Diese isolierten Polynukleotide stellen Expressionseinheiten aus Corynebacterium glutamicum dar, die entweder zur Erhöhung oder aber zur Verringerung einer Genexpression genutzt werden können. Weiterhin sind in dieser Druckschrift rekombinante Plasmide beschrieben, auf denen die Expressionseinheiten aus Corynebacterium glutamicum mit heterologen Genen assoziiert sind. Die hier beschrieben Methode, Fusion von einem Promotor aus Corynebacterium glutamicum mit einem hererlogen Gen, kann unter anderem zur Regulation der Gene der Aminosäurebiosynthese eingesetzt werden.

Aus den älteren, nicht vorveröffentlichten Patentanmeldungen DE-A-103 59 594, DE-A-103 59 595, DE-A-103 59 660 und DE-A-10 2004 035 065 sind spezielle Einzelpromotoren aus C. glutamicum bekannt.

### KURZBESCHREINUNG DER ERFINDUNG

Der Erfindung lag die Aufgabe zugrunde weitere genetisch veränderte coryneforme Bakterien, die mit einem Vektor enthaltend eine Expressionskassette transformiert sind, bereitzustellen. Diese genetisch veränderten coryneformen Bakterien weisen in Bezug auf die Expression bestimmter Gene vorteilhafte Eigenschaften auf.
Die Aufgabe wurde erfindungsgemäß gelöst durch Bereitstellung von einem genetisch veränderten coryneformen Bakterium, transformiert mit wenistens einem Vektor enthaltend wenigstens ein Expressionskassette, wobei die Expressionskassette in 5' - 3' - Richtung ein Sequenzmodul der folgenden allgemeinen Formel umfasst,:

5' -P₁-(-Aₓ-Pₓ-)ₙ-A_{y}-P_{y}-G-3' (II)

worin
n für einen ganzzahligen Wert von 0 bis 10 steht,
Aₓ und A_{y} gleich oder verschieden sind und für eine chemische Bindung oder eine Linker-Nukleinsäuresequenz stehen;
P₁, Pₓ und P_{y} für gleiche oder verschiedene Promotorsequenzen abgeleitet von gleichen oder verschiedenen coryneformen Bakterien kodieren, welche wenigstens einen RNA-Polymerase-bindenden Abschnitt umfassen; und wenigstens P_{y} einen die Ribosomenbindung vermittelnden, 3' -terminalen Sequenzabschnitt umfasst,
G für wenigstens eine kodierende Nukleinsäuresequenz steht, welche mit der 5' - stromaufwärts gelegenen regulatorischen Sequenz funktionell verknüpft ist.

Gemäß einer Ausführungsform sind P₁, Pₓ und P_{y} von gleichen oder verschiedenen coryneformen Bakterien abgeleitet.

Gemäß einer bevorzugten Ausführungsform sind P₁, Pₓ und P_{y} jeweils abgeleitet von einer zusammenhängenden Sequenz von 35 bis 500 Nukleotidresten, bevorzugt 35 bis 300 Nukleotidresten, noch bevorzugter 35 bis 210 Nukleotidresten und ganz besonders bevorzugt 35 bis 100 Nukleotidresten, die im Genom des Organismus 5'-stromaufwärts von der kodierenden Sequenz für ein Protein, z.B für ein an einem Biosyntheseweg des Organismus beteiligtes Protein, liegt.

Gemäß einer bevorzugten Ausführungsform sind P₁, Pₓ und P_{y} der Expressionseinheit unabhängig voneinander ausgewählt unter Promotorsequenzen für die kodierende Sequenz eines Proteins mit hoher Abundanz in einem coryneformen Bakterium, wie z.B. in einem der Gattung Corynebacterium oder Brevibacterium, wie z.B. einer Spezies oder einem Stamm gemäß der Aufstellung in Tabelle 3.

Zusätzlich können einzelne oder alle dieser Promotorsequenzen ausgewählt sein unter Promotorsequenzen für die kodierende Sequenz eines in der Tabelle 1 aufgelisteten und an der Aminosäurebiosynthese eines Organismus beteiligten Proteins.Wenigstens einer der Promatoren der Expressionskassette sollte dabei aber hohe Abundanz wie hierin definiert aufweisen.

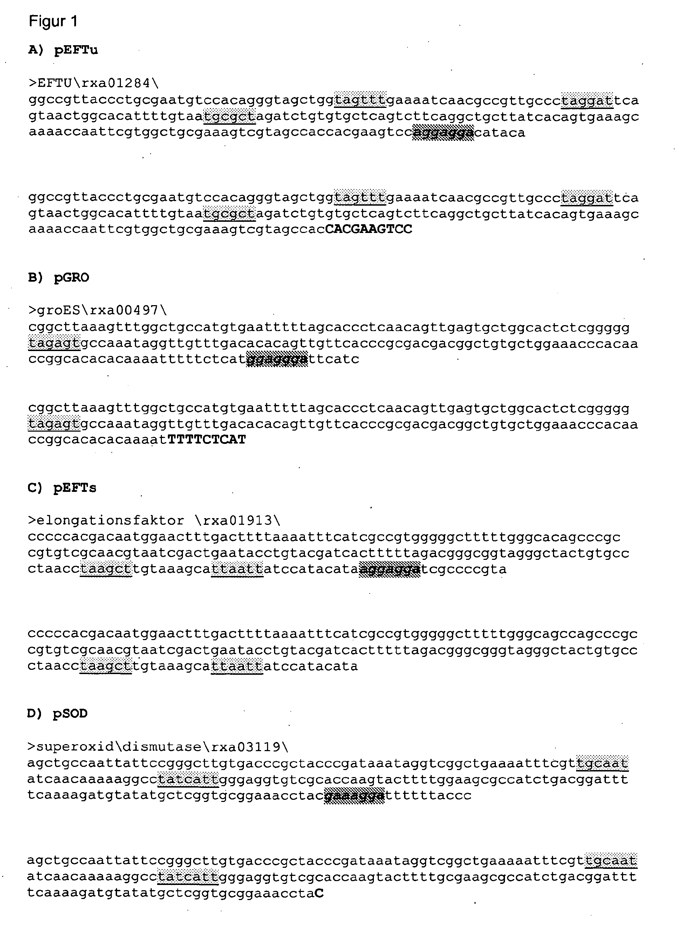

Gemäß einer weiteren bevorzugten Ausführungsform sind P₁, Pₓ und P_{y} der Expressionskassette unabhängig voneinander ausgewählt unter den in Figur 1 dargestellten Nukleotidsequenzen, beziehungsweise, wie später noch näher erläutert, abgeleitet von SEQ ID NO:1 (pGRO), SEQ ID NO:2 (pEFTs), SEQ ID NO:3 (pEFTu) und SEQ ID NO:4 (pSOD).

Gemäß einer weiteren Ausführungsform sind P₁, Pₓ und P_{y} der Expressionskassette unabhängig voneinander ausgewählt unter starken, konstitutiven oder regulierbaren Promotoren.

Das erfindungsgemäße Bakterium enthält eine Expressionskassette, umfassend in 5'-3'-Richtung ein Sequenzmodul der folgenden allgemeinen Formel II:

5'-P₁-(-Aₓ-Pₓ-)ₙ-A_{y}-P_{y}-G-3' (II)

worin
n, Aₓ, A_{y}, P_{1.} Pₓ und P_{y} wie oben definiert sind und G für wenigstens eine kodierende Nukleinsäuresequenz steht, welche mit der 5'-stromaufwärts gelegenen regulatorischen Sequenz funktionell oder operativ verknüpft ist.

Gemäß einer bevorzugten Ausführungsform der Expressionskassette ist G ausgewählt unter
a) Nukleinsäuren kodierend ein Protein aus den Biosyntheseweg von proteinogenen und nicht-proteinogenen Aminosäuren,
b) Nukleinsäuren kodierend ein Protein aus dem Biosyntheseweg von Nukleotiden und Nukleosiden,
c) Nukleinsäuren kodierend ein Protein aus dem Biosyntheseweg von organischen Säuren,
d) Nukleinsäuren kodierend ein Protein aus dem Biosyntheseweg von Lipiden und Fettsäuren,
e) Nukleinsäuren kodierend ein Protein aus dem Biosyntheseweg von Diolen,
f) Nukleinsäuren kodierend ein Protein aus dem Biosyntheseweg von Kohlenhydraten,
g) Nukleinsäuren kodierend ein Protein aus dem Biosyntheseweg von aromatischen Verbindungen,
h) Nukleinsäuren kodierend ein Protein aus dem Biosyntheseweg von Vitaminen,
i) Nukleinsäuren kodierend ein Protein aus dem Biosyntheseweg von Cofaktoren,
j) Nukleinsäuren kodierend ein Protein aus dem Biosyntheseweg von Enzymen, und
k) Nukleinsäuren kodierend ein Protein aus dem Zentralstoffwechsel.

Gemäß einer besonders bevorzugten Ausführungsform der Expressionskassette ist G ausgewählt unter Nukleinsäuren für Proteine aus dem Biosyntheseweg von Aminosäuren, ausgewählt unter Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystathionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase, Tetrahydropicolinat-Succinylase, Succinyl-aminoketopimelat-Aminotransferase, Succinyl-Diaminopimelat-Desuccinylase, Diaminopimelat-Epimerase, 6-Phosphogluconat-Dehydrogenase, Glucosephosphat-Isomerase, Phosphoglycerat-Mutase, Enolase, Pyruvat-Kinase, Aspartat-Transaminase, Malat-Enzym kodieren.

Das erfindungsgemäße Bakterium wird mit einem Vektor transformiert, der wenigstens eine der vorstehend genannten Expressionskassetten umfasst.

Die vorliegende Erfindung betrifft ein genetisch verändertes coryneformes Bakterium, das mit wenigstens einem der vorstehend genannten Vektoren transformiert ist, oder wenigstens eine der vorstehend genannten Expressionskassetten, vorzugsweise in integrierter Form, enthält.

Gemäß einem weiteren Aspekt betrifft die vorliegende Erfindung ein Verfahren zur Herstellung eines biosynthetischen Produkts, wobei man einen der vorstehend genannten, genetisch veränderten coryneformen Bakterien kultiviert und das gewünschte Produkt aus der Kultur isoliert.

Gemäß einer Ausführungsform des Verfahrens ist das biosynthetische Produkt ausgewählt unter organischen Säuren, Proteinen, Nukleotiden und Nukleosiden, sowohl proteinogenen als auch nicht-proteinogenen Aminosäuren, Lipiden und Fettsäuren, Diolen, Kohlehydraten, aromatischen Verbindungen, Vitaminen und Cofaktoren, Enzymen und Proteinen. Ganz besonders bevorzugte Biosyntheseprodukte sind Lysin, Methionin, Threonin und Trehalose.

### FIGURENBESCHREIBUNG

Figur 1 zeigt spezifische Sequenzen für vier Promotoren, welche zur Herstellung der in den Ausführungsbeispielen beschriebenen Mehrfachpromotoren verwendet wurden. Dargestellt sind die Promotorsequenzen für pEFTu (Figur 1A); für Promotor pGRO (Figur 1 B), für Promotor pEFTs (Figur 1 C) und für Promotor pSOD (Figur 1D). Für jeden Promotor sind zwei Sequenzen angegeben, die längere (obere Sequenz) gibt jeweils die vollständige Promotorsequenz einschließlich Ribosomenbindungsstelle (RBS), fett und kursiv gedruckt, an. Im Falle einer 3'-terminalen Anordnung des jeweiligen Promotors im Mehrfach-Promotorkonstrukt wird bevorzugt jeweils die längere Nukleotidsequenz (einschließlich RBS) verwendet. Promotorsequenzen, 5'-stromaufwärts zur 3'-terminalen Promotoreinheit umfassen keine RBS und wurden in der jeweils in zweiter Position angegebenen, verkürzten Nukleinsäuresequenz verwendet (ohne RBS). 3'-terminale, in Großbuchstaben und Fettschrift angegebene Teilsequenzen der kürzeren Promotorsequenzen können gegebenenfalls zusätzlich fehlen. Potenzielle "-10-Regionen" sind unterstrichen dargestellt.

### DETAILLIERTE BESCHREIBUNG DER ERFINDUNG

### I. Allgemeine Definitionen:

Ein "biosynthetisches Produkt" im Sinne der Erfindung werden solche verstanden, die über natürliche Stoffwechselprozesse (Biosynthesewege) in Zellen produziert werden. Sie finden vielfältige Verwendung, insbesondere der Nahrungsmittel-, Futtermittel-, Kosmetik-, Feed-, Food- und pharmazeutischen Industrie. Diese Substanzen, die zusammen als Feinchemikalien/Proteine bezeichnet werden, umfassen unter anderem organische Säuren, sowohl proteinogene als auch nicht-proteinogene Aminosäuren, Nukleotide und Nukleoside, Lipide und Fettsäuren, Diole, Kohlenhydrate, aromatische Verbindungen, Vitamine und Cofaktoren, sowie Proteine und Enzyme.

Unter einem "Promotor", einer "Nukleinsäure mit Promotoraktivität" oder einer "Promotorsequenz" wird eine Nukleinsäure verstanden, die in funktioneller Verknüpfung mit einer zu transkribierenden Nukleinsäure die Transkription dieser Nukleinsäure reguliert.

Sofern nicht ausdrücklich auf "Einzelpromotoren" Bezug genommen wird, umfasst der Begriff "Promotor" je nach Zusammenhang auch die sequentielle Anordnung von mehr als einer Nukleinsäuresequenz (also "Mehrfachpromotoren"), von denen jede einzelne bei funktioneller Verknüpfung mit einer zu transkribierenden Nukleinsäure zur Regulation von deren Transkription befähigt wäre.

Der Begriff "Einzelpromotor" bezieht sich entsprechend auf eine einzige, zur Regulation der Transkription einer zu transkribierenden Nukleinsäure befähigte Nukleinsäuresequenz, die bei funktioneller Verknüpfung mit einer zu transkribierenden Nukleinsäuresequenz diese transkribieren kann.

Bei "Mehrfachpromotoren" sind mindestens zwei gleiche oder unterschiedliche Nukleinsäuresequenzen, von denen jede einzelne bei funktioneller Verknüpfung mit einer zu transkribierenden Nukleinsäure zur Regulation von deren Transkription befähigt wäre (Einzelpromotoren), sequentiell so verknüpft, dass ein Transkriptionsstart von wahlweise einer der Nukleinsäuresequenzen ein die zu transkribierende Nukleinsäure enthaltendes Transkript hervorbringen könnte. Zwischen jeweils zwei Einzelpromotoren können dabei weitere Sequenzen ohne Promotorfunktion eingefügt werden, beispielsweise ein Linker, der beispielsweise eine oder mehrere Restriktionsschnittstellen aufweist. Wahlweise können jeweils zwei Einzelpromotoren auch direkt miteinander verknüpft werden. Vorzugsweise enthalten die Mehrfachpromotoren nur eine Ribosomenbindungsstelle (RBS), insbesondere im Bereich des 3'-terminalen Endes des Promotors.

Unter Promotorsequenzen für die kodierende Sequenz eines Proteins mit "hoher Abundanz" versteht man insbesondere "starke Promotoren". Wenn man Mehrfachpromotoren zur Verstärkung von Genen einsetzen möchte, werden in geeigneter Weise bevorzugt solche starken Promotoren kombiniert. Starke Promotoren regulieren die Transkription von Genen derart, dass die Ablesehäufigkeit durch die RNA-Polymerase größer ist als bei der Mehrzahl der Gene des Organismus. In den meisten Fällen resultiert das Vorhandensein eines starken Promoters darin, dass auch eine große Menge an Transkript in der Zelle vorliegt. Wenn das Transkript in Relation zu den anderen zellulären Transkripten einen prozentual größeren Anteil einnimmt, spricht man von einem "abundanten Transkript". In Bakterien korreliert die Menge an Transkript und dem entsprechenden davon kodierten Protein in der Regel, d.h. "abundante Transkripte" führen meist auch zu "abundanten Proteinen". Im Folgenden ist beispielhaft eine Methode beschrieben, die die Identifizierung von "starken Promotoren" über die Abundanz von Proteinen erlaubt. Details zum methodischen Vorgehen sowie weitere Referenzen können z.B. aus Proteomics in Practice - A Laboratory Manual of Proteome Analysis (Autoren: R. Westermeier, T. Naven; Verlag: Wiley-VCH Verlag GmbH, 2002) entnommen werden. Die Bakterienzellen werden aufgeschlossen und die Proteine des Zellextraktes werden mittels 2D-Gelelektrophorese aufgetrennt. Anschließend werden die Proteine durch gängige Methoden, wie z.B. Coomassie-, Silber- oder Fluoreszenzfarbstofffärbung angefärbt. Dabei ist eine Überfärbung der Proteine zu vermeiden, um eine spätere Quantifizierung der einzelnen Proteinspots zu ermöglichen. Im Anschluss daran wird das Gel gescannt und das erhaltenen Bild mit Hilfe einer geeigneten Software (z.B. Melanie, Amersham Biosciences) ausgewertet. Dazu werden zunächst alle detektierbaren Spots identifiziert und das Spotvolumen bestimmt. Die Summe aller Spotvolumina entspricht in erster Annäherung dem Gesamtprotein. Mit Hilfe der Bildanalysesoftware können dann die Spots mit besonders großen Spotvolumina ausgewählt werden. Beispielsweise können Spots mit Volumina, die mehr als 0.1 % des Gesamtspotvolumens einnehmen, als abundant bezeichnet werden. Anschließend werden Spots mit besonders abundanten Proteinen aus dem Gel ausgestochen. Normalerweise wird dann das im Gelstück vorhandene Protein proteolytisch verdaut (z.B. mit Trypsin) und die Molmasse der entstehenden Peptide, z.B. mit Hilfe von MALDI-ToF MS (Matrix assisted laser desorption ionization- Time of Flight Mass Spectrometry) bestimmt. Auf Basis der Molmasse von einigen der tryptischen Peptide des Proteins kann dann in Datenbankrecherchen das zugehörige Protein identifziert werden. Dies ist insbesondere dann möglich, wenn das Genom des zu untersuchenden Organismus sequenziert ist, wie das für C. glutamicum, E. coli und viele andere Bakterien der Fall ist. Auf Basis der Identität des Proteins kann dann das dieses Protein kodierende ORF (offenes Leseraster) bestimmt werden. In Bakterien liegen die dieses Gen regulierenden Promotoren meist direkt stromaufwärts vom Start-Codon. Daher befinden sich auch die "starken" Promoteren häufig in einem Bereich von ca. 200 Nukleotiden stromaufwärts des Start-Codons "abundanter" Proteine.

"Künstliche" Promotoren umfassen insbesondere solche Sequenzen, die in situ keine Transkriptionsaktivität aufweisen, in einem anderen Sequenzkontext, wie insbesondere im Kontext einer Expressionskassette, transkriptionell aktiv sind.

Unter "Promotoraktivität" wird die in einer bestimmten Zeit durch den Promotor gebildete Menge RNA, also die Transkriptionsrate verstanden.

Unter "spezifischer" Promotoraktivität wird die in einer bestimmten Zeit durch den Promotor gebildete Menge RNA pro Promotor verstanden.

Bei einer "verursachten" Promotoraktivität oder Transkriptionsrate im Bezug auf ein Gen im Vergleich zum Wildtyp wird somit im Vergleich zum Wildtyp die Bildung einer RNA verursacht, die im Wildtyp so nicht vorhanden war.

Bei einer "veränderten" Promotoraktivität oder Transkriptionsrate im Bezug auf ein Gen im Vergleich zum Wildtyp wird somit im Vergleich zum Wildtyp in einer bestimmten Zeit die gebildete Menge der RNA verändert.

Unter "verändert" wird in diesem Zusammenhang bevorzugt erhöht oder erniedrigt verstanden.

Unter einer "funktionellen" oder "operativen" Verknüpfung versteht man in diesem Zusammenhang beispielsweise die sequentielle Anordnung einer Nukleinsäure mit Promotoraktivität und einer zu transkribierenden Nukleinsäuresequenz und ggf. weiterer regulativer Elemente, wie zum Beispiel Nukleinsäuresequenzen, die die Transkription von Nukleinsäuren gewährleisten, sowie zum Beispiel einen Terminator derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Als Sequenz einer "ribosomalen Bindungsstelle" oder "Ribosomenbindungsstelle" (RBS) (oder als Shine-Dalgarno-Sequenz bezeichnet) werden A/G-reiche Polynukleotidsequenzen verstanden, die sich bis zu 30 Basen stromauf des Initiationscodon der Translation befinden.

Unter "Transkription" wird der Prozess verstanden, durch den ausgehend von einer DNA-Matrize ein komplementäres RNA-Molekül hergestellt wird. An diesem Prozess sind Proteine, wie die RNA-Polymerase, sogenannte Sigma-Faktoren und transkriptionelle Regulatorproteine beteiligt. Die synthetisierte RNA dient dann als Matrize im Prozess der Translation, der dann zum biosynthetisch aktiven Protein führt.

Unter einer "Kernregion" eines Promotors versteht man eine zusammenhängende Nukleinsäuresequenz von etwa 20 bis 80, oder 30 bis 60 Nukleotiden, die wenigstens eine potentielle sogenannte "-10-Region" umfasst. Beispiele für potentielle -10-Regionen sind für einzelne bevorzugte Promotorsequenzen hierin beschrieben; vergleiche insbesondere Figur 1. "-10-Regionen" werden auch als TATA-Boxen oder Pribnow-Schaller-Sequenzen bezeichnet. Jeder eingesetzte Promotor sollte mindestens eine dieser potentiellen -10-Regionen, wie z. B. 1, 2, 3, 4 oder 5, enthalten. Die Kernegion liegt 5'-stromaufwärts zur RBS und kann von dieser 1 bis 200 oder 10 bis 150 oder 20 bis 100 Nukleotidreste entfernt sein .

Unter einer "Expressionseinheit" wird eine Nukleinsäure mit Expressionsaktivität verstanden, die einen Mehrfachpromotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Mehrfachpromotor-Konstrukt können weitere, regulative Elemente, wie z.B. Enhancer, enthalten sein.

Unter einer "Expressionskassette" wird eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Unter "Expressionsaktivität" wird die in einer bestimmten Zeit durch die Expressionskassette bzw. deren Expressionseinheit gebildete Menge Protein, also die Expressionsrate verstanden.

Unter "spezifischer Expressionsaktivität" wird die in einer bestimmten Zeit durch die Expressionskassette bzw. deren Expressionseinheit gebildete Menge Protein pro Expressionseinheit verstanden.

Bei einer "verursachten Expressionsaktivität" oder Expressionsrate im Bezug auf ein Gen im Vergleich zum Wildtyp wird somit im Vergleich zum Wildtyp die Bildung eines Proteins verursacht, das im Wildtyp so nicht vorhanden war.

Bei einer "veränderten Expressionsaktivität" oder Expressionsrate im Bezug auf ein Gen im Vergleich zum Wildtyp wird somit im Vergleich zum Wildtyp in einer bestimmten Zeit die gebildete Menge des Proteins verändert. Unter "verändert" wird in diesem Zusammenhang bevorzugt erhöht oder erniedrigt verstanden. Dies kann beispielsweise durch Erhöhung oder Reduzierung der spezifischen Aktivität der endogenen Expressionseinheit, beispielsweise durch Mutation, oder durch Stimulierung oder Hemmung erfolgen. Weiterhin kann die veränderte Expressionsaktivität oder Expressionsrate beispielsweise durch Regulation der Expression von Genen im Mikroorganismus durch erfindungsgemäße Expressionseinheiten erreicht werden, wobei die Gene im Bezug auf die Expressionseinheiten heterolog sind.

Die "Bildungsrate", mit der ein biosynthetisch aktives Protein hergestellt wird, ist ein Produkt aus der Rate der Transkription und der Translation. Beide Raten können beeinflusst werden und damit die Rate der Bildung von Produkten/Feinchemikalien in einem Mikroorganismus beeinflussen.

Unter dem Begriff "Wildtyp" wird der entsprechende Ausgangsmikroorganismus verstanden und muss nicht zwingend einem natürlich vorkommenden Organismus entsprechen.

Je nach Zusammenhang kann unter dem Begriff Mikroorganismus" der Ausgangsmikroorganismus (Wildtyp) oder ein erfindungsgemäßer, genetisch veränderter Mikroorganismus oder beides verstanden werden.

Vorzugsweise und insbesondere in Fällen, in denen der Mikroorganismus oder der Wildtyp nicht eindeutig zugeordnet werden kann, wird unter "Wildtyp" für die Veränderung oder Verursachung der Promotoraktivität oder Transkriptionsrate, für die Veränderung oder Verursachung der Expressionsaktivität oder Expressionsrate und für die Erhöhung des Gehalts an biosynthetischen Produkten jeweils ein "Referenzorganismus" verstanden. In einer bevorzugten Ausführungsform ist dieser "Referenzorganismus" Corynebacterium glutamicum ATCC 13032 oder ein Mikroorganismus, der durch gezielte oder ungerichtete Mutation von ATCC 13032 abgeleitet ist.

Insbesondere werden "Ausgangsmikroorganismen" verwendet, die bereits in der Lage sind, das gewünschte Produkt (Feinchemikalie/Protein) herzustellen.

Unter einer abgeleiteten" Sequenz, z.B. einer abgeleiteten Promotorsequenz, wird wenn keine anderen Angaben gemacht werden, eine Sequenz verstanden, die mit der Ausgangssequenz eine Identität von mindestens 80% oder mindestens 90%, insbesondere 91%, 92%, 93%, 94%, 95%, 96%, 97%, 98% und 99% aufweist.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Ap;5(2):151-1) unter Einstellung folgender Parameter berechnet wird:
Multiple alignment parameter:
   Gap opening penalty 10
   Gap extension penalty 10
   Gap separation penalty range 8
   Gap separation penalty off
   % identity for alignment delay 40
   Residue specific gaps off
   Hydrophilic residue gap off
   Transition weighing 0
Pairwise alignment parameter:
   FAST algorithm on
   K-tuplesize 1
   Gap penalty 3
   Window size 5
   Number of best diagonals 5

Unter "hybridisieren" versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Die "Hybridisierung" erfolgt unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben. Unter stringenten Hybridisierungs-Bedingungen werden insbesondere verstanden:
Die Inkubation bei 42°C über Nacht in einer Lösung bestehend aus 50 % Formamid, 5 x SSC (750 mM NaCl, 75 mM Tri-Natrium Citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Unter "im wesentlichen gleich" wird eine spezifische Promotoraktivität verstanden die mindestens 50%, wie z.B. mindestens 60%, 70%, 80%, 90%, oder mindestens 95% der spezifischen Promotoraktivität der Ausgangssequenz aufweist.

### II. Expressionseinheiten, Expressionskassetten und deren Komponenten

### a) Expressionseinheiten

Die Erfindung betrifft genetisch veränderte coryneforme Bakterien, die mit wenigstens einem Vektor enthaltend wenigstens eine Expressionskassette transformiert wurden. Expressionskassetten enthalten Mehrfachpromotoren - enthaltende Expressionseinheiten, funktionell verknüpft mit einer translatierbaren Nukleinsäuresequenz.

Diese Mehrfachpromotor - enthaltende Expressionseinheit, umfaßt in 5'-3'-Richtung ein Sequenzmodul der folgenden allgemeinen Formel (I)

5'-P₁-(-Aₓ-Pₓ-)ₙ-A_{y}-P_{y}-3'

worin
n für einen ganzzahligen Wert von 0 bis 10, wie z. B.1, 2, 3, 4, 5 oder 6 steht;
Aₓ und A_{y} gleich oder verschieden sind und für eine chemische, insbesondere kovalente, Bindung oder eine chemisch, insbesondere kovalent eingebundene, Linker-Nukleinsäuresequenz stehen;
P₁, Pₓ und P_{y} für gleiche oder verschiedene Promotorsequenzen abgeleiten von gleichen oder verschiedenen coryneformen Bakterien kodieren, welche wenigstens einen RNA-Polymerase-bindenden Bereich, wie z.B. eine Kernregion, umfassen; und wenigstens, wie z.B. nur, P_{y} einen, die Ribosomenbindung vermittelnden, 3'-terminalen Sequenzabschnitt umfasst.

Die Promotorsequenzen P₁, Pₓ und P_{y} können von Genen von coryneformen Bakterien abgeleitet, die für Proteine kodieren, welche an einem Biosyntheseweg des Organismus beteiligt sind. Die Promotorsequenzen liegen dabei im Genom des Organismus 20 bis 500 Nukleotidreste, oder 20 bis 300 Nukleotidreste, beispielsweise 20 bis 210 Nukleotidreste und insbesondere 20 bis 100 oder 35 bis 100 Nukleotidreste 5'-stromaufwärts von der kodierenden Sequenz des jeweiligen Proteins.

Beispiele für Sequenzen, von denen Promotorsequenzen P₁, Pₓ und P_{y} abgeleitet werden können, sind die in folgender Tabelle 1 angeführten Gene.

Nichtlimitierende Beispiele für spezielle Promotorsequenzen sind abgeleitet von den Nukleinsäuresequenzen nach SEQ ID NO:1, SEQ ID NO:2, SEQ ID NO:3 und SEQ ID NO:4, sowie Figur 1, ohne jedoch darauf beschränkt zu sein.

Dabei entspricht SEQ ID NO:1 der Sequenz, die stromauf gelegen ist von der kodierenden Region des GroES Chaperonins (pGRO), SEQ ID NO:2 der Sequenz, die stromauf gelegen ist von der kodierenden Region des Protein Elongations Faktor TS (pEFTs), SEQ ID NO:3 der Sequenz, die stromauf gelegen ist von der kodierenden Region des Protein Elongationsfaktor TU (pEFTu) und SEQ ID NO:4 der Sequenz, die stromauf gelegen ist von der kodierenden Region der Superoxiddismutase (pSOD), jeweils aus Corynebacterium glutamicum. SEQ. ID.NO. 1, SEQ ID NO:2, SEQ ID NO:3 und SEQ ID NO:4 entsprechen den Promotorsequenzen des Wildtyps.

Unter einer "abgeleiteten" Sequenz wird eine Sequenz verstanden, die mit der Ausgangssequenz eine Identität von mindestens 80% oder mindestens 90%, wie 91 %, 92%, 93%, 94%, 95%, 96%, 97%, 98% und insbesondere 99% aufweist. Dieser Identitätsgrad gilt insbesondere für die oben definierte "Kernregion" des jeweiligen Promotors. Außerhalb der jeweiligen Kernregion kann dabei der Identitätsgrad geringer sein, und im Bereich von 0 bis 80%, wie z.B. 10 bis 80%, 20 bis 70% , 30 bis 60% oder 40 bis 50% betragen.

Brauchbare Kernregionen liegen z.B.
für pGRO im Bereich von Position 50 bis 80 von SEQ ID NO:1;
für pEFTs im Bereich von Position 130 bis 170 von SEQ ID NO:2;
Für pEFTu im Bereich von Position 30 bis 110 von SEQ ID NO:3;
für pSOD im Bereich von Position 50 bis 100 von SEQ ID NO:4.

Die Expressionseinheiten enthalten insbesondere
zwei oder mehr Nukleinsäuresequenzen mit Promotoraktivität,
a) vorzugsweise abgeleitet von gleichen oder verschiedenen Sequenzen, ausgewählt unter SEQ. ID. NO. 1, SEQ ID NO:2, SEQ ID NO:3 und SEQ ID NO:4; oder anderen Promotorsequenzen für Gene mit vergleichbarer oder höherer Abundanz;
b) wobei gegebenenfalls eine oder mehrere dieser Sequenzen eine durch Substitution, Insertion, Inversion oder Deletion oder Addition von Nukleotiden abgeleitete Sequenz darstellen, die insbesondere im Kernbereich, eine Identität von mindestens 80 % auf Nukleinsäureebene mit einer vorzugsweise unter Sequenz SEQ ID NO: 1, 2, 3 oder 4 oder anderen Promotorsequenzen für Gene mit vergleichbarer oder höherer Abundanz ausgewählten Sequenz aufweist,
c) oder wobei gegebenenfalls eine oder mehrere dieser enthaltenen Nukleinsäuresequenzen mit einer Sequenz unter stringenten Bedigungen hybridisiert, die zu einer der Nukleinsäuresequenzen gemäß SEQ ID NO: 1, 2, 3 oder 4 und oder zu anderen Promotorsequenzen für Gene mit vergleichbarer oder höherer Abundanz komplementär ist,

Weitere natürliche Promotoren, die als Bestandteil eines Mehrfachpromotors brauchbar sind, lassen sich beispielsweise aus verschiedenen coryneformen Bakterien, deren genomische Sequenz bekannt ist, durch Identitätsvergleiche der Nukleinsäuresequenzen aus Datenbanken mit den vorstehend beschriebenen Sequenzen SEQ ID NO: 1, 2, 3 oder 4 oder gemäß Figur 1 oder anderen Promotorsequenzen für Gene mit vergleichbarer oder höherer Abundanz leicht auffinden.

Weitere geeignete natürliche Promotoren lassen sich ausgehend von den vorstehend beschriebenen Nukleinsäuresequenzen, insbesondere ausgehend von Sequenz SEQ ID NO: 1, 2, 3 oder 4 und/oder Promotorsequenzen für Gene mit vergleichbarer oder höherer Abundanz aus verschiedenen coryneformen Bakterien, deren genomische Sequenz nicht bekannt ist, unter Anwendung von Hybridisierungstechniken in an sich bekannter Weise leicht auffinden.

Weitere geeignete natürliche Promotoren lassen sich auch nach dem Fachmann bekannten Methoden isolieren, wie sie z.B. in Cadenas et al (1991) Gene 98, 117-121; Eikmanns et al, (1991) Gene 102, 93-98.; Patek et al (1996) Microbiology 142, 1297-1309 oder Santamaria et al (1987) Gene 56, 199-208 beschrieben sind. Üblicherweise werden dazu sogenannte "Promoter probe" Vektoren verwendet, in die genomische Fragmente aus dem betreffenden Genom inseriert werden. Die Promoteraktivität eines einzelnen Fragmentes kann in dem genannten experimentellen Setup dann durch Messen der Aktivität eines nachgeschalteten Reportergens, z.B. Chloramphenicol-Acetyltransferase, bestimmt werden.

DieMehrfachpromotoren - enthaltende Expressionseinheit umfasst daher auch die Kombination von solchen Promotoren, die hier nicht namentlich aufgeführt sind. Dies trifft auch zu für die Kombination von schon bekannten Einzelpromotoren, wie sie z.B. in (Patek et al (2003). J of Biotechnology 104, 311-323; Vasicova et al. (1999) J. Bacteriol 181, 6188-6191) beschrieben sind.

Nukleinsäuren mit Promotoraktivität zum Einbau in eine, Mehrfachpromotoren-enthaltende Expressionseinheit,umfassen auch die Nukleinsäuren mit Promotoraktivität eine Nukleinsäuresequenz umfassen, die mit der Nukleinsäuresequenz SEQ ID NO: 1, 2, 3 oder 4 oder Promotorsequenzen für Gene mit vergleichbarer oder höherer Abundanz unter stringenten Bedingungen hybnidisieren. Diese Nukleinsäuresequenz umfasst mindestens 10, bevorzugt mehr als 12, 15, 30, 50 oder mehr als 150 Nukleotide.

Künstliche Promotorsequenzen zum Einbau in eine, Mehrfachpromotor-enthaltende Expressionseinheit lassen sich ausgehend von der Sequenz SEQ ID NO: 1, 2, 3 oder 4 oder gemäß Figur 1 und/oder Promotorsequenzen für Gene mit vergleichbarer oder höherer Abundanz durch künstliche Variation und Mutation, beispielsweise durch Addition, Substitution, Insertion, Inversion oder Deletion von einem oder mehreren, einzelnen oder zusammenhängenden Nukleotidresten leicht herstellen. (Patek et al (2003). J of Biotechnology 104, 311-323; Vasicova et al. (1999) J. bacteriol 181, 6188-6191) und darin genannte Referenzen).

Geeignete Ribosomenbindung vermittelnde 3'-terminale Sequenzabschnitte einer Promotorsequenz P_{y} sind beispielsweise die RBS von pGRO: GGAGGGA; die RBS von pEFTs: AGGAGGA; die RBS von pEFTu: AGGAGGA; sowie die RBS von pSOD : GGAGGGA ( vgl. auch beiliegenden Figur 1). Die theoretisch optimale RBS, d.h. die Sequenz die 100% komplementär ist zur Anti-Shine-Dalgarno-Sequenz auf der 16S rRNA von C. glutamicum lautet: 5' GAAAGGAGG 3'. Weitere geeignete RBS Sequenzabschnitte lassen sich beispielsweise durch künstliche Variation und Mutation, beispielsweise durch Substitution, Insertion, Inversion, Addition oder Deletion von Nukleotiden herstellen, oder über Homologievergleiche mit Promotorsequenzen in Datenbanken leicht auffinden.

Nichtlimitierende Beispiele für, Mehrfachpromotoren-enthaltende Expressionseinheiten sind ausgewählt unter:
e) Sequenzen, kodierend für
   PeftuPsod, von Position 18 bis 390 in SEQ ID NO: 45;
   PsodPeftu, von Position 18 bis 395 in SEQ ID NO: 52;
   PgroPsod, von Position 18 bis 369 in SEQ ID NO: 55;
   PgroPsodPefts, von Position 11 bis 538 in SEQ ID NO: 59;
   PeftuPsodPefts; von Position 11 bis 559 in SEQ ID NO: 60;
   oder
f) von Sequenzen gemäß e) durch Substitution, Insertion, Inversion, Addition oder Deletion von Nukleotiden abgeleitete Sequenzen, die im Vergleich mit dieser Ausgangssequenz eine Identität von mindestens 80 % oder mindestens 90% auf Nukleinsäureebene aufweist; oder
g) Nukleinsäuresequenzen, die mit einer zu e) komplementären Sequenz unter stringenten Bedingungen hybridisiert;

Die Expressionseinheiten können vorzugsweise eines oder mehrere der folgenden genetischen Elemente umfassen: eine -10-Region; einen Transkriptionsstart, eine Enhancer Region; und eine Operator Region.

Diese genetischen Elemente sind spezifisch für die Spezies Corynebacterien, speziell für Corynebacterium glutamicum.

Bakterielle Promotoren bestehen normalerweise aus drei RNA-Polymerase Erkennungsstellen, nämlich der -10-Region, der -35-Region und dem UP-Element. Diese Promotorelemente sind in E. coli stark konserviert, nicht jedoch in C. glutamicum. Dies trifft in besonderem Maße für die -35-Region zu. Diese kann daher oft gar nicht oder nur unzuverlässig von der Sequenz abgeleitet werden. Auch die -10-Region ist weniger stark konserviert als in Organismen mit AT-reichen Genomen. Als Konsensus-Sequenzen sind hier TGNGNTA(C/T)AATGG oder GNTANAATNG vorbeschrieben (Patek et al (2003), J. of Biotechnology 104, 311-323; Vasicova et al. (1999) J. Bacteriol 181, 6188-6191 ).

Es ist allgemein bekannt, dass bei Bakterien mit moderatem oder hohem GC-Gehalt (wie z.B. C. glutamicum.) im allgemeinen eine hohe Variabilität in den Konsensus-Sequenzen auftritt (Boum and Babb, 1995., Bashyam et al, 1996). Dies gilt besonders für die -35-Region, die daher oft nicht vorhergesagt werden kann. Dies ist z.B. in Patek et al (2003). J of Biotechnology 104, 325-334 beschrieben.

### b) Expressionskassetten

Mehrfachpromotoren-enthaltenden Expressionseinheiten, funktionell verknüpft mit einer translatierbaren Nukleinsäuresequenz, werden als Expressionskassetten bezeichnet.

Unter einer "funktionellen Verknüpfung" versteht man in diesem Zusammenhang beispielsweise die sequentielle Anordnung einer der Expressionseinheiten und einer transgen zu exprimierenden Nukleinsäuresequenz und ggf. weiterer regulativer Elemente, wie zum Beispiel einem Terminator, derart, dass jedes der regulativen Elemente eine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter (d.h. am 3'-Ende) der Expressionseinheitssequenz positioniert wird, so dass beide Sequenzen kovalent miteinander verbunden sind. Bevorzugt ist dabei der Abstand zwischen der Expressionseinheitssequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Durch die, Mehrfachpromotoren enthaltenden Expressionskassetten wird die Möglichkeit geschaffen, eine im Vergleich zur ursprünglichen Expressionsaktivität unterschiedliche Expressionsaktivität zu erreichen und somit eine Feinregulierung der Expression des gewünschten Gens vorzunehmen.

Vorzugsweise wird die Regulation der Expression von Genen im coryneformen Bakterium durch Expressionseinheiten dadurch erreicht, dass man
- eine oder mehrere Expressionseinheiten, gegebenenfalls mit veränderter spezifischer Expressionsaktivität, in das Genom des coryneformen Bakteriums einbringt, so dass die Expression eines oder mehrerer endogener Gene unter der Kontrolle der eingebrachten Expressionseinheiten erfolgt; oder
- ein oder mehrere Nukleinsäurekonstrukte, enthaltend eine Expressionseinheit, gegebenenfalls mit veränderter spezifischer Expressionsaktivität, und funktionell verknüpft eine oder mehrere, zu exprimierende Nukleinsäuren, d.h. eine Expressionskassette; in das coryneforme Bakterium einbringt.

In den Expressionskassetten ist die physikalische Lage der Expressionseinheit relativ zum zu exprimierenden Gen so gewählt, dass die Expressionseinheit die Transkription und vorzugsweise auch die Translation des zu exprimierenden Gens reguliert und damit die Bildung eines oder mehrerer Proteine ermöglicht. Die "Bildung ermöglichen" beinhaltet dabei die konstitutive Steigerung der Bildung, Abschwächung bzw. Blockierung der Bildung unter spezifischen Bedingungen und /oder die Steigerung der Bildung unter spezifischen Bedingungen. Die "Bedingungen" umfassen dabei: (1) Zugabe einer Komponente zum Kulturmedium, (2) Entfernen einer Komponente vom Kulturmedium, (3) Austausch einer Komponente im Kulturmedium durch eine zweite Komponente, (4) Erhöhung der Temperatur des Kulturmediums, (5) Erniedrigung der Temperatur des Kulturmediums, und (6) Regulierung der atmosphärischen Bedingungen, wie z.B. die Sauerstoff- oder Stickstoffkonzentration, in der das Kulturmedium gehalten wird.

### c) Allgemeine Angaben:

Alle vorstehend erwähnten Nukleinsäuren mit Promotoraktivität sowie Expressionseinheiten und Expressionskassetten sind weiterhin in an sich bekannter Weise durch chemische Synthese aus den Nukleotidbausteinen, wie beispielsweise durch Fragmentkondensation, einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Für die vorliegende Erfindungen wurden Methoden und Techniken genutzt, die dem Fachmann, der in mikrobiologischen und rekombinanten DNA-Techniken geübt ist, bekannt sind. Methoden und Techniken für das Wachstum von Bakterienzellen, das Einschleusen von isolierten DNA-Molekülen in die Wirtszelle, und die Isolierung, Klonierung und Sequenzierung von isolierten Nukleinsäuremolekülen usw. sind Beispiele für solche Techniken und Methoden. Diese Methoden sind in vielen Standardliteraturstellen beschrieben: Davis et al., Basic Methods In Molecular Biology (1986); J. H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes Genomes, University Science Books, Mill Valley, Carlifornia (1991); J. Sambrook, E.F. Fritsch and T. Maniatis, Molecular Cloning: A Laboratory Manual, 2nd ed., Cold Spring Harbor Laboratory Press, Cold Spring Harbor, New York (1989); P.B. Kaufmann et al., Handbook of Molcular and Cellular Methods in Biology and Medicine, CRC Press, Boca Raton, Florida (1995); Methods in Plant Molecular Biology and Biotechnology, B.R. Glick and J.E. Thompson, eds., CRC Press, Boca Raton, Florida (1993); and P.F. Smith-Keary, Molecular Genetics of Escherichia coli, The Guilford Press, New York, NY (1989).

Alle Nukleinsäuremoleküle liegen bevorzugt in Form eines isolierten Nukleinsäuremoleküls vor. Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Die Nukleotidsequenzen ermöglichen ferner die Erzeugung von Sonden und Primern, die zur Identifizierung und/oder Klonierung von homologer Sequenzen in anderen Zelltypen und Mikroorganismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

### III. Expressionsvektoren

Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Plasmide eignen sich solche, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS (siehe Beispiel 1), oder solche, die auf pCG4 (US-A 4,489,160) oder pNG2 (Serwold-Davis et al., FEMS Microbiology Letters 66, 119-124 (1990)) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Sirnon et al., Bio/ Technology 1,784-791 (1983)), pK18mob oder pK19mob (Schäfer et al., Gene 145,69-73 (1994)), Bernard et al., Journal of Molecular Biology, 234: 534-541 (1993)), pEM1 (Schrumpf et al. 1991, Journal of Bacteriology 173: 4510--4516) oder pBGS8 (Spratt et as.,1986, Gene 41: 337-342) in Frage. Der Plasmidvektor, der das zu amplifizierende Gen enthält, wird anschließend durch Transformation in den gewünschten Stamm von C. glutamicum überführt. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

### IV. Gene und davon kodierte Proteine

Die Veränderung oder Verursachung der Transkriptionsrate und/oder der Translationsrate von Genen in coryneformen Bakterien im Vergleich zum Wildtyp kann dadurch erfolgen, dass man die Transkription von Genen im coryneformen Bakterium durch erfindungsgemäße Expressionseinheiten reguliert, wobei die Gene in Bezug auf die Expressionseinheiten heterolog sind.

Dies wird bevorzugt dadurch erreicht, dass man
- eine oder mehrere Nukleinsäuren mit Promotoraktivität in das Genom des coryneformen Bakteriums einbringt, so dass die Transkription eines oder mehrerer endogenen Gene unter der Kontrolle der eingebrachten Nukleinsäure mit Promotoraktivität, gegebenenfalls mit veränderter spezifischer Promotoraktivität, erfolgt oder
- ein oder mehrere Nukleinsäurekonstrukte, enthaltend eine Nukleinsäure mit Promotoraktivität und funktionell verknüpft eine oder mehrere, zu transkribierende exogener oder endogener Nukleinsäuren, in das coryneforme Bakterium einbringt.

Werden ein oder mehrere Gene in das Genom des coryneformen Bakteriums eingebracht, so dass die Transkription eines oder mehrerer eingebrachter Gene unter der Kontrolle der Nukleinsäuren erfolgt, dann kann die Insertion so erfolgen, dass das Gen bzw. die Gene in kodierende oder nicht kodierende Bereiche integriert wird/werden. Vorzugsweise erfolgt die Insertion in nicht kodierende Bereiche.

Die Insertion von Nukleinsäurekonstrukten kann dabei chromosomal oder extrachromosomal erfolgen. Vorzugsweise erfolgt die Insertion der Nukleinsäurekonstrukte chromosomal. Eine "chromosomale° Integration ist die Insertion eines DNA-Fragmentes in das Chromosom einer Wirtszelle.

Unter "endogen° werden genetische Informationen, wie beispielsweise Gene, verstanden, die bereits im Wildtypgenom (wie oben definiert) enthalten sind.

Unter "exogen" werden genetische Informationen, wie beispielsweise Gene, verstanden, die im Wildtypgenom nicht enthalten sind. Werden exogene genetische Informationen, beispielsweise die Mehrfachpromotoren-enthaltenden Expressionseinheiten, in das Genom eines Wildtypstammes eingebracht und dadurch ein genetisch veränderter Stamm erzeugt, so sind diese genetischen Informationen im Vergleich des erstmals erzeugten genetischen Stammes mit seinen Nachkommen endogen, dagegen exogen im Vergleich mit dem ursprünglichen Wildtypstamm, der diese genetischen Informationen nicht enthielt.

Unter dem Begriff "Gene" in Bezug auf Regulation der Transkription durch die erfindungsgemäßen Nukleinsäuren mit Promotoraktivität werden vorzugsweise Nukleinsäuren verstanden, die einen zu transkribierenden Bereich, also beispielsweise einen Bereich der die Translation reguliert, einen kodierenden Bereich, sowie gegebenenfalls weitere Regulationselemente, wie beispielsweise einen Terminator, enthalten.

Unter dem Begriff "Gene" in Bezug auf die Regulation der Expression durch die erfindungsgemäßen Expressionseinheiten werden vorzugsweise Nukleinsäuren verstanden, die einen kodierenden Bereich, sowie gegebenenfalls weitere Regulationselemente, wie beispielsweise einen Terminator, enthalten.

Unter einem "kodierenden Bereich" wird eine Nukleinsäuresequenz verstanden, die ein Protein kodiert.

Unter "heterolog" in Bezug auf Nukleinsäuren mit Promotoraktivität und Gene wird verstanden, dass die verwendeten Gene im Wildtyp nicht unter Regulation der Nukleinsäuren mit Promotoraktivität transkribiert werden, sondern das eine neue, im Wildtyp nicht vorkommende funktionelle Verknüpfung entsteht und die funktionelle Kombination aus Nukleinsäure mit Promotoraktivität und spezifischem Gen im Wildtyp nicht vorkommt.

Unter "heterolog" in Bezug auf Expressionseinheiten und Gene wird verstanden, dass die verwendeten Gene im Wildtyp nicht unter Regulation der Expressionseinheiten exprimiert werden, sondern das eine neue, im Wildtyp nicht vorkommende funktionelle Verknüpfung entsteht und die funktionelle Kombination aus erfindungsgemäßer Expressionseinheit und spezifisches Gen im Wildtyp nicht vorkommt.

Die Gene sind ausgewählt aus der Gruppe Nukleinsäuren kodierend ein Protein aus den Biosyntheseweg von Feinchemikalien, wobei die Gene gegebenenfalls weitere Regulationselemente enthalten können.

Die Gene sind bevorzugt ausgewählt aus:Nukleinsäuren, kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, Nukleinsäuren kodierend ein Protein aus dem Zentralstoffwechsel, wobei die Gene gegebenenfalls weitere Regulationselemente enthalten können.

Besonders bevorzugt sind die Proteine aus dem Biosyntheseweg von Aminosäuren ausgewählt, nämlich:
Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase , 6-Phosphofruktokinase, Tetrahydropicolinat-Succinylase, Succinyl-aminoketopimelat-Aminotransferase, Succinyl-Diaminopimelat-Desuccinylase, Diaminopimelat-Epimerase, 6-Phosphogluconat-Dehydrogenase, Glucosephosphat-Isomerase, Phosphoglycerat-Mutase, Enolase, Pyruvat-Kinase, Aspartat-Transaminase, Malat-Enzym.

Proteine und Nukleinsäuren, kodierend diese Proteine sind Proteinsequenzen bzw. Nukleinsäuresequenzen mikrobiellen Ursprungs, vorzugsweise aus Bakterien der Gattung Corynebakterium oder Brevibacterium, bevorzugt aus coryneformen Bakterien, besonders bevorzugt aus Corynebacterium glutamicum.

Beispiele für besonders bevorzugte Proteinsequenzen und die entsprechenden Nukleinsäuresequenzen, kodierend diese Proteine aus dem Biosyntheseweg von Aminosäuren, deren Bezugsdokument, sowie deren Bezeichnung im Bezugsdokument sind in Tabelle 1 aufgelistet:

**Tabelle 1**

| Protein | Nukleinsäure kodierend Protein | Bezugsdokument | SEQ. ID. NO. im Bezugsdokument |
|---|---|---|---|
| Aspartatkinase | ask oder lysC | EP1108790 | DNA: 281 |
| | | | Protein: 3781 |
| Aspartat-Semialdehyd-Dehydrogenase | asd | EP1108790 | DNA:331 |
| | | | Protein: 3831 |
| Dihydrodipicolinate-Synthetase | dapA | WO 0100843 | DNA: 55 |
| | | | Protein: 56 |
| Dihydrodipicolinate-Reduktase | dapB | WO 0100843 | DNA: 35 |
| | | | Protein: 36 |
| Meso-Diaminopimelat-D-Dehydrogenase | ddh | EP1108790 | DNA : 3494 |
| | | | Protein : 6944 |
| Diaminopicolinat-Decarboxylase | lysA | EP1108790 | DNA:3451 |
| | | | Prot.:6951 |
| Lysin-Exporter | lysE | EP1108790 | DNA: 3455 |
| | | | Prot.: 6955 |
| Tetrahydropicolinat-Succinylase | dapD | EP1108790 | DNA : 1229 |
| | | | Prot : 4729 |
| Succinyl-aminoketopimelat-Aminotransferase | dapC | WO 0100843 | DNA : 327 |
| | | | Prot: 328 |
| Succinyl-Diaminopimelat-Desuccinylase | dapE | WO 0100843 | DNA: 31 |
| | | | Prot: 32 |
| Diaminopimelat-Epimerase | dapF | EP1108790 | DNA:2131 |
| | | | Prot: 5632 |
| Arginyl-t-RNA Synthetase | argS | EP1108790 | DNA: 3450 |
| | | | Prot.: 6950 |
| Glucose-6-Phosphat-Dehydrognease | zwf | WO 0100844 | DNA: 243 |
| | | | Prot.: 244 |
| Transketolase | RXA2739 | EP 1108790 | DNA: 1740 |
| | | | Prot: 5240 |
| Transaldolase | RXA2738 | WO 0100844 | DNA: 245 |
| | | | Prot: 246 |
| OPCA | opcA | WO 0100804 | DNA: 79 |
| | | | Prot: 80 |
| 6-Phosphogluconolactonase | RXA2735 | WO 0100844 | DNA: 1 |
| | | | Prot.: 2 |
| 6-Phosphogluconat-Dehydrogenase | | EP1108790 | DNA:1605 |
| | | | Prot: 5105 |
| Glucosephosphat-Isomerase | gpi | WO 0100844 | DNA: 41 |
| | | | Prot.: 42 |
| Malat-Quinon-Oxodoreduktase | mqo | WO 0100844 | DNA: 569 |
| | | | Protein: 570 |
| Glycerinaldehyd-3-Phosphat-Dehydrogenase | gap | WO 0100844 | DNA: 187 |
| | | | Prot.: 188 |
| 3-Phosphoglyceratkinase | pgk | WO 0100844 | DNA: 69 |
| | | | Prot.: 70 |
| Triosephosphat-Isomerase | tpi | WO 0100844 | DNA: 61 |
| | | | Prot.: 62 |
| 1-Phosphofructokinase 1 | pfk1 | WO0100844 | DNA: 55 |
| | | | Protein: 56 |
| 1-Phosphofructokinase 2 | pfk2 | WO0100844 | DNA: 57 |
| | | | Protein: 58 |
| 6-Phosphofructokinase 1 | 6-pfk1 | EP 1108790 | DNA: 1383 |
| | | | Protein: 4883 |
| 6-Phosphofructokinase 2 | 6-pfk2 | DE 10112992 | DNA: 1 |
| | | | Protein: 2 |
| Fructose-1,6-bisphosphatase 1 | fbr1 | EP1108790 | DNA: 1136 |
| | | | Protein: 4636 |
| Pyruvat Oxidase | poxB | WO 0100844 | DNA : 85 |
| | | | Protein: 86 |
| Phosphoglycerat-Mutase | | WO 0100844 | DNA: 49 |
| | | | Prot.: 50 |
| Enolase | eno | WO 0100844 | DNA: 71 |
| | | | Prot.: 72 |
| Pyruvat-Kinase | pykA | WO 0100844 | DNA: 75 |
| | | | Prot.: 76 |
| Pyruvat-Kinase | pykA | EP1108790 | DNA: 3328 |
| | | | Prot.: 6828 |
| Pyruvat-Carboxylase | pycA | EP1108790 | DNA: 765 |
| | | | Prot.: 4265 |
| Aspartat-Transaminase | | EP1108790 | DNA: 3226 |
| | | | Prot: 4726 |
| | | | DNA: 3134 |
| | | | Prot.: 6634 |
| | | | DNA: 2861 |
| | | | Prot.: 6361 |
| | | | DNA: 911 |
| | | | Prot.: 4411 |
| PEP-Carboxylase | pck | EP1108790 | DNA: 3470 |
| | | | Prot.: 6970 |
| Malat-Enzym | malE | EP1108790 | DNA: 3328 |
| | | | Prot: 6828 |
| Biotin-Ligase | birA | EP1108790 | DNA: 786 |
| | | | Prot.: 4286 |
| Homoserin Kinase | thrB | WO 0100843 | DNA: 173 |
| | | | Prot.: 174 |
| Threonin Synthase | thrC | WO 0100843 | DNA: 175 |
| | | | Prot.: 176 |
| Threonin Export Carrier | thrE | WO 0251231 | DNA: 41 |
| | | | Prot.: 42 |
| Threonin Efflux Protein | RXA2390 | WO 0100843 | DNA: 7 |
| | | | Prot.: 8 |
| Threonin Dehydratase | ilva | EP 1108790 | DNA: 2328 |
| | | | Prot.: 5828 |
| Homoserin-O-Acetyltransferase | metA | EP 1108790 | DNA:727 |
| | | | Prot: 4227 |
| Cystathionin-gammasynthase | metB | EP 1108790 | DNA:3491 |
| | | | Prot: 6991 |
| Cystathionin-beta-Lyase | metC | EP 1108790 | DNA:2535 |
| | | | Prot: 6035 |
| Coenzym B12-abhängige Methionin-Synthase, - | metH | EP 1108790 | DNA:1663 |
| | | | Prot: 5163 |
| O-Acetylhomoserin-Sulfhydrylase | metY | EP 1108790 | DNA:726 |
| | | | Prot: 4226 |
| Methylentetrahydrofolat-Reduktase | metF | EP 1108790 | DNA:2379 |
| | | | Prot: 5879 |
| D-3-Phosphoglycerat-Dehydrogenase | serA | EP 1108790 | DNA:1415 |
| | | | Prot: 4915 |
| Phosphoserin-Phosphatase 1 | serB | WO 0100843 | DNA: 153 |
| | | | Prot.: 154 |
| Phosphoserin-Phosphatase 2 | serB | EP 1108790 | DNA: 467 |
| | | | Prot: 3967 |
| Phosphoserin-Phosphatase 3 | serB | EP 1108790 | DNA: 334 |
| | | | Prot.: 3834 |
| Phosphoserin-Aminotransferase | serC | WO 0100843 | DNA: 151 |
| | | | Prot.: 152 |
| Serin Acetyl-Transferase | cysE | WO 0100843 | DNA: 243 |
| | | | Prot.: 244 |
| Cystein-Synthase I | cysK | EP 1108790 | DNA: 2817 |
| | | | Prot.: 6317 |
| Cystein Synthase II | CysM | EP 1108790 | DNA: 2338 |
| | | | Prot.: 5838 |
| Homoserin-Dehydrogenase | hom | EP 1108790 | DNA: 3452 |
| | | | Prot.: 6952 |
| Coenzym B12-unabhängige Methionin-Synthase | metE | WO 0100843 | DNA:755 |
| | | | Prot.: 756 |
| Serin-Hydroxymethyltransferase | glyA | WO 0100843 | DNA: 143 |
| | | | Prot.: 144 |
| Protein in Sulfat-Reduktion | RXA247 | EP 1108790 | DNA: 3089 |
| | | | Prot.: 6589 |
| Protein in Sulfat-Reduktion | RXA248 | EP 1108790 | DNA: 3090 |
| | | | Prot.: 6590 |
| Sulfatadenyltransferase Untereinheit 1 | CysN | EP 1108790 | DNA: 3092 |
| | | | Prot.: 6592 |
| Sulfatadenyltransferase Untereinheit 2 | CysD | EP 1108790 | DNA: 3093 |
| | | | Prot.: 6593 |
| Phosphoadenosin Phosphosulfat Reduktase | CysH | WO 02729029 | DNA: 7 |
| | | | Prot.: 8 |
| Ferredoxin-Sulfit-Reduktase | RXA073 | WO 0100842 | DNA: 329 |
| | | | Prot.: 330 |
| Ferredoxin NADP Reduktase | RXA076 | WO 0100843 | DNA: 79 |
| | | | Prot.: 80 |
| Transkriptioneller Regulator LuxR | luxR | WO 0100842 | DNA: 297 |
| | | | Protein: 298 |
| Transkriptioneller Regulator LysR1 | lysR1 | EP 1108790 | DNA: 676 |
| | | | Protein: 4176 |
| Transkriptioneller Regulator LysR2 | lysR2 | EP 1108790 | DNA: 3228 |
| | | | Protein: 6728 |
| Transkriptioneller Regulator LysR3 | lysR3 | EP 1108790 | DNA: 2200 |
| | | | Protein: 5700 |
| RXA00655-Regulator | RXA655 | US2003162267.2 | DNA: 1 |
| | | | Prot.: 2 |
| RXN02910-Regulator | RXN291 0 | US2003162267.2 | DNA: 5 |
| | | | Prot.: 6 |

Aus den oben aufgelisteten Genen ist bevorzugt das zu regulierende Target-Gen G ausgewählt.

Weitere besonders bevorzugte Proteinsequenzen aus dem Biosyntheseweg von Aminosäuren, weisen jeweils die in Tabelle 1 für dieses Protein angegebene Aminosäuresequenz auf, wobei das jeweilige Protein jeweils an mindestens einer der in Tabelle 2, Spalte 2 für diese Aminosäuresequenz angegebenen Aminosäurepositionen eine andere proteinogene Aminosäure aufweist als die jeweilige, in Tabelle 2, Spalte 3 in der gleichen Zeile angegebene Aminosäure. In einer weiter bevorzugten Ausführungsform, weisen die Proteine an mindestens einer der in Tabelle 2, Spalte 2 für die Aminosäuresequenz angegebenen Aminosäureposition die in Tabelle 2, Spalte 4 in der gleichen Zeile angegebene Aminosäure auf. Es handelt sich bei den in Tabelle 2 angegebenen Proteinen um mutierte Proteine des Biosyntheseweges von Aminosäuren, die besonders vorteilhafte Eigenschaften aufweisen und sich deshalb insbesondere zur Expression der entsprechenden Nukleinsäuren durch ein erfindungsgemäßes Promotorkonstrukt und zur Herstellung von Aminosäuren eignen. Beispielsweise führt die Mutation T311l zu einem Ausschalten der feedback-Inhibierung von ask.

Die entsprechenden Nukleinsäuren, die ein vorstehend beschriebenes mutiertes Protein aus Tabelle 2 kodieren, lassen sich durch übliche Verfahren herstellen.

Als Ausgangspunkt zur Herstellung der Nukleinsäuresequenzen, kodierend ein mutiertes Protein, eignet sich beispielsweise das Genom eines *Corynebacterium glutamicum-*Stammes, der von der American Type Culture Collection unter der Bezeichnung ATCC 13032 erhältlich ist oder die in Tabelle 1 in Bezug genommenen Nukleinsäuresequenzen. Für die Rückübersetzung der Aminosäuresequenz der mutierten Proteine in die Nukleinsäuresequenzen, kodierend diese Proteine ist es vorteilhaft, die codon usage desjenigen Organismus zu verwenden, in den die Nukleinsäuresequenz eingebracht werden soll oder in der die Nukleinsäuresequenz vorliegt. Beispielsweise ist es vorteihaft für *Corynebacterium glutamicum* die codon usage von *Corynebacterium glutamicum* zu verwenden. Die codon usage des jeweiligen Organismus lässt sich in an sich bekannter Weise aus Datenbanken oder Patentanmeldungen ermitteln, die zumindest ein Protein und ein Gen, das dieses Protein kodiert, aus dem gewünschten Organismus beschreiben.

Die Angaben in Tabelle 2 sind folgendermaßen zu verstehen:
In Spalte 1 "Identifikation" wird eine eindeutige Bezeichnung für jede Sequenz in Bezug auf Tabelle 1 aufgeführt.
In Spalte 2 "AS-POS" bezieht sich die jeweilige Zahl auf die Aminosäureposition der entsprechenden Polypeptidsequenz aus Tabelle 1. Eine "26" in der Spalte "AS-POS" bedeutet demzufolge die Aminosäureposition 26 der entsprechend angegebenen Polypeptidsequenz. Die Zählung der Position beginnt N-Terminal bei +1.
In Spalte 3 "AS-Wildtyp" bezeichnet der jeweilige Buchstabe die Aminosäure - dargestellt im Ein-Buchstaben-Code- an der in Spalte 2 angegebenen Position beim entsprechenden Wildtyp-Stamm der Sequenz aus Tabelle 1.
In Spalte 4 "AS-Mutante" bezeichnet der jeweilige Buchstabe die Aminosäure - dargestellt im Ein-Buchstaben-Code- an der in Spalte 2 angegebenen Position beim entsprechenden Mutanten-Stamm.
In Spalte 5 "Funktion" wird die physiologische Funktion der entsprechenden Polypeptidsequenz aufgeführt.

Für ein mutiertes Protein mit einer bestimmten Funktion (Spalte 5) und einer bestimmten Ausgangsaminosäuresequenz (Tabelle 1) werden in den Spalten 2,3 und 4 mindestens eine Mutation, bei einigen Sequenzen auch mehrere Mutationen beschrieben. Diese mehreren Mutationen beziehen sich immer auf die jeweils obenstehende, nächstliegendste Ausgangsaminosäuresequenz (Tabelle 1). Unter dem Begriff "mindestens eine der Aminsäurepositionen" einer bestimmten Aminosäuresequenz wird vorzugsweise mindestens eine der für diese Aminosäuresequenz in Spalte 2, 3 und 4 beschriebenen Mutationen verstanden.

Ein-Buchstaben-Code der proteinogenen Aminosäuren:

| | | |
|---|---|---|
| A Alanin | I Isoleucin | Q Glutamin |
| C Cystein | K Lysin | R Arginin |
| D Aspartat | L Leucin | S Serin |
| E Glutamat | M Methionin | T Threonin |
| F Phenylalanin | N Asparagin | V Valin |
| G Glycin | P Prolin | W Tryptophan |
| H His | | Y Tyrosin |

**Tabelle 2**

| Spalte 1 | Spalte 2 | Spalte 3 | Spalte 4 | Spalte 5 |
|---|---|---|---|---|
| Identifikation | AS Position | AS Wildtyp | AS Mutante | Funktion |
| ask | 317 | S | A | Aspartatkinase |
| | 311 | T | I | |
| | 279 | A | T | |
| asd | 66 | D | G | Aspartat-Semialdehyd-Dehydrogenase |
| | 234 | R | H | |
| | 272 | D | E | |
| | 285 | K | E | |
| | 20 | L | F | |
| dapA | 2 | S | A | Dihydrodipicolinat Synthetase |
| | 84 | K | N | |
| | 85 | L | V | |
| dapB | 91 | D | A | Dihydrodipicolinat-Reduktase |
| | 83 | D | N | |
| ddh | 174 | D | E | Meso-Diaminopimelat-D-Dehydrogenase |
| | 235 | F | L | |
| | 237 | S | A | |
| lysA | 265 | A | D | Diaminopicolinat-Decarboxylase |
| | 320 | D | N | |
| | 332 | I | V | |
| argS | 355 | G | D | Arginyl-t-RNA-Synthetase |
| | 156 | A | S | |
| | 513 | V | A | |
| | 540 | H | R | |
| zwf | 8 | S | T | Glucose-6-Phosphat-Dehydrogenase |
| | 150 | T | A | |
| | 321 | G | S | |
| gap | 264 | G | S | Glycerinaldehyd-3-Phosphat-Dehydrogenase |
| pycA | 7 | S | L | Pyruvat-Carboxylase |
| | 153 | E | D | |
| | 182 | A | S | |
| | 206 | A | S | |
| | 227 | H | R | |
| | 455 | A | G | |
| | 458 | P | S | |
| | 639 | S | T | |
| | 1008 | R | H | |
| | 1059 | S | P | |
| | 1120 | D | E | |
| pck | 162 | H | Y | PEP-Carboxylase |
| | 241 | G | D | |
| | 829 | T | R | |
| thrB | 103 | S | A | Homoserin Kinase |
| | 190 | T | A | |
| | 133 | A | V | |
| | 138 | P | S | |
| thrC | 69 | G | R | Threonin Synthase |
| | 478 | T | I | |
| RXA330 | 85 | I | M | Threonin-Efflux Protein |
| | 161 | F | I | |
| | 195 | G | D | |
| hom | 104 | V | I | Homoserin-Deydrogenase |
| | 116 | T | I | |
| | 148 | G | A | |
| | 59 | V | A | |
| | 270 | T | S | |
| | 345 | R | P | |
| | 268 | K | N | |
| | 61 | D | H | |
| | 72 | E | Q | |
| lysR1 | 80 | R | H | transkriptioneller Regulator LysR1 1 |
| lysR3 | 142 | R | W | transkriptioneller Regulator LysR3 |
| | 179 | A | T | |
| RXA2739 | 75 | N | D | Transketolase |
| | 329 | A | T | |
| | 332 | A | T | |
| | 556 | V | I | |
| RXA2738 | 242 | K | M | Transaldolase |
| opcA | 107 | Y | H | OpcA |
| | 219 | K | N | |
| | 233 | P | S | |
| | 261 | Y | H | |
| | 312 | S | F | |
| | 65 | G | R | Aspartat-1-Decarboxylase |
| | 33 | G | S | 6- Phosphogluconolactonase |

### Genetisch veränderte Mikroorganismen

Das Einbringen der vorstehend beschriebenen Expressionskassetten in coryneforme Bakterien, erfolgt nach dem Fachmann bekannten Methoden, wie Konjugation oder Elektroporation (wie z.B. Liebl et al (1989) FEMS Microbiology Letters 53, 299-303).

Die Selektion von integrierten Expressionskassetten erfolgt vorzugsweise durch die SacB-Methode. Die SacB-Methode ist dem Fachmann bekannt und beispielsweise in Schäfer A, Tauch A, Jäger W, Kalinowski J, Thierbach G, Pühler A.; Small mobilizable multi-purpose cloning vectors derived from the Escherichia coli plasmids pK18 and K19: selection of defined deletions in the chromosome of Corynebacterium glutamicum, Gene. 1994 Jul 22;145(1):69-73 und Blomfield IC, Vaughn V, Rest RF, Eisenstein BI.; Allelic exchange in Escherichia coli using the Bacillus subtilis sacB gene and a temperature-sensitive pSC101 replicon; Mol Microbiol. 1991 Jun;5(6): 1447-57 beschrieben.

Bevorzugt werden erfindungsgemäß als Ausgangsmikroorganismen solche verwendet, welche das gewünschte Wertprodukt (Feinchemikalie/Protein) bereits produzieren.

Die Erfindung betrifft daher auch einen genetisch verändertes coryneformes Bakterium, das eine Expressionskassette oder einen die Expressionskassette umfassenden Vektor enthält.

Besonders bevorzugt betrifft die vorliegende Erfindung genetisch veränderte coryneforme Bakterien, die einen Vektor, insbesondere Pendelvektor oder Plasmidvektor, der wenigstens eine Expressionskassette trägt, enthalten.

Bevorzugte coryneforme Bakterien sind Bakterien der Gattung Corynebacterium, insbesondere der Arten Corynebacterium glutamicum, Corynebacterium acetoglutamicum, Corynebacterium acetoacidophilum, Corynebacterium thermoaminogenes, Corynebacterium melassecola und Corynebacterium efficiens oder der Gattung Brevibacterium, insbesondere der Arten Brevibacterium flavum, Brevibacterium lactofermentum und Brevibacterium divaricatum.

Besonders bevorzugte Bakterien der Gattungen Corynebacterium und Brevibacterium sind ausgewählt aus der Gruppe Corynebacterium glutamicum ATCC 13032, Corynebacterium acetoglutamicum ATCC 15806, Corynebacterium acetoacidophilum ATCC 13870, Corynebacterium thermoaminogenes FERM BP-1539, Corynebacterium melassecola ATCC 17965, Corynebacterium efficiens DSM 44547, Corynebacterium efficiens DSM 44548. Corynebacterium efficiens DSM 44549, Brevibacterium flavum ATCC 14067, Brevibacterium lactofermentum ATCC 13869, Brevibacterium divaricatum ATCC 14020, Corynebacterium glutamicum KFCC10065 und Corynebacterium glutamicum ATCC 21608 sowie Stämme, die davon abgeleitet sind, z.B. durch klassische Mutation und Selektion oder durch gerichtete Mutation.

Mit der Abkürzung KFCC ist die Korean Federation of Culture Collection gemeint, mit der Abkürzung ATCC die American type strain culture collection, mit der Abkürzung DSM (bzw. DSMZ) die Deutsche Sammlung von Mikroorganismen (Deutsche Sammlung von Mikroorganismen und Zellkulturen).

Weitere besonders bevorzugte Bakterien der Gattungen Corynebacterium und Brevibacterium sind in Tabelle 3 aufgelistet:

**Tabelle 3**

| Bakterium | | Hinterlegungsnummer | | | | | | | |
|---|---|---|---|---|---|---|---|---|---|
| Genus | species | ATCC | FERM | NRRL | CEC T | NCIMB | CBS | NCT C | DSM Z |
| Brevibacterium | ammoniagenes | 21054 | | | | | | | |
| Brevibacterium | ammoniagenes | 19350 | | | | | | | |
| Brevibacterium | ammoniagenes | 19351 | | | | | | | |
| Brevibacterium | ammoniagenes | 19352 | | | | | | | |
| Brevibacterium | ammoniagenes | 19353 | | | | | | | |
| Brevibacterium | ammoniagenes | 19354 | | | | | | | |
| Brevibacterium | ammoniagenes | 19355 | | | | | | | |
| Brevibacterium | ammoniagenes | 19356 | | | | | | | |
| Brevibacterium | ammoniagenes | 21055 | | | | | | | |
| Brevibacterium | ammoniagenes | 21077 | | | | | | | |
| Brevibacterium | ammoniagenes | 21553 | | | | | | | |
| Brevibacterium | ammoniagenes | 21580 | | | | | | | |
| Brevibacterium | ammoniagenes | 39101 | | | | | | | |
| Brevibacterium | butanicum | 21196 | | | | | | | |
| Brevibacterium | divaricatum | 21792 | P928 | | | | | | |
| Brevibacterium | flavum | 21474 | | | | | | | |
| Brevibacterium | flavum | 21129 | | | | | | | |
| Brevibacterium | flavum | 21518 | | | | | | | |
| Brevibacterium | flavum | | | B11474 | | | | | |
| Brevibacterium | flavum | | | B11472 | | | | | |
| Brevibacterium | flavum | 21127 | | | | | | | |
| Brevibacterium | flavum | 21128 | | | | | | | |
| Brevibacterium | flavum | 21427 | | | | | | | |
| Brevibacterium | flavum | 21475 | | | | | | | |
| Brevibacterium | flavum | 21517 | | | | | | | |
| Brevibacterium | flavum | 21528 | | | | | | | |
| Brevibacterium | flavum | 21529 | | | | | | | |
| Brevibacterium | flavum | | | B11477 | | | | | |
| Brevibacterium | flavum | | | B11478 | | | | | |
| Brevibacterium | flavum | 21127 | | | | | | | |
| Brevibacterium | flavum | | | B11474 | | | | | |
| Brevibacterium | healii | 15527 | | | | | | | |
| Brevibacterium | ketoglutamicum | 21004 | | | | | | | |
| Brevibacterium | ketoglutamicum | 21089 | | | | | | | |
| Brevibacterium | ketosoreductum | 21914 | | | | | | | |
| Brevibacterium | lactofermentum | | | | 70 | | | | |
| Brevibacterium | lactofermentum | | | | 74 | | | | |
| Brevibacterium | lactofermentum | | | | 77 | | | | |
| Brevibacterium | lactofermentum | 21798 | | | | | | | |
| Brevibacterium | lactofermentum | 21799 | | | | | | | |
| Brevibacterium | lactofermentum | 21800 | | | | | | | |
| Brevibacterium | lactofermentum | 21801 | | | | | | | |
| Brevibacterium | lactofermentum | | | B11470 | | | | | |
| Brevibacterium | lactofermentum | | | B11471 | | | | | |
| Brevibacterium | lactofermentum | 21086 | | | | | | | |
| Brevibacterium | lactofermentum | 21420 | | | | | | | |
| Brevibacterium | lactofermentum | 21086 | | | | | | | |
| Brevibacterium | lactofermentum | 31269 | | | | | | | |
| Brevibacterium | linens | 9174 | | | | | | | |
| Brevibacterium | linens | 19391 | | | | | | | |
| Brevibacterium | linens | 8377 | | | | | | | |
| Brevibacterium | paraffinolyticum | | | | | 11160 | | | |
| Brevibacterium | spec. | | | | | | 717.73 | | |
| Brevibacterium | spec. | | | | | | 717.73 | | |
| Brevibacterium | spec. | 14604 | | | | | | | |
| Brevibacterium | spec. | 21860 | | | | | | | |
| Brevibacterium | spec. | 21864 | | | | | | | |
| Brevibacterium | spec. | 21865 | | | | | | | |
| Brevibacterium | spec. | 21866 | | | | | | | |
| Brevibacterium | spec. | 19240 | | | | | | | |
| Corynebacterium | acetoacidophilum | 21476 | | | | | | | |
| Corynebacterium | acetoacidophilum | 13870 | | | | | | | |
| Corynebacterium | acetoglutamicum | | | B11473 | | | | | |
| Corynebacterium | acetoglutamicum | | | B11475 | | | | | |
| Corynebacterium | acetoglutamicum | 15806 | | | | | | | |
| Corynebacterium | acetoglutamicum | 21491 | | | | | | | |
| Corynebacterium | acetoglutamicum | 31270 | | | | | | | |
| Corynebacterium | acetophilum | | | B3671 | | | | | |
| Corynebacterium | ammoniagenes | 6872 | | | | | | 2399 | |
| Corynebacterium | ammoniagenes | 15511 | | | | | | | |
| Corynebacterium | fujiokense | 21496 | | | | | | | |
| Corynebacterium | glutamicum | 14067 | | | | | | | |
| Corynebacterium | glutamicum | 39137 | | | | | | | |
| Corynebacterium | glutamicum | 21254 | | | | | | | |
| Corynebacterium | glutamicum | 21255 | | | | | | | |
| Corynebacterium | glutamicum | 31830 | | | | | | | |
| Corynebacterium | glutamicum | 13032 | | | | | | | |
| Corynebacterium | glutamicum | 14305 | | | | | | | |
| Corynebacterium | glutamicum | 15455 | | | | | | | |
| Corynebacterium | glutamicum | 13058 | | | | | | | |
| Corynebacterium | glutamicum | 13059 | | | | | | | |
| Corynebacterium | glutamicum | 13060 | | | | | | | |
| Corynebacterium | glutamicum | 21492 | | | | | | | |
| orynebacterium | glutamicum | 21513 | | | | | | | |
| Corynebacterium | glutamicum | 21526 | | | | | | | |
| Corynebacterium | glutamicum | 21543 | | | | | | | |
| Corynebacterium | glutamicum | 13287 | | | | | | | |
| Corynebacterium | glutamicum | 21851 | | | | | | | |
| Corynebacterium | glutamicum | 21253 | | | | | | | |
| Corynebacterium | glutamicum | 21514 | | | | | | | |
| Corynebacterium | glutamicum | 21516 | | | | | | | |
| Corynebacterium | glutamicum | 21299 | | | | | | | |
| Corynebacterium | glutamicum | 21300 | | | | | | | |
| Corynebacterium | glutamicum | 39684 | | | | | | | |
| Corynebacterium | glutamicum | 21488 | | | | | | | |
| orynebacterium | glutamicum | 21649 | | | | | | | |
| Corynebacterium | glutamicum | 21650 | | | | | | | |
| Corynebacterium | glutamicum | 19223 | | | | | | | |
| Corynebacterium | glutamicum | 13869 | | | | | | | |
| Corynebacterium | glutamicum | 21157 | | | | | | | |
| Corynebacterium | glutamicum | 21158 | | | | | | | |
| Corynebacterium | glutamicum | 21159 | | | | | | | |
| Corynebacterium | glutamicum | 21355 | | | | | | | |
| orynebacterium | glutamicum | 31808 | | | | | | | |
| Corynebacterium | glutamicum | 21674 | | | | | | | |
| Corynebacterium | glutamicum | 21562 | | | | | | | |
| Corynebacterium | glutamicum | 21563 | | | | | | | |
| Corynebacterium | glutamicum | 21564 | | | | | | | |
| Corynebacterium | glutamicum | 21565 | | | | | | | |
| Corynebacterium | glutamicum | 21566 | | | | | | | |
| Corynebacterium | glutamicum | 21567 | | | | | | | |
| Corynebacterium | glutamicum | 21568 | | | | | | | |
| Corynebacterium | glutamicum | 21569 | | | | | | | |
| Corynebacterium | glutamicum | 21570 | | | | | | | |
| Corynebacterium | glutamicum | 21571 | | | | | | | |
| Corynebacterium | glutamicum | 21572 | | | | | | | |
| Corynebacterium | glutamicum | 21573 | | | | | | | |
| Corynebacterium | glutamicum | 21579 | | | | | | | |
| Corynebacterium | glutamicum | 19049 | | | | | | | |
| Corynebacterium | glutamicum | 19050 | | | | | | | |
| Corynebacterium | glutamicum | 19051 | | | | | | | |
| Corynebacterium | glutamicum | 19052 | | | | | | | |
| Corynebacterium | glutamicum | 19053 | | | | | | | |
| Corynebacterium | glutamicum | 19054 | | | | | | | |
| Corynebacterium | glutamicum | 19055 | | | | | | | |
| Corynebacterium | glutamicum | 19056 | | | | | | | |
| Corynebacterium | glutamicum | 19057 | | | | | | | |
| Corynebacterium | glutamicum | 19058 | | | | | | | |
| Corynebacterium | glutamicum | 19059 | | | | | | | |
| Corynebacterium | glutamicum | 19060 | | | | | | | |
| Corynebacterium | glutamicum | 19185 | | | | | | | |
| Corynebacterium | glutamicum | 13286 | | | | | | | |
| Corynebacterium | glutamicum | 21515 | | | | | | | |
| Corynebacterium | glutamicum | 21527 | | | | | | | |
| Corynebacterium | glutamicum | 21544 | | | | | | | |
| Corynebacterium | glutamicum | 21492 | | | | | | | |
| Corynebacterium | glutamicum | | | B8183 | | | | | |
| Corynebacterium | glutamicum | | | B8182 | | | | | |
| Corynebacterium | glutamicum | | | B12416 | | | | | |
| Corynebacterium | glutamicum | | | B12417 | | | | | |
| Corynebacterium | glutamicum | | | B12418 | | | | | |
| Corynebacterium | glutamicum | | | B11476 | | | | | |
| Corynebacterium | glutamicum | 21608 | | | | | | | |
| Corynebacterium | lilium | | P973 | | | | | | |
| Corynebacterium | nitrilophilus | 21419 | | | | 11594 | | | |
| Corynebacterium | spec. | | P4445 | | | | | | |
| Corynebacterium | spec. | | P4446 | | | | | | |
| Corynebacterium | spec. | 31088 | | | | | | | |
| Corynebacterium | spec. | 31089 | | | | | | | |
| Corynebacterium | spec. | 31090 | | | | | | | |
| Corynebacterium | spec. | 31090 | | | | | | | |
| Corynebacterium | spec. | 31090 | | | | | | | |
| Corynebacterium | spec. | 15954 | | | | | | | 20145 |
| Corynebacterium | spec. | 21857 | | | | | | | |
| Corynebacterium | spec. | 21862 | | | | | | | |
| Corynebacterium | spec. | 21863 | | | | | | | |

Die Abkürzungen haben folgende Bedeutung:
ATCC: American Type Culture Collection, Rockville, MD, USA
FERM: Fermentation Research Institute, Chiba, Japan
NRRL: ARS Culture Collection, Northern Regional Research Laboratory, Peoria, IL, USA
CECT: Coleccion Espanola de Cultivos Tipo, Valencia, Spain
NCIMB: National Collection of Industrial and Marine Bacteria Ltd., Aberdeen, UK
CBS: Centraalbureau voor Schimmelcultures, Baarn, NL
NCTC: National Collection of Type Cultures, London, UK
DSMZ: Deutsche Sammlung von Mikroorganismen und Zellkulturen, Braunschweig, Germany

Besonders bevorzugt sind dabei Mikroorganismen der Bakterien der Gattung Corynebacterium, insbesondere diejenigen, die bereits in der Lage sind, L-Lysin, L-Methionin und/oder L-Threonin herzustellen. Dies sind insbesondere Corynebakterien bei denen beispielsweise das Gen kodierend für eine Aspartatkinase (ask-Gen) dereguliert ist oder die feed-back-Inhibierung aufgehoben oder reduziert ist. Beispielsweise weisen solche Bakterien im ask-Gen eine Mutation auf, die zu einer Reduzierung oder Aufhebung der feed-back-Inhibierung führen, wie beispielsweise die Mutation T311I.

Durch die Expressionseinheiten ist es möglich, in den vorstehend beschriebenen, erfindungsgemäßen genetisch veränderten coryneformen Bakterien die Stoffwechselwege zu spezifischen biosynthetischen Produkten zu regulieren.

Dazu werden beispielsweise Stoffwechselwege, die zu einem spezifischen biosynthetischen Produkt führen, durch Verursachung oder Erhöhung der Transkriptionsrate bzw. Expressionsrate von Genen dieses Biosyntheseweges verstärkt in dem die erhöhte Proteinmenge zu einer erhöhten Gesamtaktivität dieser Proteine des gewünschten Biosyntheseweges und damit zu einem verstärkten Stoffwechselfluss zu dem gewünschten biosynthetischen Produkt führt.

Weiterhin können Stoffwechselwege, die von einem spezifischen biosynthetischen Produkt wegführen, durch Reduzierung der Transkriptionsrate bzw. Expressionsrate von Genen dieses wegführenden Biosyntheseweges abgeschwächt werden in dem die reduzierte Proteinmenge zu einer reduzierten Gesamtaktivität dieser Proteine des unerwünschten Biosyntheseweges und damit zusätzlich zu einem verstärkten Stoffwechselfluss zu dem gewünschten biosynthetischen Produkt führt.

Die erfindungsgemäßen genetisch veränderten coryneformen Bakterien sind beispielsweise in der Lage aus Glucose, Saccharose, Lactose, Fructose, Maltose, Melasse, Stärke, Cellulose oder aus Glycerin und Ethanol biosynthetische Produkte herzustellen.

Die Erfindung betrifft daher ein Verfahren zur Herstellung von biosynthetischen Produkten durch Kultivierung von erfindungsgemäßen, genetisch veränderten coryneformen Bakterien .

Je nach gewünschtem biosynthetischen Produkt muss die Transkriptionsrate bzw. Expressionsrate verschiedener Gene erhöht bzw. reduziert werden. In der Regel ist es vorteilhaft die Transkriptionsrate bzw. Expressionsrate mehrere Gene zu verändern, d.h. die Transkriptionsrate bzw. Expressionsrate einer Kombination von Gene zu Erhöhen und/oder die Transkriptionsrate bzw. Expressionsrate einer Kombination von Gene zu reduzieren.

In den erfindungsgemäßen genetisch veränderten coryneformen Bakterien ist mindestens eine veränderte, das heißt erhöhte oder reduzierte Transkriptionsrate bzw. Expressionsrate eines Gens auf eine Expressionseinheit zurückzuführen.

Weitere, zusätzliche veränderte, d.h. zusätzlich erhöhte oder zusätzlich reduzierte Expressionsraten von weiteren Genen im genetisch veränderten coryneformen Bakterien können, müssen aber nicht auf die Expressionseinheiten zurück gehen.

Die Erfindung betrifft deshalb weiterhin ein Verfahren zur Herstellung von biosynthetischen Produkten durch Kultivierung von erfindungsgemäßen, genetisch veränderten coryneformen Bakterien.

### V. Anwendungsgebiete der Erfindung

Die Expressionskassetten und Vektoren lassen sich beispielsweise besonders vorteilhaft in verbesserten Verfahren zur fermentativen Herstellung von biosynthetischen Produkten wie nachstehend beschrieben verwenden.

Die Mehrfachpromotoren-enthaltenden Expressionseinheiten umfassenden Expressionskassetten weisen insbesondere den Vorteil auf, dass sie eine Modulation der Expression des funktionell verknüpften Strukturgens erlauben.

Die Expressionseinheiten umfassenden Expressionskassetten können zur Veränderung, also zur Erhöhung oder Reduzierung, oder zur Verursachung der Expressionsrate von Genen in Mikroorganismen im Vergleich zum Wildtyp verwendet werden. Dadurch wird die Möglichkeit bereitgestellt, im Falle einer Erhöhung der Expressionsrate die Expression des betreffenden Gens zu maximieren. Durch Wahl einer geeigneten Expressionseinheit kann die Expression aber auch in einer Stärke erfolgen, die unter dem durch eine andere Expressionseinheit erhältlichen Maximalwert liegt, gleichzeitig aber das Expressionsprodukt in einer für den exprimierenden Mikroorganismus verträglicheren Menge liefert. Dies ist insbesondere dann vorteilhaft, wenn zu hohe Konzentrationen an Expressionsprodukt für den exprimierenden Mikroorganismus toxisch sind. Die Expressionseinheiten ermöglichen somit durch Auswahl unter Expressionseinheiten mit jeweils unterschiedlicher Expressionsstärke die Feinregulierung der Expression auf einen - insbesondere bei Langzeitexpression - optimierten Wert.

Ferner können die Expressionseinheiten umfassende Expressionskassetten zur Regulation und Verstärkung der Bildung von verschiedenen biosynthetischen Produkten, wie beispielsweise Feinchemikalien, Proteinen, insbesondere Aminosäuren, in Mikroorganismen, insbesondere in Corynebacterium Species dienen.

### VI. Biosynthetische Produkte und bevorzugte Herstellungsverfahren

Bevorzugte erfindungsgemäß hergestellte biosynthetische Produkte sind Feinchemikalien.

Der Begriff "Feinchemikalie" ist dem Fachmann geläufig und beinhaltet Verbindungen, die von einem Organismus produziert werden und in verschiedenen Industriezweigen Anwendungen finden, wie beispielsweise, jedoch nicht beschränkt auf die pharmazeutische Industrie, die Landwirtschafts-, Kosmetik , Food und Feed-Industrie. Diese Verbindungen umfassen organische Säuren, wie beispielsweise Milchsäure, Bernsteinsäure, Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien.

Besonders bevorzugte biosynthetische Produkte sind ausgewählt aus der Gruppe organische Säuren, Proteine, Nukleotide und Nukleoside, sowohl proteinogene als auch nicht-proteinogene Aminosäuren, Lipide und Fettsäuren, Diole, Kohlehydrate, aromatische Verbindungen, Vitamine und Cofaktoren, Enzyme und Proteine.

Bevorzugte organische Säuren sind Milchsäure, Bernsteinsäure, Weinsäure, Itaconsäure und Diaminopimelinsäure.

Bevorzugte Nukleoside und Nukleotide sind beispielsweise beschrieben in Kuninaka, A. (1996) Nucleotides and related compounds, S. 561-612, in Biotechnology Bd. 6, Rehm et al., Hrsg. VCH: Weinheim und den darin enthaltenen Zitaten.

Bevorzugte biosynthetische Produkte sind weiterhin Lipide, gesättigte und ungesättigte Fettsäuren, wie beispielsweise Arachidonsäure, Diole wie beispielsweise Propandiol und Butandiol, Kohlenhydrate, wie beispielsweise Hyaluronsäure und Trehalose, aromatische Verbindungen, wie beispielsweise aromatische Amine, Vanillin und Indigo, Vitamine und Cofaktoren, wie beispielsweise beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme Polyketide (Cane et al. (1998) Science 282: 63-68), und sämtliche anderen von Gutcho (1983) in Chemicals by Fermentation, Noyes Data Corporation, ISBN: 0818805086 und den darin angegebenen Literaturstellen, beschriebenen Chemikalien.

Besonders bevorzugte biosynthetische Produkte sind Aminosäuren, besonders bevorzugt essentielle Aminosäuren, insbesondere L-Glycin, L-Alanin, L-Leucin, L-Methionin, L-Phenylalanin, L-Tryptophan, L-Lysin, L-Glutamin, L-Glutaminsäure, L-Serin, L-Prolin, L-Valin, L-Isoleucin, L-Cystein, L-Tyrosin, L-Histidin, L-Arginin, L-Asparagin, L-Asparaginsäure und L-Threonin, L-Homoserin, insbesondere L-Lysin, L-Methionin und L-Threonin. Im folgenden wird unter einer Aminosäure, wie beispielsweise Lysin, Methionin und Threonin, sowohl jeweils die L- und die D-Form der Aminosäure, vorzugsweise die L-Form, also beispielsweise L-Lysin, L-Methionin und L-Threonin verstanden.

In den folgenden Abschnitten werden die Lysin-, Methionin- und Threoninherstellung, welche besonders bevorzugt sind, genauer beschrieben.

### a) Lysinherstellung

Die Erfindung betrifft insbesondere ein Verfahren zur Herstellung von Lysin durch Kultivierung von genetisch veränderten coryneformen Bakterien mit erhöhter oder verursachter Expressionsrate mindestens eines Gens im Vergleich zum Wildtyp, wobei man
- die Expression mindestens eines Gens im coryneformen Bakterium durch Einbringen einer das Gen umfassenden erfindungsgemäßen Expressionskassette in den coryneformen Bakterien verursacht oder verändert.

Dabei sind die Gene insbesondere ausgewählt unter Nukleinsäuren kodierend eine Aspartatkinase, Nukleinsäuren kodierend eine Aspartat-Semialdehyd-Dehydrogenase, Nukleinsäuren kodierend eine Diaminopimelat- Dehydrogenase, Nukleinsäuren kodierend eine Diaminopimelat- Decarboxylase, Nukleinsäuren kodierend eine Dihydrodipicolinate-Synthetase, Nukleinsäuren kodierend eine Dihydridipicolinate-Reduktase, Nukleinsäuren kodierend eine Glycerinaldehyd-3-Phosphat Dehydrogenase, Nukleinsäuren kodierend eine 3-Phosphoglycerat-Kinase, Nukleinsäuren kodierend eine Pyruvat Carboxylase, Nukleinsäuren kodierend eine Triosephosphat-Isomerase, Nukleinsäuren kodierend einen Transkriptionellen Regulator LuxR, Nukleinsäuren kodierend einen Transkriptionellen Regulator LysR1, Nukleinsäuren kodierend einen Transkriptionellen Regulator LysR2, Nukleinsäuren kodierend eine Malat-Quinon-Oxodoreduktase, Nukleinsäuren kodierend eine Glucose-6-Phosphat-Deydrognease, Nukleinsäuren kodierend eine 6-Phosphogluconat-Dehydrognease, Nukleinsäuren kodierend eine Transketolase, Nukleinsäuren kodierend eine Transaldolase, Nukleinsäuren kodierend einen Lysin Exporter, Nukleinsäuren kodierend eine Biotin-Ligase, Nukleinsäuren kodierend eine Arginyl-t-RNA-Synthetase, Nukleinsäuren kodierend eine Phosphoenolpyruvat-Carboxylase, Nukleinsäuren kodierend eine Fruktose-1,6-bisphosphatase, Nukleinsäuren kodierend ein Protein OPCA, Nukleinsäuren kodierend eine 1-Phosphofructokinase, Nukleinsäuren kodierend eine 6-Phosphofructokinase Nukleinsäuren kodierend eine Tetrahydropicolinat-Succinylase, Nukleinsäuren kodierend eine Succinyl-aminoketopimelat-Aminotransferase, Nukleinsäuren kodierend eine Succinyl-Diaminopimelat-Desuccinylase, Nukleinsäuren kodierend eine Diaminopimelat-Epimerase, Nukleinsäuren kodierend eine 6-Phosphogluconat-Dehydrogenase, Nukleinsäuren kodierend eine Glucosephosphat-Isomerase, Nukleinsäuren kodierend eine Phosphoglycerat-Mutase, Nukleinsäuren kodierend eine Enolase, Nukleinsäuren kodierend eine Pyruvat-Kinase, Nukleinsäuren kodierend eine Aspartat-Transaminase und Nukleinsäuren kodierend eine Malat-Enzym.

Eine weiter bevorzugte Ausführungsform des vorstehend beschriebenen Verfahrens zur Herstellung von Lysin ist dadurch gekennzeichnet, dass die genetisch veränderten coryneformen Bakterien im Vergleich zum Wildtyp zusätzlich eine erhöhte Aktivität, mindestens einer der Aktivitäten, ausgewählt unter:
Aspartatkinase-Aktivität, Aspartat-Semialdehyd-Dehydrogenase-Aktivität, Diaminopimelat- Dehydrogenase-Aktivität, Diaminopimelat-Decarboxylase-Aktivität, Dihydrodipicolinate-Synthetase-Aktivität, Dihydridipicolinate-Reduktase-Aktivität, Glycerinaldehyd-3-Phosphat Dehydrogenase-Aktivität, 3-Phosphoglycerat-Kinase-Aktivität, Pyruvat Carboxylase-Aktivität, Triosephosphat-Isomerase-Aktivität, Aktivität des Transkriptionellen Regulators LuxR, Aktivität des Transkriptionellen Regulators LysR1, Aktivität des Transkriptionellen Regulators LysR2, Malat-Quinon-Oxodoreduktase-Aktivität, Glucose-6-Phosphat-Deydrognease-Aktivität, 6-Phosphogluconat-Dehydrognease-Aktivität, Transketolase-Aktivität, Transaldolase-Aktivität, Lysin-Exporter-Aktivität, Arginyl-t-RNA-Synthetase-Aktivität, Phosphoenolpyruvat-Carboxylase-Aktivität, Fruktose-1,6-bisphosphatase-Aktivität, Protein OpcA-Aktivität, 1-Phosphofructokinase-Aktivität, 6-Phosphofructokinase-Aktivität, Biotin-Ligase-Aktivität und Tetrahydropicolinat-Succinylase-Aktivität, Succinyl-aminoketopimelat-Aminotransferase-Aktivität, Succinyl-Diaminopimelat-Desuccinylase-Aktivität, Diaminopimelat-Epimerase-Aktivität, 6-Phosphogluconat-Dehydrogenase-Aktivität, Glucosephosphat-Isomerase-Aktivität, Phosphoglycerat-Mutase-Aktivität, Enolase-Aktivität, Pyruvat-Kinase-Aktivität, Aspartat-Transaminase-Aktivität und Malat-Enzym-Aktivität aufweisen.

Eine weiter besonders bevorzugte Ausführungsform des vorstehend beschriebenen Verfahrens zur Herstellung von Lysin ist dadurch gekennzeichnet, dass die genetisch veränderten coryneformen Bakterien im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, O-Acetylhomoserin-Sulfhydrylase-Aktivität, Phosphoenolpyruvat-Carboxykinase-Aktivität, Pyruvat-Oxidase-Aktivität, Homoserine-Kinase-Aktivität, Homoserin-Dehydrogenase-Aktivität, Threonin-Exporter-Aktivität, Threonin-Efflux-Protein-Aktivität, Asparaginase-Aktivität, Aspartat-Decarboxylase-Aktivität und Threonin-Synthase-Aktivität aufweisen.

### b) Methioninherstellung

Die Erfindung betrifft ferner ein Verfahren zur Herstellung von Methionin durch Kultivierung von genetisch veränderten coryneformen Bakterien mit erhöhter oder verursachter Expressionsrate mindestens eines Gens im Vergleich zum Wildtyp, wobei man
- die Expression mindestens eines Gens im coryneformen Bakterium durch Einbringen einer das Gen umfassenden Expressionskassette in den coryneformen Bakterien verursacht oder verändert.

Dabei sind die Gene insbesondere ausgewählt unter Nukleinsäuren kodierend eine Aspartatkinase, Nukleinsäuren kodierend eine Aspartat-Semialdehyd-Dehydrogenase, Nukleinsäuren kodierend eine Homoserin Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin O-Acetyltransferase, Nukleinsäuren kodierend eine Cystahionin-gamma-Synthase, Nukleinsäuren kodierend eine Cystahionin-beta-Lyase, Nukleinsäuren kodierend eine Serin-Hydroxymethyltransferase, Nukleinsäuren kodierend eine O-Acetylhomoserin-Sulfhydrylase, Nukleinsäuren kodierend eine Methylen-Tetrahydrofolat-Reduktase, Nukleinsäuren kodierend eine Phosphoserin-Aminotransferase, Nukleinsäuren kodierend eine Phosphoserin-Phosphatase, Nukleinsäuren kodierend eine Serine Acetyl-Transferase, Nukleinsäuren kodierend eine Cystein-Synthase Aktivität I, Nukleinsäuren kodierend eine Cystein-Synthase Aktivität II , Nukleinsäuren kodierend eine Coenzym B12-abhängige Methionin-Synthase-Aktivität, Nukleinsäuren kodierend eine Coenzym B12-unabhängige Methionin-Synthase-Aktivität, Nukleinsäuren kodierend eine Sulfat-Adenylyltransferase-Aktivität, Nukleinsäuren kodierend eine Phosphoadenosin-Phosphosulfat-Reductase-Aktivität, Nukleinsäuren kodierend eine Ferredoxin-Sulfit-Reduktase-Aktivität, Nukleinsäuren kodierend eine Ferredoxin NADPH-Reduktase Aktivität, Nukleinsäuren kodierend eine Ferredoxin-Aktivität, Nukleinsäuren kodierend ein Protein der Sulfat-Reduktion RXA077, Nukleinsäuren kodierend ein Protein der Sulfat-Reduktion RXA248, Nukleinsäuren kodierend ein Protein der Sulfat-Reduktion RXA247, Nukleinsäuren kodierend eine, RXA0655 Regulator undNukleinsäuren kodierend einen RXN2910 Regulator. Nukleinsäuren kodierend eine 6-Phosphogluconat-Dehydrogenase, Glucosephosphat-Isomerase, Phosphoglycerat-Mutase, Enolase, Pyruvat-Kinase, Aspartat-Transaminase oder Malat-Enzym.

Eine weiter bevorzugte Ausführungsform des vorstehend beschriebenen Verfahrens zur Herstellung von Methionin ist dadurch gekennzeichnet, dass die genetisch veränderten coryneformen Bakterien im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Homoserin Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Homoserin O-Acetyltransferase-Aktivität, Cystahionin-gamma-Synthase-Aktivität, Cystahionin-beta-Lyase-Aktivität Serin-Hydroxymethyltransferase-Aktivität, O-Acetylhomoserin-Sulfhydrylase-Aktivität, Methylen-Tetrahydrofolat-Reduktase-Aktivität, Phosphoserin-Aminotransferase-Aktivität, Phosphoserin-Phosphatase-Aktivität, Serine Acetyl-Transferase-Aktivität, Cystein-Synthase-Aktivität, Cystein-Synthase II -Aktivität, Coenzym B12-abhängige Methionin-Synthase-Aktivität, Coenzym B12-unabhängige Methionin-Synthase-Aktivität, Sulfat-Adenylyltransferase-Aktivität, Phosphoadenosin-Phosphosulfat-Reductase-Aktivität, Ferredoxin-Sulfit-Reduktase-Aktivität, Ferredoxin NADPH-Reduktase Aktivität, Ferredoxin-Aktivität Aktivität Proteins der Sulfat-Reduktion RXA077, Aktivität eines Proteins der Sulfat-Reduktion RXA248, Aktivität eines Proteins der Sulfat-Reduktion RXA247, Aktivität eines RXA655-Regulators und Aktivität eines RXN2910-Regulators, Aktivität einer 6-Phosphogluconat-Dehydrogenase, Aktivität einer Glucosephosphat-Isomerase, Aktivität einer Phosphoglycerat-Mutase, Aktivität einer Enolase, Aktivität einer Pyruvat-Kinase, Aktivität einer Aspartat-Transaminase und Aktivität des Malat-Enzyms aufweisen.

Eine weiter besonders bevorzugte Ausführungsform des vorstehend beschriebenen Verfahrens zur Herstellung von Methionin ist dadurch gekennzeichnet, dass die genetisch veränderten coryneformen Bakterien im Vergleich zum Wildtyp zusätzlich eine reduzierte Aktivität, mindestens einer der Aktivitäten, ausgewählt aus der Homoserine-Kinase-Aktivität, Threonin-Dehydratase-Aktivität, Threonin Synthase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthase-Aktivität, Dihydrodipicolinat Reduktase-Aktivität, und Diaminopicolinat Decarboxylase-Aktivität aufweisen.

### c) Threoninherstellung

Die Erfindung betrifft ferner ein Verfahren zur Herstellung von Threonin durch Kultivierung von genetisch veränderten coryneformen Bakterien mit erhöhter oder verursachter Expressionsrate mindestens eines Gens im Vergleich zum Wildtyp, wobei man
- die Expression mindestens eines Gens im coryneformen Bakterium durch Einbringen einer das Gen umfassenden Expressionskassette in das coryneformen Bakterium verursacht oder verändert.

Dabei sind die Gene insbesondere ausgewählt unter Nukleinsäuren kodierend eine Aspartatkinase, Nukleinsäuren kodierend eine Aspartat-Semialdehyd-Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin-Kinase, Nukleinsäuren kodierend eine Threonin Synthase, Nukleinsäuren kodierend einen Threonin Exporter Carrier, Nukleinsäuren kodierend eine Glucose-6-Phosphat-Dehydrogenase, Nukleinsäuren kodierend eine Transaldolase, Nukleinsäuren kodierend eine Transketolase, Nukleinsäuren kodierend einer Malat-Quinon-Oxidoreductase, Nukleinsäuren kodierend eine 6-Phosphogluconat-Dehydrogenase, Nukleinsäuren kodierend einen Lysin-Exporter, Nukleinsäuren kodierend eine Biotin-Ligase, Nukleinsäuren kodierend eine Phosphoenolpyruvat-Carboxylase, Nukleinsäuren kodierend ein Threonin Efflux-Protein, Nukleinsäuren kodierend eine Fruktose-1,6-bisphosphatase, Nukleinsäuren kodierend ein OpcA Protein, Nukleinsäuren kodierend eine 1-Phosphofructokinase, Nukleinsäuren kodierend eine 6-Phosphofructokinase, und Nukleinsäuren kodierend eine Homoserin-Dehydrogenase und Kukleinsäuren kodierend eine 6-Phosphogluconat-Dehydrogenase, Glucosephosphat-Isomerase, Phosphoglycerat-Mutase, Enolase, Pyruvat-Kinase, Aspartat-Transaminase und Malat-Enzym.

Eine weiter bevorzugte Ausführungsform des vorstehend beschriebenen Verfahrens zur Herstellung von Threonin ist dadurch gekennzeichnet, dass die genetisch veränderten coryneformen Bakterien im Vergleich zum Wildtyp zusätzlich eine erhöhte Aktivität, mindestens einer der Aktivitäten, ausgewählt aus der Gruppe :
Aspartatkinase-Aktivität, Aspartat-Semialdehyd-Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Threonin Synthase-Aktivität, Aktivität eines Threonin Export-Carriers, Transaldolase-Aktivität, Transketolase-Aktivität, Glucose-6-Phosphat-dehydrogenase-Aktivität, Malat-Qinon-Oxidoreductase-Aktivität, Homoserin-Kinase-Aktivität, Biotin-Ligase-Aktivität, Phosphoenolpyruvat-Carboxylase-Aktivität, Threonin-Efflux-Protein-Aktivität, Protein OpcA-Aktivität, 1-Phosphofructokinase-Aktivität, 6-Phosphofructokinase-Aktivität, Fruktose 1,6 bisphosphatase-Aktivität, 6-Phosphogluconat-Dehydrogenase , Homoserin-Dehydrogenase-Aktivität und Aktivität einer 6-Phosphogluconat-Dehydrogenase, Aktivität einer Glucosephosphat-Isomerase, Aktivität einer Phosphoglycerat-Mutase, Aktivität einer Enolase, Aktivität einer Pyruvat-Kinase, Aktivität einer Aspartat-Transaminase und Aktivität des Malat-Enzyms aufweisen.

Eine weiter besonders bevorzugte Ausführungsform des vorstehend beschriebenen Verfahrens zur Herstellung von Threonin ist dadurch gekennzeichnet, dass die genetisch veränderten coryneformen Bakterien im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, Serin-Hydroxymethyltransferase-Aktivität, O-Acetylhomoserin-Sulfhydrylase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthetase-Aktivität, Dihydrodipicolinat Reduktase-Aktivität, Asparaginase-Aktivität, Aspartat-Decarboxylase-Aktivität, Lysin-Exporter-Aktivität, Acetolactat-Synthase-Aktivität , Ketol-Aid-Reductoisomerase-Aktivität , Branched chain aminotransferase- Aktivität , Coenzym B12-abhängige Methionin Synthase- Aktivität , Coenzym B12-unabhängige Methion Synthase- Aktivität, Dihydroxy-acid Dehydratase- Aktivität und Diaminopicolinat Decarboxylase-Aktivität aufweisen.

### d) Weitere Angaben zur erfindungsgemäßen Herstellung von Bioprodukten

Diese zusätzlichen, erhöhten oder reduzierten Aktivitäten mindestens einer der vorstehend beschriebenen Aktivitäten können, müssen aber nicht durch eine oder eine Expressionskassette verursacht sein.

Unter dem Begriff der "Aktivität" eines Proteins wird bei Enzymen die Enzymaktivität des entsprechenden Proteins, bei anderen Proteinen, wie beispielsweise Struktur oder Transport-Proteinen, die physiologische Aktivität der Proteine verstanden.

Die Enzyme sind in der Regel in der Lage ein Substrat in ein Produkt umzuwandeln bzw. diesen Umwandlungsschritt zu katalysieren. Dementsprechend wird unter der "Aktivität" eines Enzyms die in einer bestimmten Zeit durch das Enzym umgesetzte Menge Substrat bzw. gebildete Menge Produkt verstanden.

Bei einer erhöhten Aktivität im Vergleich zum Wildtyp wird somit im Vergleich zum Wildtyp in einer bestimmten Zeit durch das Enzym die umgesetzte Menge Substrat bzw. die gebildete Menge Produkt erhöht.

Beispielsweise beträgt diese Erhöhung der "Aktivität" bei allen vorstehend und nachstehend beschriebenen Aktivitäten mindestens 1 bis 5 %, wie z.B. mindestens 20 %, mindestens 50 %, mindestens 100 %, mindestens 300 %, oder mindestens 500%, oder mindestens 600 % der "Aktivität des Wildtyps".

Bei einer reduzierten Aktivität im Vergleich zum Wildtyp wird somit im Vergleich zum Wildtyp in einer bestimmten Zeit durch das Enzym die umgesetzte Menge Substrat bzw. die gebildete Menge Produkt reduziert.

Unter einer reduzierten Aktivität wird vorzugsweise die teilweise oder im wesentlichen vollständige, auf unterschiedliche zellbiologische Mechanismen beruhende Unterbindung oder Blockierung der Funktionalität dieses Enzyms in einem Mikroorganismus verstanden.

Eine Reduzierung der Aktivität umfasst eine mengenmäßige Verringerung eines Enzyms bis hin zu einem im wesentlichen vollständigen Fehlen des Enzyms (d.h. fehlende Nachweisbarkeit der entsprechenden Aktivität oder fehlende immunologische Nachweisbarkeit des Enzyms). Vorzugsweise wird die 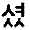 Aktivität im Mikroorganismus im Vergleich zum Wildtyp um mindestens 5 %, wie mindestens 20 %, mindestens 50 %, oder um etwa 100 % reduziert. Insbesondere meint "Reduzierung" auch das vollständige Fehlen der entsprechenden Aktivität.

Die Aktivität bestimmter Enzyme in genetisch veränderten Mikroorganismen sowie im Wildtyp und damit die Erhöhung oder Reduzierung der Enzymaktivität lassen sich nach bekannten Verfahren, wie beispielsweise Enzymassays ermitteln.

Eine zusätzliche Erhöhung von Aktivitäten kann auf verschiedenen Wegen erfolgen, beispielsweise durch Ausschalten von hemmenden Regulationsmechanismen auf Expressions- und Proteinebene oder durch Erhöhung der Genexpression von Nukleinsäuren kodierend die vorstehend beschriebenen Proteine im Vergleich zum Wildtyp.

Die Erhöhung der Genexpression der Nukleinsäuren kodierend die vorstehend beschriebenen Proteine gegenüber dem Wildtyp kann ebenfalls auf verschiedenen Wegen erfolgen, beispielsweise durch Induzierung des Gens durch Aktivatoren oder wie vorstehend beschrieben durch Erhöhung der Promotoraktivität oder Erhöhung der Expressionsaktivität oder durch Einbringen von einer oder mehrerer Genkopien in den Mikroorganismus.

Unter Erhöhung der Genexpression einer Nukleinsäure kodierend ein Protein wird erfindungsgemäß auch die Manipulation der Expression der dem Mikroorganismus eigenen, endogenen Proteine verstanden. Dies kann beispielsweise, wie vorstehend beschrieben durch Veränderung der Promotorsequenzen der Gene, Einbringen von Expressionseinheit zur regulatorischen Kontrolle der Gene und Veränderung der eingebrachten Expressionseinheiten erreicht werden. Eine solche Veränderung, die eine erhöhte Expressionsrate des Gens zur Folge hat, kann beispielsweise durch Deletion oder Insertion von DNA Sequenzen erfolgen.

Es ist, wie vorstehend beschrieben, möglich, die Expression der endogenen Proteine durch die Applikation exogener Stimuli zu verändern. Dies kann durch besondere physiologische Bedingungen, also durch die Applikation von Fremdsubstanzen erfolgen.

Zur Erzielung einer Erhöhung der Genexpression kann der Fachmann weitere unterschiedliche Maßnahmen einzeln oder in Kombination ergreifen. So kann beispielsweise die Kopienzahl der entsprechenden Gene erhöht werden, oder es kann die Promotor-und Regulationsregion oder die Ribosomenbindungsstelle, die sich stromaufwärts des Strukturgens befindet, mutiert werden. Durch induzierbare Promotoren ist es zusätzlich möglich, die Expression im Verlaufe der fermentativen Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biontechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der EP-A-0472869, im US -A-4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der WO 96/15246, bei Malumbres et al. (Gene 134, 15-24 (1993)), in der JP-A-10-229891, bei Jensen und Hammer (Biotechnology and Bioengineering 58,.191-195 (1998)), bei Makrides (Microbiological Reviews 60 : 512-538 (1996) und in bekannten Lehrbüchern der Genetik und Molekularbiologie.

Weiterhin kann es für die Produktion von biosynthetischen Produkten, insbesondere L-Lysin, L-Methionin und L-Threonin, vorteilhaft sein, neben der Expression bzw. Verstärkung eines Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

In einer bevorzugten Ausführungsform erfolgt die Erhöhung der Genexpression einer Nukleinsäure, kodierend eines der vorstehend beschriebenen Proteine, durch Einbringen von mindestens einer Nukleinsäure kodierend ein entsprechendes Protein in den Mikroorganismus. Das Einbringen der Nukleinsäure kann chromosomal oder extrachromosomal erfolgen, also durch Erhöhung der Kopienzahl auf dem Chromosom und oder eine Kopie des Gens auf einem sich replizierenden Plasmid in dem Wirtsmikroorganismus.

Vorzugsweise erfolgt das Einbringen der Nukleinsäure, beispielsweise in Form einer erfindungsgemäßen Expressionskassette, enthaltend die Nukleinsäure, chromosomal, insbesondere durch die vorstehend beschriebene SacB-Methode. Dazu kann prinzipiell jedes Gen, das eines der vorstehend beschriebenen Proteine kodiert, verwendet werden.

Bei genomischen Nukleinsäure-Sequenzen aus eukaryontischen Quellen, die Introns enthalten, sind für den Fall, dass der Wirtsmikroorganismus nicht in der Lage ist oder nicht in die Lage versetzt werden kann, die entsprechenden Proteine zu exprimieren, bevorzugt bereits prozessierte Nukleinsäuresequenzen, wie die entsprechenden cDNAs zu verwenden.

Beispiele für die entsprechenden Gene sind in Tabelle 1 und 2 aufgelistet.

Vorzugsweise erfolgt die Reduzierung der vorstehend beschriebenen Aktivitäten in coryneformen Bakterien durch mindestens eines der nachfolgenden Verfahren:
- Einbringen mindestens einer sense-Ribonukleinsäuresequenz zur Induktion einer Cosuppression oder einer deren Expression gewährleistenden Expressionskassette
- Einbringen mindestens eines DNA- oder Protein-bindenden Faktors für ein entsprechedes Gen, eine RNA oder ein Protein, oder einer dessen Expression gewährleistenden Expressionskassette
- Einbringen mindestens einer den RNA-Abbau bewirkenden viralen Nukleinsäuresequenz oder einer deren Expression gewährleistenden Expressionskassette
- Einbringen mindestens eines Konstruktes zur Erzeugung eines Funktionsverlustes, wie beispielsweise die Generierung von Stop-Codons oder von Verschiebungen im Leseraster, an einem Gen, beispielsweise durch Erzeugung einer Insertion, Deletion, Inversion oder Mutation in einem Gen. Bevorzugt können Knockout-Mutanten mittels gezielter Insertion in das gewünschte Zielgen durch homologe Rekombination oder Einbringen von sequenzspezifischen Nukleasen gegen 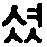 das Zielgen generiert werden.
- Einbringen eines Promotors mit reduzierter Promotoraktivität oder einer Expressionseinheit mit reduzierter Expressionsaktivität.
- Einbringen einer antisense-RNA, die die Translation der sense RNA (mRNA) vermindert.

Dem Fachmann ist bekannt, dass auch weitere Verfahren im Rahmen der vorliegenden Erfindung zur Reduzierung seiner Aktivität oder Funktion eingesetzt werden können. Beispielsweise kann auch das Einbringen einer dominant-negativen Variante eines Proteins oder einer deren Expression gewährleistenden Expressionskassette vorteilhaft sein.

Dabei kann jedes einzelne dieser Verfahren eine Verminderung der Proteinmenge, mRNA-Menge und/oder Aktivität eines Proteins bewirken. Auch eine kombinierte Anwendung ist denkbar. Weitere Methoden sind dem Fachmann bekannt und können die Behinderung oder Unterbindung der Prozessierung des Proteins, des Transports des Proteins oder dessen mRNA, Hemmung der Ribosomenanlagerung, Hemmung des RNA-Spleißens, Induktion eines 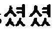 RNA-abbauenden Enzyms und/oder Hemmung der Translationselongation oder -termination umfassen.

### VII. Kultivierung von coryneformen Bakterien und Produktisolierung

Im erfindungsgemäßen Verfahren zur Herstellung von biosynthetischen Produkten wird vorzugsweise dem Kultivierungsschritt der genetisch veränderten coryneformen Bakterien ein Isolieren von biosynthetischen Produkten aus den Mikroorganismen oder/oder aus der Fermentationsbrühe angeschlossen. Diese Schritte können gleichzeitig und/oder vorzugsweisie nach dem Kultivierungsschritt stattfinden.

Die erfindungsgemäßen genetisch veränderten coryneformen Bakterien können kontinuierlich oder diskontinuierlich im batch-Verfahren (Satzkultivierung) oder im fed batch (Zulaufverfahren) oder repeated fed batch Verfahren (repetitives Zulaufverfahren) zur Produktion von biosynthetischen Produkten, insbesondere L-Lysin, L-Methionin und L-Threonin, kultiviert werden. Eine Zusammenfassung über bekannte Kultivierungsmethoden ist im Lehrbuch von Chemiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen

Als Schwefelquelle für die Herstellung von Feinchemikalien, insbesondere von Methionin, können anorganische Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden, Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden entweder durch Hitze (20 min bei 1,5 bar und 121 °C) oder durch Sterilfiltration sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Temperatur der Kultur liegt normalerweise bei 20°C bis 45°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf 0 bis 3 g/l gehalten, beziehungsweise abgesenkt wird.

Die Isolierung von biosynthetischen Produkten aus der Fermentationsbrühe und/oder den Mikroorganismen erfolgt in an sich bekannter Weise entsprechend den physikalisch-chemischen Eigenschaften des biosynthetischen Wertprodukts und den biosynthetischen Nebenprodukten.

Die Fermentationsbrühe kann anschließend beispielsweise weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich die biosynthetischen Produkte, insbesondere L-Lysin, L-Methionin und L-Threonin, weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die biosynthetischen Produkte können in unterschiedlichen Formen anfallen, beispielsweise in Form ihrer Salze oder Ester.

Die Identität und Reinheit der isolierten Verbindung(en) kann durch Techniken des Standes der Technik bestimmt werden. Diese umfassen Hochdruckflüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele näher beschrieben:

### Beispiel 1:

### Herstellung des Vektors pCLiK5MCS

Zunächst wurden Ampicillinresistenz und Replikationsursprung des Vektors pBR322 mit den Oligonukleotidprimern SEQ ID NO:5 und SEQ ID NO: 6 mit Hilfe der Polymerase-Kettenreaktion (PCR) amplifiziert.

Neben den zu pBR322 komplementären Sequenzen, enthält der Oligonukleotidprimer SEQ ID NO: 5 in 5'-3' Richtung die Schnittstellen für die Restriktionsendonukleasen Smal, BamHI, Nhel und AscI und der Oligonukleotidprimer SEQ ID NO: 6 in 5'-3' Richtung die Schnittstellen für die Restriktionsendonukleasen Xbal, Xhol, NotI und Dral. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,1 kb wurde mit dem GFX™PCR, DNA and Gel Band Purification Kit (Amersham Pharmacia, Freiburg) nach Angaben des Herstellers gereinigt. Die stumpfen Enden des DNA-Fragmentes wurden mit dem Rapid DNA Ligation Kit (Roche Diagnostics, Mannheim) nach Angaben des Herstellers miteinander ligiert und der Ligationsansatz nach Standardmethoden wie in Sambrook et al. (Molecular Cloning. A Laboratory Manual, Cold Spring Harbor, beschrieben (1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK1.

Ausgehend vom Plasmid pWLT1 (Liebl et al.,1992) als Template für eine PCR Reaktion wurde mit den Oligonukleotidprimern SEQ ID NO:7 und SEQ ID NO:8 eine Kanamycin-Resistenzcassette amplifiziert.
SEQ ID NO:7:
5'-GAGATCTAGACCCGGGGATCCGCTAGCGGGCTGCTAAAGGAAGCGGA-3`
SEQ ID NO:8
5'-GAGAGGCGCGCCGCTAGCGTGGGCGAAGAACTCCAGCA-3'

Neben den zu pWLT1 komplementären Sequenzen, enthält der Oligonukleotidprimer SEQ ID NO: 7 in 5'-3' Richtung die Schnittstellen für die Restriktionsendonukleasen Xbal, Smal, BamHI, Nhel und der Oligonukleotidprimer SEQ ID NO:8 in 5'-3' Richtung die Schnittstellen für die Restriktionsendonukleasen AscI und Nhel. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,3 kb wurde mit dem GFX™PCR, DNA and Gel Band Purification Kit (Amersham Pharmacia, Freiburg) nach Angaben des Herstellers gereinigt. Das DNA-Fragment wurde mit den Restriktionsendonukleasen Xbal und AscI (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK1 wurde ebenfalls mit den Restriktionsendonukleasen Xbal und AscI geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,1kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben (1989)), in kompetente E.coli XL-1Blue (Stratagene, La Jolla, USA) transformiert. Eine Selektion auf Plasmid tragende Zellen wurde durch das Ausplattieren auf Ampicillin (50µg/ml) und Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK2.

Der Vektor pCLiK2 wurde mit der Restriktionsendonuklease Dral (New England Biolabs, Beverly, USA) geschnitten. Nach Elektrophorese in einem 0,8%igen Agarosegel wurde ein ca. 2,3 kb großes Vektorfragment mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers religiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK3.

Ausgehend vom Plasmid pWLQ2 (Liebl et al., 1992) als Template für eine PCR Reaktion wurde mit den Oligonukleotidprimern SEQ ID NO:9 und SEQ ID NO:10 der Replikationsursprung pHM1519 amplifiziert.
SEQ ID NO:9:
5'-GAGAGGGCGGCCGCGCAAAGTCCCGCTTCGTGAA-3'
SEQ ID NO:10:
5'-GAGAGGGCGGCCGCTCAAGTCGGTCAAGCCACGC-3'

Neben den zu pWLQ2 komplementären Sequenzen, enthalten die Oligonukleotidprimer SEQ ID NO:9 und SEQ ID NO:10 Schnittstellen für die Restriktionsendonuklease Notl. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,7 kb wurde mit dem GFX™PCR, DNA and Gel Band Purification Kit (Amersham Pharmacia, Freiburg) nach Angaben des Herstellers gereinigt. Das DNA-Fragment wurde mit der Restriktionsendonuklease Notl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK3 wurde ebenfalls mit der Restriktionsendonuklease Notl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,3kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5.

Für die Erweiterung von pCLiK5 um eine "multiple cloning site" (MCS) wurden die beide synthetischen, weitestgehend komplementären Oligonukleotide SEQ ID NO:11 und SEQ ID NO:12, die Schnittstellen für die Restriktionsendonukleasen Swal, Xhol, Aatl, Apal, Asp718, MluI, NdeI, SpeI, EcoRV, SalI, ClaI, BamHI, Xbal und Smal enthalten, durch gemeinsames erhitzen auf 95°C und langsames abkühlen zu einem doppelsträngigen DNA-Fragment vereinigt.

Der Vektor pCLiK5 wurde mit den Restriktionsendonuklease XhoI und BamHI (New England Biolabs, Beverly, USA) geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 5,0 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem synthetischen Doppelsträngigen DNA-Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS.

Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS ist als SEQ ID NO:13 aufgeführt.

### Beispiel 2:

### Herstellung des Plasmids PmetA metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO: 14 und SEQ ID NO: 15, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein das metA Gen inklusice des nichkodierenden 5'-Bereichs amplifiziert.
SEQ ID NO: 14
5`-GCGCGGTACCTAGACTCACCCCAGTGCT -3'
und
SEQ ID NO: 15
5'-CTCTACTAGTTTAGATGTAGAACTCGATGT -3'

Das erhaltene DNA Fragment von ca. 1,3 kb Größe wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Asp718 und Spel (Roche Diagnostics, Mannheim) gespalten und das DNA Fragment mit GFX™PCR, DNA and Gel Band Purification Kit aufgereinigt.

Der Vektor pClik5MCS SEQ ID NO: 13 wurde mit den Restriktionsenzymen Asp718 und SpeI geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PmetA metA ist als SEQ ID NO 16: aufgeführt.

### Beispiel 3:

### Herstellung des Plasmids pCLiK5MCS Psod metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 17 und SEQ ID NO 18, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 200 Basenpaaren aus dem nichtkodierenden 5'-Bereich (Region der Expressionseinheit) der Superoxiddismutase (Psod) amplifiziert.
SEQ ID NO: 17
5'-GAGACTCGAGAGCTGCCAATTATTCCGGG-3'
und
SEQ ID NO: 18
5'-CCTGAAGGCGCGAGGGTGGGCATGGGTAAAAAATCCTTTCG -3'

Das erhaltene DNA Fragment wurde mit dem GFX™PCR, DNA and Gel Band Purification Kit (Amersham Pharmacia, Freiburg) nach Angaben des Herstellers gereinigt.

Ausgehend vom Plasmid PmetA metA SEQ ID NO: 16 als Template für eine PCR Reaktion wurde mit den Oligonukleotidprimern SEQ ID NO: 19: und SEQ ID NO: 20: ein Teil von metA amplifiziert.
SEQ ID NO: 19
5'-CCCACCCTCGCGCCTTCAG -3'
und
SEQ ID NO: 20
5'-CTGGGTACATTGCGGCCC -3'

Das erhaltene DNA Fragment von ungefähr 470 Basenpaaren wurde mit dem GFX™PCR, DNA and Gel Band Purification Kit nach Angaben des Herstellers gereinigt.

In einer weiteren PCR Reaktion wurden die beiden oben erhaltenen Fragmente gemeinsam als Template eingesetzt. Durch die mit dem Oligonukleotidprimer SEQ ID NO: 18 eingebrachten, zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 17 und SEQ ID NO: 20 erst mit Beginn des 2. Zykluses dem Reaktionsansatz zugegeben wurden.

Das amplifizierte DNA Fragment von ungefähr 675 Basenpaaren wurde mit dem GFX™PCR, DNA and Gel Band Purification Kit nach Angaben des Herstellers gereinigt. Im Anschluß daran wurde es mit den Restriktionsenzymen Xhol und Ncol (Roche Diagnostics, Mannheim) gespalten und gelelektrophoretisch aufgetrennt. Anschließend wurde das ca. 620 Basenpaar große DNA Fragment mit GFX™CR, DNA and Gel Band Purification Kit (Amersham Pharmacia, Freiburg) aus der Agarose aufgereinigt.

Das Plasmid PmetA metA SEQ ID NO 16: wurde mit den Restriktionsenzymen NcoI und SpeI (Roche Diagnostics, Mannheim) gespalten. Nach gelelektrophoretischer Auftrennung wurde ein ca. 0,7 kb großes metA Fragment mit GFX™PCR, DNA and Gel Band Purification Kit aus der Agarose aufgereinigt.

Der Vektor pClik5MCS SEQ ID NO:13 wurde mit den Restriktionsenzymen Xhol und SpeI (Roche Diagnostics, Mannheim) geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment und dem metA-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PSODmetA ist als SEQ ID NO: 21: aufgeführt.

### Beispiel 4:

### Herstellung des Plasmids pCLiK5MCS P EF-TU metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 22 und SEQ ID NO 23, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 200 Basenpaaren aus dem nichtkodierenden 5'-Bereich (Promotorregion) der Superoxiddismutase (Psod) amplifiziert.
SEQ ID NO: 22
5'-GAGACTCGAGGGCCGTTACCCTGCGAATG -3'
und
SEQ ID NO: 23
5'-CCTGAAGGCGCGAGGGTGGGCATTGTATGTCCTCCTGGAC -3'

Das erhaltene DNA Fragment wurde mit dem GFX™PCR, DNA and Gel Band Purification Kit (Amersham Pharmacia, Freiburg) nach Angaben des Herstellers gereinigt.

Ausgehend vom Plasmid PmetA metA SEQ ID NO: 16 als Template für eine PCR Reaktion wurde mit den Oligonukleotidprimern SEQ ID NO: 24 und SEQ ID NO: 25 ein Teil von metA amplifiziert.
SEQ ID NO: 24
5'-CCCACCCTCGCGCCTTCAG -3'
und
SEQ ID NO: 25
5'-CTGGGTACATTGCGGCCC -3'

Das erhaltene DNA Fragment von ungefähr 470 Basenpaaren wurde mit dem GFX™PCR, DNA and Gel Band Purification Kit nach Angaben des Herstellers gereinigt.

In einer weiteren PCR Reaktion wurden die beiden oben erhaltenen Fragmente gemeinsam als Template eingesetzt. Durch die mit dem Oligonukleotidprimer SEQ ID NO: 18 eingebrachten, zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 22 und SEQ ID NO: 25 erst mit Beginn des 2. Zykluses dem Reaktionsansatz zugegeben wurden.

Das amplifizierte DNA Fragment von ungefähr 675 Basenpaaren wurde mit dem GFX™PCR, DNA and Gel Band Purification Kit nach Angaben des Herstellers gereinigt. Im Anschluß daran wurde es mit den Restriktionsenzymen Xhol und Ncol (Roche Diagnostics, Mannheim) gespalten und gelelektrophoretisch aufgetrennt. Anschließend wurde das ca. 620 Basenpaar große DNA Fragment mit GFX™PCR, DNA and Gel Band Purification Kit (Amersham Pharmacia, Freiburg) aus der Agarose aufgereinigt.

Das Plasmid PmetA metA SEQ ID NO: 16 wurde mit den Restriktionsenzymen Ncol und SpeI (Roche Diagnostics, Mannheim) gespalten. Nach gelelektrophoretischer Auftrennung wurde ein ca. 0,7 kb großes metA Fragment mit GFX™PCR, DNA and Gel Band Purification Kit aus der Agarose aufgereinigt.

Der Vektor pClik5MCS SEQ ID NO: 13 wurde mit den Restriktionsenzymen Xhol und SpeI (Roche Diagnostics, Mannheim) geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment und dem metA-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS P_EFTUmetA ist als SEQ ID NO: 26 aufgeführt.

### Beispiel 5:

### Herstellung des Plasmid pCLiK5MCS Pgro metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 27 und SEQ ID NO 28, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 200 Basenpaaren aus dem nichtkodierenden 5'-Bereich (Region der Expressionseinheit) der Gens GroES (Pgro) amplifiziert.
SEQ ID NO: 27
5'-GAGACTCGAGCGGCTTAAAGTTTGGCTGCC -3'
SEQ ID NO :28
5'-CCTGAAGGCGCGAGGGTGGGCATGATGAATCCCTCCATGAG -3'

Das erhaltene DNA Fragment wurde mit dem GFX™PCR, DNA and Gel Band Purification Kit (Amersham Pharmacia, Freiburg) nach Angaben des Herstellers gereinigt. Ausgehend vom Plasmid PmetA metA als Template für eine PCR Reaktion wurde mit den Oligonukleotidprimern SEQ ID NO:29 und SEQ ID NO: 30 ein Teil von metA amplifiziert.
SEQ ID NO: 29
5'-CCCACCCTCGCGCCTTCAG -3'
SEQ ID NO: 30
5'-CTGGGTACATTGCGGCCC -3'

Das erhaltene DNA Fragment von ungefähr 470 Basenpaaren wurde mit dem GFX™PCR, DNA and Gel Band Purification Kit nach Angaben des Herstellers gereinigt.

In einer weiteren PCR Reaktion wurden die beiden oben erhaltenen Fragmente gemeinsam als Template eingesetzt. Durch die mit dem Oligonukleotidprimer SEQ ID NO: 28 eingebrachten, zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 27 und SEQ ID NO: 30 erst mit Beginn des 2. Zykluses dem Reaktionsansatz zugegeben wurden.

Das amplifizierte DNA Fragment von ungefähr 675 Basenpaaren wurde mit dem GFX™PCR, DNA and Gel Band Purification Kit nach Angaben des Herstellers gereinigt. Im Anschluß daran wurde es mit den Restriktionsenzymen Xhol und Ncol (Roche Diagnostics, Mannheim) gespalten und gelelektrophoretisch aufgetrennt. Anschließend wurde das ca. 620 Basenpaar große DNA Fragment mit GFX™PCR, DNA and Gel Band Purification Kit (Amersham Pharmacia, Freiburg) aus der Agarose aufgereinigt.

Das Plasmid PmetA metA SEQ ID NO: 16 wurde mit den Restriktionsenzymen Ncol und SpeI (Roche Diagnostics, Mannheim) gespalten. Nach gelelektrophoretischer Auftrennung wurde ein ca. 0,7 kb großes metA Fragment mit GFX™PCR, DNA and Gel Band Purification Kit aus der Agarose aufgereinigt.

Der Vektor pClik5MCS SEQ ID NO: 13 wurde mit den Restriktionsenzymen Xhol und SpeI (Roche Diagnostics, Mannheim) geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment und dem metA-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS Pgro metA ist als SEQ ID NO: 31 aufgeführt.

### Beispiel 6:

### Herstellung des Plasmids P EF-TS metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer BK 1849 (SEQ ID NO: 32) und BK 1862 (SEQ ID NO: 33), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, das metA Gen, das für die Homoserin-O-Acetyltransferase kodiert, amplifiziert.
BK 1849 (SEQ ID NO: 32)
5'- GTGTGTCGACTTAGATGTAGAACTCGATGTAG -3'
und
BK 1862 (SEQ ID NO: 33)
5'-ATGCCCACCCTCGCGCC -3'

Das erhaltene DNA Fragment von ca. 1134 bp Größe wurde mit dem GFX™PCR, DNA and Gel Band Purification Kit (Amersham Pharmacia, Freiburg) nach Angaben des Herstellers gereinigt.

Mit den Oligonukleotidprimer Haf 26 (SEQ ID NO: 34) und Haf 27 (SEQ ID NO: 35), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment von ca. 200 Basenpaaren aus dem nichtkodierenden 5'-Bereich (Region der Expressionseinheit) der Gens, das für den Elongationsfaktor TS kodiert, amplifiziert.
Haf 26 (SEQ ID NO: 34)
5'-GAGAGGATCCCCCCCACGACAATGGAAC-3`
und
Haf 27 (SEQ ID NO: 35)
5'-CCTGAAGGCGCGAGGGTGGGCATTACGGGGCGATCCTCCTTATG -3'

Das erhaltene DNA Fragment von ca. 195 bp Größe wurde mit dem GFX™PCR, DNA and Gel Band Purification Kit (Amersham Pharmacia, Freiburg) nach Angaben des Herstellers gereinigt.

Die Primer Haf 27 und BK 1862enthalten eine überlappende Sequenz und sind an ihren 5'-Enden homolog zueinander.

Die oben erhaltenen PCR-Produkte wurden als Template für eine weitere PCR eigensetzt, in der die Primer BK 1849 (SEQ ID NO: 32) und Haf 26 (SEQ ID NO: 34) genutzt wurden.

Mit diesem Ansatz konnte ein DNA-Fragment amplifiziert werden, das der erwarteten Größe von 1329 bp entsprach. Diese P EF-TS/metA-Fusion wurde dann über die Restriktionschnittstellen BamHI und SalI in den Vektor pClik 5a MCS (SEQ ID NO: 13) kloniert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das resultierende Plasmid wurde mit pClik 5a MCS P EF-TS metA (SEQ ID NO: 36) bezeichnet.

### Beispiel 7:

### Herstellung des Plasmids P EF-Tu pSOD metA (H473)

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer BK 1753 (SEQ ID NO: 37) und BK 1754 (SEQ ID NO: 38) , der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, der GroEL-Terminator amplifiziert.
BK 1753 (SEQ ID NO: 37)
GGATCTAGAGTTCTGTGAAAAACACCGTG
BK 1754 (SEQ ID NO: 38)
GCGACTAGTGCCCCACAAATAAAAAACAC

Das erhaltene DNA Fragment von ca. 77 bp Größe wurde mit dem GFX™PCR, DNA and Gel Band Purification Kit (Amersham Pharmacia, Freiburg) nach Angaben des Herstellers gereinigt, mit den Restriktionsenzymen XbaI und Bcu I geschnitten und nochmals gereinigt.

Der Vektor pClik5MCS (SEQ ID NO: 13) wurde mit dem Restriktionsenzym Xbal linearisiert und mit dem GFX™PCR, DNA and Gel Band Purification Kit (Amersham Pharmacia, Freiburg) nach Angaben des Herstellers gereinigt,

Der linearisierte Vektor wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das resultierende Plasmid wurde mit H247 bezeichnet (SEQ ID NO: 39) bezeichnet.

Dieses Plasmid wurde mit den Restriktionsenzymen Bcul und SalI behandelt und mit dem GFX™PCR, DNA and Gel Band Purification Kit (Amersham Pharmacia, Freiburg) nach Angaben des Herstellers gereinigt,

Eine PCR mit den Oligonukleotiden BK 1848 (SEQ ID NO: 40) und BK 1849 (SEQ ID NO: 41), dem Plasmid pClik5MCS Psod metA (SEQ ID NO: 21) als Template und Pfu Turbo Polymerase (Fa. Stratagene) wurde nach Standardmethoden, wie in Innis et al. (1990) PCR Protocols. A Guide to Methods and Applications, Academic Press beschrieben, durchgeführt. Das amplifizierte Fragment hatte eine erwartete Länge von ca 1350 bp und wurde mit dem GFX™PCR, DNA and Gel Band Purification Kit (Amersham Pharmacia, Freiburg) nach Angaben des Herstellers gereinigt, mit den Restriktionsenzymen Bcul und SalI geschnitten und nochmals gereinigt.

Dieses Fragment wurde mit dem Plasmid H247 (Schnittstellen Bcul und Sall) mit Hilfe des Rapid DNA Ligation Kits (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das resultierende Plasmid wurde als pG A4 (H344) bezeichnet und ist unter der SEQ ID NO: 42 aufgeführt.
SEQ ID NO: 40 (BK 1848)
GAGAACTAGTAGCTGCCAATTATTCCGGG
SEQ ID NO: 41 (BK 1849)
GTGTGTCGACTTAGATGTAGAACTCGATGTAG

Das Plasmid pG A4 (SEQ ID NO: 42) wurde mit den Restriktionsenzymen Xhol und Bcul geschnitten und mit dem GFX™PCR, DNA and Gel Band Purification Kit (Amersham Pharmacia, Freiburg) nach Angaben des Herstellers gereinigt.

### Konstruktion von H473:

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer BK 1695 (SEQ ID NO: 43) und Haf16 (SEQ ID NO: 44), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment von ca. 182 Basenpaaren aus dem nichtkodierenden 5'-Bereich (Region der Expressionseinheit) der Gens EF Tu (Peftu) amplifiziert.
SEQ ID NO: 43 (BK1695)
GAGACTCGAGGGCCGTTACCCTGCGAATG
SEQ ID NO: 44 (Haf16)
GAGAACTAGTGTGGCTACGACTTTCGCAGC

Das erhaltene DNA Fragment von ca. 200 bp Größe wurde mit dem GFX™PCR, DNA and Gel Band Purification Kit (Amersham Pharmacia, Freiburg) nach Angaben des Herstellers gereinigt, mit den Restriktionsenzymen Xho I und Bcu I geschnitten und nochmals gereinigt.

Dieses Fragment wurde mit dem Plasmid Plasmid pG A4 (H344) (Schnittstellen Xhol und Bcul) mit Hilfe des Rapid DNA Ligation Kits (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Das erhaltene Plasmid wurde pClik5MCS PeftuPsod metA genannt (H473). Es ist unter SEQ ID NO: 45 aufgeführt. Es enthält (von 5' nach 3') einen Peftu-Promoter, eine Psod Expressionseinheit und direkt anschließend das metA open reading frame.

### Beispiel 8:

### Herstellung des Plasmides PsodPeftu metA (H505)

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO: 46 (Haf64) und SEQ ID NO: 47 (Haf65), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment von ca. 200 aus dem nichtkodierenden 5'-Bereich (Region der Expressionseinheit) des Gens EF Tu (Peftu) amplifiziert.
SEQ ID NO: 46 (Haf64)
GAGAACTAGTGGCCGTTACCCTGCGAATG
SEQ ID NO: 47 (Haf65)
GCGCGAGGGTGGGCATTGTATGTCCTCCTGGACTTC

Das erhaltene DNA Fragment von ca. 200 bp Größe wurde mit dem GFX™PCR, DNA and Gel Band Purification Kit (Amersham Pharmacia, Freiburg) nach Angaben des Herstellers gereinigt.

In einer zweiten PCR mit den Oligonukleotidprimern BK1862 (SEQ ID NO: 48) und BK1849 (SEQ ID NO: 49) und dem Plasmid H344 (SEQ ID NO: 42) als Template wurde das metA open reading frame nach Standardmethoden, wie in Innis et al. (1990) PCR Protocols. A Guide to Methods and Applications, Academic Press beschrieben, amplifiziert. Das erhaltene Fragment von ca. 1140 bp Länge wurde mit dem GFX™PCR, DNA and Gel Band Purification Kit (Amersham Pharmacia, Freiburg) nach Angaben des Herstellers gereinigt.
SEQ ID NO: 48 (BK1862)
ATGCCCACCCTCGCGCC
SEQ ID NO: 49 (BK1849)
GTGTGTCGACTTAGATGTAGAACTCGATGTAG

In einer weiteren PCR Reaktion wurden die beiden oben erhaltenen Fragmente gemeinsam als Template eingesetzt. Durch die mit dem Oligonukleotidprimer Haf 65 SEQ ID NO: 47 eingebrachten zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer Haf 64 und BK 1849 SEQ ID NO: 46 und SEQ ID NO: 49 erst mit Beginn des 2. Zykluses dem Reaktionsansatz zugegeben wurden.

Das amplifizierte DNA Fragment von ungefähr 1350 Basenpaaren wurde mit dem GFX™PCR, DNA and Gel Band Purification Kit nach Angaben des Herstellers gereinigt. Im Anschluß daran wurde es mit den Restriktionsenzymen Bcul und SalI (Roche Diagnostics, Mannheim) gespalten und gelelektrophoretisch aufgetrennt. Anschließend wurde das ca. 1340 Basenpaar große DNA Fragment mit GFX™PCR, DNA and Gel Band Purification Kit (Amersham Pharmacia, Freiburg) aus der Agarose aufgereinigt. Dieses Fragment enthält die Peftu-Expressionseinheit fusioniert mit dem metA ORF (= Fragment Peftu-metA).

Mit den Oligonukleotidprimer BK 1697 (SEQ ID NO: 50) und Haf17 (SEQ ID NO:51), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment (Gesamtlänge: 193 bp, davon 173 pSOD) aus dem nichtkodierenden 5'-Bereich (Region der Expressionseinheit) des Gens SOD (Psod) amplifiziert.
SEQ ID NO: 50 (BK 1697)
GAGACTCGAGAGCTGCCAATTATTCCGGG
SEQ ID NO: 51 (Haf 17)
GAGAACTAGTTAGGTTTCCGCACCGAGC

Das amplifizierte DNA Fragment wurde mit dem GFX™PCR, DNA and Gel Band Purification Kit nach Angaben des Herstellers gereinigt. Im Anschluß daran wurde es mit den Restriktionsenzymen Xhol und Bcul (Roche Diagnostics, Mannheim) gespalten und gelelektrophoretisch aufgetrennt. Anschließend wurde das ca. 180 Basenpaar große DNA Fragment mit GFX™PCR, DNA and Gel Band Purification Kit (Amersham Pharmacia, Freiburg) aus der Agarose aufgereinigt (= Fragment Psod).

Das Plasmid (H344) pG A4 SEQ ID NO: 42 wurde mit den Restriktionsenzymen Xhol und Sall (Roche Diagnostics, Mannheim) gespalten und gelelektrophoretisch aufgetrennt. Anschließend wurde linearisierte Vektor mit dem GFX™PCR, DNA and Gel Band Purification Kit (Amersham Pharmacia, Freiburg) aus der Agarose aufgereinigt.

Der linearisierte Vektor wurde mit den beiden Fragmenten (Fragment Peftu-metA und Fragment Psod) mit Hilfe des Rapid DNA Ligation Kits (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pClik5MCS PsodPeftu metA ist als SEQ ID NO: 52 aufgeführt.

### Beispiel 9:

### Herstellung des Plasmides pClik5MCS Pgro Psod metA (H472)

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimern SEQ ID NO: 53 (BK1701) und SEQ ID NO: 54 (Haf18), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment von ca. 175 Nt amplifiziert. Es enthält 155 Basenparre aus dem nichtkodierenden 5'-Bereich (Region der Expressionseinheit) des Gens GRO EL (Pgro) und je eine Restriktionsschnittstelle am 5' und 3' Ende (Xhol bzw. Bcul).
SEQ ID NO: 53 (BK1701)
GAGACTCGAGCGGCTTAAAGTTTGGCTGCC
SEQ ID NO: 54 (Haf018)
GAGAACTAGTATTTTGTGTGTGCCGGTTGTG

Das erhaltene DNA Fragment von ca. 175 bp Größe wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Xhol und Bcul (Roche Diagnostics, Mannheim) gespalten und das DNA Fragment mit GFX™PCR, DNA and Gel Band Purification Kit aufgereinigt.

Das Plasmid H344 (SEQ ID NO: 42) wurde mit den Restriktionsenzymen Xhol und Bcul geschnitten und ein ca. 6.4 kb großes Fragment nach elektrophoretischer Auftrennung mit GFX™PCR, DNA and Gel Band Purification Kit isoliert.

Das PCR-Produkt und das aufgeschnittene Plasmid H344 wurden mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt.
- Die Sequenzierreaktionen wurden mittels ABI Prism 377 (PE Applied Biosystems, Weiterstadt) aufgetrennt und ausgewertet.

Das resultierende Plasmid enthält einen Pgro-Promoter direkt gefolgt von einer Psod-Expressionseinheit und dem metA ORF.
Es ist als pCLiK5MCS PgroPsod metA (H472) ist als SEQ ID NO: 55 aufgeführt.

### Beispiel 10:

### Herstellung des Plasmides PgroPsod Pefts metA (H501)

Mit den Oligonukleotidprimern BK1782 (SEQ ID NO: 56) und Haf63 (SEQ ID NO: 57) und dem Plasmid pClik5MCS Pgro Psod metA (SEQ ID NO: 55 (H472)) als template wurde ein PCR nach Standardmethoden, wie in Innis et al. (1990) PCR Protocols. A Guide to Methods and Applications, Academic Press beschrieben, durchgeführt. Das amplifizierte Fragment umfasst ca. 474 Basenparre und enthält eine Pgro-Psod-Kassette mit einer Acc651-Schnittstelle am 3'-Ende. Das erhaltene DNA Fragment wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Xhol und Acc65I gespalten und das DNA Fragment (333 Basenpaare) mit GFX™PCR, DNA and Gel Band Purification Kit aufgereinigt.
SEQ ID NO: 56 (BK1782)
TCGAGAGATTGGATTCTTAC
SEQ ID NO: 57 (Haf 63)
TCTCGGTACCCCGCACCGAGCATATACATC

Das Plasmid H479 (SEQ ID NO: 58) enthält die Pefts Expressionseinheit fusioniert mit dem metA ORF. Es wurde mit den Restriktionsenzymen Xhol und Acc651 (direkt upstream von Pefts) geschnitten und ein ca. 6.4 kb großes Fragment nach elektrophoretischer Auftrennung mit GFX™PCR, DNA and Gel Band Purification Kit isoliert.

Anschließend wurde es mit Hilfe des Rapid DNA Ligation Kit (Roche Diagnostics, Mannheim) nach Angaben des Herstellers mit dem PCR-Fragment (Pgro-Psod-Kassette) ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das resultierende Plasmid enthält die 3 Promotoren Pgro Psod und P EF -Ts fusioniert mit dem metA ORF.
Es ist als pCLiK5MCS Pgro Psod Pefts metA (H501) ist als SEQ ID NO: 59 aufgeführt.

### Beispiel 11:

### Herstellung des Plasmides Peftu Psod Pefts metA (H502)

Mit den Oligonukleotidprimern BK1782 (SEQ ID NO: 56) und Haf63 (SEQ ID NO: 57) und dem Plasmid pClik5MCS Peftu Psod (SEQ ID NO: 45 (H473)) als Template wurde ein PCR nach Standardmethoden, wie in Innis et al. (1990) PCR Protocols. A Guide to Methods and Applications, Academic Press beschrieben, durchgeführt. Das amplifizierte Fragment umfasst ca. 495 Basenparre und enthält eine Peftu-Psod-Kassette mit einer Acc65I-Schnittstelle am 3'-Ende. Das erhaltene DNA Fragment wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Xhol und Acc651 gespalten und das DNA Fragment (354 Basenpaare) mit GFX™PCR, DNA and Gel Band Purification Kit aufgereinigt.
SEQ ID NO: 56 (BK1782)
TCGAGAGATTGGATTCTTAC
SEQ ID NO: 57 (Haf 63)
TCTCGGTACCCCGCACCGAGCATATACATC

Das Plasmid H479 (SEQ ID NO: 58) enthält die Pefts Expressionseinheit fusioniert mit dem metA ORF. Es wurde mit den Restriktionsenzymen Xhol und Acc65I (direkt upstream von Pefts) geschnitten und ein ca. 6.4 kb großes Fragment nach elektrophoretischer Auftrennung mit GFX™PCR, DNA and Gel Band Purification Kit isoliert.

Anschließend wurde es mit Hilfe des Rapid DNA Ligation Kit (Roche Diagnostics, Mannheim) nach Angaben des Herstellers mit dem PCR-Fragment (Peftu-Psod-Kassette) ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das resultierende Plasmid enthält die 3 regulatorischen Sequenzen Peftu Psod und P EF -Ts fusioniert mit dem metA ORF. Es ist als pCLiK5MCS Peftu Psod Pefts metA (H501) ist als SEQ ID NO: 60 aufgeführt.

### Beispiel 12:

### Messung der metA-Aktivitäten

Der Stamm Corynebacterium glutamicum ATCC13032 wurde jeweils mit den Plasmiden pClik5 MCS, pClik MCS Psod metA, pClik MCS Peftu metA, pClik5 MCS Pefts metA, pClik5 MCS Peftu Psod metA, pClik5 MCS Psod Peftu metA, pClik5 MCS Pgro Psod meta, pClik5 MCS Pgro Psod Pefts metA, pClik5 MCS Peftu Psod Pefts metA nach der beschriebenen Methode (Liebl, et al. (1989) FEMS Microbiology Letters 53:299-303) transformiert. Die Transformationsmischung wurde auf CM-Platten plattiert, die zusätzlich 20mg/l Kanamycin enthielten, um eine Selektion auf Plasmid-haltige Zellen zu erreichen. Erhaltene Kan-resistente Klone wurden gepickt und vereinzelt.

C. glutamicum Stämme, die eines dieser Plasmidkonstrukte enthielten, wurden in MMA-Medium ((40 g/l Saccharose, 20 g/l (NH₄)₂SO₄, 1 g/l KH₂PO₄, 1 g/l K₂HPO₄, 0,25g/l MgSO₄ x 7H₂O, 54 g Aces, 1 ml CaCl2 (10 g/l), 1 ml Protocatechoat (300 mg/10 ml), 1 ml Spurenelementelösung (10 g/l FeSO₄ x /H₂O, 10 g/l MnSO₄ x H₂O, 2 g/l ZnSO₄ x 7 H₂O, 0,2 g/l CuSO₄, 0,02 g/l NiCl₂ x 6 H₂O),100 µg/l Vitamin B₁₂, 0,3 mg/l Thiamin, 1mM Leucin, 1 mg/l Pyridoxal HCl, 1 ml Biotin ( 100 mg/l), pH7,0) bei 30°C über Nacht angezogen. Die Zellen wurden bei 4°C abzentrifugiert und das zweimal mit kaltem Tris-HCl-Puffer (0,1%, pH 8,0) gewaschen. Nach erneuter Zentrifugation wurden die Zellen in kalten Tris-HCl-Puffer ( 0,1%, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die enzymatische Aktivität von MetA wurde wie folgt durchgeführt. Die Reaktionsansätze von 1 ml enthielten 100 mM Kaliumphosphatpuffer (pH 7,5), 5mM MgCl2, 100 µM Acetyl CoA, 5mM L-Homoserine, 500 µM DTNB (Ellmans Reagenz) und Zellextrakt. Der Test wurde durch Zugabe von dem jeweiligen Proteinlysat gestartet und bei Raumtemperatur inkubiert. Es wurde dann eine Kinetik bei 412 nm über 10 min aufgenommen.

Die Ergebnisse sind in Tabelle 1 a gezeigt.

**Tabelle 1 a**

| Stamm | interne Konstruktbezeichnung | spezifische Aktivität [nMol/mg/min] |
|---|---|---|
| ATCC 13032 pClik5MCS | H356 (metZ) | 3,1 |
| ATCC 13032 pCLiK5MCS Psod metA | H144 | 1308,7 |
| ATCC 13032 pCLiK5MCS Peftu metA | H146 | 1233,0 |
| ATCC 13032 pCLiK5MCS Pefts metA | H479 | 2339,0 |
| ATCC 13032 pCLiK5MCS Peftu Psod metA | H473 | 2308,1 |
| ATCC 13032 pCLiK5MCS Psod Peftu metA | H505 | 2061,9 |
| ATCC 13032 pCLiK5MCS Pgro Psod metA | H472 | 1501,6 |
| ATCC 13032 pCLiK5MCS Pgro Psod Pefts metA | H501 | 1773,4 |
| ATCC 13032 pCLiK5MCS Peftu Psod Pefts metA | H502 | 2262,2 |

Die Aktivität von MetA konnte durch die Verwendung der verschiedenen Kombinationen von Promotern/Expressionseinheiten moduliert werden.

### Beispiel 13:

### Konstruktion von Plasmid pCIS lysC

Um einen Lysin-produzierenden Stamm zu generieren wurde ein allelischer Austausch des lysC Wildtypgens in Corynebakterium glutamicum ATCC13032 durchgeführt. Dabei wurde im lysC Gen ein Nukleotidaustausch durchgeführt, so daß im resultierenden Protein die Aminosäure Thr an der Position 311 durch ein Ile ausgetauscht wurde. Ausgehend von der chromosomalen DNA aus ATCC13032 als Template für eine PCR Reaktion wurde mit den Oligonukleotidprimern SEQ ID NO:61 und SEQ ID NO:62 lysC mit Hilfe des Pfu-Turbo PCR Systems (Stratagene USA) nach Angaben des Herstellers amplifiziert.
SEQ ID NO:61
5'-GAGAGAGAGACGCGTCCCAGTGGCTGAGACGCATC-3'
SEQ ID NO:62
5`-CTCTCTCTGTCGACGAATTCAATCTTACGGCCTG-3'

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Das amplifizierte Fragment wird an seinem 5'-Ende von einem Sall Restriktionsschnitt und an seinem 3'-Ende von einem Mlul Restriktionsschnitt flankiert. Vor der Klonierung wurde das amplifizierte Fragment durch diese beiden Restriktionsenzyme verdaut und mit GFX™PCR, DNA and Gel Band Purification Kit (Amersham Pharmacia, Freiburg) aufgereinigt.

Das erhaltenen Polynukleotid wurde über die Sall und Mlul Restriktionsschnitte in pCLIK5 MCS integrativ SacB im folgenden pCIS genannt (SEQ ID NO: 63) kloniert und in E.coli XL-1 blue transformiert. Eine Selektion auf Plasmid tragende Zellen wurde durch das Ausplattieren auf Kanamycin (20µg/ml) haltigen LB Agar (Lennox, 1955, Virology, 1:190) erreicht. Das Plasmid wurdn isoliert und durch Sequenzierung die erwartete Nukleotidsequenz bestätigt. Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet. Das erhaltene Plasmid pCIS lysC ist als SEQ ID NO:64 aufgeführt.

### Beispiel 14:

### Mutagenese des lysC Gens aus C. glutamicum

Die gerichtete Mutagenese des lysC Gens aus C. glutamicum wurde mit dem QuickChange Kit (Fa. Stratagene/USA) nach Angaben des Herstellers durchgeführt. Die Mutagenese wurde im Plasmid pCIS lysC, SEQ ID NO:64 durchgeführt. Für den Austausch von thr 311 nach 311 ile mit Hilfe der Quickchange Methode (Stratagene) wurden folgende Oligonukleotidprimer synthetisiert:
SEQ ID NO:65
5'-CGGCACCACCGACATCATCTTCACCTGCCCTCGTTCCG -3'
SEQ ID NO:66
5'-CGGAACGAGGGCAGGTGAAGATGATGTCGGTGGTGCCG -3'

Der Einsatz dieser Oligonukleotidprimer in der Quickchange Reaktion führt in dem lysC Gen SEQ ID NO:67 zu einem Austausch des Nukleotids in Position 932 (von C nach T). Der resultierende Aminosäureaustausch Thr311Ile im lysC Gen wurde nach Transformation in E.coli XL1-blue und Plasmidpräparation durch ein Sequenzierungsreaktionen bestätigt. Das Plasmid erhielt die Bezeichnung pCIS lysC thr311ile und ist als SEQ ID NO:68 aufgeführt.

Das Plasmid pCIS lysC thr311ile wurde in C. glutamicum ATCC13032 mittels Elektroporation wie bei Liebl, et al. (1989) FEMS Microbiology Letters 53:299-303 beschrieben, transformiert. Modifikationen des Protokolls sind in DE 10046870 beschrieben. Die chromosomale Anordnung des lysC-Lokus einzelner Transformanten wurde mit Standardmethoden durch Southernblot und Hybridisierung wie in Sambrook et al. (1989), Molecular Cloning. A Laboratory Manual, Cold Spring Harbor, beschrieben, überprüft. Dadurch wurde sichergestellt, daß es sich bei den Transformanten um solche handelt, die das transformierte Plasmid durch homologe Rekombination am lysC-Lokus integriert haben. Nach Wachstum solcher Kolonien über Nacht in Medien, die kein Antibiotikum enthalten, werden die Zellen auf ein Saccharose-CM-Agarmedium (10% Saccharose, 10 g/l Glucose; 2,5 g/l NaCl; 2 g/l Harnstoff, 10 g/l Bacto Peptone (Fa. Difco); 10 g/l Hefeextrakt, 22,0 g/L Agar (Difco)) ausplattiert und bei 30°C für 24 Stunden inkubiert.

Da das im Vektor pCIS lysC thr311ile enthaltende sacB Gen Saccharose in ein toxisches Produkt umwandelt, können nur solche Kolonien anwachsen, die das sacB Gen durch einen zweiten homologen Rekombinationsschritt zwischen dem Wildtyp lysC Gen und dem mutierten Gen lysC thr311ile deletiert haben. Während der homologen Rekombination kann entweder das Wildtyp Gen, oder das mutierte Gen zusammen mit dem sacB Gen deletiert werden. Wenn das sacB Gen zusammen mit dem Wildtyp Gen entfernt wird, resultiert eine mutierte Transformante.

Anwachsende Kolonien wurden gepickt, und auf eine Kanamycin-sensitiven Phänotyp hin untersucht. Klone mit deletiertem SacB Gen müssen gleichzeitg Kanamycin sensitives Wachstumsverhalten zeigen. Solche Kan-sensitiven Klone wurde im einem Schüttelkolben auf ihre Lysin-Produktivivät hin untersucht (siehe Beispiel 19). Zum Vergleich wurde der nicht behandelte C. glutamicum ATCC13032 angezogen. Klone mit einer gegenüber der Kontrolle erhöhten Lysin-Produktion wurden selektiert, chromosomale DNA gewonnen und der entsprechende Bereich des lysC Gens durch eine PCR-Reaktion amplifiziert und sequenziert. Ein solcher Klon mit der Eigenschaft erhöhter Lysin-Synthese und nachgewiesener Mutation in lysC an der Stelle 932 wurde mit ATCC13032 lysC^{for} bezeichnet.

### Beispiel 15:

### Herstellung des Plasmides pCIS Peftu ddh

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimern SEQ ID NO: 69 (Old38) und SEQ ID NO: 70 (Old 39), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment von ca. 200 aus dem nichtkodierenden 5'-Bereich (Region der Expressionseinheit) des Gens EF Tu (Peftu) amplifiziert.
SEQ ID NO: 69 (OId38)
ACATCCATGGTGGCCGTTACCCTGCGAAT
SEQ ID NO: 70 (Old 39)
TGTATGTCCTCCTGGACTTC

Das erhaltene DNA Fragment von ca. 200 bp Größe wurde mit dem GFX™PCR, DNA and Gel Band Purification Kit (Amersham Pharmacia, Freiburg) nach Angaben des Herstellers gereinigt.

In einer zweiten PCR mit den Oligonukleotidprimern Old40 (SEQ ID NO: 71) und SEQ ID NO: 72 (Old 37) und chromosomaler DNA aus C. glutamicum ATCC 13032 als template wurde das ddh open reading frame nach Standardmethoden, wie in Innis et al. (1990) PCR Protocols. A Guide to Methods and Applications, Academic Press beschrieben, amplifiziert. Das erhaltene Fragment von ca. 1017 bp Länge wurde mit dem GFX™PCR, DNA and Gel Band Purification Kit (Amersham Pharmacia, Freiburg) nach Angaben des Herstellers gereinigt.
OId40 (SEQ ID NO: 71)
GAAGTCCAGGAGGACATACAATGACCAACATCCGCGTAGC
Old 37 (SEQ ID NO: 72)
GAAACCACACTGTTTCCTTGC

In einer weiteren PCR Reaktion wurden die beiden oben erhaltenen Fragmente gemeinsam als Template eingesetzt. Durch die mit dem Oligonukleotidprimer OId40 SEQ ID NO: 71 eingebrachten zu Peftu homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer OId37 und Old 38 (SEQ ID NO: 72 und SEQ ID NO: 69) erst mit Beginn des 2. Zykluses dem Reaktionsansatz zugegeben wurden.

Das amplifizierte DNA Fragment von ungefähr 1207 Basenpaaren wurde mit dem GFX™PCR, DNA and Gel Band Purification Kit nach Angaben des Herstellers gereinigt. Im Anschluß daran wurde es mit den Restriktionsenzymen Ncol und Xhol (Roche Diagnostics, Mannheim) gespalten und gelelektrophoretisch aufgetrennt. Anschließend wurde das ca. 1174 Basenpaar große DNA Fragment mit GFX™PCR, DNA and Gel Band Purification Kit (Amersham Pharmacia, Freiburg) aus der Agarose aufgereinigt. Dieses Fragment enthält die Peftu-Expressionseinheit fusioniert mit dem ddh ORF (= Fragment Peftu-ddh).

Mit den Oligonukleotidprimer Old 32 (SEQ ID NO: 73) und Old 33 (SEQ ID NO: 74), der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein DNA Fragment aus dem nichtkodierenden 5'-Bereich des Gens ddh amplifiziert.
SEQ ID 73 (OId32)
ATCAACGCGTCGACACCACATCATCAATCAC
SEQ ID 74 (OId33)
ATCACCATGGGTTCTTGTAATCCTCCAAAATTG

Das amplifizierte DNA Fragment wurde mit dem GFX™PCR, DNA and Gel Band Purification Kit nach Angaben des Herstellers gereinigt. Im Anschluß daran wurde es mit den Restriktionsenzymen Mlul und NcoI (Roche Diagnostics, Mannheim) gespalten und gelelektrophoretisch aufgetrennt. Anschließend wurde das ca. 720 Basenpaar große DNA Fragment mit GFX™PCR, DNA and Gel Band Purification Kit (Amersham Pharmacia, Freiburg) aus der Agarose aufgereinigt (= Fragment 5' ddh).

Das Plasmid pCIS (SEQ ID NO: 63) wurde mit den Restriktionsenzymen MluI und Xhol (Roche Diagnostics, Mannheim) gespalten und gelelektrophoretisch aufgetrennt. Anschließend wurde linearisierte Vektor mit dem GFX™PCR, DNA and Gel Band Purification Kit (Amersham Pharmacia, Freiburg) aus der Agarose aufgereinigt.

Der linearisierte Vektor wurde mit den beiden Fragmenten (Fragment Peftu-ddh und Fragment 5' ddh) mit Hilfe des Rapid DNA Ligation Kits (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCIS Peftu ddh ist als SEQ ID NO: 75 aufgeführt.

### Beispiel 16:

### Herstellung von pCIS PeftuPsod ddh

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO: 76 und SEQ ID NO: 77, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 677 Basenpaaren aus dem 5'-Bereich der ddh-Gens amplifiziert (Fragment 5'ddh).
SEQ ID NO: 76 (CK461)
CCTGACGTCGCAATATAGGCAGCTGAATC
SEQ ID NO : 77 (CK466)
GCCCAATTGGTTCTTGTAATCCTCCAAAA

Das erhaltene DNA Fragment wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Aatll und MunI (Roche Diagnostics, Mannheim) gespalten und gelelektrophoretisch aufgetrennt. Anschließend wurde das ca. 661 Basenpaar große DNA Fragment mit GFX™PCR, DNA and Gel Band Purification Kit (Amersham Pharmacia, Freiburg) aus der Agarose aufgereinigt. Das entstandene Fragment enthät den 5'-Bereich des ddh-ORFs.

Ausgehend vom Plasmid P EF-Tu pSOD metA (H473) (SEQ ID 45) als Template für eine PCR Reaktion wurde mit den Oligonukleotidprimern SEQ ID NO:-78 und SEQ ID NO: 79 eine PeftuPsod Kassette amplifiziert.
SEQ ID NO: 78 (CK426)
CGCCAATTGTCGAGGGCCGTTACCCT
SEQ ID NO: 79 (CK463)
GCTACGCGGATGTTGGTCATGGGTAAA,4AATCCTTTCGTA

Das erhaltene DNA Fragment von ungefähr 378 Basenpaaren wurde mit dem GFX™PCR, DNA and Gel Band Purification Kit nach Angaben des Herstellers gereinigt.

In einer nachfolgenden PCR wurde das ddh ORF amplifiziert. Dazu wurde chromosomale DNA aus C. glutamicum ATCC 13032 nach Tauch et al. (1995) Plasmid 33:168-179 oder Eikmanns et al. (1994) Microbiology 140:1817-1828 präpariert. Mit den Oligonukleotidprimer SEQ ID NO: 80 (CK464) und SEQ ID NO: 81 (CK467=, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 992 Basenpaaren (ddh-ORF) amplifiziert (Fragment ddh). Das amplifizierte DNA Fragment wurde mit dem GFX™PCR, DNA and Gel Band Purification Kit nach Angaben des Herstellers gereinigt.
SEQ ID NO: 80 (CK464)
TACGAAAGGATTTTTTACCCATGACCAACATCCGCGTAGC
SEQ ID NO: 81 (CK467)
AGACCCGGGTTAGACGTCGCGTGCGATCA

In einer weiteren PCR Reaktion wurden die beiden oben erhaltenen Fragmente (PeftuPsod-Kassette und ddh-ORF) gemeinsam als Template eingesetzt. Durch die mit dem Oligonukleotidprimer SEQ ID NO: 80 (CK464) eingebrachten, zu Psod homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 79 (CK426) und SEQ ID NO: 81 (CK467) erst mit Beginn des 2. Zyklus dem Reaktionsansatz zugegeben wurden.
Das amplifizierte DNA Fragment von ungefähr 1359 Basenpaaren wurde mit dem GFX™PCR, DNA and Gel Band Purification Kit nach Angaben des Herstellers gereinigt.

Im Anschluß daran wurde es mit den Restriktionsenzymen MunI und Smal (Roche Diagnostics, Mannheim) gespalten und gelelektrophoretisch aufgetrennt. Anschließend wurde das ca. 1349 Basenpaar große DNA Fragment mit GFX™PCR, DNA and Gel Band Purification Kit (Amersham Pharmacia, Freiburg) aus der Agarose aufgereinigt. Das entstandene Fragment enthält (von 5' nach 3') einen Peftu-Promoter, eine Psod Expressionseinheit und direkt anschließend das ddh open reading frame (PeftuPsod ddh).

Der Vektor pCIS wurde mit den Restriktionsendonukleasen AatlI und Smal geschnitten und mit alkalischer Phosphatase (Roche Diagnostics, Mannheim) nach Angaben des Herstellers dephosphoryliert. Nach Elektrophorese in einem 0,8%igen Agarosegel wurde der linearisierte Vektor (ca. 4.2 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit den beiden geschnittenen PCR Fragmenten (5'ddh sowie PeftuPsod ddh) ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA von individuellen Klonen wurde mit dem Qiaprep Spin Miniprep Kit (Qiagen, Hilden) nach Angaben des Herstellers isoliert und über Restriktionsverdaus überprüft. 2 korrekte Plasmide wurden sequenziert. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet. Das entstandene Plasmid pClik Peftu Psod ddh ist dazu geeignet, um mit Hilfe von 2 aufeinanderfolgenden Rekombinationsereignissen die PeftuPsod-Kassette vor das ddh-ORF chromosomal zu integrieren.
Das Plasmid pCIS PeftuPsod ddh ist als SEQ ID NO: 82 aufgeführt.

### Beispiel 17:

### Generierung der Stämme ATCC13032 lysC^{for} Peftu ddh und ATCC13032 lysC^{for} PeftuPsod ddh

Zellen des E. coli-Stammes Mn522 (Firma Stratagene, Amsterdam, Nierderlande) wurde mit einem der Plasmide pCIS Peftu ddh und pCIS PeftuPsod ddh sowie mit dem Plasmid pTc15AcgIM nach Liebl, et al. (1989) FEMS Microbiology Letters 53:299-303 transformiert. Das Plasmid pTc15AcgIM ermöglicht die Methylierung von DNA nach dem Methylierungsmuster von Corynebacterium glutamicum (DE 10046870). Durch diese Methylierung wird die Stabilität der Plasmide pCIS Peftu ddh und pCIS PeftuPsod ddh in C. glutamicum-Zellen erhöht. Die Plasmid-DNA wurde anschließend aus den Mn522-Zellen nach Standardmethoden isoliert und mit Hilfe von Elektroporation den oben beschriebenen Corynebacterium glutamicum -Stamm ATCC 13032 ask (fbr) eingebracht. Aus dieser Elektroporation und der nachfolgenden Selektion auf CM-Platten mit Kanamycin (25 µg/ml) wurden mehrere Transkonjuganten erhalten. Zur Selektion auf das zweite Rekombinationsereignis, das zur Excision des Vektors führen soll, wurden diese Transkonjuganten in CM-Medium über Nacht ohne Kanamycin angezogen und anschließend zur Selektion auf CM-Platten mit 10% Saccharose ausplattiert. Das auf dem Vektor pCIS vorhandenen sacB-Gen kodiert für das Enzym Laevansucrase und führt bei Wachstum auf Saccharose zur Synthese von Laevan. Da Laevan für C. glutamicum toxisch ist, können nur C. glutamicum Zellen, die das Integrationsplasmid durch den zweiten Rekombinationsschritt verloren haben, auf Saccharosehaltigem Medium wachsen (Jäger et al., Journal of Bacteriology 174 (1992) 5462-5466). 100 Saccharose-resistente Klone wurden auf ihre Kanamycin-Sensitivität hin überprüft. Für mehrere der getesteten Klone konnte neben der Resistenz gegenüber Saccharose auch eine Sensitivität gegenüber Kanamycin nachgewiesen werden, was bei der erwünschten Excision der Vektorsequenzen erwartet wird. Ob auch die gewünschte Integration der Peftu bzw PeftuPsod-Expressionseinheit erfolgt war, wurde mittels Polymerase-Kettenreaktion (PCR) überprüft. Hierzu wurden die jeweiligen Klone mit einem Zahnstocher von der Agarplatte abgenommen und in 100 µl H₂O suspendiert und 10 min bei 95°C aufgekocht. Jeweils 10 µl der erhaltenen Lösung wurden als Template in die PCR eingesetzt. Als Primer wurden Oligonukleotide verwendet, die zur einzuführenden Expressionseinheit und dem ddh-Gen homolog sind. Die PCR-Bedingungen wurden wie folgt gewählt: Vorabdenaturierung:5 min bei 95°C; Denaturierung 30 sec bei 95°C; Hybridisierung 30 sec bei 55°C; Amplifizierung 2 min bei 72°C; 30 Zyklen,; End-Extension 5 min bei 72°C. Im Ansatz mit der DNA des Ausgangsstammes konnte durch Wahl der Oligonukleotide kein PCR-Produkt entstehen. Nur bei Klonen, die durch die 2. Rekombination die Integration der Peftu oder PeftuPsod-Expressionseinheiten direkt 5' des ddh-Gens vollzogen haben, wurde ein Bande erwartet. Die erfolgreiche Integration der dabei positiv getesteten Klone wurde zudem noch per Southern blot Analyse bestätigt (nach Standardmethoden, wie z.B. in Sambrook et al. (Molecular Cloning. A Laboratory Manual, Cold Spring Harbor, beschrieben (1989)). Für jeden zu generierenden Stamm wurden dabei 2 unterschiedliche Hydrolysen mit Restriktionsenzymen durchgeführt, die eine eindeutige Unterscheidung von Ausgangsstamm (ATCC13032 ask (fbr)) und erwünschtem Stamm zulässen. Zudem wurde mit Hilfe einer Sonde, die homolog zum Kanamycin-Resistenz-Gen ist, bestätigt, dass die erhaltenen Stämme keine Kanamycin-Gene im Chromosom aufweisen.
So konnten folgende Stämme generiert werden:
ATCC13032 lysC^{fbr} Peftu ddh
ATCC13032 lysC^{fbr} PeftuPsod ddh

All diese Stämme enthalten ein feedback-dereguliertes ask-Gen und produzieren daher Lysin. Sie unterscheiden sich ausschließlich durch die Regulationseinheit, die die Transkription des ddh-Gens steuert.

### Beispiel 18:

### Auswirkung der Verstärkung von ddh durch einen Doppelpromoter auf die Lysinproduktion

Zur Untersuchung der Auswirkung der Verstärkung des ddh-Gens mit Hilfe eines Doppelpromoters wurden folgende Stämme auf Lysin-Produktion untersucht:
ATCC13032 lysCfbr
ATCC13032 lysCfbr Peftu ddh
ATCC13032 lysCfbr Peftu Psod ddh

Dazu wurden die Stämme auf CM-Platten (10,0 g/L D-glucose, 2,5 g/L NaCl, 2,0 g/L Harnstoff, 10,0 g/L Bacto Pepton (Difco), 5,0 g/L Yeast Extract (Difco), 5,0 g/L Beef Extract (Difco), 22,0 g/L Agar (Difco), autoklaviert (20 min. 121°C)) für 2 Tage bei 30°C angezogen. Anschließend wurden die Zellen von der Platte abgekratzt und in Saline resuspendiert. Für die Hauptkultur wurden 10 ml Medium I und 0,5 g autoklaviertes CaCO₃ (Riedel de Haen) in einem 100 ml Erlenmeyerkolben mit der Zellsuspension bis zu einer OD₆₀₀ von 1,5 beimpft und für 40h auf einem vom Typ Infors AJ118 (Fa. Infors, Bottmingen, Schweiz) bei 220 upm inkubiert. Anschließend wurde die Konzentration des in das Medium ausgeschiedene Lysins bestimmt.

Medium I:
40g/l Saccharose
60g/l Melasse (auf 100% Zuckergehalt berechnet)
10g/l (NH₄)₂SO₄
0.4g/l MgSO₄*7H₂O
0.6g/l KH₂PO₄
0.3mg/l Thiamin*HCl
1mg/l Biotin (aus einer 1 mg/ml steril flitrierten Stammlösung die mit NH₄OH auf pH 8,0 eingestellt wurde)
2mg/l FeSO₄
2mg/l MnSO₄
mit NH₄OH auf pH 7,8 eingestellt, autoklaviert (121 °C, 20 min).
zusäztlich wird Vitamin B12 (Hydroxycobalamin Sigma Chemicals) aus einer Stammlösung (200 µg/ml, steril filtriert) bis zu einer Endkonzentration von 100 µg/l zugegeben

Die Bestimmung der Aminosäurekonzentration erfolgte mittels Hochdruckflüssigkeitschromatographie nach Agilent auf einer Agilent 1100 Series LC System HPLC. Eine Vorsäulenderivatisierung mit Ortho-Pthalaldehyd erlaubt die Quantifizierung der gebildeten Aminosäuren, die Auftrennung des Aminosäuregemisch findet auf einer Hypersil AA-Säule (Agilent) statt.
Das Ergebnis der Untersuchung ist in folgender Tabelle dargestellt

| Stamm | Relative Lysinkonzentration (%) |
|---|---|
| ATCC13032 lysC^{fbr} | 100 |
| ATCC13032 lysC^{fbr} Peftu ddh | 102,9 |
| ATCC13032 lysC^{fbr} PeftuPsod ddh | 106,9 |

## Patentansprüche

1. Genetisch verändertes coryneformes Bakterium, transformiert mit wenigstens einem Vektor enthaltend wenigstens eine Expressionskassette, wobei die Expressionskassette in 5' -3' -Richtung ein Sequenzmodul der folgenden allgemeinen Formel II umfasst:
5' -P₁-(-Aₓ-Pₓ-)ₙ-A_{y}-P_{y}-G-3' (II)
worin
n für einen ganzzahligen Wert von 0 bis 10 steht,
Aₓ und A_{y} gleich oder verschieden sind und für eine chemische Bindung oder eine Linker-Nukleinsäuresequenz stehen;
P₁, Pₓ und P_{y} für gleiche oder verschiedene Promotorsequenzen abgeleitet von gleichen oder verschiedenen coryneformen Bakterien kodieren, welche wenigstens einen RNA-Polymerase-bindenden Abschnitt umfassen; und wenigstens P_{y} einen die Ribosomenbindung vermittelnden, 3' -terminalen Sequenzabschnitt umfasst,
G für wenigstens eine kodierende Nukleinsäuresequenz steht, welche mit der 5' -stromaufwärts gelegenen regulatorischen Sequenz funktionell verknüpft ist.

2. Genetisch verändertes coryneformes Bakterium nach Anspruch 1, wobei coryneforem Bakterien Bakterien der Gattung Corynebacterium oder Brevibacterium sind.

3. Genetisch verändertes coryneformes Bakterium nach einem der Ansprüche 1 oder 2, enthaltend wenigstens eine Expressionskassette in integrierter Form.

4. Genetisch verändertes coryneformes Bakterium nach einem der Ansprüche 1 bis 3, wobei P₁, Pₓ und P_{y} jeweils zur Ausgangssequenz eine Identität von mindestens 80% aufweisen oder künstliche Promotorsequenzen sind.

5. Genetisch verändertes coryneformes Bakterium nach einem der Ansprüche 1 bis 4, wobei P₁, Pₓ und P_{y} jeweils abgeleitet sind von einer zusammenhängenden Sequenz von 35 bis 500 Nukleotidresten, die im Genom des Organismus 5' - stromaufwärts von der kodierenden Sequenz für ein Protein liegt und zur Ausgangssequenz eine Identität von mindestens 80% aufweisen.

6. Genetisch verändertes coryneformes Bakterium nach einem der Ansprüche 1 bis 5, wobei P₁, Pₓ und P_{y} unabhängig voneinander ausgewählt sind unter Promotorsequenzen für die kodierende Sequenz eines Proteins mit hoher Abundanz in einem Organismus.

7. Genetisch verändertes coryneformes Bakterium nach einem der Ansprüche 1 bis 6, wobei P₁, Pₓ und P_{y} unabhängig voneinander ausgewählt sind unter SEQ ID NO:1, SEQ ID NO:2, SEQ ID NO:3 und SEQ ID NO:4.

8. Genetisch verändertes coryneformes Bakterium nach einem der Ansprüche 1 bis 7, wobei die Promotoren ausgewählt sind unter starken, konstitutiven oder regulierbaren Promotoren.

9. Genetisch verändertes coryneformes Bakterium nach einem der Ansprüche 1-8, wobei G ausgewählt ist unter
a. Nukleinsäuren kodierend ein Protein aus den Biosyntheseweg von proteinogenen und nicht-proteinogenen Aminosäuren,
b. Nukleinsäuren kodierend ein Protein aus dem Biosyntheseweg von Nukleotiden und Nukleosiden,
c. Nukleinsäuren kodierend ein Protein aus dem Biosyntheseweg von organischen Säuren,
d. Nukleinsäuren kodierend ein Protein aus dem Biosyntheseweg von Lipiden und Fettsäuren,
e. Nukleinsäuren kodierend ein Protein aus dem Biosyntheseweg von Diolen,
f. Nukleinsäuren kodierend ein Protein aus dem Biosyntheseweg von Kohlenhydraten,
g. Nukleinsäuren kodierend ein Protein aus dem Biosyntheseweg von aromatische Verbindungen,
h. Nukleinsäuren kodierend ein Protein aus dem Biosyntheseweg von Vitaminen,
i. Nukleinsäuren kodierend ein Protein aus dem Biosyntheseweg von Cofaktoren und
j. Nukleinsäuren kodierend ein Protein aus dem Biosyntheseweg von Enzymen.
k. Nukleinsäuren kodierend ein Protein aus dem Zentralstoffwechsel.

10. Genetisch verändertes coryneformes Bakterium, nach Anspruch 9a) wobei die Nukleinsäuren für Proteine aus dem Biosyntheseweg von Aminosäuren, ausgewählt unter Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase, 6-Phosphofruktokinase, Tetrahydropicolinat-Succinylase, Succinyl-aminoketopimelat-Aminotransferase, Succinyl-Diaminopimelat-Desuccinylase, Diaminopimelat-Epimerase, 6-Phosphogluconat-Dehydrogenase, Glucosephosphat-Isomerase, Phosphoglycerat-Mutase, Enolase, Pyruvat-Kinase, Aspartat-Transaminase, und Malat-Enzym, kodieren.

11. Verfahren zur Herstellung eines biosynthetischen Produkts, wobei man einen genetisch verändertes coryneformes Bakterium nach einem der Ansprüche 1 bis 10 kultiviert und das gewünschte Produkt aus der Kultur isoliert.

12. Verfahren nach Anspruch 11, wobei das biosynthetische Produkt ausgewählt ist unter organischen Säuren, Proteinen, Nukleotiden und Nukleosiden, sowohl proteinogenen als auch nicht-proteinogenen Aminosäuren, Lipiden und Fettsäuren, Diolen, Kohlenhydraten, aromatischen Verbindungen, Vitaminen und Cofaktoren, Enzymen und Proteinen.

## Claims

1. A genetically modified coryneform bacterium, transformed with at least one vector comprising at least one expression cassette, the expression cassette comprising, in the 5'-3' direction, a sequence module of the following formula II:
5'-P₁-(-Aₓ-Pₓ-)ₙ-A_{y}-P_{y}-G-3' (II)
where
n is an integer from 0 to 10,
Aₓ and A_{y} are identical or different and are a chemical bond or a linker nucleic acid sequence;
P₁, Pₓ and P_{y} code for identical or different promoter sequences derived from identical or different coryneform bacteria which comprise at least one DNA polymerase-binding section; and at least P_{y} comprises a ribosome binding-mediating, 3-terminal sequence section,
G is at least one coding nucleic acid sequence which is functionally linked to the 5' upstream regulatory sequence.

2. The genetically modified coryneform bacterium according to claim 1, where coryneform bacteria are bacteria of the genus Corynebacterium or Brevibacterium.

3. The genetically modified coryneform bacterium according to either of claims 1 or 2, comprising at least one expression cassette in an integrated form.

4. The genetically modified coryneform bacterium according to any of claims 1 to 3, wherein P₁, Pₓ and P_{y} are in each case at least 80% identical to the starting sequence or are artificial promoter sequences.

5. The genetically modified coryneform bacterium according to any of claims 1 to 4, wherein P₁, Pₓ and P_{y} in each case are derived from a contiguous sequence of from 35 to 500 nuclotide residues, which is located 5' upstream of the coding sequence for a protein in the genome of the organism, and are at least 80% identical to the starting sequence.

6. The genetically modified coryneform bacterium according to any of claims 1 to 5, wherein P₁, Pₓ and P_{y} are, independently of one another, selected from among promoter sequences for the coding sequence of a protein with high abundance in an organism.

7. The genetically modified coryneform bacterium according to any of claims 1 to 6, wherein P₁, Pₓ and P_{y} are, independently of one another, selected from among SEQ ID NO:1, SEQ ID NO:2, SEQ ID NO:3 and SEQ ID NO:4.

8. The genetically modified coryneform bacterium according to any of claims 1 to 7, wherein the promoters are selected from among strong, constitutive or regulatable promoters.

9. The genetically modified coryneform bacterium according to any of claims 1-8, wherein G is selected from among
a. nucleic acids encoding a protein of the biosynthetic pathway of proteinogenic and nonproteinogenic amino acids,
b. nucleic acids encoding a protein of the biosynthetic pathway of nucleotides and nucleosides,
c. nucleic acids encoding a protein of the biosynthetic pathway of organic acids,
d. nucleic acids encoding a protein of the biosynthetic pathway of lipids and fatty acids,
e. nucleic acids encoding a protein of the biosynthetic pathway of diols,
f. nucleic acids encoding a protein of the biosynthetic pathway of carbohydrates,
g. nucleic acids encoding a protein of the biosynthetic pathway of aromatic compounds,
h. nucleic acids encoding a protein of the biosynthetic pathway of vitamins,
i. nucleic acids encoding a protein of the biosynthetic pathway of cofactors, and
j. nuclei acids encoding a protein of the biosynthetic pathway of enzymes.
k. nuclei acids encoding a protein of the central metabolism.

10. The genetically modified coryneform bacterium according to claim 9a), wherein the nucleic acids coding for proteins of the biosynthetic pathway of amino acids, selected from among aspartate kinase, aspartate semialdehyde dehydrogenase, diaminopimelate dehydrogenase, diaminopimelate decarboxylase, dihydrodipicolinate Synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR1, transcriptional regulator LysR2, malate-quinone oxidoreductase, glucose-6-phosphate dehydrogenase, 6-phosphogluconate dehydrogenase, transketolase, transaldolase, homoserine O-acetyltransferase, cystathionine gamma synthase, cystathionine beta lyase, serine hydroxymethyltransferase, O-acetylhomoserine sulfhydrylase, methylenetetrahydrofolate reductase, phosphoserine aminotransferase, phosphoserine phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cystéine synthase II, coenzyme B12-dependent methionine synthase, coenzyme B12-independent methionine synthase activity, sulfate adenylyltransferase subunits 1 and 2, phosphoadenosine phosphosulfate reductase, ferredoxin sulfite reductase, ferredoxin NADP reductase, 3-phosphoglycerate dehydrogenase, RNA00655 regulator, RXN2910 regulator, arginyl-t-RNA synthetase, phosphoenolpyruvate carboxylase, thréonine efflux protein, serine hydroxymethyltransferase, fructose 1,6-bisphosphatase, protein of sulfate reduction RNA077, protein of sulfate reduction RNA248, protein of sulfate reduction RXA247, OpcA protein, 1-phosphofructokinase, 6-phosphofructokinase, tetrahydropicolinate succinylase, succinyl aminoketopimelate aminotransferase, succinyl diaminopimelate desuccinylase, diaminopimelate epimerase, 6-phosphogluconate dehydrogenase, glucose phosphate isomerase, phosphoglycerate mutase, enolase, pyruvate kinase, aspartate transaminase, and malate enzyme.

11. A process for preparing a biosynthetic product, which comprises culturing a genetically modified coryneform bacterium according to any of claims 1 to 10 and isolating the desired product from the culture.

12. The process according to claim 11, wherein the biosynthetic product is selected from among organic acids, proteins, nucléotides and nucleosides, both proteinogenic and nonproteinogenic amino acids, lipids and fatty acids, diols, carbohydrates, aromatic compounds, vitamins and cofactors, enzymes and proteins.

## Revendications

1. Bactérie corényforme génétiquement modifiée, transformée avec au moins un vecteur contenant au moins une cassette d'expression, la cassette d'expression comprenant, dans la direction 5'-3' un module de séquence ayant la formule générale II ci-après :
5'-P₁-(Aₓ-Pₓ-)ₙ-A_{y}-P_{y}-G-3' (II)
dans laquelle
n est un nombre entier de 0 à 10,
Aₓ et A_{y} sont identiques ou différents et représentent chacun une liaison chimique ou une séquence d'acide nucléique de liaison ;
P₁, Pₓ et P_{y} codent pour des séquences promotrices identiques ou différentes, qui dérivent de bactéries corynéformes identiques ou différentes, qui comprennent au moins un segment de liaison à l'ARN-polymérase ; et au moins P_{y} comprend un segment de séquence 3'-terminal, réalisant une liaison à des ribosomes,
G représente au moins une séquence d'acide nucléique codante, qui est reliée d'une manière fonctionnelle à la séquence régulatrice située en amont côté 5'.

2. Bactérie corynéforme génétiquement modifiée selon la revendication 1, dans laquelle les bactéries corynéformes sont des bactéries du genre Corynebacterium ou Brevibacterium.

3. Bactérie corynéforme génétiquement modifiée selon l'une des revendications 1 ou 2, contenant au moins une cassette d'expression sous forme intégrée.

4. Bactérie corynéforme génétiquement modifiée selon l'une des revendications 1 à 3, dans laquelle P₁, Pₓ et P_{y} présentent chacun avec la séquence de départ une identité d'au moins 80 %, ou sont des séquences promotrices artificielles.

5. Bactérie corynéforme génétiquement modifiée selon l'une des revendications 1 à 4, dans laquelle P₁, Pₓ et P_{y} dérivent chacun d'une séquence continue de 35 à 500 résidus nucléotidiques, qui se trouvent dans le génome de l'organisme en amont côté 5' de la séquence codante pour une protéine, et qui avec la séquence de départ présentent une identité d'au moins 80 %.

6. Bactérie corynéforme génétiquement modifiée selon l'une des revendications 1 à 5, dans laquelle P₁, Pₓ et P_{y} sont chacun indépendamment des autres choisis parmi les séquences promotrices pour la séquence codante d'une protéine ayant une grande abondance dans un organisme.

7. Bactérie corynéforme génétiquement modifiée selon l'une des revendications 1 à 6, dans laquelle P₁, Pₓ et P_{y} sont choisis indépendamment les uns des autres parmi SEQ ID N° 1, SEQ ID N° 2, SEQ ID N° 3 et SEQ ID N° 4.

8. Bactérie corynéforme génétiquement modifiée selon l'une des revendications 1 à 7, les promoteurs étant choisis parmi les promoteurs forts, constitutifs ou régulables.

9. Bactérie corynéforme génétiquement modifiée selon l'une des revendications 1 à 8, dans laquelle G est choisi parmi :
a. les acides nucléiques codant pour une protéine de la voie de biosynthèse des acides aminés protéinogènes et non protéinogènes,
b. les acides nucléiques codant pour une protéine de la voie de biosynthèse des nucléotides et des nucléosides,
c. les acides nucléiques codant pour une protéine de la voie de biosynthèse des acides organiques,
d. les acides nucléiques codant pour une protéine de la voie de biosynthèse des lipides et des acides gras,
e. les acides nucléiques codant pour une protéine de la voie de biosynthèse des diols,
f. les acides nucléiques codant pour une protéine de la voie de biosynthèse des hydrates de carbone,
g. les acides nucléiques codant pour une protéine de la voie de biosynthèse des composés aromatiques,
h. les acides nucléiques codant pour une protéine de la voie de biosynthèse des vitamines,
i. les acides nucléiques codant pour une protéine de la voie de biosynthèse des co-facteurs, et
j. les acides nucléiques codant pour une protéine de la voie de biosynthèse des enzymes,
k. les acides nucléiques codant pour une protéine du métabolisme central.

10. Bactérie corynéforme génétiquement modifiée selon la revendication 9a), dans laquelle les acides nucléiques codent pour des protéines de la voie de biosynthèse des acides aminés, choisis parmi l'aspartate kinase, l'aspartate semi-aldéhyde déshydrogénase, la diaminopinolate déshydrogénase, la diaminopinolate décarboxylase, la dihydrodipicolinate synthétase, la dihydrodipicolinate réductase, la glycérolaldéhyde-3-phosphate déshydrogénase, la 3-phosphoglycérate kinase, la pyruvate carboxylase, la triosephosphate isomérase, le régulateur transcriptionnel LuxR, le régulateur transcriptionnel LysR1, le régulateur transcriptionnel LysR2, la malate quinone oxydoréductase, la glucose 6-phosphate déshydrogénase, la 6-phosphogluconate déshydrogénase, la transkétolase, la transaldolase, l'homosérine-0-acétyltransférase, la cystahionine gamma synthase, le cystahionine bêta lyase, la sérine hydroxyméthyltransférase, l'O-acétylhomosérine sulfhydrylase, la méthylène tétrahydrofolate réductase, la phosphosérine aminotransférase, la phosphosérine phosphatase, la sérine acétyl transférase, l'homosérine déshydrogénase, l'homosérine kinase, la thréonine synthase, le support d'exportation de la thréonine, la thréonine déshydratase, la pyruvate oxydase, l'exportateur de lysine, la biotine ligase, la cystéine synthase I, la cystéine synthase II, la méthionine synthase co-enzyme B12-dépendante, l'activité de méthionine synthase co-enzyme B12-indépendante, les sous-unités 1 et 2 de la sulfatadényltransférase, la phosphoadénosine phosphosulfate réductase, la ferrédoxine sulfite réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate carboxylase, la protéine d'efflux de la thréonine, la sérine hydroxyméthyltransférase, la fructose 1,6 bisphosphatase, la protéine de réduction des sulfates RXA077, la protéine de réduction des sulfates RXA248, la protéine de réduction des sulfates RXA247, la protéine OpcA, la 1-phosphofructokinase, la 6-phosphofructokinase, la tétrahydropicolinate succinylase, la succinyl aminocétopimélate aminotransférase, la succinyl diaminopimélate désuccinylase, la diaminopimélate épimérase, la 6-phosphogluconate déshydrogénase, la glucosephosphate isomérase, la phosphoglycérate mutase, l'énolase, la pyruvate kinase, l'aspartate transaminase et l'enzyme malate.

11. Procédé de préparation d'un produit biosynthétique, dans lequel on cultive une bactérie corynéforme génétiquement modifiée selon l'une des revendications 1 à 10, et on isole de la culture le produit souhaité.

12. Procédé selon la revendication 1, dans lequel le produit biosynthétique est choisi parmi les acides organiques, les protéines, les nucléotides et les nucléosides, ainsi que les acides aminés protéinogènes et aussi non protéinogènes, les lipides et les acides gras, les diols, les hydrates de carbone, les composés aromatiques, les vitamines et les co-facteurs, les enzymes et les protéines.
